# FASCICULE DE BREVET EUROPEEN

(11) **EP 2 875 010 B1**
(45) Date de publication et mention de la délivrance du brevet: **31.08.2016**
(21) Numéro de dépôt: 13744751.2
(22) Date de dépôt: 16.07.2013
(51) Int. Cl.: C07D 333/24, C07D 333/34, C07D 333/38, C07D 333/40, C07D 409/04, C07D 409/06, C07D 409/12, C07D 409/14, C07D 417/06, C07H 9/04, A61K 31/381, A61P 3/10

(54) **DERIVES DE THIOPHENES UTILES DANS LE TRAITEMENT DU DIABETE**
THIOPHEN-DERIVATE ZUR BEHANDLUNG VON DIABETES
THIOPHENE DERIVATIVES USEFUL FOR THE TREATMENT OF DIABETES

(30) Priorité: 20.07.2012 FR 1257079
(43) Date de publication de la demande: 27.05.2015
(73) Titulaire: Metabrain Research, 91380 Chilly Mazarin (FR)
(72) Inventeur: LEPIFRE, Franck, F., F-91400 Saclay (FR); LENA, Gersande, R., F-69840 Julienas (FR); AUTIER, Valérie, F-91190 Gif Sur Yvette (FR); KERGOAT, Micheline, R., F-91440 Bures Sur Yvette (FR); FAVERIEL, Lauren, R., F-91160 Longjumeau (FR); CHARON, Christine, G., F-91940 Gometz Le Chatel (FR); RAYNAL, Sophie, N., F-75019 Paris (FR); AUDET, Annick,M., F-91630 Leudeville (FR)
(74) Mandataire: Mendelsohn, Isabelle M. N.
(86) Numéro de dépôt international: PCT/FR2013/051702
(87) Numéro de publication internationale: WO 2014/013181

(56) Documents cités:
- WO-A1-2004/035570
- WO-A2-2008/051197

## Description

### Description de l'invention

La présente invention concerne des dérivés thiophènes utiles dans le traitement de pathologies associées au syndrome métabolique, en particulier le traitement ou la prévention du diabète

Le diabète sucré représente un groupe très hétérogène de maladies ayant toutes en commun un certain nombre de caractéristiques : une hyperglycémie, des anomalies fonctionnelles et quantitatives des cellules bêta pancréatiques, une résistance tissulaire à l'insuline et un risque accru de développer à long terme des complications, en particulier cardiovasculaires.

Le diabète de type 2 est devenu un problème majeur de santé publique. Sa prévalence est en forte augmentation dans les pays les plus industrialisés mais encore plus dans les pays en pleine expansion économique. On peut parler aujourd'hui d'épidémie pour cette maladie qui entraîne d'importantes complications pouvant devenir très invalidantes ou même létales du fait entre autres d'insuffisance rénale, d'infarctus du myocarde, ou d'accidents vasculaires cérébraux.

Quelques chiffres sur le diabète (données OMS) :
- Plus de 220 millions de personnes sont diabétiques dans le monde.
- Le diabète multiplie par 3 les risques d'accident vasculaire
- Le diabète est la première cause de cécité et d'insuffisance rénale dans le monde occidental.
- Selon les estimations, le diabète a tué 1,1 million de personnes en 2005.
- Selon les projections de l'OMS, le nombre de décès par diabète va doubler entre 2005 et 2030.

En France, les soins et traitements des diabétiques pèsent très lourds sur le budget de l'Assurance Maladie. Compte-tenu des chiffres alarmants du nombre de patients diabétiques dans le monde d'ici 2030, de nombreuses compagnies pharmaceutiques et biotechnologiques investissent de façon intense en R&D dans le domaine du métabolisme et plus particulièrement dans celui du diabète de type 2 afin de mettre sur le marché de nouvelles alternatives médicamenteuses.

A l'heure actuelle, aucun traitement du diabète de type 2 n'est capable de rétablir un équilibre glycémique normal sur les 24 heures et n'est dénué d'effets secondaires. Aucun ne prend en compte la pathologie complète de la maladie et ne vise qu'à corriger l'un ou l'autre défaut. Les antidiabétiques les plus récemment mis sur le marché n'ont pas montré d'amélioration du contrôle glycémique supérieure à celle observée avec les traitements préexistants et ont entraîné des effets secondaires indésirables, ce qui laisse la place pour de nouveaux traitements potentiels.

Il existe donc un besoin de nouvelles molécules utiles dans le traitement ou la prévention du diabète, de ses complications et/ou des pathologies associées, avantageusement du diabète de type 2.

Les inventeurs ont découvert de façon surprenante que certains dérivés thiophènes présentaient une activité inhibitrice de la production hépatique de glucose et activatrice de la sécrétion d'insuline en réponse au glucose et en particulier pouvaient être utilisés comme produits à usage pharmaceutique chez les patients qui en ont besoin, notamment pour la prévention et/ou le traitement du diabète et de ses complications et/ou des pathologies associées (obésité, hypertension, etc...), avantageusement le diabète de type 2.

### Description détaillée de l'invention

La présente invention concerne donc des dérivés thiophènes de formule générale I suivante : 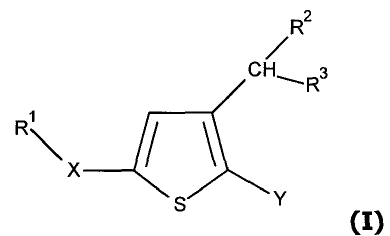 dans laquelle :
**Y** représente un groupe aryle, avantageusement phényle (Ph), un groupe hétéroaryle, avantageusement furyle, ou un groupe benzo-1,3-dioxole, le groupe aryle ou hétéroaryle étant éventuellement substitué par un ou plusieurs groupes choisi parmi -CN ; un atome d'halogène, avantageusement choisi parmi Cl ou F ; -O(alkyle en C₁-C₆) avantageusement -OMe, le groupe alkyle étant éventuellement substitué par un ou plusieurs atomes d'halogène, avantageusement F, tel que par exemple -OCF₃ ou -OCHF₂, ou par un groupe -O(alkyle en C₁-C₆), avantageusement -OMe; alkyle en C₁-C₆, avantageusement méthyle, substitué par un ou plusieurs atomes d'halogène, avantageusement F, tel que par exemple -CF₃, ou par un groupe -O(alkyle en C₁-C₆), avantageusement -OMe, tel que par exemple -CH₂OMe, ou par un groupe -OH tel que par exemple -CH₂OH; -SO₂(alkyl en C₁-C₆), avantageusement -SO₂Me; -CONRaRb dans lequel **Ra** représente un atome d'hydrogène ou un groupe alkyle en C₁-C₆, avantageusement méthyle et **Rb** représente une groupe alkyle en C₁-C₆; ou -OH ; des exemples de groupe aryle éventuellement substitué sont Ph, 4-F-Ph, 2,3-(F)2-Ph, 2-F-4-Cl-Ph, 4-Cl-Ph, 3,4-(Cl)2-Ph, 3-NC-Ph, 4-NC-Ph, 3-MeO-Ph, 4-MeO-Ph, 2-MeO-3-F-Ph et 2-OH-3-F-Ph ;
**X** représente un groupe -SO₂ ou un groupe 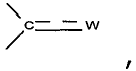 avantageusement un groupe 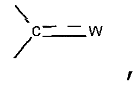 dans lequel représente une liaison et **W** représente un atome d'oxygène ou le groupe -NOR⁴, dans lequel **R⁴** représente un atome d'hydrogène, un groupe alkyle en C₁-C₆ ou un groupe (alkyle en C₁-C₆)aryle, le groupe aryle étant éventuellement substitué par un ou plusieurs groupes choisi parmi -CN ; un atome d'halogène, avantageusement choisi parmi Cl ou F ; -O(alkyle en C₁-C₆) avantageusement -OMe, le groupe alkyle étant éventuellement substitué par un ou plusieurs atomes d'halogène, avantageusement F, ou par un groupe -O(alkyle en C₁-C₆), avantageusement -OMe; alkyle en C₁-C₆ substitué par un ou plusieurs atomes d'halogène, avantageusement F, ou par un groupe -O(alkyle en C₁-C₆), avantageusement -OMe ou par un groupe -OH ; -SO₂(alkyle en C₁-C₆); -CONRa'Rb' dans lequel **Ra'** représente un atome d'hydrogène ou un groupe alkyle en C₁-C₆ et **Rb'** représente un groupe alkyle en C₁-C₆, ou -OH ou 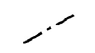 est absent et **W** représente -OH ;
**R¹** représente
   - un groupe alkyle en C₁-C₆, avantageusement méthyle ou éthyle, le groupe alkyle étant éventuellement substitué par un atome d'halogène, avantageusement CI, tel que par exemple -(CH₂)₂Cl ;
   - un groupe cycloalkyle en C₃-C₆, avantageusement cyclopropyle ou cyclohexyle ;
   - un groupe (alkyle en C₁-C₆)O(alkyle en C₁-C₆) ;
   - un groupe (alkyle en C₁-C₆)NR(alkyle en C₁-C₆) dans lequel **R** représente un atome d'hydrogène ou un groupe alkyle en C₁-C₆, avantageusement méthyle ;
   - un groupe aryle, avantageusement phényle (Ph), le groupe aryle étant éventuellement substitué par un ou plusieurs groupes choisi parmi -CN ; un atome d'halogène, avantageusement choisi parmi Cl ou F ; -O(alkyle en C₁-C₆) avantageusement -OMe, le groupe alkyle étant éventuellement substitué par un ou plusieurs atomes d'halogène, avantageusement F, tel que par exemple -OCF₃ ou -OCHF₂, ou par un groupe -O(alkyle en C₁-C₆), avantageusement -OMe; -SO₂(alkyle en C₁-C₆) avantageusement -SO₂Me; -CONRa"Rb" dans lequel **Ra"** représente un atome d'hydrogène ou un groupe alkyle en C₁-C₆ avantageusement méthyle et **Rb"** représente un groupe alkyle en C₁-C₆ avantageusement méthyle ; ou alkyle en C₁-C₆ avantageusement méthyle, le groupe alkyle étant éventuellement substitué par un ou plusieurs atomes d'halogène, avantageusement F, tel que par exemple -CF₃, ou par un groupe -O(alkyle en C₁-C₆), avantageusement -OMe, tel que par exemple -CH₂OMe, ou par un groupe -OH tel que par exemple -CH₂OH ; des exemples de groupe aryle éventuellement substitués sont Ph, 2-F-Ph, 3-F-Ph, 4-F-Ph, 2,3-(F)2-Ph, 2,4-(F)2-Ph, 2,5-(F)2-Ph, 3,5-(F)2-Ph, 3-Cl-Ph, 2,4-(Cl)2-Ph, 3,4-(CI)2-Ph, 4-NC-Ph, 2-MeO-Ph, 4-MeO-Ph, 3-MeO-Ph, 3-F-4-MeO-Ph et 3-Me-4-F-Ph;
   - un groupe (alkyle en C₁-C₆)aryle ; avantageusement (alkyle en C₁-C₆) phényle, en particulier benzyle ou(CH₂)₂phényle, le groupe aryle étant éventuellement substitué par un ou plusieurs groupes choisi parmi -CN ; un atome d'halogène, avantageusement choisi parmi F ou CI, en particulier F; -O(alkyle en C₁-C₆) avantageusement -OMe; ou alkyle en C₁-C₆, avantageusement méthyle ; des exemples de groupe (alkyle en C₁-C₆)aryle éventuellement substitués sont CH2Ph, CH2-4-F-Ph et (CH2)2Ph ;
   - un groupe -NH-aryle, avantageusement -NH-phényle, le groupe aryle étant éventuellement substitué par un ou plusieurs groupes choisi parmi - CN ; un atome d'halogène, avantageusement F ou CI ; -O(alkyle en C₁-C₆) avantageusement -OMe; ou alkyle en C₁-C₆, avantageusement méthyle ; des exemples de groupe -NH-aryle éventuellement substitués sont NH-4-Br-Ph, NH-3-MeO-Ph et NH-4-MeO-Ph ;
   - un groupe -NH(alkyle en C₁-C₆)aryle, avantageusement -NH(alkyle en C₁-C₆)phényle, en particulier -NH(CH₂)phényle, le groupe aryle étant éventuellement substitué par un ou plusieurs groupes choisi parmi -CN ; un atome d'halogène; -O(alkyle en C₁-C₆) avantageusement -OMe ; ou alkyle en C₁-C₆, avantageusement méthyle; des exemples de groupe-NH(alkyle en C₁-C₆)aryle éventuellement substitué sont - NHCH₂-3-MeO-Ph et NHCH₂-4-MeO-Ph ;
   - un groupe hétéroaryle, avantageusement furyle, pyridyle ou thiazolyle, éventuellement substitué par un atome d'halogène, en particulier -Cl (de préférence il est non substitué) ;
   - un groupe -OH ;
   - un groupe morpholine ; ou
   - un groupe N-Phénylepipérazine ;
   - un groupe NH-NH-CO-aryle dans lequel le groupe aryle est éventuellement substitué par un ou plusieurs groupes choisi parmi un atome d'halogène, avantageusement CI et un groupe -O(alkyle en C₁-C₆) avantageusement -OMe ;
   - un groupe NH-NH-CO-hétéroaryle, avantageusement NH-NH-CO-pyridyle.
**R²** représente un atome d'hydrogène ; un groupe alkyle en C₁-C₆, avantageusement un groupe méthyle ; un groupe (alkyle en C₁-C₆)aryle, avantageusement un groupe(alkyle en C₁-C₆)phényle, en particulier un groupe benzyle ; ou un groupe (alkyle en C₁-C₆)O(alkyle en C₁-C₆), avantageusement un groupe -CH₂OCH₃ ; avantageusement **R²** représente un atome d'hydrogène ;
**R³** représente
   - un groupe -COOR⁵, dans lequel **R⁵** représente un atome d'hydrogène, un groupe alkyle en C₁-C₆, tel que par exemple un groupe méthyle, éthyle, isopropyle et t-butyle, ou le groupe glucopyranose ;
   - un groupe -COSR⁶, dans lequel **R⁶** représente un atome d'hydrogène ou un groupe alkyle en C₁-C₆;
   - un groupe -CONR⁷R⁸, dans lequel **R⁷** représente un atome d'hydrogène ou un groupe alkyle en C₁-C₆, tel qu'un groupe méthyle, et **R⁸** représente un atome d'hydrogène ; un groupe alkyle en C₁-C₆, avantageusement éthyle ou méthyle, éventuellement substitué par un groupe -OH, tel que par exemple -(CH₂)₂OH ; un groupe -OH ; un groupe -O(alkyle en C₁-C₆), avantageusement -Oéthyle ; un groupe -NH₂ ; un groupe -(alkyle en C₁-C₆)NR⁹R¹⁰, avantageusement -(CH₂)₂NR⁹R¹⁰, dans lequel **R⁹** et **R¹⁰** représentent tous les deux un groupe alkyle en C₁-C₆, avantageusement un groupe méthyle ou éthyle ; un groupe -(alkyle en C₁-C₆)COOH avantageusement -CH₂COOH ; un groupe -(alkyle en C₁-C₆)COO(alkyle en C₁-C₆) avantageusement -CH₂COOéthyle ; un groupe aryle avantageusement phényle (Ph); ou un groupe hétéroaryle ; des exemples de groupe -CONR⁷R⁸ sont CONH₂, CONHEt, CONHOH, CONHOEt, CONHNH₂, CONH(CH₂)₂OH, CONH(CH₂)₂NMe₂, CONH(CH₂)₂NEt₂, CONMeCH₂COOH, CONMeCH₂COOEt, CONMeOMe, CONHPh et CONHhétéroaryle ;
   - un groupe -CSNR¹¹R¹² dans lequel **R¹¹** et **R¹²** représentent indépendamment l'un de l'autre un atome d'hydrogène ou un groupe alkyle en C₁-C₆, tel que par exemple un groupe éthyle, avantageusement **R¹¹** représente un atome d'hydrogène et **R¹²** représente un groupe alkyle en C₁-C₆, tel que par exemple un groupe éthyle ;
   - un groupe -CN ;
   - un groupe -C(=NH)NHOH ;
   - un groupe -COmorpholine ;
   - un groupe -COpyrolidine ;
   - un groupe -CON-Me-pipérazine ;
   - un groupe -COguanidine ou -COguanidine-BOC ;
   - un groupe tétrazole ; ou
   - un groupe oxadiazolone ;
   ou un énantiomère, diastéréoisomère, hydrate, solvate, tautomère, mélange racémique ou sel pharmaceutiquement acceptable de ceux-ci, à l'exception des composés (a) à (z1) de formule suivante :

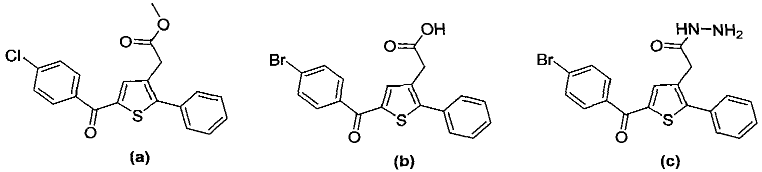

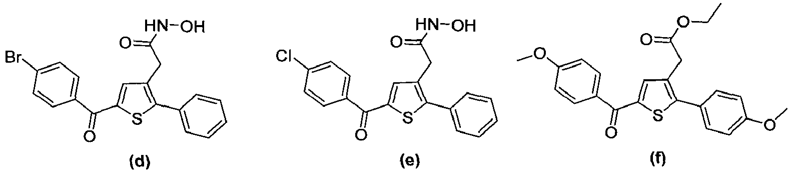

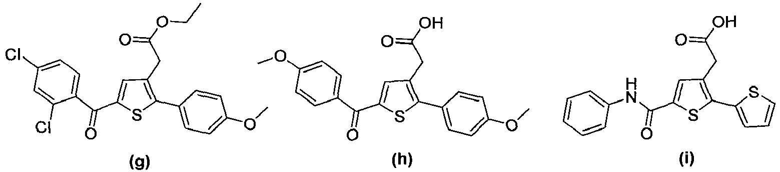

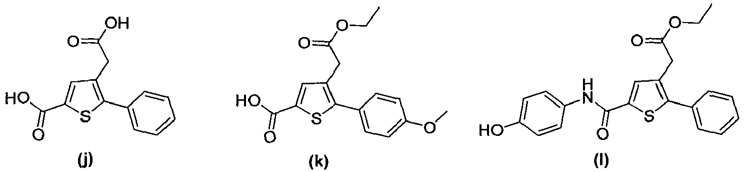

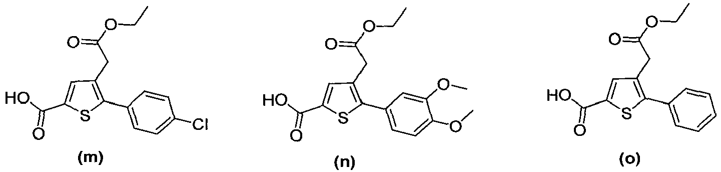

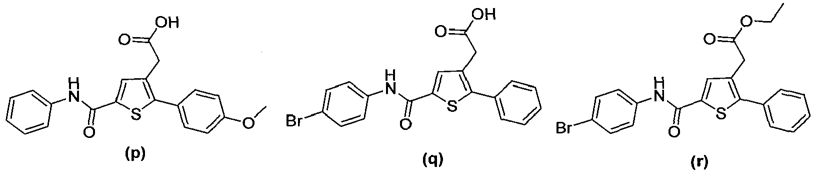

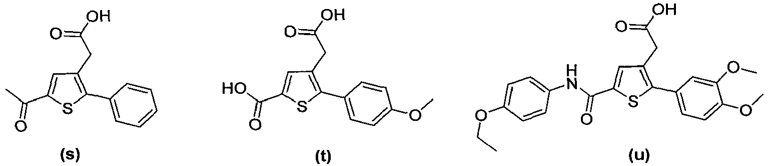

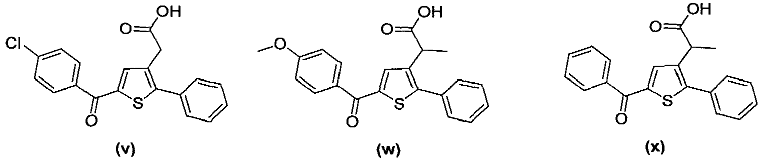

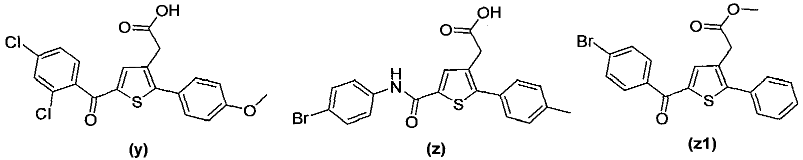

Les documents [1] à [9] divulguent 11 composés de structures couvertes par la formule générale (I) (composés (a) à (e), (q), (t), (v) à (x) et (z1) ci-dessus) sans toutefois décrire d'activité antidiabétique. Ils ont donc été exclus des produits de formule (I) mais pas de l'utilisation de ces produits dans le traitement ou la prévention du diabète.

Par ailleurs 16 composés de structures couvertes par la formule générale (I) (composés (f) à (p), (r), (s), (u), (y) et (z) ci-dessus) sont disponibles commercialement sans toutefois qu'une quelconque activité thérapeutique ne soit divulguée. Ils ont donc été exclus des produits de formule (I) mais pas de l'utilisation de ces produits à titre de médicament et en particulier dans le traitement ou la prévention du diabète.

Dans le cadre de la présente invention on entend par « groupe aryle », un cycle aromatique ayant 5 à 8 atomes de carbones ou plusieurs cycles aromatiques fusionnés ayant 5 à 14 atomes de carbones. En particulier, les groupes aryles peuvent être des groupes monocycliques ou bicycliques, de préférence phényle ou naphthyle. Avantageusement il s'agit d'un groupe phényle (Ph).

Dans le cadre de la présente invention on entend par « groupe hétéroaryle », tout groupe aromatique hydrocarboné de 3 à 9 atomes contenant un ou plusieurs hétéroatomes, tels que par exemple des atomes de soufre, azote ou oxygène. L'hétéroaryle selon la présente invention peut être constitué par un ou plusieurs cycles fusionnés. Des exemples de groupes hétéroaryle sont les groupes furyle, isoxazyle, pyridyle, thiazolyle, pyrimidyle, benzimidazole, benzoxazole, benzothiazole. Avantageusement le groupe hétéroaryle est choisi parmi les groupes furyle, pyridyle et thiazolyle. De façon avantageuse il s'agit du groupe furyle.

Dans le cadre de la présente invention on entend par «atome d'halogène» tout atome d'halogène, avantageusement choisi parmi Cl, Br, I ou F, en particulier choisi parmi F, Cl ou Br, en particulier F ou CI.

Dans le cadre de la présente invention on entend par « groupe alkyle en C₁-C₆ », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle, éthyle, iso-propyle ou t-butyle, en particulier d'un groupe méthyle ou éthyle, plus particulièrement d'un groupe méthyle.

Dans le cadre de la présente invention on entend par « groupe cycloalkyle en C₃-C₆ », tout cycle saturé et hydrocarboné comprenant de 3 à 6 atomes de carbones, en particulier, le groupe cyclopropyle, cyclobutyle, cyclopentyle ou cyclohexyle. Avantageusement il s'agit d'un groupe cyclopropyle ou cyclohexyle.

Dans le cadre de la présente invention on entend par « (groupe alkyle en C₁-C₆)aryle », tout groupe aryle tel que défini ci-dessus, lié par l'intermédiaire d'un groupe alkyle en C₁-C₆ tel que défini ci-dessus. En particulier un exemple de (groupe alkyle en C₁-C₆)aryle est un groupe benzyle ou -(CH₂)₂phényle.

Dans le cadre de la présente invention on entend par «pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine. Dans le cadre de la présente invention on entend par « sels pharmaceutiquement acceptables d'un composé » des sels qui sont pharmaceutiquement acceptables, comme défini ici, et qui possèdent l'activité pharmacologique souhaitée du composé parent. De tels sels comprennent :
(1) les sels d'addition d'acide formés avec des acides minéraux tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Dans le cadre de la présente invention on entend par « solvate d'un composé », tout composé obtenu par addition d'une molécule de solvant inerte sur le composé selon l'invention, le solvate se formant en raison de leur force d'attraction mutuelle. Les solvates sont par exemple des alcoolates du composé. Un hydrate est un solvate dans lequel le solvant inerte utilisé est l'eau. Il peut être mono, di ou trihydraté.

Dans le cadre de la présente invention on entend par « tautomère » tout isomère de constitution des composés selon la présente invention qui sont interconvertibles par la réaction chimique réversible appelée tautomérisation. Dans la plupart des cas, la réaction se produit par migration d'un atome d'hydrogène accompagnée d'un changement de localisation d'une double liaison. Dans une solution d'un composé capable de tautomérisation, un équilibre entre les 2 tautomères se crée. Le rapport entre tautomères est alors fonction du solvant, de la température et du pH. La tautomérie est donc la transformation d'un groupement fonctionnel en un autre, le plus souvent par déplacement concomitant d'un atome d'hydrogène et d'une liaison n (liaison double ou triple). Des tautomères courants sont par exemple les paires aldéhydes / cétones - alcools ou plus précisément énol; amides - acides imidiques; lactames - lactimes ; imines - énamines ; énamines - énamines. En particulier il peut inclure une tautomérie cycle - chaîne qui a lieu lorsque le mouvement du proton est accompagné par la transformation d'une structure ouverte à un cycle.

Dans un mode de réalisation avantageux de la présente invention, représente une liaison et **W** représente un atome d'oxygène ou le groupe -NOR⁴, dans lequel **R⁴** représente un atome d'hydrogène, un groupe alkyle en C₁-C₆ tel que par exemple un groupe méthyle ou éthyle, ou un groupe (alkyle en C₁-C₆)aryle tel que par exemple un groupe (alkyle en C₁-C₆)phényle, en particulier un groupe benzyle, le groupe aryle, avantageusement phényle, étant éventuellement substitué par un ou plusieurs groupes choisi parmi -CN ; un atome d'halogène, avantageusement choisi parmi Cl ou F ; -O(alkyle en C₁-C₆) avantageusement -OMe, le groupe alkyle étant éventuellement substitué par un ou plusieurs atomes d'halogène, avantageusement F, ou par un groupe -O(alkyle en C₁-C₆), avantageusement -OMe; alkyle en C₁-C₆ substitué par un ou plusieurs atomes d'halogène, avantageusement F, ou par un groupe -O(alkyle en C₁-C₆), avantageusement -OMe ou par un groupe -OH ; -SO₂(alkyle en C₁-C₆) ; -CONRa'Rb' dans lequel **Ra'** représente un atome d'hydrogène ou une groupe alkyle en C₁-C₆ et **Rb'** représente un groupe alkyle en C₁-C₆, ou -OH . Avantageusement
représente une liaison et **W** représente un atome d'oxygène ou le groupe -NOR⁴, dans lequel **R⁴** représente un atome d'hydrogène ou un groupe alkyle en C₁-C₆ tel que par exemple un groupe méthyle ou éthyle, encore plus avantageusement **W** représente un atome d'oxygène.

Dans un autre mode de réalisation avantageux de la présente invention, **Y** représente un aryle, avantageusement phényle, un groupe hétéroaryle, avantageusement furyle, ou un groupe benzo-1,3-dioxole, le groupe aryle étant éventuellement substitué par un ou plusieurs groupes choisi parmi-CN ; un atome d'halogène, avantageusement choisi parmi Cl ou F ;-O(alkyle en C₁-C₆) avantageusement -OMe ; ou -OH ; De façon avantageuse **Y** représente un groupe aryle, avantageusement phényle, le groupe aryle étant éventuellement substitué, par un ou plusieurs groupes choisis parmi -CN ; un atome d'halogène, avantageusement choisi parmi Cl ou F, en particulier CI; ou -O(alkyle en C₁-C₆) avantageusement -OMe. Avantageusement la substitution sur le groupe phényle se trouve en ortho et/ou en méta et/ou en para. De façon encore plus avantageuse **Y** représente un groupe phényle substitué, avantageusement en ortho et/ou méta et/ou para, par un ou plusieurs atomes d'halogène, avantageusement Cl et/ou F, en particulier Cl.

Dans encore un autre mode de réalisation avantageux de la présente invention, **R¹** représente un groupe cycloalkyle en C₃-C₆, avantageusement cyclopropyle ou cyclohexyle ; un groupe aryle, avantageusement phényle, le groupe aryle étant éventuellement substitué par un ou plusieurs groupes choisis parmi -CN ; un atome d'halogène, avantageusement choisi parmi Cl ou F ; -O(alkyle en C₁-C₆) avantageusement -OMe; ou alkyle en C₁-C₆ avantageusement méthyle; un groupe hétéroaryle, avantageusement furanyle, pyridyle ou thiazolyle, le groupe furanyle étant éventuellement substitué par un atome d'halogène, en particulier CI, (de préférence il est non substitué) ; ou un groupe morpholine. De façon avantageuse **R¹** représente un groupe phényle (Ph), le groupe phényle étant éventuellement substitué par un ou plusieurs groupes choisis parmi un atome d'halogène, avantageusement choisi parmi Cl ou F, en particulier CI, et -O(alkyle en C₁-C₆) avantageusement-OMe, en particulier le groupe phényle est éventuellement substitué par un ou plusieurs groupes -O(alkyle en C₁-C₆) avantageusement -OMe; ou un groupe furanyle, pyridyle ou thiazolyle, le groupe furanyle étant éventuellement substitué par un atome d'halogène, en particulier Cl, (de préférence il est non substitué).

Dans un autre mode de réalisation de la présente invention, **R²** représente un atome d'hydrogène, un groupe alkyle en C₁-C₆, avantageusement un groupe méthyle, ou un groupe (alkyle en C₁-C₆)O(alkyle en C₁-C₆), en particulier un atome d'hydrogène.

Dans encore un autre mode de réalisation avantageux de la présente invention, **R³** représente un groupe -COguanidine, un groupe -COOR⁵, dans lequel **R⁵** représente un atome d'hydrogène ou un groupe alkyle en C₁-C₆, tel que par exemple un groupe méthyle, éthyle, isopropyle et t-butyle; un groupe -CONR⁷R⁸, dans lequel **R⁷** représente un atome d'hydrogène et **R⁸** représente un atome d'hydrogène ; un groupe alkyle en C₁-C₆ , avantageusement éthyle ou méthyle, éventuellement substitué par un groupe -OH, tel que par exemple -(CH₂)₂OH ; un groupe -OH ; un groupe -O(alkyle en C₁-C₆), avantageusement -Oéthyle ; ou un groupe - (alkyle en C₁-C₆)NR⁹R¹⁰ dans lequel **R⁹** et **R¹⁰** représentent tous les deux un groupe alkyle en C₁-C₆ avantageusement un groupe méthyle ou éthyle ; ou un groupe -COmorpholine ; De façon avantageuse, **R³** représente un groupe -CONHOH, un groupe -COguanidine ou un groupe -COOR⁵, dans lequel **R⁵** représente un atome d'hydrogène ou un groupe alkyle en C₁-C₆, tel que par exemple un groupe méthyle, éthyle, isopropyle et t-butyle. De façon encore plus avantageuse **R³** représente un groupe -COOR⁵, dans lequel **R⁵** représente un atome d'hydrogène ou un groupe alkyle en C₁-C₆, tel que par exemple un groupe méthyle, éthyle, isopropyle et t-butyle. De façon particulièrement avantageuse, **R³** représente un groupe -COOH ou COOEt, en particulier COOH.

Dans un mode de réalisation particulièrement intéressant de la présente invention, les dérivés thiophènes sont choisis parmi les composés de formule 1 à 187 tels qu'indiqués dans le tableau 1 ci-après.

Dans un autre mode de réalisation encore plus intéressant les dérivés thiophènes sont choisis parmi les 102 composés numérotés 3, 5, 6, 10-15, 18, 19, 21, 22, 24-27, 29-33, 35, 39, 40, 43, 44, 46, 48-52, 56, 58-61, 63-65, 67, 68, 70-78, 81, 83, 84, 86, 89, 91, 93, 95, 96, 98, 100, 102, 104, 106, 108-110, 114, 123-125, 127, 128, 130, 133, 136, 137, 139, 140, 142-144, 148, 149, 154, 155, 156-159, 165-167, 175, 176 et 182-187 indiqués dans le tableau 1 ci-après.

Encore plus avantageusement il s'agit des composés 10, 13, 49, 56, 58, 60, 63, 100, 104, 110, 124, 127, 128, 130, 136, 143, 148, 149, 156, 157-159, 167, 175, 176, 184 et 185 indiqués dans le tableau 1 ci-après.

La présente invention concerne en outre une composition pharmaceutique comprenant un dérivé thiophène selon la présente invention et un excipient pharmaceutiquement acceptable.

Ces compositions peuvent être formulées pour l'administration aux mammifères, y compris l'homme. La posologie varie selon le traitement et selon l'affection en cause. Ces compositions pharmaceutiques sont adaptées pour une administration par n'importe quelle voie appropriée, par exemple par voie orale (incluant buccale et sublinguale), par voie rectale, nasale, topique (incluant transdermique), vaginale, intraoculaires ou parentérale (incluant sous-cutanée, intramusculaire ou intraveineuse). Avantageusement les compositions pharmaceutiques sont adaptées pour une administration par voie orale. Ces formulations peuvent être préparées en utilisant tous les procédés connus par l'homme du métier en combinant les ingrédients actifs avec les excipients pharmaceutiquement acceptables appropriés.

Les formes unitaires d'administrations appropriées par voie orale comprennent les comprimés, les gélules, les poudres, les granules et les solutions ou suspensions orales dans des liquides aqueux ou non aqueux, les mousses comestibles ou alimentaires, ou les émulsions liquides eau-dans-huile ou huile-dans-eau. Lorsque l'on prépare une composition solide sous forme de comprimé, on mélange l'ingrédient actif principal, avantageusement sous forme de poudre, avec un excipient pharmaceutique approprié tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif, avantageusement sous forme de poudre, avec un diluant et en versant le mélange obtenu dans des gélules molles ou dures, en particulier des gélules de gélatine. Des lubrifiants tels que par exemple le talc, le stéarate de magnésium, le stéarate de calcium ou le polyéthylène glycol sous forme solide peuvent être ajoutés dans la composition avant sa mise en gélule. Un désintégrant ou un solubilisant tels que par exemple le carbonate de calcium ou le carbonate de sodium peuvent également être ajoutés afin d'améliorer la disponibilité du médicament après la prise de la gélule.

En outre on peut ajouter si nécessaire dans le mélange, des liants, des lubrifiants et des désintégrant appropriés de même que des colorants. Les liants appropriés peuvent être par exemple l'amidon, la gélatine, les sucres naturels tels que par exemple le glucose ou le bêta-lactose, des agents sucrants fabriqués à partir de maïs, du caoutchouc synthétique ou naturel tel que par exemple l'acacia ou l'alginate de sodium, de la carboxyméthylcellulose, du polyéthylène glycol, des cires et similaires. Les lubrifiants utilisables dans ces formes de dosage incluent l'oléate de sodium, le stéarate de sodium, le stéarate de magnésium, le benzoate de sodium, l'acétate de sodium, le chlorure de sodium et similaires. Les désintégrant incluent l'amidon, la méthylcellulose, l'agar, la bentonite, la gomme de xanthane et similaires. Les comprimés sont formulés par exemple par préparation d'un mélange de poudre, granulation ou pressage à sec du mélange, addition d'un lubrifiant et d'un désintégrant et pressage du mélange pour donner les comprimés. Un mélange de poudre est préparé par mélange du principe actif additionné de façon appropriée avec un diluant ou une base et optionnellement avec un liant tel que par exemple la carboxyméthylcellulose, l'alginate, la gélatine ou la polyvinylpyrrolidone, un retardant de dissolution tel que par exemple la paraffine, un accélérant d'absorption tel que par exemple un sel quaternaire et/ou un absorbant tel que par exemple la bentonite, le kaolin ou le phosphate dicalcique. Les mélanges de poudres peuvent être granulés par mouillage avec un liant tel que par exemple un sirop, une pâte d'amidon, du mucilage d'acadia ou des solutions de cellulose ou de matériaux polymères et pressage à travers un tamis. Les granules peuvent être lubrifiés par addition d'acide stéarique, de sel de stéarate, de talc ou d'une huile minérale de façon à éviter qu'ils collent aux moules permettant la fabrication des comprimés. Le mélange lubrifié est alors pressé pour donner les comprimés. Une couche protectrice opaque ou transparente consistant en une couche shellac, une couche de sucre ou de matériaux polymères est éventuellement présente. Des colorants peuvent être ajoutés à ces enrobages façon à les différencier des autres comprimés. Une préparation sous forme de sirop ou d'élixir peut contenir l'ingrédient actif conjointement avec un édulcorant, un antiseptique, ainsi qu'un agent donnant du goût et un colorant approprié. En général les préparations de sirop sont obtenues par dissolution du composé dans une solution aqueuse avec un agent donnant du goût approprié alors que les élixirs sont préparés en utilisant un véhicule alcoolique non toxique.

Les poudres ou les granules dispersibles dans l'eau peuvent contenir l'ingrédient actif en mélange avec des agents de dispersion ou des agents mouillants, ou des agents de mise en suspension, tels que par exemple des alcools isostéaryle éthoxylés et des éthers de sorbitol polyoxyéthylène, et de même qu'avec des correcteurs du goût ou des édulcorants.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les compositions pharmaceutiques adaptées pour une administration par voie topique peuvent être formulées sous forme de crème, d'onguent, de suspension, de lotion, de poudre, de solution, de pâte, de gel, de spray, d'aérosols ou d'huiles.

Des compositions pharmaceutiques adaptées à l'administration par voie nasale dans lequel l'excipient support est à l'état solide comprennent des poudres ayant des tailles de particules par exemple dans la gamme de 20 à 500 microns, administrées par inhalation à partir d'un récipient contenant la poudre disposé à proximité du nez.

Les formulations pharmaceutiques adaptées à l'administration par voie vaginale peuvent assez être administrées sous forme de tampon, crème, gel, pâtes, mousse ou spray.

Dans un mode de réalisation avantageux, la composition pharmaceutique selon la présente invention comprend en outre un autre agent actif, ayant avantageusement un effet complémentaire ou synergique. En particulier cet agent actif est un autre antidiabétique, avantageusement choisi parmi l'insuline, les sulfonylurées, les glinides, les biguanides, les thiazolidinediones, les agonistes GLP-1R, les inhibiteurs de DPP-IV, les inhibiteurs SGLT-2, de façon avantageuse choisie parmi l'insuline, le glibenclamide, le gliclazide, le glipizide, le glimepiride, le repaglinide, le nateglinide, la metformine, la troglitazone, la rosiglitazone, la pioglitazone, l'exenatide, le liraglutide, la sitagliptine, la vildagliptine, la saxagliptine, l'alogliptine, la dapagliflozine. Plus particulièrement il s'agit de la metformine. Ce second agent actif peut être administré dans la même composition pharmaceutique que le dérivé thiophène de la présente invention. Il peut être aussi administré séparément, soit au même moment soit de façon étalée dans le temps. Avantageusement ce second agent actif est administré par voie orale.

La présente invention concerne de plus un dérivé thiophène selon la présente invention ou choisi parmi les composés de formule (f) à (p), (r), (s), (u), (y) et (z) tels que définis ci-dessus pour utilisation à titre de médicament. En effet, comme indiqué ci-dessus les composés (f) à (p), (r), (s), (u), (y) et (z) sont disponibles commercialement, sans qu'une quelconque activité thérapeutique ne leur soit associée. Ainsi donc ils n'ont jamais été divulgués à titre de médicament.

La présente invention concerne également l'utilisation d'un dérivé thiophène selon la présente invention ou choisi parmi les composés de formule (f) à (p), (r), (s), (u), (y) et (z) tels que définis ci-dessus pour la préparation d'un médicament.

Selon la présente invention, les composés de formule (I) présentent une activité antihyperglycémiante. Ils peuvent réduire l'hyperglycémie, plus particulièrement l'hyperglycémie du diabète de type 2. Notamment, les composés de l'invention présentent une activité antihyperglycémique et sont donc utiles dans le traitement et/ou la prévention du diabète, de ses complications et/ou des pathologies associées, tel que par exemple les pathologies associées au syndrome métabolique, avantageusement le diabète de type 2 ou l'hyperglycémie. Ces médicaments sont particulièrement actifs chez les personnes âgées. On entend par « personnes âgées » des personnes, homme ou femmes, ayant 65 ans ou plus..

Le terme « résistance à l'insuline » tel qu'utilisé dans le cadre de la présente invention, se réfère à une condition ou une quantité normale d'insuline est incapable de produire une réponse physiologique ou moléculaire normale.

La présente invention concerne donc un dérivé thiophène selon l'invention ou choisi parmi les composés de formule (a) à (z1), tels que définis ci-dessus pour utilisation à titre de médicament destiné au traitement et/ou à la prévention du diabète, de ses complications et/ou des pathologies associées, avantageusement du diabète de type 2 et de l'hyperglycémie. En effet comme indiqué ci-dessus les composés de formule (a) à (z1) n'ont jamais été divulgués en tant qu'agent antidiabétique.

Les inventeurs ont découvert que les dérivés selon la présente invention permettaient de stimuler la sécrétion d'insuline par les cellules INS1 et d'inhiber la production hépatique de glucose au niveau d'hépatocytes isolés de rat.

Avantageusement le diabète est choisi parmi les diabètes précoces, tardifs, pédiatriques, des personnes âgées et gestationnels, en particulier des personnes âgées. De façon avantageuse les défauts du diabète et les complications et/ou pathologies associées au diabète sont choisies parmi l'hyperglycémie, les anomalies fonctionnelles et quantitatives des cellules pancréatiques endocrines, la résistance à l'insuline, la neuropathie diabétique, la néphropathie diabétique, la rétinopathie diabétique, l'inflammation, l'obésité, l'hypertension, les problèmes cardiovasculaires, microvasculaires, neurologiques et de cicatrisation des plaies. Avantageusement il s'agit de l'hyperglycémie, des anomalies fonctionnelles et quantitatives des cellules pancréatiques endocrines, de la résistance à l'insuline et de l'inflammation.

De façon avantageuse le patient traité présente des facteurs de risques associés au diabète, c'est-à-dire un taux d'affection directement ou indirectement associée avec l'apparition du diabète. En particulier cela comprend l'historique familial, le diabète gestationnel, l'excès de poids, l'obésité, l'insuffisance d'exercice physique, l'hypertension, un haut niveau de triglycérides, l'inflammation et l'hyperlipidémie.

La présente invention concerne de plus l'utilisation d'un dérivé thiophène selon l'invention ou choisi parmi les composés de formule (a) à (z1) tels que définis ci-dessus pour la fabrication d'un médicament destiné au traitement et/ou à la prévention du diabète, de ses complications et/ou des pathologies associées, avantageusement du diabète de type 2 et de l'hyperglycémie.

Elle concerne enfin une méthode de traitement et/ou de prévention et/ou de traitement prophylactique et/ou pour retarder l'apparition du diabète, de ses complications et/ou des pathologies associées, avantageusement du diabète de type 2 et de l'hyperglycémie, comprenant l'administration d'une quantité efficace d'un dérivé thiophène selon l'invention ou choisi parmi les composés de formule (a) à (z1), tels que définis ci-dessus à un patient qui en a besoin.

La quantité efficace sera adaptée selon la nature et la sévérité de la pathologie à traiter, la voie d'administration et aussi le poids et l'âge du patient. En générale l'unité de doses variera entre 0,5 mg et 2000 mg par jour, en une prise ou plusieurs, avantageusement entre 1 et 1000 mg.

Les dérivés thiophènes selon l'invention sont fabriqués par des méthodes bien connues de l'homme du métier et en partie par des méthodes telles que décrites ci-après.

L'invention sera mieux comprise à la lecture de la description et des exemples qui suivent qui sont donnés à titre indicatif non limitatif.

### Description des synthèses et schémas généraux

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les procédures qui suivent.

La synthèse des molécules de formule générale (I) est proche, parfois identique, de ce qui a été décrit dans les documents [2], [5], [9], [10], [11] et [12] sans que cette liste de références puisse être considérée comme exhaustive.

Les différents groupements R¹ à R⁸ et Y des schémas 1 à 13 font référence aux définitions précédemment données.
**Schéma** 1 : La formation du noyau thiophène peut se réaliser en 3 étapes à partir d'un acide 4-phényle-4-oxo butanoique correctement substitué, la fonction acide est estérifiée selon les conditions classiques, une chloroformylation est réalisée en une étape avant la cyclisation en présence de souffre. Le produit majeur de cette réaction est l'intermédiaire **1.4** dont la formation s'accompagne de la production de sous-produit **1.5. 1.4** est ensuite acylé ou sulfonylé selon des conditions de type Friedel-Craft, la saponification de la fonction ester permet d'aboutir à des produits de type **1.7** et **1.9** respectivement. 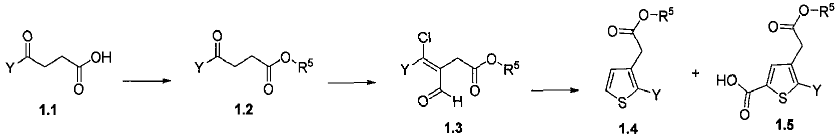 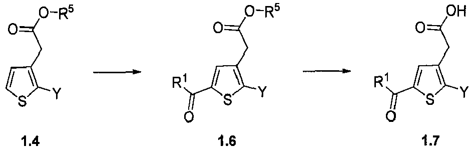 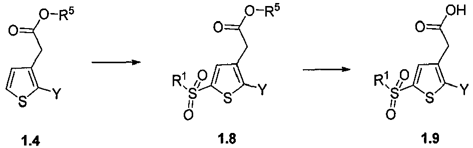
   La préparation d'esters complexes peut être envisagée à partir des acides **1.7** ou **1.9** selon des conditions d'estérification classiques ou par préparation d'un chlorure d'acide intermédiaire suivi d'une réaction d'acylation. Des réactions de trans-estérifications à partir des dérivés de type **1.6** ou **1.8** peuvent également être réalisées selon des conditions classiques.
**Schéma 2 :** La préparation d'amides de type **2.1** a pu être réalisée à partir de dérivés **1.7** en passant par un chlorure d'acide intermédiaire et en réalisant une réaction d'acylation sur une amine, ou bien en conditions de couplages peptidique telles que 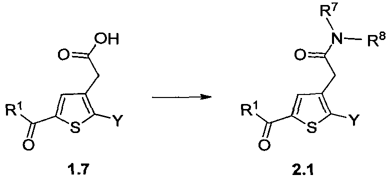
**Schéma** 3 : La réduction de la cétone a été réalisée dans les conditions classiques de réduction qui se sont avérées être sélectives. 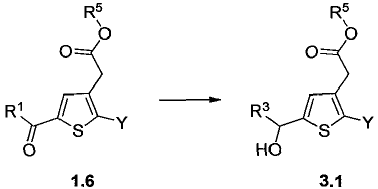
**Schéma 4 :** La préparation d'oximes a été réalisée à partir de dérivés 1.6. 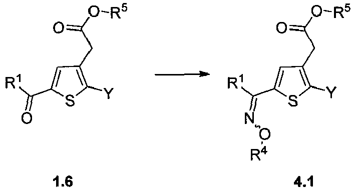
**Schéma 5 :** Des dérivés de formule **1.5** ont été utilisés pour la préparation d'amides en passant par la formation intermédiaire d'un chlorure d'acide et en réalisant une réaction d'acylation sur une amine, ou bien en conditions de couplages peptidique telles que EDC.HCl, HOBt ou encore PyBOP. A représente un groupement aryle, un groupement (alkyle en C₁-C₆)aryle ou encore un groupe arylCONH. 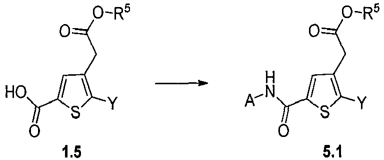
**Schéma 6** : Des dérivés substitués en alpha de l'acide ont été préparés par déprotonation dans des conditions basiques suivies de l'addition d'un réactif électrophile. Dans le cas où le réactif électrophile est le bromo(méthoxy)méthane, selon les conditions opératoires, la réaction peut conduire à la formation de l'acrylate de type **6.2.** 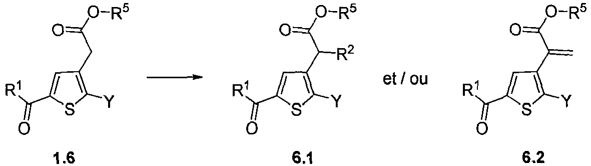
**Schéma 7 :** L'introduction spécifique de certains groupements a été possible en utilisant une méthode de synthèse alternative à celle décrite dans le schéma 1. Partant d'un thiophène acide acétique, une réaction d'estérification a été réalisée, suivie par une halogénation, l'introduction de substituants aryles ou hétéroaryles est permises en mettant en oeuvre des transformations de type Suzuki, afin d'obtenir des dérivés de type **1.4,** qui sont ensuite travaillés de la même façon que précédemment, c'est-à-dire, engagés dans une réaction d'acylation de type Friedel-Craft puis une saponification afin d'obtenir les dérivés **1.7.** 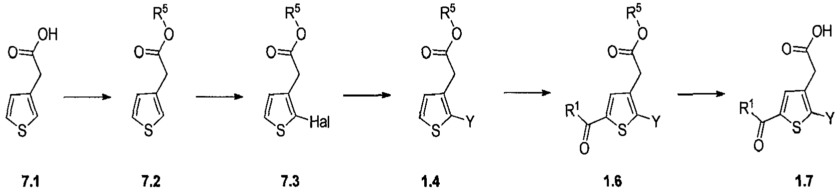 Hal : représente un atome d'halogène, avantageusement le brome.
**Schéma 8 :** La simplification de cette procédure est envisageable dans certains cas, des dérivés de type **8.1** ont été préparés par acylation de dérivés type **7.2** et ont été engagés dans des réactions d'arylation pallado catalysées impliquant des dérivés iodophényles afin d'obtenir les dérivés de type **1.6.** 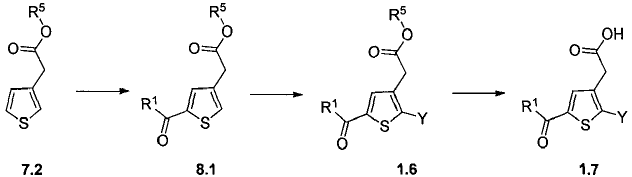
**Schéma 9 :** Une méthode alternative de préparation des composés de formule I fait appel à un dérivé 2-boronate-3-méthylthiophène qui est engagé dans une réaction de type Suzuki. Les dérivés de type **9.2** sont acylés selon des conditions de Friedel-Craft. L'obtention de dérivés **1.6** se fait par halogénation, cyanation puis hydrolyse alcoolique des dérivés **9.3** 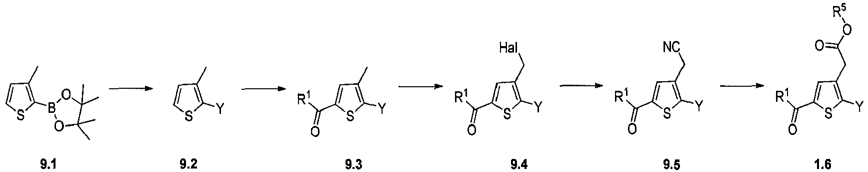
**Schéma 10 :** Dans le but de permettre l'introduction de groupements spécifiques, une autre méthode de synthèse alternative aux méthodes précédemment décrites a été mise au point. Partant d'un 5-bromo-4-méthylthiophéne-2-carboxylate de méthyle une réaction de saponification a été réalisée afin d'obtenir l'acide correspondant et pouvoir préparer un amide de Weinreb. En présence d'un organométal de type halomagnésien, les dérivés de type **10.3** ont permis d'obtenir des dérivés de type **10.4,** qui ont ensuite été engagés dans une réaction de type Suzuki, suivie d'une halogénation puis de cyanation afin d'obtenir les dérivés de type **10.7.** Ces derniers ont été engagés dans des réactions d'hydrolyses alcooliques afin d'obtenir les esters de type **1.6.** 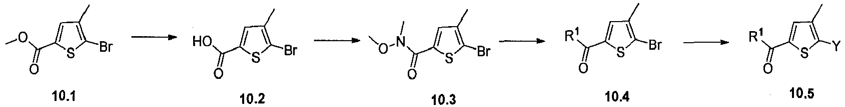 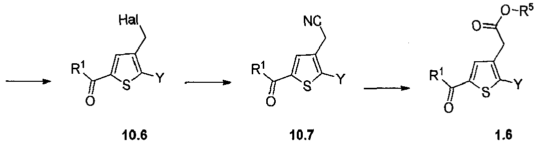
**Schéma 11 :** L'introduction de substituants à la fonction acide a nécessité une autre méthode de synthèse, qui fait appel à un 2-(thiophén-3-yl)acétonitrile comme produit de départ. ce produit a été engagé dans une réaction d'halogénation suivie d'un couplage pallado catalysé de type Suzuki pour obtenir les dérivés de type **11.3,** ces produits ont été acylés selon des conditions de Friedel-Craft pour obtenir les dérivés de type **10.7.** 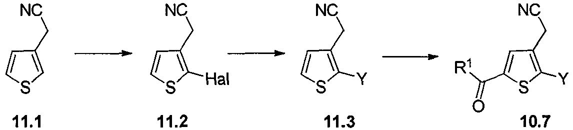
**Schéma 12 :** Les dérivés de type **10.7** ont été soumis à un traitement avec un azoture afin de former les dérivés tétrazoles **12.1.** 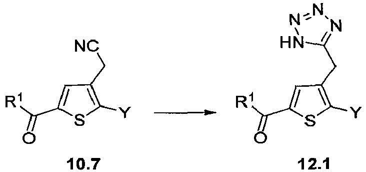
**Schéma 13** : Les dérivés de type **10.7** ont été soumis à un traitement avec une hydroxylamine afin d'obtenir les dérivés ouverts de type **13.1** qui ont ensuite été engagés dans des réactions de carbonylation en présence de carbonyle diimidazole afin d'obtenir les oxadiazolones de type **13.2.** 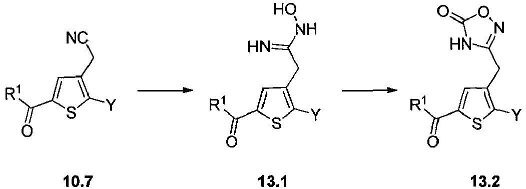
**Schéma 14 :** Les dérivés de type **1.4** ont été soumis à une hydrolyse en conditions basiques afin d'obtenir les dérivés acide intermédiaires de type **14.1** qui ont ensuite été engagés dans des réactions de couplage peptidique permettant d'obtenir les dérivés de type **14.2 .** Ces derniers traités par le réactif de Lawesson forment les thioamides **14.3** correspondants. Enfin une réaction d'acylation de Friedel-Craft donne accès aux composés de type **14.4.** 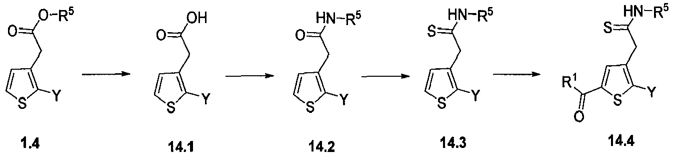
**Schéma 15 :** Des conditions de couplage peptidique en présence de guanidine-Boc conduisent à partir de dérivés **1.7** à la formation des dérivés de type **15.1** lesquels donnent ensuite accès aux composés déprotégés **15.2** dans des conditions d'hydrolyse acide.

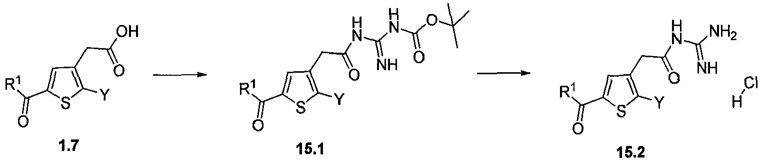

### EXEMPLES :

### Matériel et méthode

Les spectres de résonance magnétique nucléaire **(RMN)** du proton **(¹H)** sont effectués sur un appareil Bruker Avance DPX300 (300,16 MHz). Les déplacements chimiques (δ) sont mesurés en partie par million **(ppm).** Les spectres sont calibrés sur le déplacement chimique du solvant deutéré utilisé. Les constantes de coupage **(J)** sont exprimées en Hertz **(Hz)** et la multiplicité est représentée de la manière suivante, singulet (s), doublet (d), doublet de doublet (dd), triplet (t), triplet de doublet (td), quadruplet (q), multiplet (m). Les spectres de masse **(SM)** sont réalisés par un spectromètre Agilent Technologies MSD, type G1946A, les échantillons sont ionisés par une source « Atmospheric pressure chemical ionization » **(APCI).**

### Abréviations

- AIBN: azoisobutyronitrile
- EDC: *N*-(3-diméthylaminopropyl)-*N*-éthylcarbodiimide
- HOBt: 1-hydroxybenzotriazole
- CDCl₃: chloroforme deutéré
- DMSO: diméthylsulfoxyde deutéré
- PyBOP: (benzotriazol-1-yl-oxytripyrrolidinophosphonium hexafluorophosphate)
- DMPU: 1,3-Diméthyl-3,4,5,6-tetrahydro-2(1H)-pyrimidinone
- DMF: diméthylformamide
- Boc: tert-butoxycarbonyle
- mmol: milli mole(s)
- µM: micromolaire
- ml: milli litre(s)
- g: gramme(s)
- M: mol / litre
- N: normal(e)
- nm: nano mètre(s)
- min: minute(s)
- h: heure(s)
- j: jour(s)
- t.a.: température ambiante
- UV: ultra violet
- ctrl: contrôle
- HGP: Production Hépatique de Glucose

La liste des exemples ci-dessous sert à illustrer le propos de cette invention et non à en limiter le champ d'application.

**Tableau 1 : Liste des molécules dont la synthèse est exemplifiée**

| **N°** | **Structure chimique** | **Nom chimique** |
|---|---|---|
| **1** | 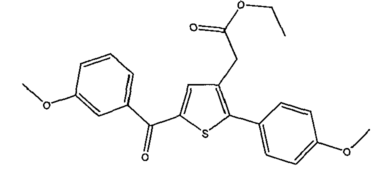 | 2-(5-(4-méthoxybenzoyl)-2-(4-méthoxyphényl)thiophén-3-yl)acétate d'éthyle |
| **2** | 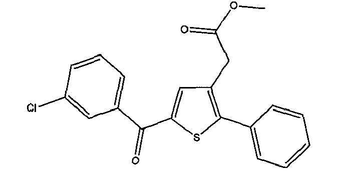 | 2-(5-(3-chlorobenzoyl)-2-phénylthiophén-3-yl)acétate de méthyle |
| **3** | 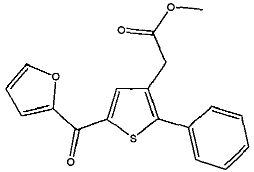 | 2-(5-(furan-2-carbonyl)-2-phénylthiophén-3-yl)acétate de méthyle |
| **4** | 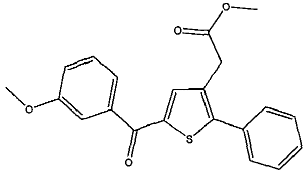 | 2-(5-(3-méthoxybenzoyl)-2-phénylthiophén-3-yl)acétate de méthyle |
| **5** | 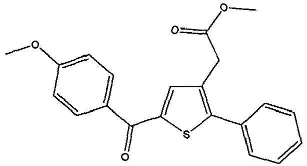 | 2-(5-(4-méthoxybenzoyl)-2-phénylthiophén-3-yl)acétate de méthyle |
| **6** | 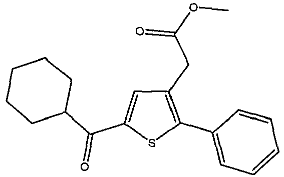 | 2-(5-(cyclohexanecarbonyl)-2-phénylthiophén-3-yl)acétate de méthyle |
| **7** | 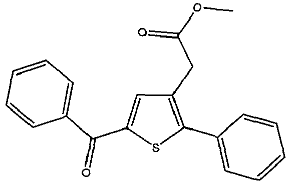 | 2-(5-benzoyl-2-phénylthiophén-3-yl)acétate de méthyle |
| **8** | 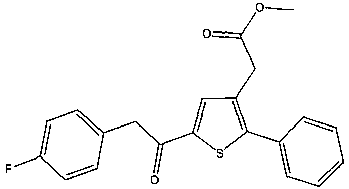 | 2-(5-(2-(4-fluorophényl)acétyl)-2-phénylthiophén-3-yl)acétate de méthyle |
| **9** | 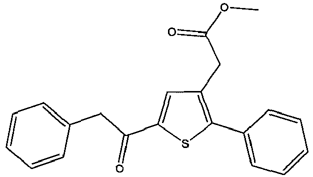 | 2-(2-phényl-5-(2-phénylacétyl)thiophén-3-yl)acétate de méthyle |
| **10** | 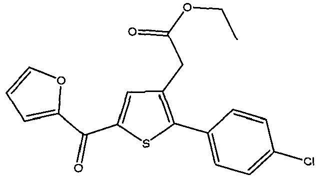 | 2-(2-(4-chlorophényl)-5-(furan-2-carbonyl)thiophén-3-yl)acétate d'éthyle |
| **11** | 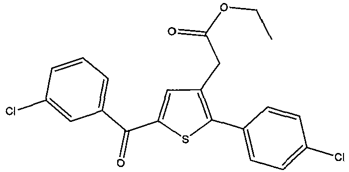 | 2-(5-(3-chlorobenzoyl)-2-(4-chlorophényl)thiophén-3-yl)acétate d'éthyle |
| **12** | 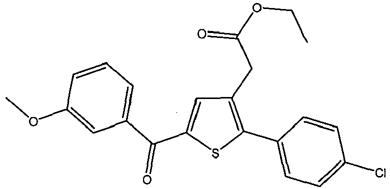 | 2-(2-(4-chlorophényl)-5-(3-méthoxybenzoyl)thiophén-3-yl)acétate d'éthyle |
| **13** | 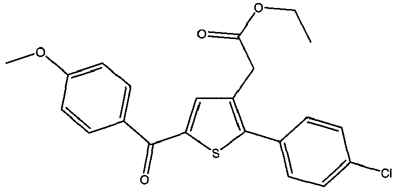 | 2-(2-(4-chlorophényl)-5-(4-méthoxybenzoyl)thiophén-3-yl)acétate d'éthyle |
| **14** | 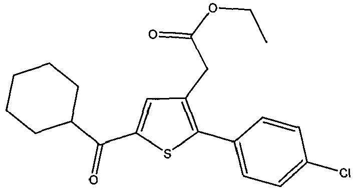 | 2-(2-(4-chlorophényl)-5-(cyclohexanecarbonyl)thiophén-3-yl)acétate d'éthyle |
| **15** | 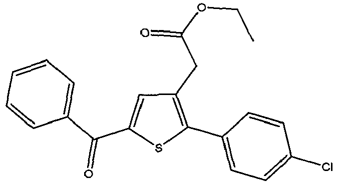 | 2-(5-benzoyl-2-(4-chlorophényl)thiophén-3-yl)acétate d'éthyle |
| **16** | 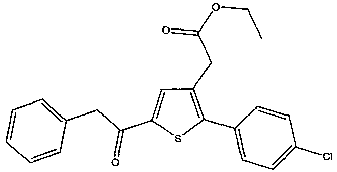 | 2-(2-(4-chlorophényl)-5-(2-phénylacétyl)thiophén-3-yl)acétate d'éthyle |
| **17** | 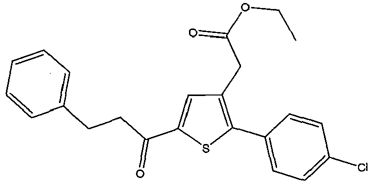 | 2-(2-(4-chlorophényl)-5-(3-phénylpropanoyl)thiophén-3-yl)acétate d'éthyle |
| **18** | 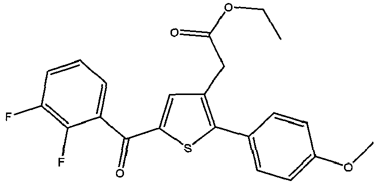 | 2-(5-(2,3-difluorobenzoyl)-2-(4-méthoxyphényl)thiophén-3-yl)acétate d'éthyle |
| **19** | 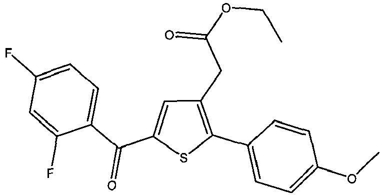 | 2-(5-(2,4-difluorobenzoyl)-2-(4-méthoxyphényl)thiophén-3-yl)acétate d'éthyle |
| **20** | 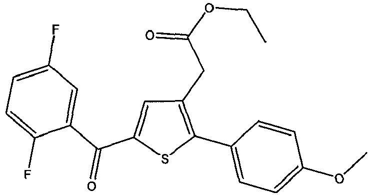 | 2-(5-(2,5-difluorobenzoyl)-2-(4-méthoxyphényl)thiophén-3-yl)acétate d'éthyle |
| **21** | 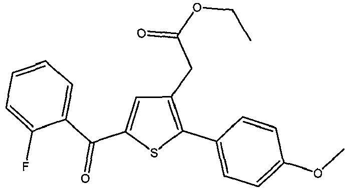 | 2-(5-(2-fluorobenzoyl)-2-(4-méthoxyphényl)thiophén-3-yl)acétate d'éthyle |
| **22** | 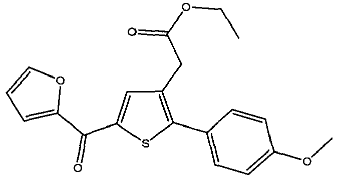 | 2-(5-(furan-2-carbonyl)-2-(4-méthoxyphényl)thiophén-3-yl)acétate d'éthyle |
| **23** | 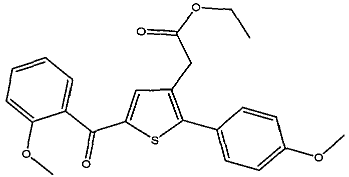 | 2-(5-(2-méthoxybenzoyl)-2-(4-méthoxyphényl)thiophén-3-yl)acétate d'éthyle |
| **24** | 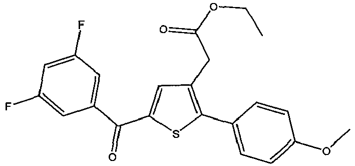 | 2-(5-(3,5-difluorobenzoyl)-2-(4-méthoxyphényl)thiophén-3-yl)acétate d'éthyle |
| **25** | 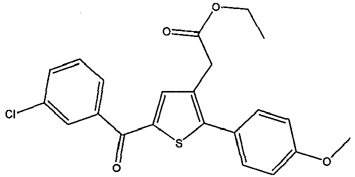 | 2-(5-(3-chlorobenzoyl)-2-(4-méthoxyphényl)thiophén-3-yl)acétate d'éthyle |
| **26** | 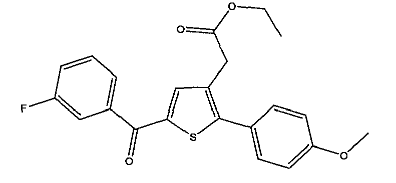 | 2-(5-(3-fluorobenzoyl)-2-(4-méthoxyphényl)thiophén-3-yl)acétate d'éthyle |
| **27** | 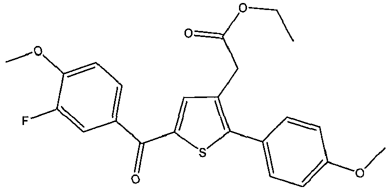 | 2-(5-(3-fluoro-4-méthoxybenzoyl)-2-(4-méthoxyphényl)thiophén-3-yl) acétate d'éthyle |
| **28** | 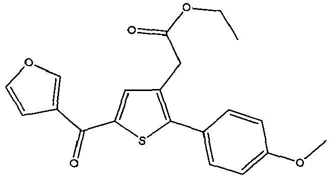 | 2-(5-(furan-3-carbonyl)-2-(4-méthoxyphényl)thiophén-3-yl)acétate d'éthyle |
| **29** | 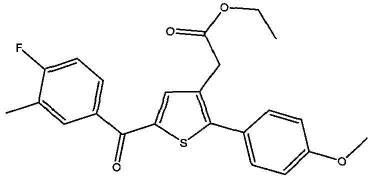 | 2-(5-(4-fluoro-3-méthylbenzoyl)-2-(4-méthoxyphényl)thiophén-3-yl) acétate d'éthyle |
| **30** | 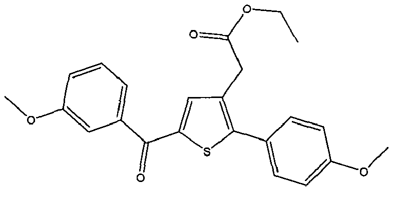 | 2-(5-(3-méthoxybenzoyl)-2-(4-méthoxyphényl)thiophén-3-yl)acétate d'éthyle |
| **31** | 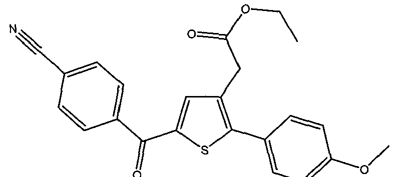 | 2-(5-(4-cyanobenzoyl)-2-(4-méthoxyphényl)thiophén-3-yl)acétate d'éthyle |
| **32** | 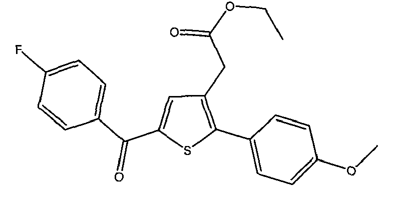 | 2-(5-(4-fluorobenzoyl)-2-(4-méthoxyphényl)thiophén-3-yl)acétate d'éthyle |
| **33** | 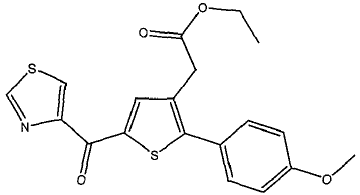 | 2-(2-(4-méthoxyphényl)-5-(thiazole-4-carbonyl)thiophén-3-yl)acétate d'éthyle |
| **34** | 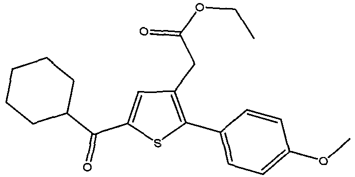 | 2-(5-(cyclohexanecarbonyl)-2-(4-méthoxyphényl)thiophén-3-yl)acétate d'éthyle |
| **35** | 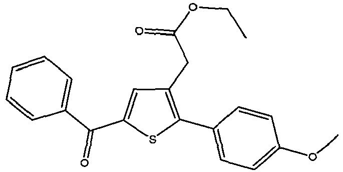 | 2-(5-benzoyl-2-(4-méthoxyphényl)thiophén-3-yl)acétate d'éthyle |
| **36** | 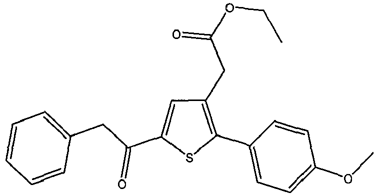 | 2-(2-(4-méthoxyphényl)-5-(2-phénylacétyl)thiophén-3-yl)acétate d'éthyle |
| **37** | 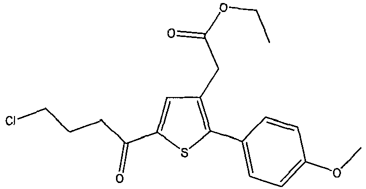 | 2-(5-(4-chlorobutanoyl)-2-(4-méthoxyphényl)thiophén-3-yl)acétate d'éthyle |
| **38** | 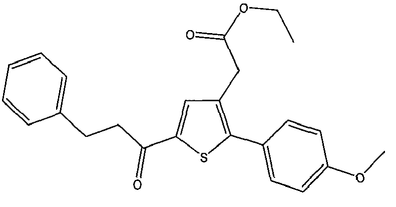 | 2-(2-(4-méthoxyphényl)-5-(3-phénylpropanoyl)thiophén-3-yl)acétate d'éthyle |
| **39** | 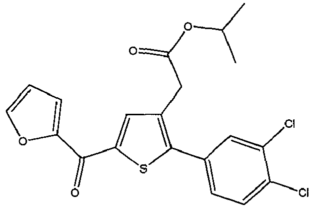 | 2-(2-(3,4-dichlorophényl)-5-(furan-2-carbonyl)thiophén-3-yl) acétate d'isopropyle |
| **40** | 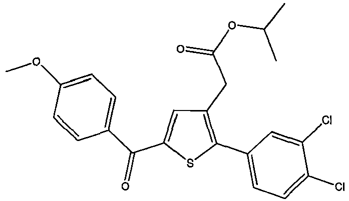 | 2-(2-(3,4-dichlorophényl)-5-(4-méthoxybenzoyl)thiophén-3-yl) acétate d'isopropyle |
| **41** | 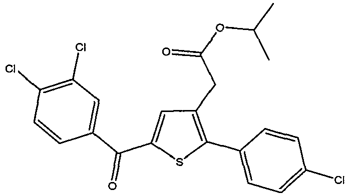 | 2-(2-(4-chlorophényl)-5-(3,4-dichlorobenzoyl)thiophén-3-yl)acétate d'isopropyle |
| **42** | 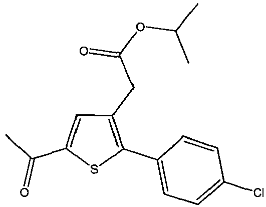 | 2-(5-acétyl-2-(4-chlorophényl)thiophén-3-yl)acétate d'isopropyle |
| **43** | 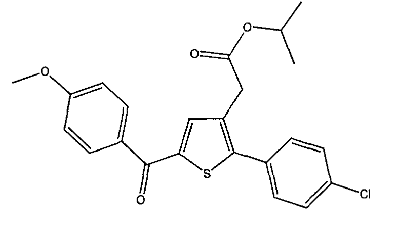 | 2-(2-(4-chlorophényl)-5-(4-méthoxybenzoyl)thiophén-3-yl) acétate d'isopropyle |
| **44** | 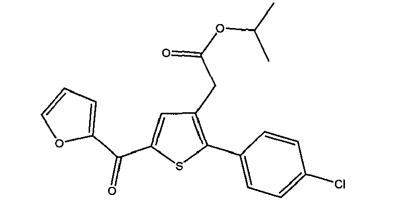 | 2-(2-(4-chlorophényl)-5-(furan-2-carbonyl)thiophén-3-yl)acétate d'isopropyle |
| **45** | 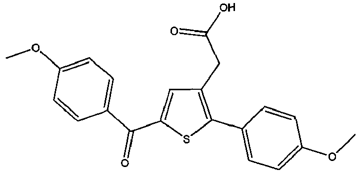 | Acide 2-(5-(4-méthoxybenzoyl)-2-(4-méthoxyphényl)thiophén-3-yl)acétique |
| **46** | 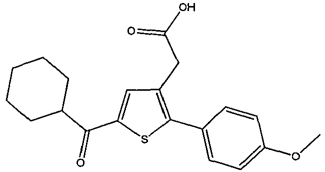 | Acide 2-(5-(cyclohexanecarbonyl)-2-(4-méthoxyphényl)thiophén-3-yl) acétique |
| **47** | 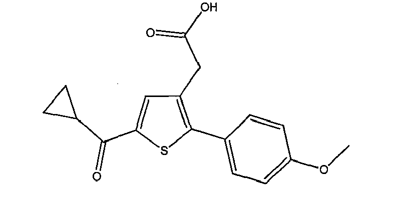 | Acide 2-(5-(cyclopropanecarbonyl)-2-(4-méthoxyphényl)thiophén-3-yl) acétique |
| **48** | 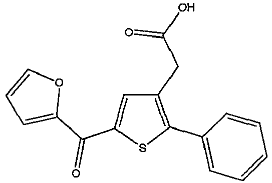 | Acide 2-(5-(furan-2-carbonyl)-2-phénylthiophén-3-yl) acétique |
| **49** | 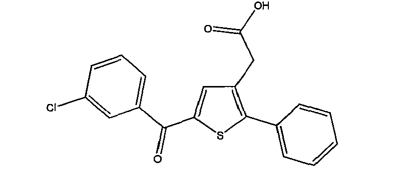 | Acide 2-(5-(3-chlorobenzoyl)-2-phénylthiophén-3-yl) acétique |
| **50** | 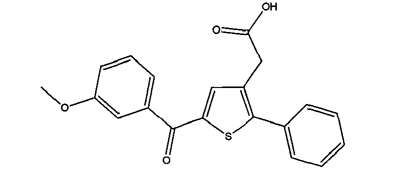 | Acide 2-(5-(3-méthoxybenzoyl)-2-phénylthiophén-3-yl) acétique |
| **51** | 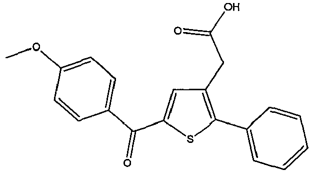 | Acide 2-(5-(4-méthoxybenzoyl)-2-phénylthiophén-3-yl) acétique |
| **52** | 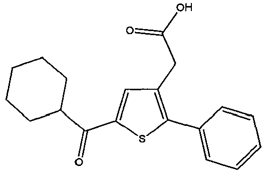 | Acide 2-(5-(cyclohexanecarbonyl)-2-phénylthiophén-3-yl) acétique |
| **53** | 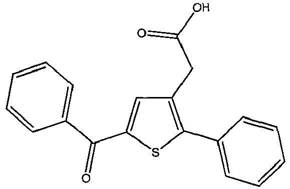 | Acide 2-(5-benzoyl-2-phénylthiophén-3-yl) acétique |
| **54** | 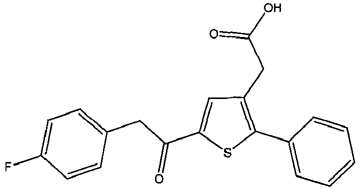 | Acide 2-(5-(2-(4-fluorophényl)acétyl)-2-phénylthiophén-3-yl) acétique |
| **55** | 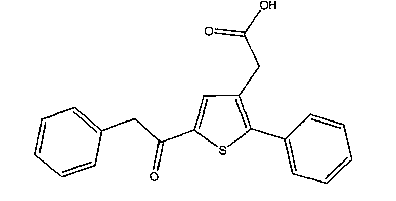 | Acide 2-(2-phényl-5-(2-phénylacétyl)thiophén-3-yl) acétique |
| **56** | 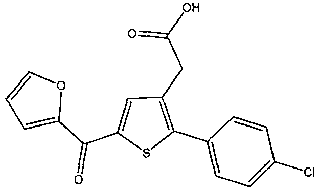 | Acide 2-(2-(4-chlorophényl)-5-(furan-2-carbonyl)thiophén-3-yl) acétique |
| **57** | 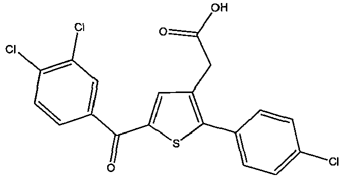 | Acide 2-(2-(4-chlorophényl)-5-(3,4-dichlorobenzoyl)thiophén-3-yl) acétique |
| **58** | 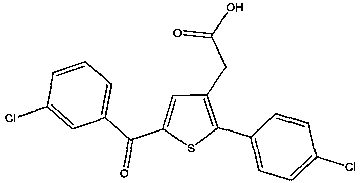 | Acide 2-(5-(3-chlorobenzoyl)-2-(4-chlorophényl)thiophén-3-yl) acétique |
| **59** | 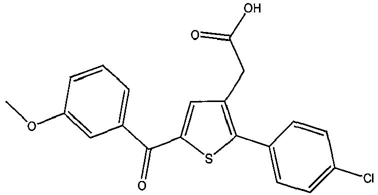 | Acide 2-(2-(4-chlorophényl)-5-(3-méthoxybenzoyl)thiophén-3-yl) acétique |
| **60** | 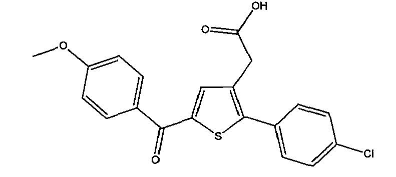 | Acide 2-(2-(4-chlorophényl)-5-(4-méthoxybenzoyl)thiophén-3-yl) acétique |
| **61** | 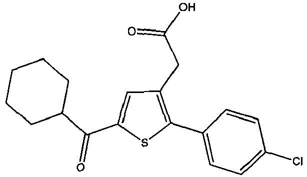 | Acide 2-(2-(4-chlorophényl)-5-(cyclohexanecarbonyl)thiophén-3-yl)acétique |
| **62** | 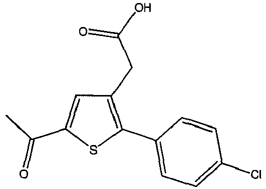 | Acide 2-(5-acétyl-2-(4-chlorophényl)thiophén-3-yl) acétique |
| **63** | 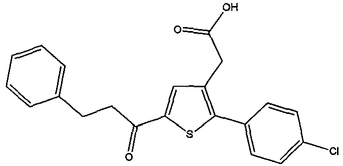 | Acide 2-(2-(4-chlorophényl)-5-(3-phénylpropanoyl)thiophén-3-yl) acétique |
| **64** | 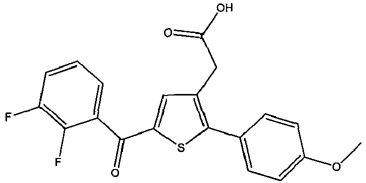 | Acide 2-(5-(2,3-difluorobenzoyl)-2-(4-méthoxyphényl)thiophén-3-yl) acétique |
| **65** | 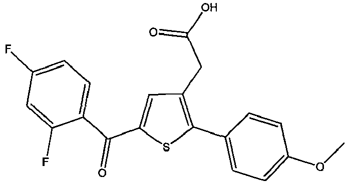 | Acide 2-(5-(2,4-difluorobenzoyl)-2-(4-méthoxyphényl)thiophén-3-yl) acétique |
| **66** | 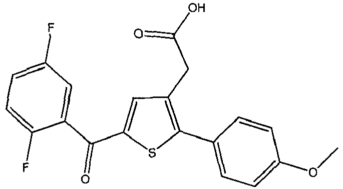 | Acide 2-(5-(2,5-difluorobenzoyl)-2-(4-méthoxyphényl)thiophén-3-yl) acétique |
| **67** | 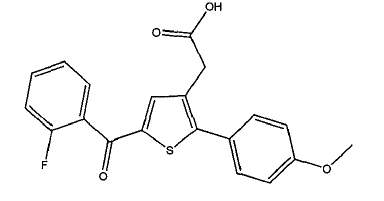 | Acide 2-(5-(2-fluorobenzoyl)-2-(4-méthoxyphényl)thiophén-3-yl) acétique |
| **68** | 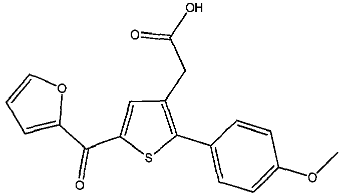 | Acide 2-(5-(furan-2-carbonyl)-2-(4-méthoxyphényl)thiophén-3-yl) acétique |
| **69** | 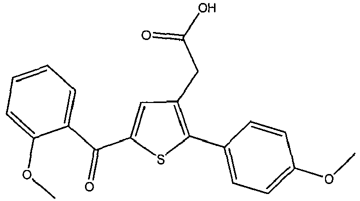 | Acide 2-(5-(2-méthoxybenzoyl)-2-(4-méthoxyphényl)thiophén-3-yl) acétique |
| **70** | 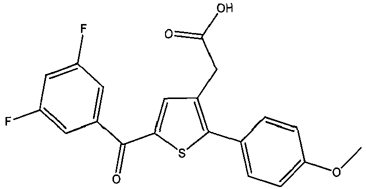 | Acide 2-(5-(3,5-difluorobenzoyl)-2-(4-méthoxyphényl)thiophén-3-yl) acétique |
| **71** | 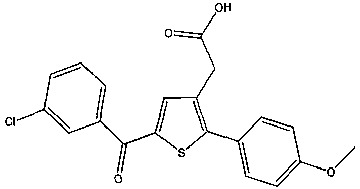 | Acide 2-(5-(3-chlorobenzoyl)-2-(4-méthoxyphényl)thiophén-3-yl) acétique |
| **72** | 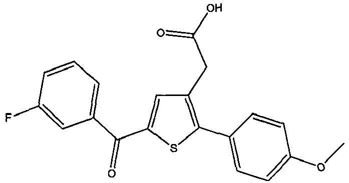 | Acide 2-(5-(3-fluorobenzoyl)-2-(4-méthoxyphényl)thiophén-3-yl) acétique |
| **73** | 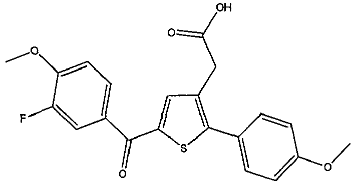 | Acide 2-(5-(3-fluoro-4-méthoxybenzoyl)-2-(4-méthoxyphényl)thiophén-3-yl) acétique |
| **74** | 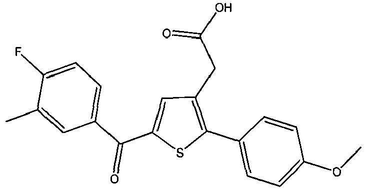 | Acide 2-(5-(4-fluoro-3-méthylbenzoyl)-2-(4-méthoxyphényl)thiophén-3-yl) acétique |
| **75** | 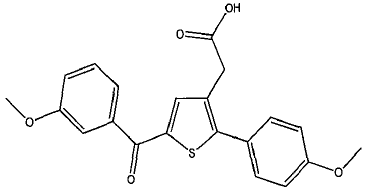 | Acide 2-(5-(3-méthoxybenzoyl)-2-(4-méthoxyphényl)thiophén-3-yl) acétique |
| **76** | 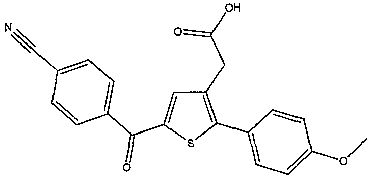 | Acide 2-(5-(4-cyanobenzoyl)-2-(4-méthoxyphényl)thiophén-3-yl) acétique |
| **77** | 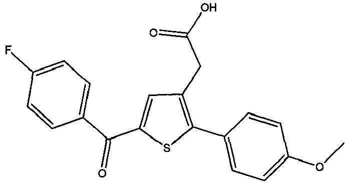 | Acide 2-(5-(4-fluorobenzoyl)-2-(4-méthoxyphényl)thiophén-3-yl) acétique |
| **78** | 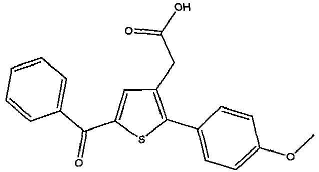 | Acide 2-(5-benzoyl-2-(4-méthoxyphényl)thiophén-3-yl) acétique |
| **79** | 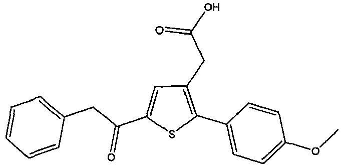 | Acide 2-(2-(4-méthoxyphényl)-5-(2-phénylacétyl)thiophén-3-yl) acétique |
| **80** | 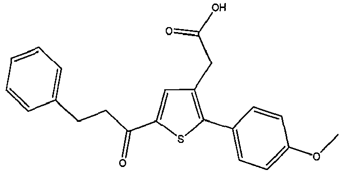 | Acide 2-(2-(4-méthoxyphényl)-5-(3-phénylpropanoyl)thiophén-3-yl)ace acétique |
| **81** | 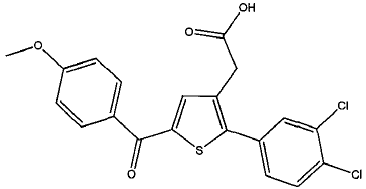 | Acide 2-(2-(3,4-dichlorophényl)-5-(4-méthoxybenzoyl)thiophén-3-yl) acétique |
| **82** | 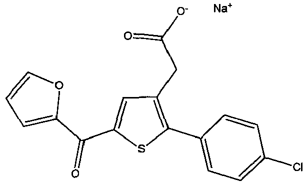 | 2-(2-(4-chlorophényl)-5-(furan-2-carbonyl)thiophén-3-yl)acétate de sodium |
| **83** | 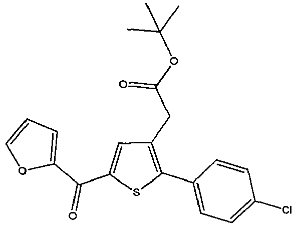 | 2-(2-(4-chlorophényl)-5-(furan-2-carbonyl)thiophén-3-yl)acétate de tert-butyle |
| **84** | 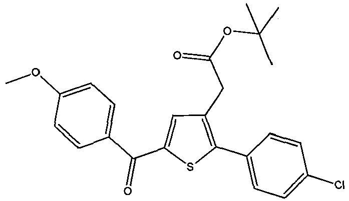 | 2-(2-(4-chlorophényl)-5-(4-méthoxybenzoyl)thiophén-3-yl) acétate de tert-butyle |
| **85** | 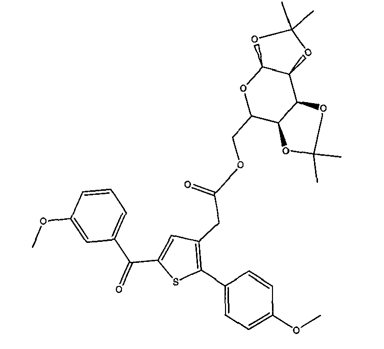 | 2-(5-(3-méthoxybenzoyl)-2-(4-méthoxyphényl)thiophén-3-yl)acétate de ((3aS,5aS,8aS,8bS)-2,2,7,7-tetraméthyltetrahydro-3aH-bis([1,3]dioxolo)[4,5-b:4',5'-d]pyran-5-yl)méthyle |
| **86** | 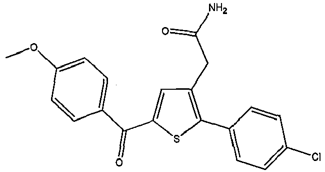 | 2-(2-(4-chlorophényl)-5-(4-méthoxybenzoyl)thiophén-3-yl)acétamide |
| **87** | 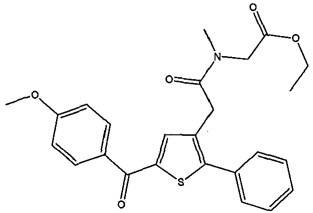 | 2-(2-(5-(4-méthoxybenzoyl)-2-phénylthiophén-3-yl)-N-méthylacétamido)acétate d'éthyle |
| **88** | 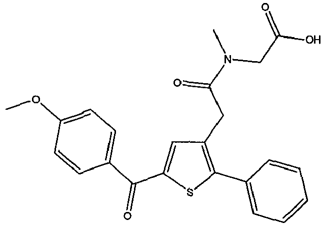 | Acide 2-(2-(5-(4-méthoxybenzoyl)-2-phénylthiophén-3-yl)-N-méthylacétamido)acétique |
| **89** | 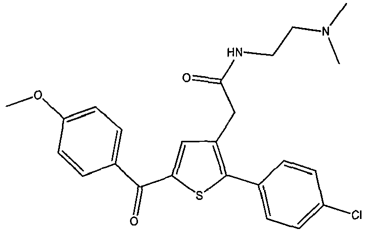 | 2-(2-(4-chlorophényl)-5-(4-méthoxybenzoyl)thiophén-3-yl)-N-(2-(diméthyl amino)éthyl)acétamide |
| **90** | 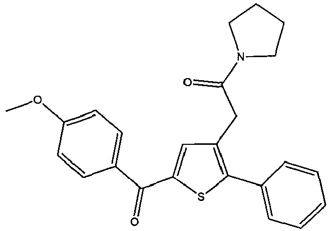 | 2-(5-(4-méthoxybenzoyl)-2-phénylthiophén-3-yl)-1-(pyrrolidin-1-yl)éthanone |
| **91** | 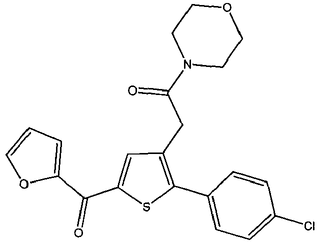 | 2-(2-(4-chlorophényl)-5-(furan-2-carbonyl)thiophén-3-yl)-1-morpholino éthanone |
| **92** | 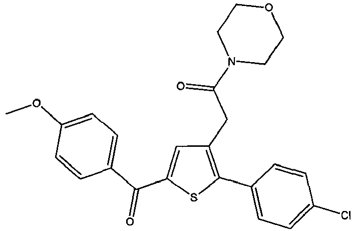 | 2-(2-(4-chlorophényl)-5-(4-méthoxybenzoyl)thiophén-3-yl)-1-morpholinoéthanone |
| 93 | 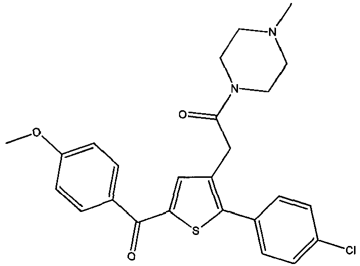 | 2-(2-(4-chlorophényl)-5-(4-méthoxybenzoyl)thiophén-3-yl)-1-(4-méthylpipérazin-1-yl)éthanone |
| **94** | 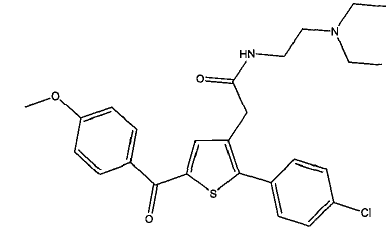 | 2-(2-(4-chlorophényl)-5-(4-méthoxybenzoyl)thiophén-3-yl)-N-(2-(diéthylamino)éthyl)acétamide |
| 95 | 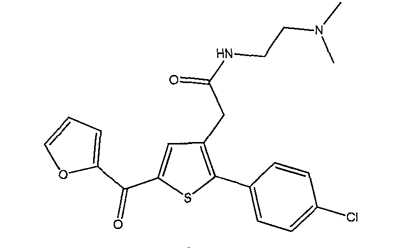 | 2-(2-(4-chlorophényl)-5-(furan-2-carbonyl)thiophén-3-yl)-N-(2-(diméthylamino)éthyl)acétamide |
| 96 | 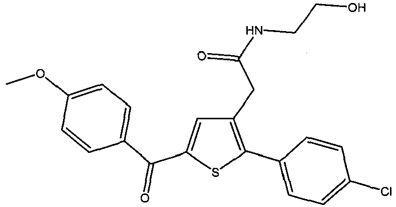 | 2-(2-(4-chlorophényl)-5-(4-méthoxybenzoyl)thiophén-3-yl)-N-(2-hydroxyéthyl)acétamide |
| 97 | 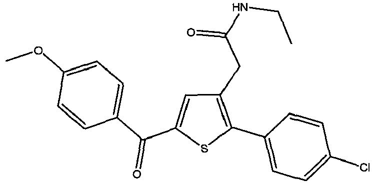 | 2-(2-(4-chlorophényl)-5-(4-méthoxybenzoyl)thiophén-3-yl)-N-éthylacétamide |
| **98** | 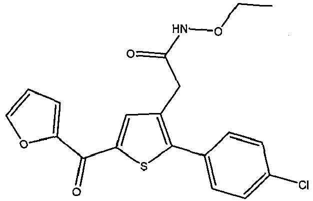 | 2-(2-(4-chlorophényl)-5-(furan-2-carbonyl)thiophén-3-yl)-N-éthoxyacétamide |
| **99** | 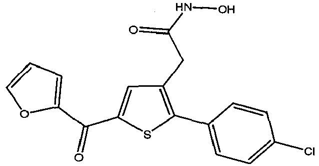 | 2-(2-(4-chlorophényl)-5-(furan-2-carbonyl)thiophén-3-yl)-N-hydroxyacétamide |
| **100** | 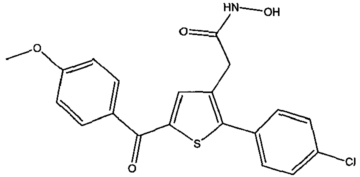 | 2-(2-(4-chlorophényl)-5-(4-méthoxybenzoyl)thiophén-3-yl)-N-hydroxyacétamide |
| **101** | 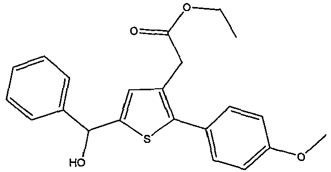 | 2-(5-(hydroxy(phényl)méthyl)-2-(4-méthoxyphényl)thiophén-3-yl)acétate d'éthyle |
| **102** | 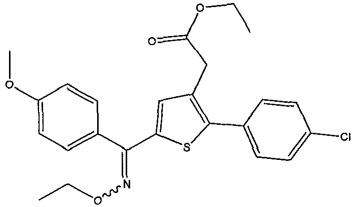 | 2-(2-(4-chlorophényl)-5-((éthoxyimino)(4-méthoxyphényl) méthyl)thiophén-3-yl)acétate d'éthyle |
| **103** | 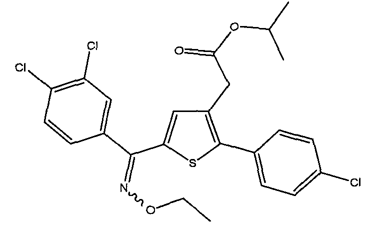 | 2-(2-(4-chlorophényl)-5-((3,4-dichlorophényl)(éthoxyimino)méthyl) thiophén-3-yl)acétate d'isopropyle |
| **104** | 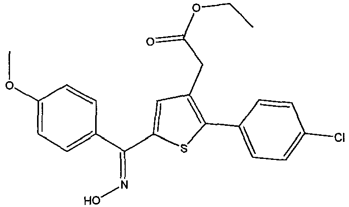 | 2-(2-(4-chlorophényl)-5-((hydroxyimino)(4-méthoxyphényl) méthyl)thiophén-3-yl)acétate d'éthyle |
| **105** | 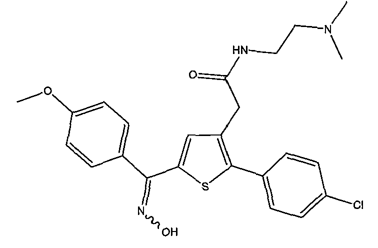 | 2-(2-(4-chlorophényl)-5-((hydroxyimino)(4-méthoxyphényl)méthyl)thiophén-3-yl)-N-(2-(diméthylamino)éthyl)acétamide |
| **106** | 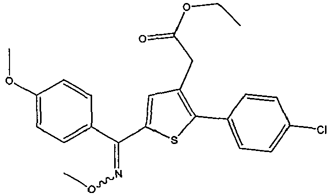 | 2-(2-(4-chlorophényl)-5-((méthoxyimino)(4-méthoxyphényl) méthyl)thiophén-3-yl)acétate d'éthyle |
| **107** | 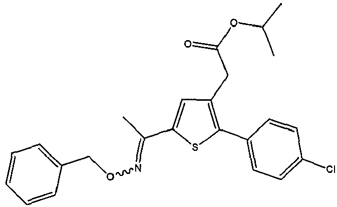 | 2-(5-(1-((benzyloxy)imino)éthyl)-2-(4-chlorophényl)thiophén-3-yl)acétate d'isopropyle |
| **108** | 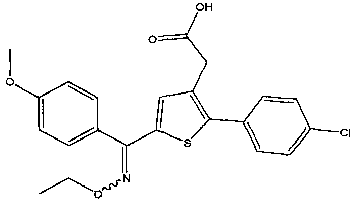 | Acide 2-(2-(4-chlorophényl)-5-((éthoxyimino)(4-méthoxyphényl) méthyl)thiophén-3-yl)acétique |
| **109** | 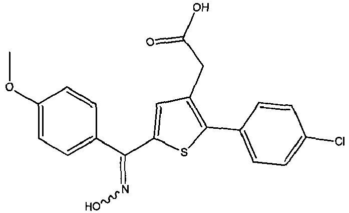 | Acide 2-(2-(4-chlorophényl)-5-((hydroxyimino)(4-méthoxyphényl) méthyl)thiophèn-3-yl)acétique |
| **110** | 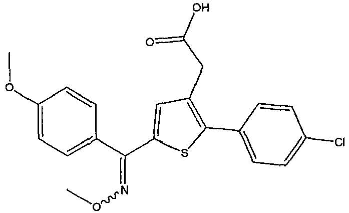 | Acide 2-(2-(4-chlorophényl)-5-((méthoxyimino)(4-méthoxyphényl) méthyl)thiophén-3-yl)acétique |
| **111** | 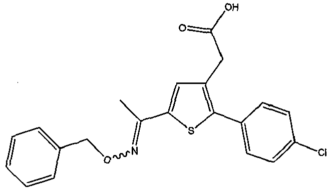 | Acide 2-(5-(1-((benzyloxy)imino) éthyl)-2-(4-chlorophényl)thiophén-3-yl)acétique |
| **112** | 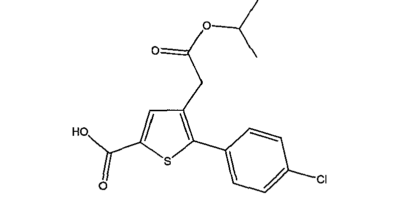 | Acide 5-(4-chlorophényl)-4-(2-isopropoxy-2-oxoéthyl)thiophène-2-carboxylique |
| **113** | 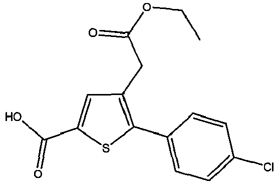 | Acide 5-(4-chlorophényl)-4-(2-ethoxy-2-oxoéthyl)thiophène-2-carboxylique |
| **114** | 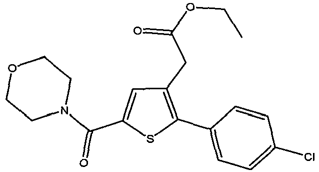 | 2-(2-(4-chlorophényl)-5-(morpholine-4-carbonyl)thiophén-3-yl)acétate d'éthyle |
| **115** | 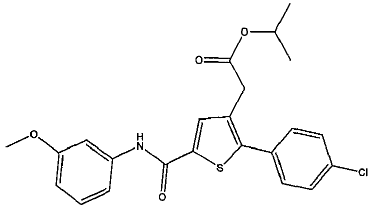 | 2-(2-(4-chlorophényl)-5-((3-méthoxyphényl)carbamoyl)thiophén-3-yl)acétate d'isopropyle |
| **116** | 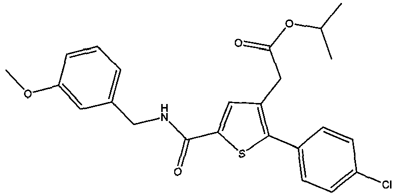 | 2-(2-(4-chlorophényl)-5-((3-méthoxybenzyl)carbamoyl)thiophén-3-yl)acétate d'isopropyle |
| **117** | 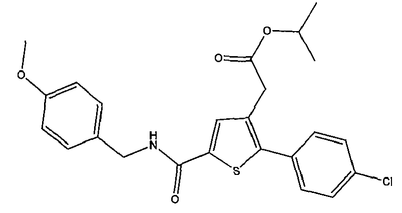 | 2-(2-(4-chlorophényl)-5-((4-méthoxybenzyl)carbamoyl)thiophén-3-yl)acétate d'isopropyle |
| **118** | 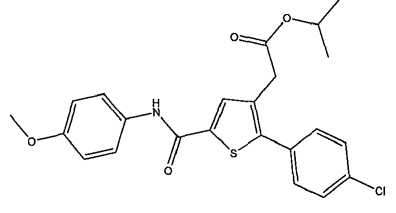 | 2-(2-(4-chlorophényl)-5-((4-méthoxyphényl)carbamoyl)thiophén-3-yl)acétate d'isopropyle |
| **119** | 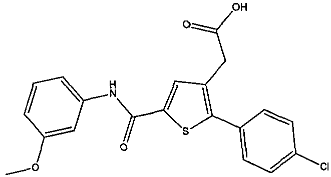 | Acide 2-(2-(4-chlorophényl)-5-((3-méthoxyphényl)carbamoyl)thiophén-3-yl)acétique |
| **120** | 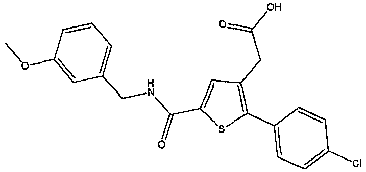 | Acide 2-(2-(4-chlorophényl)-5-((3-méthoxybenzyl)carbamoyl)thiophén-3-yl)acétique |
| **121** | 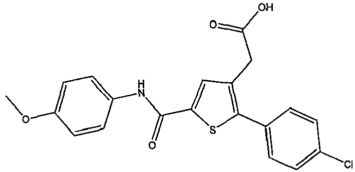 | Acide 2-(2-(4-chlorophényl)-5-((4-méthoxyphényl)carbamoyl)thiophén-3-yl)acétique |
| **122** | 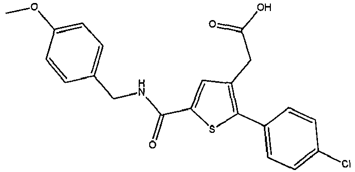 | Acide 2-(2-(4-chlorophényl)-5-((4-méthoxybenzyl)carbamoyl)thiophén-3-yl) acétique |
| **123** | 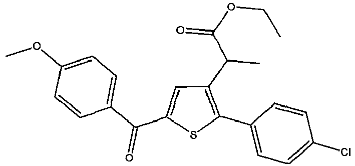 | 2-(2-(4-chlorophényl)-5-(4-méthoxybenzoyl)thiophén-3-yl) propanoate d'éthyle |
| **124** | 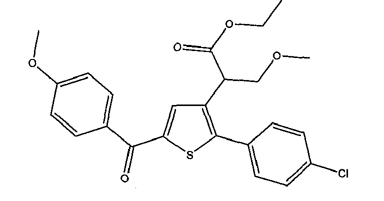 | 2-(2-(4-chlorophényl)-5-(4-méthoxybenzoyl)thiophén-3-yl)-3-méthoxypropanoate d'éthyle |
| **125** | 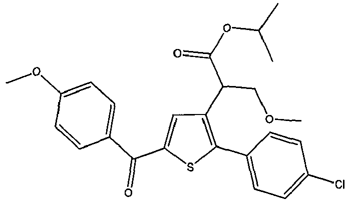 | 2-(2-(4-chlorophényl)-5-(4-méthoxybenzoyl)thiophén-3-yl)-3-méthoxypropanoate d'isopropyle |
| **126** | 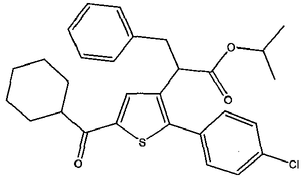 | 2-(2-(4-chlorophényl)-5-(cyclohexanecarbonyl)thiophén-3-yl)-3-phénylpropanoate d'isopropyle |
| **127** | 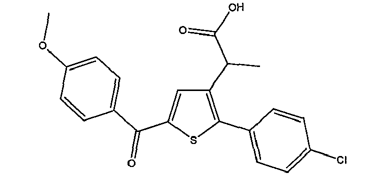 | Acide 2-(2-(4-chlorophényl)-5-(4-méthoxybenzoyl)thiophén-3-yl) propanoïque |
| **128** | 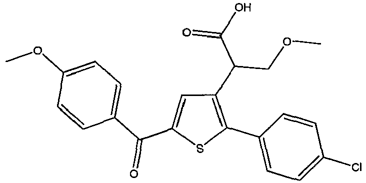 | Acide 2-(2-(4-chlorophényl)-5-(4-méthoxybenzoyl)thiophén-3-yl)-3-méthoxypropanoïque |
| **129** | 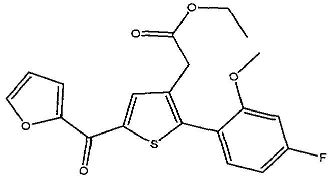 | 2-(2-(4-fluoro-2-méthoxyphényl)-5-(furan-2-carbonyl)thiophén-3-yl)acétate d'éthyle |
| **130** | 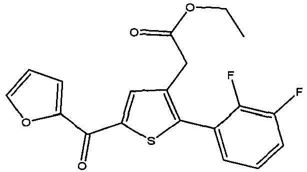 | 2-(2-(2,3-difluorophényl)-5-(furan-2-carbonyl)thiophén-3-yl)acétate d'éthyle |
| **131** | 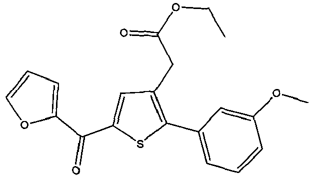 | 2-(5-(furan-2-carbonyl)-2-(3-méthoxyphényl)thiophén-3-yl)acétate d'éthyle |
| **132** | 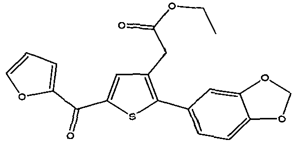 | 2-(2-(benzo[d][1,3]dioxol-5-yl)-5-(furan-2-carbonyl)thiophén-3-yl)acétate d'éthyle |
| **133** | 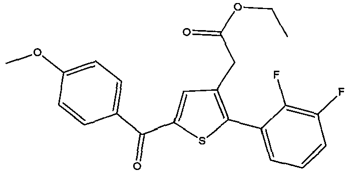 | 2-(2-(2,3-difluorophényl)-5-(4-méthoxybenzoyl)thiophén-3-yl)acétate d'éthyle |
| **134** | 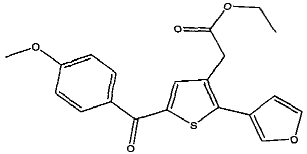 | 2-(2-(furan-3-yl)-5-(4-méthoxybenzoyl)thiophén-3-yl)acétate d'éthyle |
| **135** | 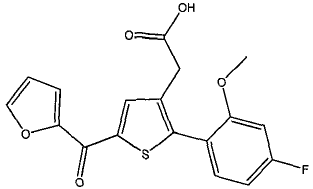 | Acide 2-(2-(4-fluoro-2-méthoxyphényl)-5-(furan-2-carbonyl)thiophén-3-yl)acétique |
| **136** | 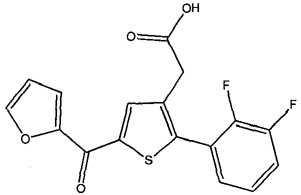 | Acide 2-(2-(2,3-difluorophényl)-5-(furan-2-carbonyl)thiophén-3-yl) acétique |
| **137** | 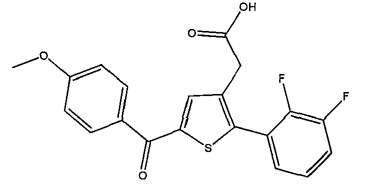 | Acide 2-(2-(2,3-difluorophényl)-5-(4-méthoxybenzoyl)thiophén-3-yl)acétique |
| **138** | 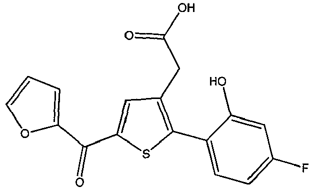 | Acide 2-(2-(4-fluoro-2-hydroxyphényl)-5-(furan-2-carbonyl)thiophén-3-yl)acétique |
| **139** | 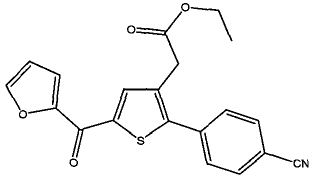 | Acide 2-(2-(4-cyanophényl)-5-(furan-2-carbonyl)thiophén-3-yl)acétique |
| **140** | 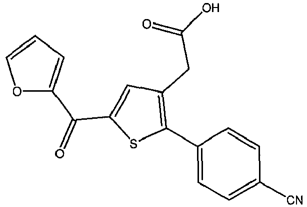 | Acide 2-(2-(4-cyanophényl)-5-(furan-2-carbonyl)thiophén-3-yl)acétique |
| **141** | 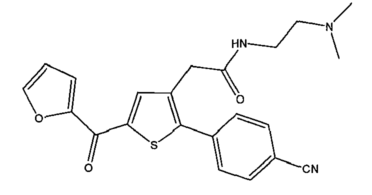 | 2-(2-(4-cyanophényl)-5-(furan-2-carbonyl)thiophén-3-yl)-N-(2-(diméthylamino)éthyl)acétamide |
| **142** | 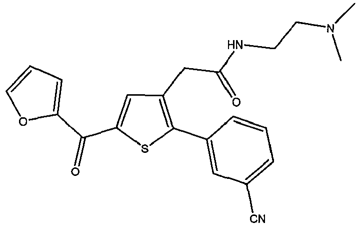 | 2-(2-(3-cyanophényl)-5-(furan-2-carbonyl)thiophén-3-yl)-N-(2-(diméthyl amino)éthyl)acétamide |
| **143** | 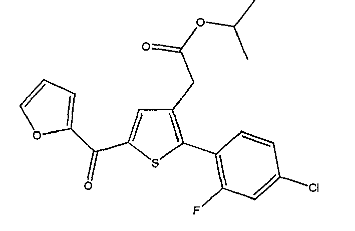 | 2-(2-(4-chloro-2-fluorophényl)-5-(furan-2-carbonyl)thiophén-3-yl)acétate d'isopropyle |
| **144** | 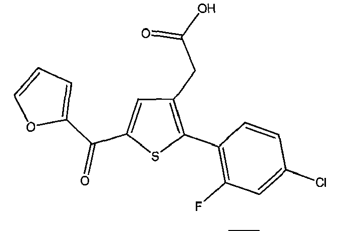 | Acide 2-(2-(4-chloro-2-fluorophényl)-5-(furan-2-carbonyl)thiophén-3-yl) acétique |
| **145** | 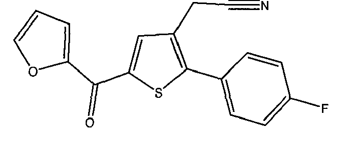 | 2-(2-(4-fluorophényl)-5-(furan-2-carbonyl)thiophén-3-yl)acéetonitrile |
| **146** | 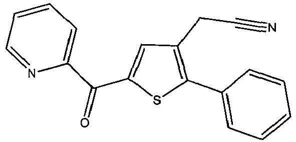 | 2-(2-phényl-5-picolinoylthiophén-3-yl)acétonitrile |
| **147** | 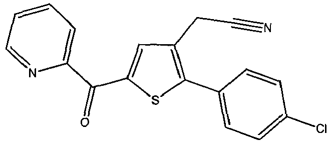 | 2-(2-(4-chlorophényl)-5-picolinoylthiophén-3-yl)acétonitrile |
| **148** | 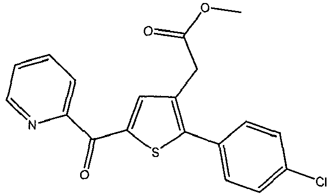 | 2-(2-(4-chlorophényl)-5-picolinoylthiophén-3-yl)acétate de méthyle |
| **149** | 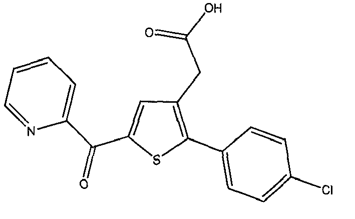 | Acide 2-(2-(4-chlorophényl)-5-picolinoylthiophén-3-yl)acétique |
| **150** | 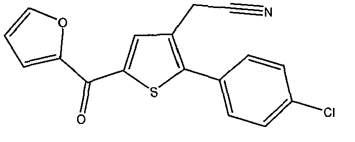 | 2-(2-(4-chlorophényl)-5-(furan-2-carbonyl)thiophén-3-yl)acétonitrile |
| **151** | 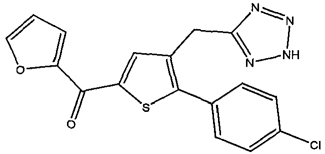 | (4-((2H-tetrazol-5-yl)méthyl)-5-(4-chlorophényl)thiophén-2-yl)(furan-2-yl)méthanone |
| **152** | 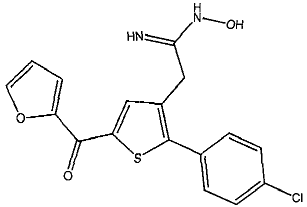 | 2-(2-(4-chlorophényl)-5-(furan-2-carbonyl)thiophén-3-yl)-N-hydroxyacétimidamide |
| **153** | 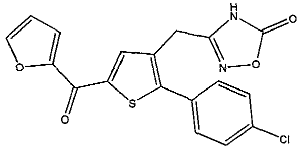 | 3-((2-(4-chlorophényl)-5-(furan-2-carbonyl)thiophén-3-yl)méthyl)-1,2,4-oxadiazol-5(4H)-one |
| **154** | 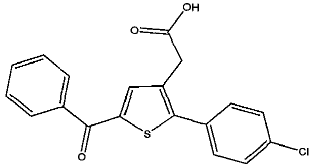 | Acide 2-[5-benzoyl-2-(4-chlorophényl)-3-thiényl]acétique |
| **155** | 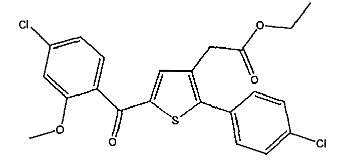 | 2-[5-(4-chloro-2-méthoxy-benzoyl)-2-(4-chlorophényl)-3-thiényl]acétate d'éthyle |
| **156** | 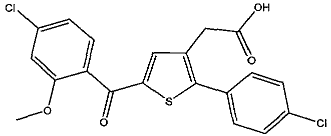 | Acide 2-[5-(4-chloro-2-méthoxy-benzoyl)-2-(4-chlorophényl)-3-thiényl]acétique |
| **157** | 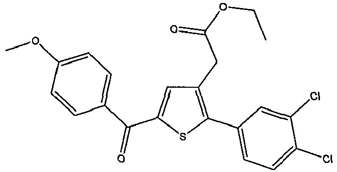 | 2-[2-(3,4-dichlorophényl)-5-(4-méthoxybenzoyl)-3-thiényl]acétate d'éthyle |
| **158** | 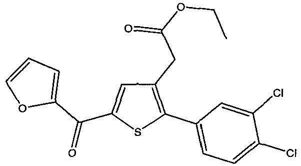 | 2-[2-(3,4-dichlorophényl)-5-(furan-2-carbonyl)-3-thiényl]acétate d'éthyle |
| **159** | 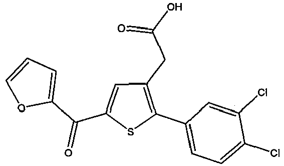 | Acide 2-[2-(3,4-dichlorophényl)-5-(furan-2-carbonyl)-3-thiényl]acétique |
| **160** | 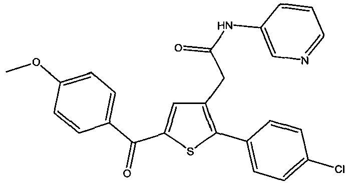 | 2-[2-(4-chlorophényl)-5-(4-méthoxybenzoyl)-3-thiényl]-N-(3-pyridyl)acétamide |
| **161** | 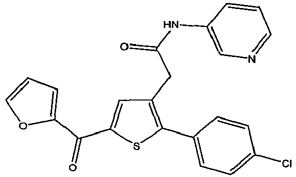 | 2-[2-(4-chlorophényl)-5-(furan-2-carbonyl)-3-thiényl]-N-(3-pyridyl)acétamide |
| **162** | 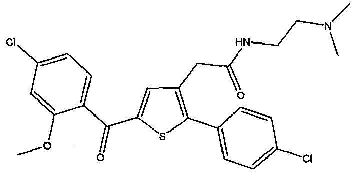 | 2-[5-(4-chloro-2-méthoxy-benzoyl)-2-(4-chlorophényl)-3-thiényl]-N-(2-diméthylaminoéthyl)acétamide |
| **163** | 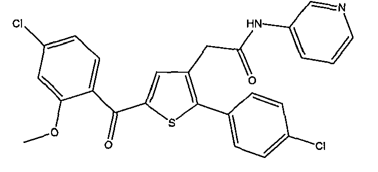 | 2-[5-(4-chloro-2-méthoxy-benzoyl)-2-(4-chlorophényl)-3-thiényl]-N-(3-pyridyl)acétamide |
| **164** | 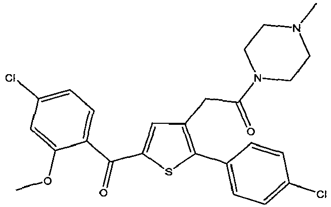 | 2-[5-(4-chloro-2-méthoxy-benzoyl)-2-(4-chlorophényl)-3-thiényl]-1-(4-méthylpipérazin-1-yl)éthanone |
| **165** | 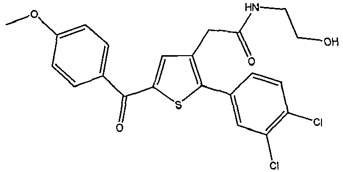 | 2-[2-(3,4-dichlorophényl)-5-(4-méthoxybenzoyl)-3-thiényl]-N-(2-hydroxyéthyl)acétamide |
| **166** | 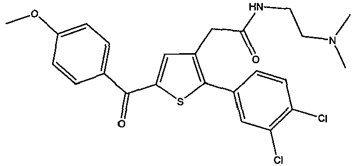 | 2-[2-(3,4-dichlorophényl)-5-(4-méthoxybenzoyl)-3-thiényl]-N-(2-diméthylaminoéthyl)acétamide |
| **167** | 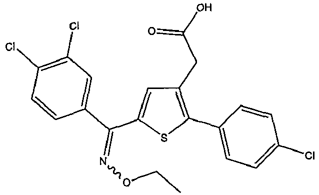 | Acide 2-[2-(4-chlorophényl)-5-[C-(3,4-dichlorophényl)-N-ethoxy-carbonimidoyl]-3-thiényl]acétique |
| **168** | 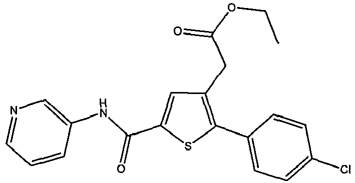 | 2-[2-(4-chlorophényl)-5-(3-pyridylcarbamoyl)-3-thiényl]acétate d'éthyle |
| **169** | 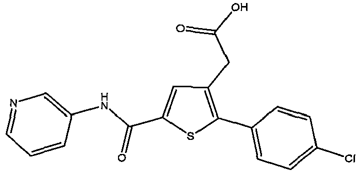 | Acide 2-[2-(4-chlorophényl)-5-(3-pyridylcarbamoyl)-3-thiényl]acétique |
| **170** | 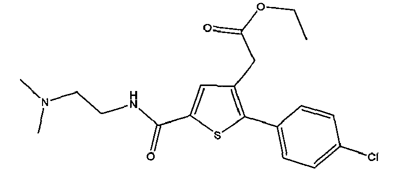 | 2-[2-(4-chlorophényl)-5-(2-diméthylaminoéthylcarbamoyl)-3-thiényl]acétate d'éthyle |
| **171** | 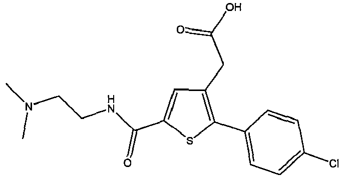 | Acide 2-[2-(4-chlorophényl)-5-(2-diméthylaminoéthylcarbamoyl)-3-thiényl]acétique |
| **172** | 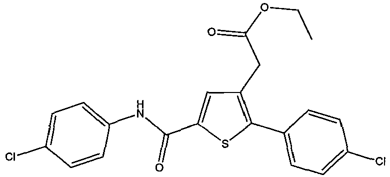 | 2-[2-(4-chlorophényl)-5-[(4-chlorophényl)carbamoyl]-3-thiényl]acétate d'éthyle |
| **173** | 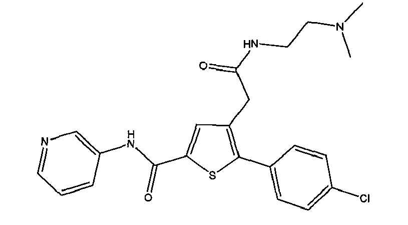 | 5-(4-chlorophényl)-4-[2-(2-diméthylaminoéthylamino)-2-oxo-éthyl]-N-(3-pyridyl)thiophène-2-carboxamide |
| **174** | 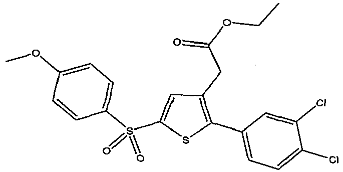 | 2-[2-(3,4-dichlorophényl)-5-(4-méthoxyphényl)sulfonyl-3-thiényl]acétate d'éthyle |
| **175** | 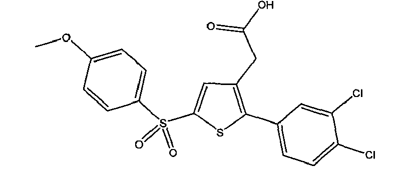 | Acide 2-[2-(3,4-dichlorophényl)-5-(4-méthoxyphényl)sulfonyl-3-thiényl]acétique |
| **176** | 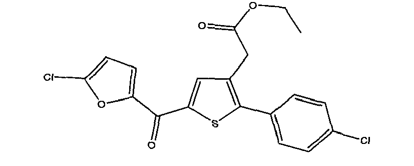 | 2-[5-(5-chlorofuran-2-carbonyl)-2-(4-chlorophényl)-3-thiényl]acétate d'éthyle |
| **177** | 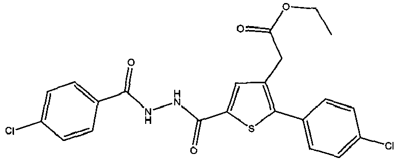 | 2-[5-[[(4-chlorobenzoyl)amino]carbamoyl]-2-(4-chlorophényl)-3-thiényl]acétate d'éthyle |
| **178** | 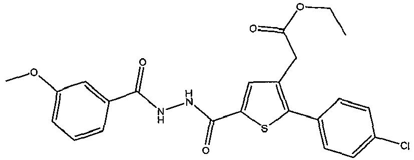 | 2-[2-(4-chlorophényl)-5-[[(3-méthoxybenzoyl)amino]carbamoyl]-3-thiényl]acétate d'éthyle |
| **179** | 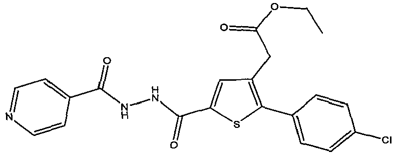 | 2-[2-(4-chlorophényl)-5-[(pyridine-4-carbonylamino)carbamoyl]-3-thiényl]acétate d'éthyle |
| **180** | 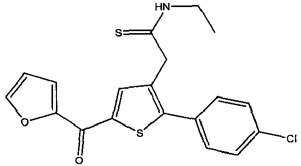 | 2-[2-(4-chlorophényl)-5-(furan-2-carbonyl)-3-thiényl]-N-éthyl-thioacétamide |
| **181** | 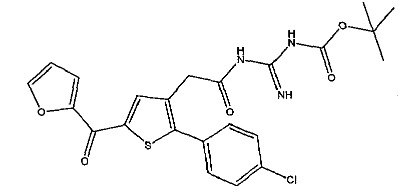 | N-[N-[2-[2-(4-chlorophényl)-5-(furan-2-carbonyl)-3-thiényl]acétyl]carbamimidoyl]carbam ate de tert-butyle |
| **182** | 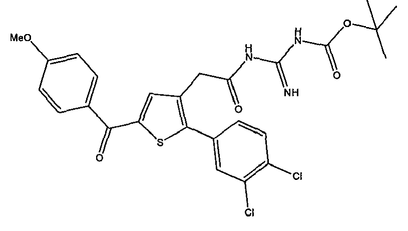 | N-[N-[2-[2-(3,4-dichlorophényl)-5-(4-méthoxybenzoyl)-3-thiényl]acétyl]carbamimidoyl]carbam ate de tert-butyle |
| **183** | 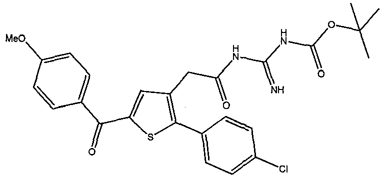 | N-[N-[2-[2-(4-chlorophényl)-5-(4-méthoxybenzoyl)-3-thiényl]acétyl]carbamimidoyl]carbam ate de tert-butyle |
| **184** | 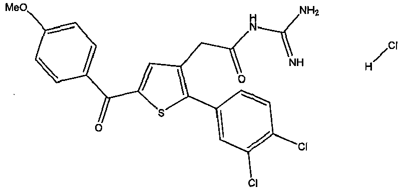 | chlorhydrate de N-carbamimidoyl-2-[2-(3,4-dichlorophényl)-5-(4-méthoxybenzoyl)-3-thiényl]acétamide |
| **185** | 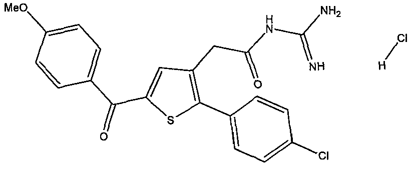 | chlorhydrate de N-carbamimidoyl-2-[2-(4-chlorophényl)-5-(4-méthoxybenzoyl)-3-thiényl]acétamide |
| **186** | 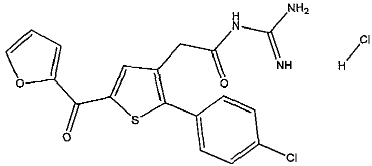 | chlorhydrate de N-carbamimidoyl-2-[2-(4-chlorophényl)-5-(furan-2-carbonyl)-3-thiényl]acétamide |
| **187** | 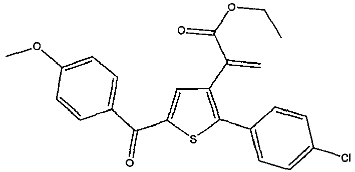 | 2-[2-(4-chlorophényl)-5-(4-méthoxybenzoyl)-3-thiényl]prop-2-énoate d'éthyle |

### Exemple 1 : Préparation du dérivé N°1: 2-(5-(4-méthoxy benzoyl)-2-(4-méthoxyphényl)thiophén-3-yl)acétate d'éthyle

### Etape 1 : Préparation du 4-méthoxyphényl 4-oxobutanoate d'éthyle

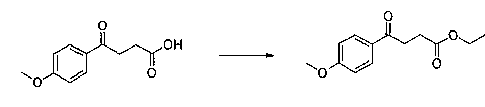

50 g (240 mmol) d'acide 4-méthoxyphényle-4-oxobutanoique ont été solubilisés dans 320 ml d'éthanol, 0,64 ml (12 mmol) d'acide sulfurique ont été ajouté à cette solution. Le mélange a été chauffé à reflux pendant 16 h sous agitation magnétique. Après retour à t.a., le mélange a été concentré sous vide, le résidu brut a été directement purifié par chromatographie flash sur cartouche de gel de silice (éluant : 100% Dichlorométhane). 54,04 g (rendement = 95%) de 4-méthoxyphényle-4-oxobutanoate d'éthyle ont été obtenus sous forme d'une huile incolore. LC-MS: m/z = 237 (MH⁺) pureté UV à 254 nm = 84%. RMN ¹H (300 MHz, DMSO) δ 7.97 (d, *J* = 8.9 Hz, 2H), 7.05 (d, *J* = 8.9 Hz, 2H), 4.05 (q, *J* = 7.1 Hz, 2H), 3.85 (s, 3H), 3.30 - 3.18 (m, 2H), 2.69 - 2.56 (m, 2H), 1.18 (t, *J* = 7.1 Hz, 3H).

### Etape 2 : Préparation du (Z/E)-4-chloro-3-formyle-4-(4-méthoxyphényl)but-3-énoate d'éthyle

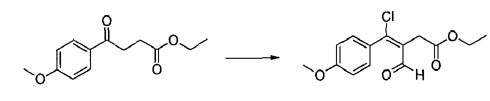

54,8 g (228 mmol) de 4-méthoxyphényle-4-oxobutanoate d'éthyle ont été solubilisés dans 52,9 ml de diméthylformamide (683 mmol), 53,1 ml (569 mmol) de trichlorure de phosphoryle ont été lentement additionnés à cette solution, la réaction étant très exothermique. Le mélange obtenu a été chauffé à 80°C pendant 3 h sous agitation magnétique. Après retour à t.a., le mélange a été versé sur 11 d'un mélange composé d'eau et de glace. La phase aqueuse a été extraite avec 2 x 200 ml d'acétate d'éthyle. Les phases organiques combinées ont été lavées avec 300 ml d'eau, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : 100% Dichlorométhane). 57 g (rendement = 75%) de (Z/E)-4-chloro-3-formyl-4-(4-méthoxyphényl)but-3-énoate d'éthyle ont été obtenu sous forme d'une huile orangée. LC-MS: m/z = 283 (MH⁺) pureté UV à 254 nm = 93%. RMN ¹H (300 MHz, DMSO) δ 9.37 (s, 1H), 7.50 (d, J = 8.8 Hz, 2H), 7.08 (d, J = 8.8 Hz, 2H), 4.12 (d, J = 7.1 Hz, 2H), 3.84 (s, 3H), 3.55 (s, 2H), 1.19 (t, J = 7.1 Hz, 3H).

### Etape 3 : Préparation du 2-(2-(4-méthoxyphényl)thiophén-3-yl)acétate d'éthyle

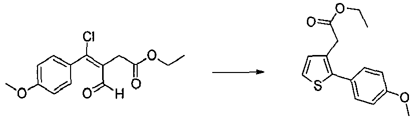

57 g (189 mmol) de (Z/E)-4-chloro-3-formyle-4-(4-méthoxyphényl)but-3-énoate d'éthyle ont été solubilisés dans 400 ml de tétrahydrofurane, à cette solution ont été ajoutés 19,71 ml (284 mmol) d'acide 2-mercapto acétique et 79 ml (567 mmol) de triéthylamine. Le mélange obtenu a été chauffé à reflux pendant 6 h sous agitation magnétique. Après retour à température ambiante, le mélange a été concentré sous vide. Le résidu a été repris dans 200 ml de diméthylformamide et le mélange a été chauffé à 130°C pendant 2 h sous agitation magnétique. Après retour à t.a., le mélange a été traité avec 600 ml d'eau. La phase aqueuse a été extraite avec 2 x 200 ml d'acétate d'éthyle. Les phases organiques combinées ont été lavées avec 2 x 200 ml d'eau, 300 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : cyclohexane / Dichlorométhane , 3/1, v/v). 29,09 g (rendement = 53%) de 2-(2-(4-méthoxyphényl)thiophén-3-yl)acétate d'éthyle ont été obtenu sous forme d'une huile incolore. LC-MS: m/z = 277 (MH⁺) pureté UV à 254 nm = 98%. RMN ¹H (300 MHz, DMSO) δ 7.46 (d, J = 5.2 Hz, 1H), 7.36 (d, J = 8.7 Hz, 2H), 7.03 (t, J = 6.5 Hz, 3H), 4.07 (q, J = 7.1 Hz, 2H), 3.79 (s, 3H), 3.62 (s, 2H), 1.16 (t, J = 7.1 Hz, 3H).
NB : Un traitement différent est décrit dans l'exemple 15 (étape 3) et permet d'isoler le sous-produit de type 1.5 décrit dans le schéma 1.

### Etape 4 : Préparation du 2-(5(4-méthoxybenzoyl)-2-(4-méthoxyphényl)thiophén-3-yl)acétate d'éthyle (dérivé N°1)

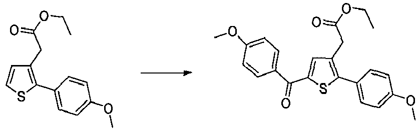

Dans un ballon placé sous un flux d'argon, ont été introduits sous agitation magnétique : 5 ml de Dichlorométhane, 0,5 g (1,809 mmol) de 2-(2-(4-méthoxyphényl)thiophén-3-yl)acétate d'éthyle et 0,367 ml (2,71 mmol) de chlorure de 4-méthoxybenzoyle. Le mélange a été placé à 5°C sous agitation magnétique et 0,362 g (2,71 mmol) de chlorure d'aluminium ont été additionnés par portions. Le mélange obtenu a été agité à t.a. pendant 5 j puis versé sur de la glace et agité pendant 1 h. La phase aqueuse a été extraite avec 2 x 20 ml de Dichlorométhane. Les phases organiques combinées ont été séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de cyclohexane / Dichlorométhane, 100% à 0% de cyclohexane, v/v). 0,652 g (rendement = 87%) de 2-(5-(4-méthoxy benzoyl)-2-(4-méthoxyphényl)thiophén-3-yl)acétate d'éthyle ont été obtenu sous forme d'une huile marron claire. LC-MS: m/z = 411 (MH⁺) pureté UV à 254 nm = 99%.. RMN ¹H (300 MHz, DMSO) δ 7.87 (d, J = 8.8 Hz, 2H), 7.70 (s, 1H), 7.46 (d, J = 8.8 Hz, 2H), 7.10 (dd, J = 13.7, 8.9 Hz, 4H), 4.06 (q, J = 7.1 Hz, 2H), 3.87 (s, 3H), 3.81 (s, 3H), 3.74 (s, 2H), 1.14 (t, J = 7.1 Hz, 3H). Les dérivés **2** à **43, 155, 157** et **158** ont été préparés selon le même enchainement des étapes 1 à 4 :

| **N°** | **Poids moléculaire g/mol** | **Apparence** | **Pureté LCMS UV à 254 nm** | **(Spectrométrie de masse m/z** | | **RMN¹H (300 MHz, DMSO) δ** |
|---|---|---|---|---|---|---|
| | | | | **MH⁺** | **M-H⁺** | |
| **2** | 370,85 | huile incolore | 99 | 371 | | 7.75-7.85 (m, 4H), 7.60-7.67 (m, 1H), 7.45-7.55 (m, 5H), 3.79 (s, 3H), 3.59 (s, 3H) |
| **3** | 326,06 | huile | 99 | 327 | | 8.17 (dd, J = 5.8, 5.0 Hz, 2H), 7.66 - 7.38 (m, 6H), 6.84 (dd, J = 3.6, 1.7 Hz, 1H), 3.81 (s, 2H), 3.62 (s, 3H). |
| **4** | 366,09 | huile | 94,7 | 367 | | 7.76 (s, 1H), 7.58 - 7.11 (m, 9H), 3.84 (s, 3H), 3.79 (d, J = 2.8 Hz, 2H), 3.58 (s, 3H). |
| **5** | 366,09 | solide | 99 | 367 | | 7.89 (d, J = 8.8 Hz, 2H), 7.73 (s, 1H), 7.52 (s, 5H), 7.13 (d, J = 8.8 Hz, 2H), 3.87 (s, 3H), 3.78 (s, 2H), 3.59 (s, 3H). |
| **6** | 342,13 | huile | 95,6 | 343 | | 7.97 (s, 1H), 7.49 (s, 5H), 3.75 (s, 2H), 3.61 (s, 3H), 3.24 (s, 1H), 1.76 (s, 6H), 1.39 (s, 4H). |
| **7** | 336,08 | solide | 97,5 | 337 | | 7.91 - 7.82 (m, 2H), 7.74 (s, 2H), 7.65 - 7.44 (m, 7H), 3.79 (s, 2H), 3.59 (s, 3H). |
| **8** | 368,09 | huile | 98,8 | 369 | | 8.09 (s, 1H), 7.49 (s, 5H), 7.34 (s, 2H), 7.16 (s, 2H), 4.31 (s, 2H), 3.76 (s, 2H), 3.62 (s, 3H). |
| **9** | 350,10 | solide | 99 | 351 | | 8.09 (s, 1H), 7.49 (s, 5H), 7.40 - 7.18 (m, 5H), 4.28 (s, 2H), 3.75 (s, 2H), 3.61 (s, 3H). |
| **10** | 374,04 | huile | 92,6 | 375 | | 8.19 (s, 2H), 7.58 (d, *J* = 3.1 Hz, 5H), 6.85 (dd, *J* = 3.6, 1.7 Hz, 1H), 4.07 (d, *J* = 7.1 Hz, 2H), 3.80 (s, 2H), 1.15 (s, 3H). |
| **11** | 418,02 | huile | 95,8 | 419 | | 7.88 - 7.70 (m, 4H), 7.58 (t, *J* = 8.1 Hz, 5H), 4.03 (d, *J* = 7.1 Hz, 2H), 3.77 (s, 2H), 1.11 (s, 3H). |
| **12** | 414,07 | huile | 91,5 | 415 | | 7.75 (s, 1H), 7.64 - 7.22 (m, 8H), 4.03 (d, *J* = 7.1 Hz, 2H), 3.84 (s, 3H), 3.77 (s, 2H), 1.12 (s, 3H). |
| **13** | 414,07 | huile | 92,7 | 415 | | 7.89 (d, *J* = 8.8 Hz, 2H), 7.74 (s, 1H), 7.58 (d, *J* = 4.1 Hz, 4H), 7.13 (d, *J* = 8.9 Hz, 2H), 4.05 (d, *J* = 7.1 Hz, 2H), 3.88 (s, 3H), 3.78 (s, 2H), 1.13 (s, 3H). |
| **14** | 390,11 | huile | 95,1 | 391 | | 7.97 (s, 1H), 7.54 (d, *J* = 9.2 Hz, 4H), 4.06 (d, *J* = 7.1 Hz, 2H), 3.74 (s, 2H), 3.24 (s, 1H), 1.79 (dd, *J* = 19.0, 6.5 Hz, 5H), 1.55 - 1.25 (m, 5H), 1.14 (s, 3H). |
| **15** | 384,06 | solide | 96,9 | 385 | | 7.90 - 7.81 (m, 2H), 7.77 - 7.66 (m, 2H), 7.60 (dd, *J* = 9.1, 5.7 Hz, 6H), 4.04 (d, *J* = 7.1 Hz, 2H), 3.78 (s, 2H), 1.12 (s, 3H). |
| **16** | 398,07 | huile | 92,5 | 399 | | 8.10 (s, 1H), 7.55 (d, *J* = 8.6 Hz, 4H), 7.32 (d, *J* = 4.1 Hz, 5H), 4.29 (s, 2H), 4.06 (d, *J* = 7.1 Hz, 2H), 3.75 (s, 2H), 1.14 (s, 3H). |
| **17** | 412,09 | huile | 99 | 413 | | 7.96 (s, 1H), 7.62 - 7.47 (m, 4H), 7.35 - 7.25 (m, 4H), 7.24 - 7.13 (m, 1H), 4.05 (q, *J* = 7.1 Hz, 2H), 3.72 (s, 2H), 3.29 (dd, *J* = 9.5, 5.6 Hz, 2H), 2.95 (t, *J* = 7.5 Hz, 2H), 1.13 (dd, *J* = 9.2, 5.0 Hz, 3H). |
| **18** | 416,44 | solide | 99 | 417 | | 7.84 - 7.57 (m, 2H), 7.54 - 7.29 (m, 4H), 7.16 - 6.98 (m, 2H), 4.04 (dt, *J* = 14.2, 6.5 Hz, 2H), 3.82 (d, *J* = 2.4 Hz, 3H), 3.72 (s, 2H), 1.13 (dt, *J* = 12.4, 7.1 Hz, 3H). |
| **19** | 416,44 | solide | 99 | 417 | | 7.76 (td, *J* = 8.4, 6.6 Hz, 1H), 7.65 - 7.41 (m, 4H), 7.38 - 7.23 (m, 1H), 7.13 - 7.01 (m, 2H), 4.04 (q, *J* = 7.1 Hz, 2H), 3.82 (s, 3H), 3.72 (s, 2H), 1.12 (t, *J* = 7.1 Hz, 3H). |
| **20** | 416,44 | solide | 96 | 417 | | 7.53 (dd, *J* = 39.8, 5.1 Hz, 6H), 7.08 (d, *J* = 8.8 Hz, 2H), 4.02 (q, *J* = 7.1 Hz, 2H), 3.81 (s, 3H), 3.72 (s, 2H), 1.11 (t, *J* = 7.1 Hz, 3H). |
| **21** | 398,45 | huile | 99 | 399 | | 7.75 - 7.59 (m, 2H), 7.54 (d, *J* = 1.5 Hz, 1H), 7.52 - 7.32 (m, 4H), 7.16 - 6.99 (m, 2H), 4.12 - 3.93 (m, 2H), 3.81 (s, 3H), 3.71 (s, 2H), 1.15 - 1.02 (m, 3H). |
| **22** | 370,09 | solide | 96,8 | 371 | | 8.16 (d, *J* = 8.2 Hz, 2H), 7.63 - 7.36 (m, 3H), 7.09 (d, *J* = 8.4 Hz, 2H), 6.83 (s, 1H), 4.09 (d, J = 7.0 Hz, 2H), 3.80 (d, *J* = 14.1 Hz, 5H), 1.16 (d, *J* = 7.0 Hz, 3H). |
| **23** | 412,47 | huile | 99 | 411 | | 7.54 (ddd, *J* = 8.5, 7.4, 1.8 Hz, 1H), 7.48 - 7.41 (m, 2H), 7.37 (q, *J* = 1.7 Hz, 2H), 7.21 (d, *J* = 8.2 Hz, 1H), 7.11 - 7.03 (m, 3H), 4.03 (q, *J* = 7.1 Hz, 2H), 3.81 (s, 3H), 3.77 (s, 3H), 3.68 (s, 2H), 1.11 (t, *J* = 7.1 Hz, 3H). |
| **24** | 416,44 | solide | 99 | 417 | | 7.77 (s, 1H), 7.67 - 7.56 (m, 1H), 7.55 - 7.42 (m, 4H), 7.16 - 6.98 (m, 2H), 4.05 (q, *J* = 7.1 Hz, 2H), 3.82 (s, 3H), 3.72 (d, J = 12.7 Hz, 2H), 1.14 (t, J = 7.1 Hz, 3H). |
| **25** | 414,07 | huile | 99 | 415 | | 7.78 (dd, *J* = 19.4, 10.4 Hz, 4H), 7.62 (s, 1H), 7.47 (d, *J* = 8.7 Hz, 2H), 7.08 (d, *J* = 8.8 Hz, 2H), 4.05 (d, *J* = 7.1 1Hz, 2H), 3.81 (s, 3H), 3.75 (s, 2H), 1.14 (s, 3H). |
| **26** | 398,45 | huile | 99 | 399 | | 7.74 (s, 1H), 7.72 - 7.51 (m, 4H), 7.50 - 7.44 (m, 2H), 7.12 - 7.04 (m, 2H), 4.06 (q, *J* = 7.1 Hz, 2H), 3.82 (s, 3H), 3.75 (s, 2H), 1.14 (t, *J* = 7.1 Hz, 3H). |
| **27** | 428,47 | solide | 96 | 429 | | 7.84 - 7.63 (m, 3H), 7.52 - 7.43 (m, 2H), 7.38 (t, *J* = 8.5 Hz, 1H), 7.14 - 7.02 (m, 2H), 4.09 (t, *J* = 7.1 Hz, 2H), 3.97 (s, 3H), 3.83 (s, 3H), 3.75 (s, 2H), 1.16 (t, *J* = 7.1 Hz, 3H). |
| **28** | 370,42 | huile | 99 | 371 | | 8.63 (d, *J* = 0.9 Hz, 1H), 7.94 (dd, *J* = 10.6, 8.9 Hz, 2H), 7.47 (d, *J* = 8.8 Hz, 2H), 7.08 (d, *J* = 8.8 Hz, 2H), 6.96 (dd, *J* = 1.8, 0.7 Hz, 1H), 4.20 - 3.96 (m, 2H), 3.82 (s, 3H), 3.75 (s, 2H), 1.31- 1.00 (m, 3H). |
| **29** | 412,47 | solide | 99 | 413 | | 7.89 - 7.64 (m, 3H), 7.56 - 7.42 (m, 2H), 7.42 - 7.28 (m, 1H), 7.14 - 6.96 (m, 2H), 4.06 (q, *J* = 7.1 Hz, 2H), 3.82 (s, 3H), 3.74 (s, 2H), 2.33 (d, *J* = 1.6 Hz, 3H), 1.14 (t, *J* = 7.1 Hz, 3H). |
| **30** | 410,12 | huile | 96,5 | 411 | | 7.72 (s, 1H), 7.57 - 7.36 (m, 4H), 7.35 - 7.20 (m, 2H), 7.08 (d, *J* = 8.7 Hz, 2H), 4.06 (q, *J* = 7.1 Hz, 2H), 3.83 (d, J = 8.0 Hz, 6H), 3.75 (s, 2H), 1.14 (t, *J* = 7.1 Hz, 3H). |
| **31** | 405,47 | huile | 99 | 406 | | 8.08 (d, *J* = 8.5 Hz, 2H), 7.97 (d, *J* = 8.5 Hz, 2H), 7.70 (s, 1H), 7.48 (d, *J* = 8.8 Hz, 2H), 7.09 (d, *J* = 8.8 Hz, 2H), 4.05 (q, *J* = 7.1 Hz, 2H), 3.82 (s, 3H), 3.74 (s, 2H), 1.22 - 1.02 (m, 3H). |
| **32** | 398,45 | huile | 99 | 399 | | 8.01 - 7.85 (m, 2H), 7.72 (s, 1H), 7.55 - 7.35 (m, 4H), 7.18 - 7.01 (m, 2H), 4.06 (q, *J* = 7.1 Hz, 2H), 3.83 (s, 3H), 3.75 (s, 2H), 1.15 (t, *J* = 7.1 Hz, 3H). |
| **33** | 387,47 | solide | 99 | 388 | | 9.34 (d, *J* = 2 Hz, 1H), 8.72 (d, J = 2 Hz, 1H), 8.45 (s, 1H), 7.47 (d, J = 8.8 Hz, 2H), 7.07 (d, J = 8.8 Hz, 2H), 4.07 (q, J = 7.1 Hz, 2H), 3.81 (s, 3H), 3.75 (s, 2H), 1.15 (t, J = 7.1 Hz, 3H), |
| **34** | 386,16 | huile | 98,1 | 387 | | 7.93 (s, 1H), 7.42 (d, *J* = 8.8 Hz, 2H), 7.06 (d, *J* = 8.8 Hz, 2H), 4.07 (q, *J* = 7.1 Hz, 2H), 3.80 (s, 3H), 3.70 (s, 2H), 3.23 (s, 1H), 1.91 - 1.27 (m, 10H), 1.16 (t, *J* = 7.1 Hz, 3H). |
| **35** | 380,11 | solide | 99 | 381 | | 7.89 - 7.81 (m, 2H), 7.70 (s, 2H), 7.61 (d, *J* = 7.6 Hz, 2H), 7.47 (d, *J* = 8.8 Hz, 2H), 7.08 (d, *J* = 8.8 Hz, 2H), 4.06 (d, *J* = 7.1 Hz, 2H), 3.82 (s, 3H), 3.74 (s, 2H), 1.14 (s, 3H). |
| **36** | 394,12 | solide | 99 | 395 | | 8.06 (s, 1H), 7.42 (d, *J* = 8.8 Hz, 2H), 7.32 (d, *J* = 4.1 Hz, 5H), 7.05 (d, *J* = 8.8 Hz, 2H), 4.26 (s, 2H), 4.07 (d, *J* = 7.1 Hz, 2H), 3.80 (s, 3H), 3.71 (s, 2H), 1.15 (s, 3H). |
| **37** | 380,08 | solide | 99 | 381 | | 7.90 (s, 1H), 7.43 (d, *J* = 8.8 Hz, 2H), 7.06 (d, *J* = 8.8 Hz, 2H), 4.07 (q, *J* = 7.1 Hz, 2H), 3.80 (s, 3H), 3.71 (d, *J* = 1.3 Hz, 4H), 3.10 (s, 2H), 2.07 (s, 2H), 1.16 (s, 3H). |
| **38** | 408,14 | solide | 99 | 409 | | 7.93 (s, 1H), 7.42 (d, *J =* 8.8 Hz, 2H), 7.29 (d, *J* = 4.4 Hz, 4H), 7.19 (d, *J* = 4.5 Hz, 1H), 7.06 (d, *J* = 8.8 Hz, 2H), 4.06 (d, *J* = 7.1 Hz, 2H), 3.80 (s, 3H), 3.69 (s, 2H), 3.28 (s, 2H), 2.94 (s, 2H), 1.15 (s, 3H). |
| **39** | 423,31 | solide | >99 | 423 | | 8.18 (d, J = 10.0 Hz, 2H), 7.81 (dd, J = 10.7, 5.2 Hz, 2H), 7.64 - 7.50 (m, 2H), 6.86 (dd, J = 3.6, 1.7 Hz, 1H), 4.95 - 4.80 (m, 1H), 3.82 (s, 2H), 1.14 (d, J = 6.3 Hz, 6H). |
| **40** | 463,37 | solide | >99 | 463 | 461 | 7.98 - 7.68 (m, 5H), 7.54 (d, J = 8.4 Hz, 1H), 7.13 (d, J = 8.8 Hz, 2H), 4.86 (dt, J = 12.5, 6.2 Hz, 1H), 3.88 (s, 3H), 3.78 (s, 2H), 1.14 (d, J = 6.3 Hz, 6H). |
| **41** | 467,79 | solide | 96 | 468 | | 8.01 (d, *J* = 1.9 Hz, 1H), 7.93 - 7.69 (m, 3H), 7.65 - 7.47 (m, 4H), 5.00 - 4.57 (m, 1H), 3.68 (d, *J* = 37.6 Hz, 2H), 1.11 (d, *J* = 6.3 Hz, 6H). |
| **42** | 336,83 | huile | 90 | 337 | | 7.72 - 7.37 (m, 9H), 7.31 (s, 1H), 3.61 (s, 2H), 3.39 (t, J = 6.6 Hz, 2H), 3.30 (t, J = 6.8Hz, 2H), 1.96 - 1.66 (m, 4H). |
| **43** | 428,92 | solide blanc | 97 | 429 | | 7.97 - 7.81 (m, 2H), 7.72 (s, 1H), 7.64 - 7.49 (m, 4H), 7.13 (d, *J* = 8.8 Hz, 2H), 4.96 - 4.73 (m, 1H), 3.88 (s, 3H), 3.74 (s, 2H), 1.13 (d, *J* = 6.3 Hz, 6H). |
| **155** | 449,34 | Solide | 99 | 449 | | 7.67 - 7.40 (m, 7H), 7.25 (d, J = 9.0 Hz, 1H), 4.01 (q, J = 7.1 Hz, 2H), 3.77 (s, 3H), 3.72 (s, 2H), 1.09 (t, J = 7.1 Hz, 3H) |
| **157** | 449,34 | Solide | 99 | 449 | | 7.89 (d, J = 8.8 Hz, 2H), 7.83 (d, J = 2.1 Hz, 1H), 7.79 (d, J = 8.4 Hz, 1H), 7.75 (s, 1H), 7.53 (dd, J = 8.4, 2.1 Hz, 1H), 7.13 (d, J = 8.9 Hz, 2H), 4.05 (q, J = 7.1 Hz, 2H), 3.81 (s, 2H), 1.14 (t, J = 7.1 Hz, 3H) |
| **158** | 409,28 | solide | 99 | 409 | 407 | 8.18 (d, J = 9.8 Hz, 2H), 7.81 (dd, J = 12.7, 5.0 Hz, 2H), 7.57 (dd, J = 21.4, 5.0 Hz, 2H), 6.85 (d, J = 1.9 Hz, 1H), 4.07 (q, J = 7.0 Hz, 2H), 3.83 (s, 2H), 1.15 (t, J = 7.1 Hz, 3H) |

### Exemple 2 : Préparation du dérivé N°44 : 2-(2-(4-chlorophényl)-5-(furan-2-carbonyl)thiophén-3-yl)acétate d'isopropyle

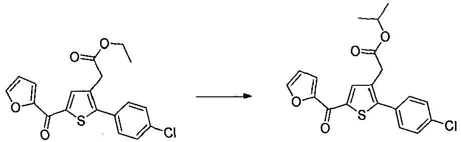

200 mg (0,518 mmol) de 2-(2-(4-chlorophényl)-5-(furan-2-carbonyl)thiophén-3-yl)acétate d'éthyle ont été solubilisés dans 5 mmol de 2-Propanol, une goutte d'acide sulfurique a été ajoutée, le mélange a été agité à reflux sous agitation magnétique pendant 17 h. Le retour à t.a. a permis la précipitation d'un solide dans le milieu réactionnel. Le solide a été isolé par filtration et séché sous cloche à vide pour donner 110 mg (rendement = 54%) de 2-(2-(4-chlorophényl)-5-(furan-2-carbonyl) thiophén-3-yl)acétate d'isopropyle sous forme d'une poudre blanche. LC-MS: m/z = 389 (MH⁺) pureté UV à 254 nm = 99%. RMN ¹H (300 MHz, DMSO) δ 8.15 (dd, *J* = 6.5, 5.5 Hz, 2H), 7.58 (d, *J* = 2.4 Hz, 5H), 6.84 (dd, *J* = 3.6, 1.7 Hz, 1H), 5.00 - 4.76 (m, 1H), 3.77 (s, 2H), 1.15 (d, *J* = 6.3 Hz, 6H).

### Exemple 3 : Préparation du dérivé numéro 45 : acide 2-(5-(4-méthoxybenzoyl)-2-(4-méthoxyphényl)thiophén-3-yl)acétique

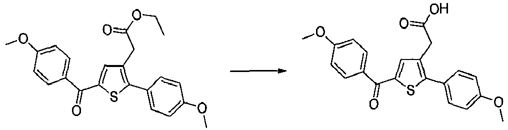

0,616 g (1,503 mmol) de 2-(5-(4-méthoxybenzoyl)-2-(4-méthoxyphényl)thiophén-3-yl)acétate d'éthyle ont été solubilisés dans 5 ml d'éthanol, à cette solution ont été ajoutés sous agitation magnétique 0,301 ml (3,01 mmol) d'une solution aqueuse d'hydroxyde de sodium à 30% en masse. Le mélange obtenu a été agité à t.a. pendant 16 h. Le mélange a été concentré sous vide, le résidu obtenu a été repris dans 10 ml d'eau, la phase aqueuse a été extraite avec 2 x 5 ml d'acétate d'éthyle. Le pH de la phase aqueuse a ensuite été abaissé par addition d'une solution aqueuse d'acide chlorhydrique 1N jusqu'à apparition d'un précipité. Le solide a été isolé par filtration, lavé avec 2 x 5 ml d'eau et séché sous cloche à vide pour donner 0,536 g (rendement = 92%) d'acide 2-(5-(4-méthoxybenzoyl)-2-(4-méthoxy phényl)thiophén-3-yl)acétique sous forme d'un solide blanc. LC-MS: m/z = 383 (MH⁺) pureté UV à 254 nm = 99%. RMN ¹H (300 MHz, DMSO) δ 12.66 (s, 1H), 7.87 (d, J = 8.8 Hz, 2H), 7.69 (s, 1H), 7.48 (d, J = 8.7 Hz, 2H), 7.10 (dd, J = 12.0, 8.8 Hz, 4H), 3.87 (s, 3H), 3.81 (s, 3H), 3.64 (s, 2H).

Les dérivés **46** à **81, 154, 156** et **159** ont été préparés selon le même protocole :

| **N°** | **Poids moléculaire g/mo)** | **Apparence** | **Pureté LCMS UV à 254 nm** | **Spectrométrie de masse m/z** | | **RMN ¹H (300 MHz, DMSO) δ** |
|---|---|---|---|---|---|---|
| | | | | **MH+** | **M-H⁺** | |
| **46** | 358,12 | solide | 99 | 359 | | 12.64 (s, 1H), 7.93 (s, 1H), 7.44 (d, *J* = 8.8 Hz, 2H), 7.06 (d, *J* = 8.8 Hz, 2H), 3.80 (s, 3H), 3.61 (s, 2H), 3.23 (s, 1H), 1.77 (d, *J* = 15.3 Hz, 5H), 1.39 (t, *J* = 10.0 Hz, 5H). |
| **47** | 316,08 | solide | 99 | 317 | 315 | 12.67 (s, 1H), 8.05 (s, 1H), 7.44 (d, *J* = 8.7 Hz, 2H), 7.06 (d, *J* = 8.8 Hz, 2H), 3.80 (s, 3H), 3.61 (s, 2H), 2.78 (s, 1H), 1.13 - 0.91 (m, 4H). |
| **48** | 312,05 | solide | 97,7 | 313 | 311 | 12.65 (s, 1H), 8.27 - 8.07 (m, 2H), 7.65 - 7.39 (m, 6H), 6.84 (dd, *J* = 3.6, 1.7 Hz, 1H), 3.70 (s, 2H). |
| **49** | 356,03 | solide | 99 | 357 | | 12.63 (s, 1H), 7.87 - 7.71 (m, 4H), 7.63 (s, 1H), 7.53 (s, 5H), 3.68 (s, 2H). |
| **50** | 352,08 | huile | 95,5 | 353 | 351 | 12.74 (s, 1H), 7.76 (s, 1H), 7.58 - 7.16 (m, 9H), 3.85 (s, 3H), 3.67 (s, 2H). |
| **51** | 352,08 | solide | 99 | 353 | 351 | 12.61 (s, 1H), 7.88 (d, *J* = 8.8 Hz, 2H), 7.73 (s, 1H), 7.53 (d, J = 1.0 Hz, 5H), 7.13 (d, *J* = 8.8 Hz, 2H), 3.87 (s, 3H), 3.67 (s, 2H). |
| **52** | 328,11 | solide | 99 | 329 | 327 | 12.62 (s, 1H), 7.96 (s, 1H), 7.50 (s, 5H), 3.63 (s, 2H), 3.25 (s, 1H), 1.79 (dd, *J* = 19.5, 6.5 Hz, 5H), 1.39 (s, 4H), 1.19 (d, *J* = 12.0 Hz, 1H). |
| **53** | 322,07 | solide | 99 | 323 | 321 | 7.88 - 7.79 (m, 2H), 7.69 (d, *J* = 7.5 Hz, 2H), 7.63 - 7.40 (m, 7H), 3.55 (s, 2H). |
| **54** | 354,07 | solide | 96,4 | 355 | 353 | 12.72 (s, 1H), 8.10 (s, 1H), 7.50 (s, 5H), 7.34 (d, *J* = 5.6 Hz, 2H), 7.15 (s, 2H), 4.31 (s, 2H), 3.63 (d, *J* = 11.6 Hz, 2H). |
| **55** | 336,08 | solide | 98 | 337 | | 12.65 (s, 1H), 8.11 (s, 1H), 7.68 - 7.17 (m, 10H), 4.29 (s, 2H), 3.65 (s, 2H). |
| **56** | 346,01 | solide | 95,8 | 347 | 345 | 12.68 (s, 1H), 8.26 - 8.11 (m, 2H), 7.69 - 7.50 (m, 5H), 6.85 (dd, *J* = 3.6, 1.7 Hz, 1H), 3.71 (s, 2H). |
| **57** | 425,71 | solide jaune pale | >99 | non ionisé | | 8.00 (d, *J* = 1.8 Hz, 1H), 7.91-7.76 (m, 4H), 7.67 (s, 1H), 7.54 (d, *J* = 8.5 Hz, 2H), 3.22 (s, 2H). |
| **58** | 389,99 | solide | 97,3 | 391 | | 12.62 (s, 1H), 7.89 - 7.70 (m, 4H), 7.59 (s, 5H), 3.69 (s, 2H). |
| **59** | 386,04 | solide | 93,1 | 387 | 385 | 12.63 (s, 1H), 7.76 (s, 1H), 7.65 - 7.22 (m, 8H), 3.84 (s, 3H), 3.68 (s, 2H). |
| **60** | 386,04 | solide | 97,1 | 387 | 385 | 12.62 (s, 1H), 7.89 (d, *J* = 8.8 Hz, 2H), 7.73 (s, 1H), 7.58 (d, J = 3.3 Hz, 4H), 7.13 (d, *J* = 8.8 Hz, 2H), 3.88 (s, 3H), 3.68 (s, 2H). |
| **61** | 362,07 | solide | 99 | 363 | | 12.62 (s, 1H), 7.96 (s, 1H), 7.55 (d, *J* = 8.3 Hz, 4H), 3.63 (s, 2H), 3.24 (s, 1H), 1.93 - 1.57 (m, 5H), 1.38 (t, *J* = 10.3 Hz, 5H). |
| **62** | 294,75 | solide | >99 | | 293 | 12.66 (s, 1H), 7.90 (s, 1H), 7.56 (q, J = 8.7 Hz, 4H), 3.63 (s, 2H), 2.54 (s, 3H). |
| **63** | 384,06 | solide | 95,5 | 385 | | 12.63 (s, 1H), 7.97 (s, 1H), 7.55 (td, *J* = 8.7, 6.5 Hz, 4H), 7.29 (d, *J* = 4.4 Hz, 4H), 7.19 (d, *J* = 4.3 Hz, 1H), 3.62 (s, 2H), 3.33 - 3.26 (m, 3H), 2.94 (s, 2H). |
| **64** | 388,39 | solide | 99 | 389 | 387 | 13.06 - 11.89 (m, 1H), 7.77-7.64 (m, 1H), 7.62 (d, *J* = 1.5 Hz, 1H), 7.59 - 7.32 (m, 4H), 7.14 - 7.02 (m, 2H), 3.82 (d, *J* = 2.1 Hz, 3H), 3.61 (s, 2H). |
| **65** | 388,39 | solide | 97 | 389 | 387 | 13.08 - 12.07 (m, 1H), 7.76 (td, *J* = 8.3, 6.6 Hz, 1H), 7.65-7.39 (m, 4H), 7.29 (td, *J* = 8.4, 2.3 Hz, 1H), 7.18 - 6.96 (m, 2H), 3.82 (s, 3H), 3.61 (s, 2H). |
| **66** | 388,39 | solide | 99 | 389 | 387 | 12.83 - 12.21 (m, 1H), 7.71-7.34 (m, 6H), 7.08 (dd, *J* = 9.2, 2.6 Hz, 2H), 3.81 (s, 3H), 3.61 (s, 2H). |
| **67** | 370,39 | solide | 93 | 371 | | 12.95 - 12.19 (m, 1H), 7.74-7.59 (m, 2H), 7.58 - 7.34 (m, 5H), 7.14 - 7.01 (m, 2H), 3.82 (s, 3H), 3.62 (s, 2H). |
| **68** | 342,06 | solide | 99 | 343 | 341 | 12.66 (s, 1H), 8.16 (d, *J* = 5.8 Hz, 2H), 7.53 (dd, *J* = 23.6, 6.0 Hz, 3H), 7.09 (d, *J* = 8.6 Hz, 2H), 6.93 - 6.74 (m, 1H), 3.82 (s, 3H), 3.68 (s, 2H). |
| **69** | 382,43 | solide | 99 | 383 | 381 | 7.52 (dd, *J* = 7.2, 1.3 Hz, 1H), 7.49 - 7.41 (m, 2H), 7.41-7.32 (m, 2H), 7.20 (d, *J* = 8.3 Hz, 1H), 7.07 (dt, *J* = 3.5, 2.5 Hz, 3H), 3.81 (s, 3H), 3.77 (s, 3H), 3.55 (d, *J =* 14.3 Hz, 2H). |
| **70** | 388,39 | solide | 99 | 389 | 387 | 13.06 - 12.16 (m, 1H), 7.77 (s, 1H), 7.72 - 7.41 (m, 5H), 7.08 (dd, *J* = 9.4, 2.6 Hz, 2H), 3.82 (s, 3H), 3.65 (s, 2H). |
| **71** | 386,04 | solide | 99 | 387 | 385 | 12.60 (s, 1H), 7.85 - 7.69 (m, 4H), 7.62 (s, 1H), 7.49 (d, *J* = 8.7 Hz, 2H), 7.09 (d, *J* = 8.8 Hz, 2H), 3.82 (s, 3H), 3.66 (s, 2H). |
| **72** | 370,39 | huile | 97 | 371 | 369 | 12.95 - 12.12 (m, 1H), 7.78-7.43 (m, 7H), 7.10 (d, *J* = 8.8 Hz, 2H), 3.83 (s, 3H), 3.66 (s, 2H). |
| **73** | 400,42 | solide | 97 | 401 | 399 | 7.71 (dt, *J* = 11.9, 4.7 Hz, 3H), 7.48 (d, *J* = 8.7 Hz, 2H), 7.37 (t, *J* = 8.5 Hz, 1H), 7.08 (d, *J* = 8.8 Hz, 2H), 3.96 (s, 3H), 3.82 (s, 3H), 3.65 (s, 2H). |
| **74** | 384,42 | solide | 99 | 385 | 383 | 7.90 - 7.65 (m, 3H), 7.56-7.41 (m, 2H), 7.41 - 7.26 (m, 1H), 7.15 - 6.96 (m, 2H), 3.81 (s, 3H), 3.61 (d, *J* = 22.1 Hz, 2H), 2.31 (dd, *J* = 12.0, 5.1 Hz, 3H). |
| **75** | 382,09 | huile | 99 | 383 | 381 | 7.72 (s, 1H), 7.44 (ddd, *J* = 45.8, 19.3, 2.0 Hz, 6H), 7.09 (d, *J* = 8.8 Hz, 2H), 3.84 (s, 3H), 3.82 (s, 3H), 3.65 (s, 2H). |
| **76** | 377,41 | solide | 92 | | 376 | 7.93 (t, *J* = 9.4 Hz, 2H), 7.82 (t, *J* = 8.3 Hz, 2H), 7.59 (d, *J* = 4.7 Hz, 1H), 7.48 - 7.37 (m, 2H), 7.07 - 6.90 (m, 2H), 3.87 (s, 3H), 3.70 (s, 2H). |
| **77** | 370,94 | solide | 99 | 371 | 369 | 8.04 - 7.85 (m, 2H), 7.71 (s, 1H), 7.58 - 7.30 (m, 4H), 7.08 (dd, *J =* 8.8, 3.2 Hz, 2H), 3.82 (s, 3H), 3.64 (s, 2H). |
| **78** | 352,08 | solide | 99 | 353 | 351 | 12.58 (s, 1H), 7.88 - 7.81 (m, 2H), 7.70 (s, 2H), 7.61 (d, *J* = 7.6 Hz, 2H), 7.49 (d, *J =* 8.8 Hz, 2H), 7.09 (d, *J* = 8.8 Hz, 2H), 3.82 (s, 3H), 3.65 (s, 2H). |
| **79** | 366,09 | solide | 95,1 | 367 | 365 | 12.64 (s, 1H), 8.07 (s, 1H), 7.44 (d, *J* = 8.7 Hz, 2H), 7.38-7.20 (m, 5H), 7.06 (d, *J* = 8.8 Hz, 2H), 4.27 (s, 2H), 3.80 (s, 3H), 3.62 (s, 2H). |
| **80** | 380,11 | huile | 99 | 381 | 379 | 12.62 (s, 1H), 7.93 (s, 1H), 7.44 (d, *J* = 8.7 Hz, 2H), 7.29 (d, *J* = 4.3 Hz, 4H), 7.19 (dq, *J* = 8.7, 4.2 Hz, 1H), 7.06 (d, *J* = 8.8 Hz, 2H), 3.80 (s, 3H), 3.59 (s, 2H), 3.28 (s, 2H), 2.94 (s, 4H). |
| **81** | 421,29 | solide | 97 | 421 | | 12.61 (s, 1H), 7.52 (m, 6H), 7.33 (s, 1H), 6.99 (d, J = 8.8 Hz, 2H), 3.80 (s, 3H), 3.59 (s, 2H). |
| **154** | 356,82 | solide gris | 97 | 357 | | 12.63 (s, 1H), 7.93 - 7.80 (m, 2H), 7.74 (s, 2H), 7.67 - 7.51 (m, 6H), 3.68 (s, 2H). |
| **156** | 421,29 | solide | 99 | 421 | | 7.57 (q, J = 9.0 Hz, 5H), 7.47 (d, J = 2.6 Hz, 2H), 7.25 (d, J = 9.0 Hz, 1H), 3.77 (s, 3H), 3.62 (s, 2H) |
| **159** | 381,22 | solide | 97 | 381 | | 12.70 (s, 1H), 8.18 (d, J = 8.4 Hz, 2H), 7.90 - 7.74 (m, 2H), 7.58 (dd, J = 17.4, 5.9 Hz, 2H), 6.85 (d, J = 5.2 Hz, 1H), 3.73 (s, 2H) |

### Exemple 4 : Préparation du dérivé N°82 : 2-(2-(4-chlorophényl)-5-(furan-2-carbonyl)thiophén-3-yl)acétate de sodium

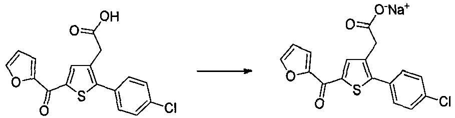

0,21 g (0,606 mmol) d'acide 2-(2-(4-chlorophényl)-5-(furan-2-carbonyl)thiophén-3-yl)acétique ont été solubilisés dans 3 ml de méthanol, à cette solution ont été ajoutés sous agitation magnétique 0,112 ml (0,606 mmol) d'une solution de méthoxyde de sodium à 30% en masse. Le mélange obtenu a été agité à t.a. pendant 1 h. Le mélange a été traité avec 10 ml d'eau et le méthanol a été évaporé sous vide. La phase aqueuse restante a été lyophilisée pour donner 0,222 g (rendement = 97%) de 2-(2-(4-chlorophényl)-5-(furan-2-carbonyl)thiophén-3-yl)acétate de sodium sous forme d'un solide jaune. LC-MS: m/z = 347 (MH⁺) pureté UV à 254 nm = 98%. RMN ¹H (300 MHz, DMSO) δ 8.13 (dd, *J* = 3.4, 2.4 Hz, 2H), 7.89 - 7.74 (m, 2H), 7.58 - 7.47 (m, 3H), 6.81 (dd, *J* = 3.6, 1.7 Hz, 1H), 3.30 (s, 2H).

### Exemple 5 : Préparation du dérivé N°83 : 2-(2-(4-chlorophényl)-5-(furan-2-carbonyl)thiophén-3-yl)acétate de tert-butyle

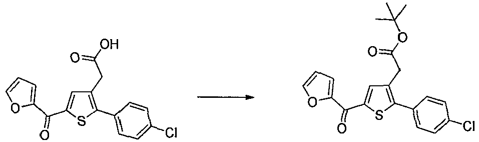

280 mg (0,783 mmol) d'acide 2-(2-(4-chlorophényl)-5-(furan-2-carbonyl) thiophén-3-yl)acétique ont été solubilisés dans 5 ml de Dichlorométhane, à cette solution ont été ajoutés sous agitation magnétique, 0,205 ml (2,35 mmol) de chlorure d'oxalyle et une goutte de diméthylformamide. Le mélange a été agité à t.a. pendant 2 h avant d'être concentré sous vide. Ce produit a été additionné goute à goute sous agitation magnétique à une solution à 5°C de 0,225 ml (2,349 mmol) tert-butanol dans 5 ml de dichlorométhane. Le mélange a été maintenu 15 min à 5°C puis le bain a été retiré et le mélange a été agité à t.a. pendant 40 h. Le milieu réactionnel a été versé dans 30 ml d'eau, la phase aqueuse a été extraite avec 3 x 20 ml de Dichlorométhane. Les phases organiques combinées ont été lavées avec 30 ml d'une solution aqueuse saturée en NaHCO₃, 30 ml d'eau, 30 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient d'heptane / acétate d'éthyle , 95% à 90% d'heptane, v/v). 0,2 g (rendement = 62%) de 2-(2-(4-chlorophényl)-5-(furan-2-carbonyl) thiophén-3-yl)acétate de *tert*-butyle ont été obtenu sous forme d'une huile jaune claire. LC-MS: m/z = 403 (MH⁺) pureté UV à 254 nm = 97%. 1H NMR (300 MHz, DMSO) δ 8.29 - 7.99 (m, 2H), 7.77 - 7.40 (m, 5H), 6.84 (dd, J = 3.6, 1.7 Hz, 1H), 3.72 (s, 2H), 1.35 (s, 9H).

Le dérivé **84** a été préparé selon le même protocole :

| **N°** | **Poids moléculaire g/mol** | **Apparence** | **Pureté LCMS UV à 254 nm** | **Spectrométrie de masse m/z** | | **RMN ¹H (300 MHz, DMSO) δ** |
|---|---|---|---|---|---|---|
| | | | | **MH⁺** | **M-H⁺** | |
| **84** | 442,85 | solide jaune clair | 97 | 443 | | 7.88 (d, *J* = 8.8 Hz, 2H), 7.70 (s, 1H), 7.65 - 7.49 (m, 4H), 7.12 (d, *J* = 8.9 Hz, 2H), 3.87 (s, 3H), 3.69 (s, 2H), 1.33 (s, 9H). |

### Exemple 6: Préparation du dérivé numéro 85: 2-(5-(3-méthoxybenzoyl)-2-(4-méthoxyphényl)thiophén-3-yl)acétate de 1,2-3,4-Di-O-isopropylidène-α-D-galactopyranose

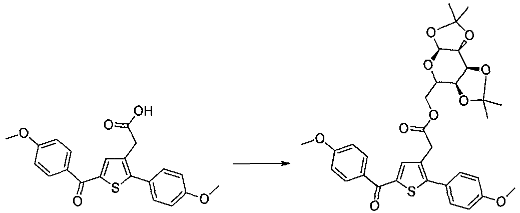

0,314 g (0,812 mmol) d'acide 2-(5-(4-méthoxybenzoyl)-2-(4-méthoxyphényl)thiophén-3-yl)acétique ont été solubilisés dans 4 ml de Dichlorométhane, sous agitation magnétique 0,145 g (0,894 mmol) de carbonyle diimidazole ont été ajoutés, le mélange a été agité à t.a. pendant 2 h. Une solution de 211 mg (0,812 mmol) de 1,2-3,4-Di-O-isopropylidène-α-D-galactopyranose dans 4 ml de dichlorométhane a été ajoutée sous agitation magnétique et le mélange a été agité à t.a. pendant 16 h. Le milieu réactionnel a été concentré sous vide et le résidu brut a été directement purifié par chromatographie flash sur cartouche de gel de silice (éluant : heptane / acétate d'éthyle , 9/1, v/v). 386 mg (rendement = 75%) de 2-(5-(3-méthoxybenzoyl)-2-(4-méthoxyphényl)thiophén-3-yl)acétate de 1,2-3,4-Di-O-isopropylidène-α-D-galactopyranose ont été obtenus sous forme d'un solide jaune clair. LC-MS: m/z = 625 (MH⁺), pureté UV à 254 nm = >99%. RMN ¹H (300 MHz, DMSO) δ 1H NMR (300 MHz, DMSO) δ 7.71 (s, 26H), 7.60 - 7.37 (m, 104H), 7.37 - 7.21 (m, 52H), 7.16 - 7.03 (m, 51H), 5.41 (d, J = 5.0 Hz, 25H), 4.58 (dd, J = 7.9, 2.4 Hz, 26H), 4.35 (dd, J = 5.0, 2.4 Hz, 26H), 4.22 - 3.99 (m, 80H), 3.89 (d, J = 2.8 Hz, 19H), 3.85 (t, J = 7.9 Hz, 160H), 3.75 (s, 50H), 1.99 (s, 3H), 1.33 (s, 78H), 1.29 (s, 79H), 1.25 (d, J = 4.6 Hz, 160H), 1.17 (s, 6H), 0.84 (d, J = 6.6 Hz, 5H).

### Exemple 7: Préparation du dérivé numéro 86: 2-(2-(4-chlorophényl)-5-(4-méthoxybenzoyl)thiophén-3-yl)acétamide

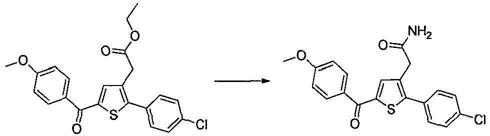

0,25 g (0,603 mmol) de 2-(2-(4-chlorophényl)-5-(4-méthoxybenzoyl)thiophén-3-yl)acétate d'éthyle ont été suspendus dans 2,15 ml (15,06 mmol) une solution à 7 M de méthanol ammoniacal. Le milieu réactionnel a été agité à t.a. sous agitation magnétique pendant 5 j avant d'être versé dans 25 ml d'eau. Une agitation magnétique pendant 10 min a permis de faire précipiter un solide qui a été isolé par filtration, lavé avec 2 x 5 ml d'eau et recristallisé dans l'éthanol. 0,137 g (rendement = 58%) de 2-(2-(4-chlorophényl)-5-(4-méthoxybenzoyl)thiophén-3-yl) acétamide ont été obtenus sous forme d'un solide blanc. LC-MS: m/z = 386 (MH⁺), pureté UV à 254 nm = 98%. RMN ¹H (300 MHz, DMSO) δ 7.88 (d, J = 8.8 Hz, 2H), 7.75 (s, 1H), 7.72 - 7.52 (m, 5H), 7.13 (d, J = 8.9 Hz, 2H), 7.05 (s, 1H), 3.88 (s, 3H), 3.47 (s, 2H).

### Exemple 8: Préparation du dérivé N°87: 2-(2-(5-(4-méthoxybenzoyl)-2-phénylthiophén-3-yl)-N-méthylacétamido) acétate d'éthyle

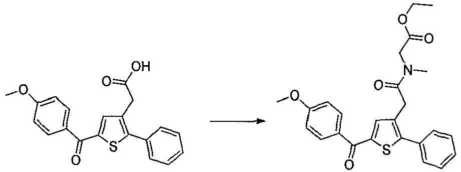

0,091 g (0,593 mmol) d'éthyle ester de sarcosine hydrochlorate ont été solubilisés dans 3 ml de tétrahydrofurane. A cette solution, ont été additionnés sous agitation magnétique, 0,149 g (1,079 mmol) de carbonate de potassium et 0,2 g (0,593 mmol) d'acide 2-(5-(4-méthoxybenzoyl)-2-phénylthiophén-3-yl)acétique. Le mélange a été agité à t.a. pendant 16 h avant d'être versé dans 20 ml d'eau. La phase aqueuse a été extraite avec 2 x 20 ml de Dichlorométhane. Les phases organiques combinées ont été séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de Dichlorométhane / acétate d'éthyle, 100% à 75% de Dichlorométhane, v/v). 0,133 g (rendement = 54%) de 2-(2-(5-(4-méthoxybenzoyl)-2-phénylthiophén-3-yl)-N-méthylacétamido)acétate d'éthyle ont été obtenus sous forme d'une huile marron claire. LC-MS: m/z = 452 (MH⁺), pureté UV à 254 nm = 98%. RMN ¹H (300 MHz, DMSO) δ 7.88 (d, J = 8.8 Hz, 2H), 7.66 - 7.39 (m, 6H), 7.12 (d, J = 8.8 Hz, 2H), 4.15 (d, J = 40.5 Hz, 4H), 3.87 (s, 3H), 3.74 (d, J = 35.2 Hz, 2H), 2.92 (d, J = 51.8 Hz, 3H), 1.15 (dd, J = 13.1, 6.0 Hz, 3H).

### Exemple 9 : Préparation du dérivé N°88: acide 2-(2-(5-(4-méthoxybenzoyl)-2-phénylthiophén-3-yl)-N-méthylacétamido) acétique

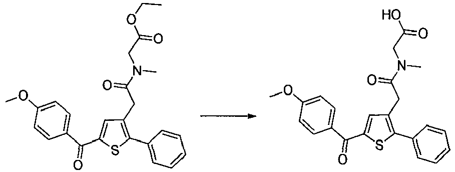

0,099 g (0,203 mmol) de 2-(2-(5-(4-méthoxybenzoyl)-2-phénylthiophén-3-yl)-N-méthylacétamido)acétate d'éthyle ont été solubilisés dans 3 ml d'éthanol, à cette solution ont été ajoutés sous agitation magnétique 0,02 ml (0,203 mmol) d'une solution aqueuse d'hydroxyde de sodium à 30% en masse. Le mélange obtenu a été agité à t.a. pendant 16 h. Le mélange a été concentré sous vide, le résidu obtenu a été repris dans 5 ml d'eau, la phase aqueuse a été extraite avec 5 ml d'acétate d'éthyle. Le pH de la phase aqueuse a ensuite été abaissé par addition d'une solution d'acide chlorhydrique 1N jusqu'à apparition d'un précipité. Le solide a été isolé par filtration, lavé avec 2 x 3 ml d'eau et séché sous cloche à vide pour donner 0,01 g (rendement = 12%) d'acide 2-(2-(5-(4-méthoxybenzoyl)-2-phénylthiophén-3-yl)-N-méthylacétamido)acétique sous forme d'un solide blanc. LC-MS: m/z = 424 (MH⁺), pureté UV à 254 nm = 99%. RMN ¹H (300 MHz, DMSO) δ 12.85 (s, 1H), 7.89 (d, J = 8.8 Hz, 2H), 7.70 - 7.42 (m, 6H), 7.18 - 7.08 (m, 2H), 4.04 (d, J = 21.1 Hz, 2H), 3.87 (s, 3H), 3.73 (d, J = 34.0 Hz, 2H), 2.91 (d, J = 49.4 Hz, 3H).

### Exemple 10 : Préparation du dérivé N°89 : 2-(2-(4-chlorophényl)-5-(4-méthoxybenzoyl)thiophén-3-yl)-N-(2-(diméthylamino) éthyl)acétamide

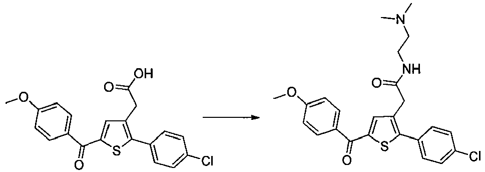

0,1 g (0,258 mmol) d'acide 2-(2-(4-chlorophényl)-5-(4-méthoxy benzoyl) thiophén-3-yl)acétique ont été solubilisés dans 2 ml de Dichlorométhane. A cette solution, ont été ajoutés sous agitation magnétique, 0,059 g (0,310 mmol) d'EDC, 0,047 g (0,310 mmol) de HOBt et 0,108 ml (0,775 mmol) de triéthylamine. Le mélange a été agité à t.a. pendant 15 min puis 0,034 ml (0,310 mmol) de N,N-diméthyléthane-1,2-diamine ont été ajoutés et le mélange a été agité à t.a. pendant 16 h. 20 ml de Dichlorométhane ont été ajoutés au milieu réactionnel. Le mélange a été lavé avec 2 x 20 ml d'eau, la phase organique a été séchée sur Na₂SO₄ qui a ensuite été éliminé par filtration et le filtrat obtenu a été concentré sous vide. Le résidu brut a été purifié par chromatographie flash sur cartouche de gel de silice (éluant : gradient de Dichlorométhane / méthanol, 100% à 90% de Dichlorométhane, v/v). 49 mg (rendement = 39%) de 2-(2-(4-chlorophényl)-5-(4-méthoxybenzoyl)thiophén-3-yl)-N-(2-(diméthylamino) éthyl)acétamide ont été obtenus sous forme d'un solide blanc. LC-MS: m/z = 457 (MH⁺), pureté UV à 254 nm = 94%. RMN ¹H (300 MHz, DMSO) δ 8.06 (t, J = 5.5 Hz, 1H), 7.87 (d, J = 8.8 Hz, 2H), 7.78 - 7.65 (m, 3H), 7.58 (d, J = 8.5 Hz, 2H), 7.13 (d, J = 8.9 Hz, 2H), 3.88 (s, 3H), 3.49 (s, 2H), 3.14 (dd, J = 12.3, 6.4 Hz, 2H), 2.25 (t, J = 6.6 Hz, 2H), 2.11 (s, 6H).

Les dérivés **160** à **166** ont été préparés selon le même protocole :

| **N°** | **Poids moléculaire** | **Apparence** | **Pureté LCMS UV à 254 nm** | **Spectrométrie de masse m/z** | | **RMN ¹H** |
|---|---|---|---|---|---|---|
| | **g/mol** | | | **MH⁺** | **M-H⁺** | **(300 MHz, DMSO) δ** |
| **160** | 462,95 | Solide jaune | 94,4 | 463 | 461 | 10.41 (s, 1H), 8.71 (d, J = 2.5 Hz, 1H), 8.27 (dd, J = 4.6, 1.2 Hz, 1H), 8.07 - 7.96 (m, 1H), 7.94 - 7.78 (m, 3H), 7.64 (dd, J = 19.5, 8.5 Hz, 4H), 7.34 (dd, J = 8.3, 4.7 Hz, 1H), 7.13 (d, J = 8.7 Hz, 2H), 3.88 (s, 3H), 3.80 (s, 2H). |
| **161** | 422,88 | solide gris | 98,9 | 423 | 421 | 10.44 (s, 1H), 8.72 (d, J = 2.3 Hz, 1H), 8.26 (dd, J = 4.0, 2.1 Hz, 2H), 8.14 (dd, J = 1.7, 0.7 Hz, 1H), 8.02 (ddd, J = 8.4, 2.5, 1.5 Hz, 1H), 7.74 - 7.51 (m, 5H), 7.34 (dd, J = 8.2, 4.6 Hz, 1H), 6.84 (dd, J = 3.6, 1.7 Hz, 1H), 3.82 (s, 2H). |
| **162** | 491,43 | solide | 99 | 491 | | 8.09 (s, 1H), 7.71 (d, J = 8.5 Hz, 2H), 7.57 (d, J = 8.4 Hz, 3H), 7.51 - 7.38 (m, 2H), 7.25 (d, J = 9.0 Hz, 1H), 3.77 (s, 3H), 3.11 (d, J = 5.9 Hz, 2H), 2.20 (t, J = 6.5 Hz, 2H), 2.09 (s, 6H). |
| **163** | 497,39 | solide | 97 | 497 | 495 | 10.40 (s, 1H), 8.68 (s, 1H), 8.26 (d, J = 3.8 Hz, 1H), 7.98 (d, J = 8.4 Hz, 1H), 7.63 (dd, J = 20.0, 7.9 Hz, 6H), 7.49 (d, J = 2.6 Hz, 1H), 7.33 (d, J = 12.9 Hz, 1H), 7.24 (d, J = 8.9 Hz, 1H), 3.73 (s, 5H) |
| **164** | 503,44 | solide | 99 | 503 | | (in CDCl3) δ 7.43 - 7.26 (m, 7H), 6.87 (d, J = 8.8 Hz, 1H), 3.74 (s, 3H), 3.55 (d, J = 8.2 Hz, 4H), 3.26 - 3.16 (m, 2H), 2.31 - 2.23 (m, 2H), 2.21 (s, 3H), 2.18 - 2.09 (m, 2H) |
| **165** | 464,36 | solide | 99 | 464 | | 8.32 - 7.57 (m, 7H), 7.13 (d, J = 7.9 Hz, 2H), 4.72 (s, 1H), 3.88 (s, 3H), 3.51 (s, 2H), 3.14 (s, 2H) |
| **166** | 491,43 | solide | 91 | 491 | | 8.13 (s, 1H), 8.03 (d, J = 1.9 Hz, 1H), 7.88 (d, J = 8.7 Hz, 2H), 7.82 - 7.72 (m, 2H), 7.66 (d, J = 6.4 Hz, 1H), 7.13 (d, J = 8.7 Hz, 2H), 3.88 (s, 3H), 3.51 (s, 2H), 3.14 (dd, J = 12.3, 6.3 Hz, 2H), 2.24 (t, J = 6.6 Hz, 2H), 2.10 (s, 6H) |

### Exemple 11 : Préparation du dérivé N°90: 2-(5-(4-méthoxybenzoyl)-2-phénylthiophén-3-yl)-1-(pyrrolidin-1-yl) éthanone

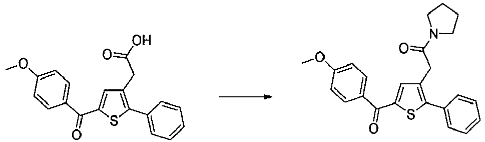

0,095 g (0,270mmol) d'acide 2-(5-(4-méthoxybenzoyl)-2-phénylthiophén-3-yl)acétique ont été solubilisés dans 3 ml de Dichlorométhane, à cette solution ont été ajoutés sous agitation magnétique, 0,047 ml (0,540mmol) de chlorure d'oxalyle et une goutte de diméthylformamide. Le mélange a été agité à t.a. pendant 2 h avant d'être concentré sous vide. Ce produit a été solubilisé dans 2 ml de tétrahydrofurane pour être ensuite additionné goute à goute sous agitation magnétique à une solution de 0,049 ml (0,593mmol) de pyrrolidine dans 2 ml de tétrahydrofurane. Le mélange a été agité à t.a. pendant 16 h. Le milieu réactionnel a été versé dans 20 ml d'eau, la phase aqueuse a été extraite avec 2 x 10 ml de Dichlorométhane. Les phases organiques combinées ont été séchées sur Na₂SO₄ qui a ensuite été éliminé par filtration et le filtrat obtenu a été concentré sous vide. Le résidu brut a été purifié par chromatographie flash sur cartouche de gel de silice (éluant : Dichlorométhane / acétate d'éthyle, 3/1, v/v). 0,077 g (rendement = 65%) de 2-(5-(4-méthoxybenzoyl)-2-phénylthiophén-3-yl)-1-(pyrrolidin-1-yl)éthanone ont été obtenu sous forme d'une huile marron claire. LC-MS: m/z = 406 (MH⁺), pureté UV à 254 nm = 93%. RMN ¹H (300 MHz, DMSO) δ 7.87 (d, J = 8.8 Hz, 2H), 7.64 (s, 1H), 7.57 - 7.42 (m, 5H), 7.13 (d, J = 8.9 Hz, 2H), 3.87 (s, 3H), 3.68 (s, 2H), 3.46 - 3.23 (m, 4H), 1.91 - 1.68 (m, 4H).

Les dérivés **91** à **100** ont été préparés selon le même protocole :

| **N°** | **Poids moléculaire** | **Apparence** | **Pureté LCMS UV à 254 nm** | **Spectrométrie de masse m/z** | | **¹H RMN ¹H** |
|---|---|---|---|---|---|---|
| | **g/mol** | | | **MH⁺** | **M-H⁺** | **(300 MHz, DMSO) δ** |
| **91** | 415,89 | huile orangée | 96,9 | 416 | 415 | 8.17 - 8.11 (m, 1H), 8.08 (s, 1H), 7.63 - 7.51 (m, 5H), 6.84 (dd, J = 3.6, 1.7 Hz, 1H), 3.80 (s, 2H), 3.55 (s, 4H), 3.47 (d, J = 5.0 Hz, 4H). |
| **92** | 455,95 | huile jaune pale | 97 | 456 | | 7.96 - 7.77 (m, 2H), 7.72 - 7.46 (m, 5H), 7.25 - 7.03 (m, 2H), 3.87 (s, 3H), 3.76 (s, 2H), 3.52 (s, 4H), 3.44 (d, J = 4.9 Hz, 4H). |
| **93** | 468,99 | huile incolore | >99 | 469 | | 7.87 (d, J = 8.8 Hz, 2H), 7.65 - 7.48 (m, 5H), 7.12 (d, J = 8.8 Hz, 2H), 3.87 (s, 3H), 3.75 (s, 2H), 3.39 (d, J = 17.5 Hz, 4H), 2.19 (d, J = 12.5 Hz, 4H), 2.16 (s, 3H). |
| **94** | 485,04 | solide blanc | >99 | 485 | | 7.96 (t, J = 5.5 Hz, 1H), 7.92 - 7.82 (m, 2H), 7.77 - 7.64 (m, 3H), 7.62 - 7.48 (m, 2H), 7.24 - 7.02 (m, 2H), 3.88 (s, 3H), 3.49 (s, 2H), 3.10 (dd, J = 13.1, 6.3 Hz, 2H), 2.48 - 2.29 (m, 6H), 0.89 (t, J = 7.1 Hz, 6H). |
| **95** | 416,92 | solide jaune | >99 | 417 | | 8.24 - 8.12 (m, 2H), 8.08 (s, 1H), 7.69 (d, J = 8.6 Hz, 2H), 7.64 - 7.47 (m, 3H), 6.84 (dd, J = 3.6, 1.7 Hz, 1H), 3.53 (s, 2H), 3.17 (dd, J = 12.3, 6.4 Hz, 2H), 2.29 (t, J = 6.5 Hz, 2H), 2.15 (s, 6H). |
| **96** | 429,92 | solide jaune | 96,2 | 430 | | 8.18 (t, *J* = 5.5 Hz, 1H), 7.88 (d, *J* = 8.8 Hz, 2H), 7.80 - 7.65 (m, 3H), 7.58 (d, *J* = 8.6 Hz, 2H), 7.13 (d, *J* = 8.9 Hz, 2H), 4.70 (t, *J* = 5.3 Hz, 1H), 3.88 (s, 3H), 3.50 (s, 2H), 3.45 - 3.36 (m, 2H), 3.13 (q, *J* = 5.8 Hz, 2H). |
| **97** | 413,92 | solide blanc | 97,2 | 414 | | 8.12 (s, 1H), 7.87 (d, J = 8.7 Hz, 2H), 7.78 - 7.48 (m, 5H), 7.13 (d, J = 8.8 Hz, 2H), 3.88 (s, 3H), 3.47 (s, 2H), 3.07 (dd, J = 7.1, 5.7 Hz, 2H), 1.00 (t, J = 7.2 Hz, 3H). |
| **98** | 389,85 | solide beige | 97 | 390 | 388 | 11.21 (s, 1H), 8.26 - 8.07 (m, 2H), 7.76 - 7.49 (m, 5H), 6.85 (dd, J = 3.6, 1.7 Hz, 1H), 3.80 (q, J = 7.0 Hz, 2H), 3.44 (s, 2H), 1.14 (t, J = 7.0 Hz, 3H). |
| **99** | 361,8 | solide blanc | >99 | | 360 | 10.73 (s, 1H), 8.93 (s, 1H), 8.22 - 8.08 (m, 2H), 7.72 (d, *J* = 8.5 Hz, 2H), 7.57 (dd, *J* = 8.4, 6.0 Hz, 4H), 6.85 (dd, *J* = 3.6, 1.6 Hz, 1H), 3.41 (s, 2H). |
| **100** | 401,86 | solide jaune | >99 | 402 | 400 | 10.71 (s, 1H), 8.91 (s, 1H), 7.87 (d, *J* = 8.8 Hz, 2H), 7.71 (d, *J* = 7.0 Hz, 3H), 7.63 - 7.54 (m, 2H), 7.13 (d, *J* = 8.8 Hz, 2H), 3.88 (s, 3H), 3.38 (s, 2H). |

### Exemple 12 : Préparation du dérivé N°101: 2-(5-(hydroxy (phényl)méthyl)-2-(4-méthoxyphényl)thiophén-3-yl)acétate d'éthyle

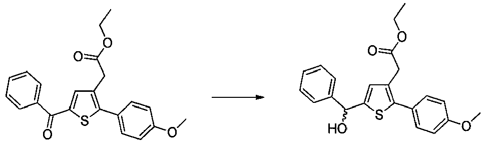

2,8 g (7,36 mmol) de 2-(5-benzoyle-2-(4-méthoxyphényl)thiophén-3-yl) acétate d'éthyle ont été solubilisés dans 50 ml d'éthanol. Sous agitation magnétique, ce mélange a été placé dans un bain à 0°C, puis 0,557 g (14,72 mmol) de borohydrure de sodium ont été ajoutés. Le mélange a été maintenu 15 min à 0°C puis le bain a été retiré et le mélange a été agité à t.a. pendant 72 h. Le milieu réactionnel a été versé dans 400 ml d'un mélange composé d'eau et de glace, la phase aqueuse a été extraite avec 2 x 200 ml d'acétate d'éthyle. Les phases organiques combinées ont été lavées avec 2 x 50 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de Dichlorométhane / acétone, 100% à 95% de Dichlorométhane, v/v). 1,05 g (rendement = 37%) de 2-(5-(hydroxy(phényle)méthyl)-2-(4-méthoxy phényl)thiophén-3-yl)acétate d'éthyle ont été obtenu sous forme d'une huile jaune claire. LC-MS: m/z = non ionisé. ¹H NMR (300 MHz, DMSO) δ 7.40(d,2H),7.20-7.35 (m, 5H), 6.80 (d, 2H), 6.75 (s, 1H), 5.92 (s,1H), 4.07 (q, 2H), 3.75 (s, 3H), 3.44 (s, 2H), 2.45 (s,1H), 1.15(t,3H).

### Exemple 13 : Préparation du dérivé numéro 102: 2-(2-(4-chlorophényl)-5-((ethoxyimino)(4-méthoxyphényl)méthyl) thiophén-3-yl)acétate d'éthyle

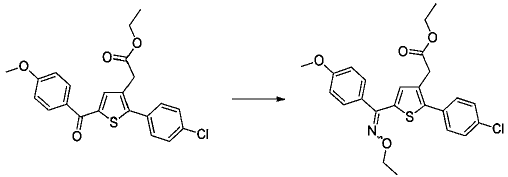

0,3 g (0,701 mmol) de 2-(2-(4-chlorophényl)-5-(4-méthoxybenzoyl) thiophén-3-yl)acétate d'éthyle ont été solubilisés dans 1,5 ml d'éthanol, sous agitation magnétique. A cette solution, 0,342 g (3,51 mmol) de chlorure de O-éthylhydroxylammonium et 0,187 ml (2,315 mmol) de pyridine ont ensuite été ajoutés. Le mélange a été agité à 50°C pendant 24 h. Après retour à t.a., le mélange a été versé dans 5 ml d'eau. La phase aqueuse a été extraite avec 2 x 5 ml d'acétate d'éthyle. Les phases organiques combinées ont été lavées avec 5 ml d'une solution aqueuse saturée en NaHCO₃, 5 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : Heptane / acétate d'éthyle, 9/1, v/v). 0,242 g (rendement = 73%) de 2-(2-(4-chlorophényl)-5-((éthoxyimino)(4-méthoxyphényl)méthyl)thiophén-3-yl) acétate d'éthyle ont été obtenu sous forme d'une huile incolore. LC-MS: m/z = 458 (MH⁺) ; pureté UV à 254 nm = 97%. 1H NMR (300 MHz, DMSO) δ 7.62 - 7.31 (m, 6H), 7.12 (s, 1H), 7.10 - 6.98 (m, 2H), 4.30 (q, J = 7.0 Hz, 1,6H), 4.11 (q, J = 7.0 Hz, 0,4H), 4.01 (p, J = 7.1 Hz, 2H), 3.82 (s, 3H), 3.64 (d, J = 12.6 Hz, 2H), 1.33 (t, J = 7.0 Hz, 2,4H), 1.26 - 1.15 (m, 0,6H), 1.09 (t, J = 7.1 Hz, 3H).

Les dérivés **103** à **107** ont été préparés selon le même protocole :

| **N°** | **Poids moléculaire** | **Apparence** | **Pureté LCMS UV à 254 nm** | **Spectrométrie de masse m/z** | | **RMN ¹H** |
|---|---|---|---|---|---|---|
| | **g/mol** | | | **MH⁺** | **M-H⁺** | **(300 MHz, DMSO) δ** |
| **103** | 510,86 | solide blanc | 92 | 512 | | 7.78 (dd, J = 5.1, 3.1 Hz, 2H), 7.65 - 7.37 (m, 5H), 7.10 (s, 1H), 4.80 (dt, J = 12.5, 6.3 Hz, 1H), 4.34 (q, J = 7.0 Hz, 2H), 3.64 (s, 2H), 1.34 (t, J = 7.0 Hz, 3H), 1.08 (d, J = 6.3 Hz, 6H). |
| **104** | 429,92 | solide jaune pâle | 97 | 430 | | 12.29 (s, 1H), 7.60 - 7.30 (m, 6H), 7.12 - 6.96 (m, 3H), 4.07 - 3.93 (m, 2H), 3.81 (s, 3H), 3.65 (s, 2H), 1.09 (t, J = 7.1 Hz, 3H). |
| **105** | 472 | solide | 99 | 472 | | 12.19 (s, 1H), 11,28 (d, J = 6.3 Hz, 1H), 7.96 (t, J = 5.5 Hz, 1H), 7.65 (ddd, J = 8.5, 4.4, 2.3 Hz, 2H), 7.57 - 7.48 (m, 2H), 7.46 - 7.31 (m, 2H), 7.07 - 6.97 (m, 2H), 3.82 (s, 3H), 3.39 (s, 2H), 3.09 (dd, J = 12.5, 6.5 Hz, 2H), 2.19 (t, J = 6.7 Hz, 2H), 2.09 (t, J = 3.2 Hz, 6H) |
| **106** | 443,94 | huile jaune | 97 | 444 | | 7.57 - 7.32 (m, 5H), 7.14 (s, 1H), 6.96 (ddt, J = 7.7, 4.9, 2.4 Hz, 3H), 4.20 - 4.03 (m, 2H), 3.90 (d, J = 25.8 Hz, 3H), 3.53 (d, J = 12.5 Hz, 2H), 1.31 - 1.12 (m, 3H). |
| **107** | 441,97 | huile | 98 | 442 | | 7.50 (q, J = 8.6 Hz, 4H), 7.38 (d, J = 7.2 Hz, 6H), 5.15 (s, 2H), 4.93 - 4.79 (m, 1H), 3.63 (s, 2H), 2.22 (s, 3H), 1.14 (d, J = 6.3 Hz, 6H). |

### Exemple 14 : Préparation du dérivé N°108 : acide 2-(2-(4-chloro phényl)-5-((éthoxyimino)(4-méthoxyphényl)méthyl) thiophén-3-yl)acétique

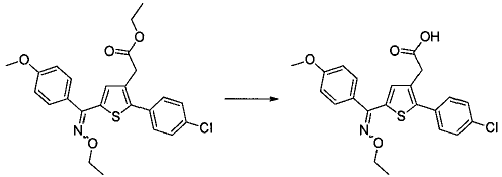

0,238 g (0,505 mmol) de 2-(2-(4-chlorophényl)-5-((éthoxyimino)(4-méthoxyphényl)méthyl)thiophén-3-yl)acétate d'éthyle ont été solubilisés sous agitation magnétique dans 1,1 ml d'un mélange méthanol-tétrahydrofurane (1/1, v/v), 0,545 ml (0,545 mmol) d'une solution aqueuse d'hydroxyde de sodium 1 M ont été ajoutés. Le mélange a été agité à t.a. pendant 16 h. Le mélange a été neutralisé par addition d'une solution aqueuse d'acide chlorhydrique 1N jusqu'à apparition d'un précipité. Le mélange a été concentré sous vide, le résidu obtenu a été repris dans l'isopropanol, les sels inorganiques ont été éliminés par filtration, le filtrat obtenu a été concentré sous vide avant d'être repris dans 2 ml d'eau et agité sous agitation magnétique à 80°C pendant 1 h. Le mélange a été concentré sous vide pour donner 0,191 g (rendement = 82%) d'acide 2-(2-(4-chlorophényl)-5-((éthoxyimino)(4-méthoxyphényl)méthyl)thiophén-3-yl) acétique sous forme d'un solide blanc. LC-MS: m/z = 430 (MH⁺) ; pureté UV à 254 nm = 97%. RMN ¹H (300 MHz, DMSO) (un mélange d'isomères Z/E est observé) δ 7.65 - 7.29 (m, 6H), 7.16 - 6.97 (m, 3H), 4.30 (q, J = 7.0 Hz, 1,5H), 4.17 - 4.04 (m, 0,5H), 3.82 (s, 3H), 3.53 (d, J = 13.9 Hz, 2H), 1.40 - 1.29 (m, 1,5H), 1.20 (dd, J = 13.0, 6.0 Hz, 0,5H).

Les dérivés **109** à **111** ont été préparés selon le même protocole :

| **N°** | **Poids moléculaire** | **Apparence** | **Puretés LCMS UV à 254 nm** | **Spectrométrie de masse m/z** | | **RMN ¹H** |
|---|---|---|---|---|---|---|
| | **g/mol** | | | **MH⁺** | **M-H⁺** | **(300 MHz, DMSO) δ** |
| **109** | 401,86 | solide blanc | 97 | 402 | | 12.31 (s, 0,7H), 11.34 (s, 0,3H), 7.98 - 7.65 (m, 2H), 7.64 - 7.20 (m, 4H), 7.20 - 6.84 (m, 3H), 3.81 (s, 3H), 3.14 (d, J = 17.0 Hz, 2H). |
| **110** | 415,89 | solide blanc | 97 | 416 | | 7.46 (ddd, J = 38.2, 30.9, 8.0 Hz, 6H), 7.21 - 6.84 (m, 3H), 4.02 (s, 3H), 3.81 (s, 3H), 3.35 (d, J = 15.1 Hz, 2H). |
| **111** | 399,89 | solide | 95 | 400 | | 7.51 (q, J = 8.7 Hz, 4H), 7.44 - 7.25 (m, 6H), 5.16 (s, 2H), 3.56 (s, 2H), 2.22 (s, 3H). |
| **167** | 468,77 | solide blanc | 94,6 | 468 | | 7.76 (dd, J = 5.1, 3.1 Hz, 2H), 7.63 - 7.43 (m, 5H), 7.13 (s, 1H), 4.33 (q, J = 7.0 Hz, 2H), 3.57 (s, 2H), 1.33 (q, J = 7.0 Hz, 3H). |

### Exemple 15: Préparation du dérivé N°112: acide 5-(4-chloro phényl)-4-(2-isopropoxy-2-oxoéthyl)thiophène-2-carboxylique

### Etape 1 : Préparation du 4-chlorophényl-4-oxobutanoate d'isopropyle

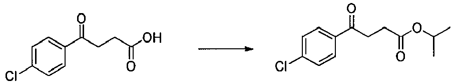

50 g (235 mmol) d'acide 4-chlorophényl-4-oxobutanoique ont été solubilisés dans 300 ml d'isopropanol, 0,63 ml (11,76 mmol) d'acide sulfurique ont été ajoutés à cette solution. Le mélange a été chauffé à reflux pendant 6 j sous agitation magnétique. Après retour à t.a., le mélange a été concentré sous vide, le résidu brut a été directement purifié par chromatographie flash sur cartouche de gel de silice (éluant : 100% Dichlorométhane). 59,83 g (rendement = >99%) de 4-chlorophényl-4-oxobutanoate d'isopropyle ont été obtenus sous forme d'une huile incolore. LC-MS: m/z = 255 (MH⁺) pureté UV à 254 nm = 95%. RMN ¹H (300 MHz, DMSO) δ 8.13 - 7.85 (m, 2H), 7.70 - 7.47 (m, 2H), 4.86 (dt, *J* = 12.5, 6.3 Hz, 1H), 3.29 - 3.22 (m, 2H), 2.65 - 2.54 (m, 2H), 1.16 (d, *J* = 6.3 Hz, 6H).

### Etape 2 : Préparation du (Z/E)-4-chloro-3-formyl-4-(4-chlorophényl)but 3-énoate d'isopropyle

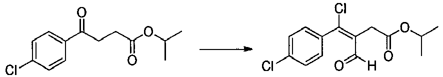

59,8 g (235 mmol) de 4-chlorophényl-4-oxobutanoate d'isopropyle ont été solubilisés dans 54,5 ml de diméthylformamide (704 mmol), 54,7 ml (587 mmol) de trichlorure de phosphoryle ont été lentement additionnés à cette solution, la réaction étant très exothermique. Le mélange obtenu a ensuite été chauffé à 80°C pendant 2 h sous agitation magnétique. Après retour à t.a., le mélange a été versé sur 1 l d'un mélange composé d'eau et de glace, une agitation magnétique a été mise en place et le mélange a été agité à t.a. pendant 16 h. La phase aqueuse a été extraite avec 300 ml puis 2 x 150 ml d'acétate d'éthyle. Les phases organiques combinées ont été lavées avec 2 x 200 ml d'eau, 300 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de Heptane / Dichlorométhane, de 50 à 100% de Dichlorométhane, v/v). 47,4 g (rendement = 62%) de (Z/E)-4-chloro-3-formyle-4-(4-chlorophényl)but-3-énoate d'isopropyle ont été obtenu sous forme d'une huile orangée. LC-MS: m/z = 301 (MH⁺) pureté UV à 254 nm = 92%. RMN ¹H (300 MHz, DMSO) δ 10.29 - 9.28 (m, 1H), 7.71 - 7.38 (m, 4H), 4.87 (dq, J = 25.0, 6.3 Hz, 1H), 3.53 (s, 19H), 3.18 - 1.16 (m, 2H).

### Etape 3 : Préparation du 2-(2-(4-chlorophényl)thiophén-3-yl)acétate d'isopropyle et de l'acide 5-(4-chlorophényl)-4-(2-isopropoxy-2-oxoéthyl)thiophène-2-carboxylique (dérivé N°112)

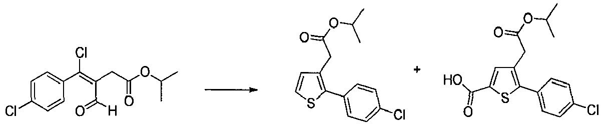

47,4 g (157 mmol) de (Z/E)-4-chloro-3-formyl-4-(4-chlorophényl)but-3-énoate d'isopropyle ont été solubilisés dans 250 ml de tétrahydrofurane, à cette solution ont été ajoutés 16,40 ml (236 mmol) d'acide 2-mercapto acétique et 65,8 ml (472 mmol) de triéthylamine. Le mélange obtenu a été chauffé à reflux pendant 4 h sous agitation magnétique. Après retour à t.a., le mélange a été concentré sous vide. Le résidu a été repris dans 175 ml de diméthylformamide et le mélange a été chauffé à 130°C pendant 2 h sous agitation magnétique. Après retour à t.a., le mélange a été traité avec 500 ml d'eau. La phase aqueuse a été extraite avec 300 ml puis 2 x 150 ml d'acétate d'éthyle. Les phases organiques combinées ont été lavées avec 2 x 200 ml d'eau, 300 ml d'une solution aqueuse saturée en NaCl, puis séchées sur Na₂SO₄ qui a ensuite été éliminé par filtration et le filtrat obtenu a été concentré sous vide. Le résidu brut a été repris et trituré dans l'heptane. Un solide a précipité et a été isolé par filtration pour donner 22,8 g (rendement = 30 %) d'acide 5-(4-chlorophényl)-4-(2-isopropoxy-2-oxoéthyl)thiophène-2-carboxylique. Le filtrat a été concentré sous vide et purifié par chromatographie flash sur cartouche de gel de silice (éluant : heptane / Dichlorométhane, 2/1, v/v). 16,18 g (rendement = 34%) de 2-(2-(4-chlorophényl)thiophén-3-yl)acétate d'isopropyle ont été obtenu sous forme d'une huile marron claire.

Acide 5-(4-chlorophényl)-4-(2-isopropoxy-2-oxoéthyl)thiophène-2-carboxylique : LC-MS: non ionisé. RMN ¹H (300 MHz, DMSO) δ 13.19 (s, 1H), 7.69 (s, 1H), 7.53 (q, *J* = 8.7 Hz, 4H), 4.86 (dt, *J* = 12.5, 6.2 Hz, 1H), 3.69 (s, 2H), 1.13 (d, *J=* 6.3 Hz, 6H).
2-(2-(4-chlorophényl)thiophén-3-yl)acétate d'isopropyle : LC-MS: m/z = 294 (M) pureté UV à 254 nm = 96%. RMN ¹H (300 MHz, DMSO) δ 7.51 (dt, *J* = 19.3, 6.9 Hz, 5H), 7.08 (d, *J* = 5.2 Hz, 1H), 4.87 (dt, *J* = 12.5, 6.3 Hz, 1H), 3.63 (s, 2H), 1.15 (d, *J =* 6.3 Hz, 6H).

Le dérivé numéro **113** a été préparé selon le même enchainement d'étapes 1 à 3

| **N°** | **Poids moléculaire** | **Apparence** | **Pureté LCMS UV à 254 nm** | **Spectrométrie de masse m/z** | | **RMN ¹H** |
|---|---|---|---|---|---|---|
| | **g/mol** | | | **MH⁺** | **M-H⁺** | **(300 MHz, DMSO) δ** |
| **113** | 324,78 | solide | 93 | | 323 | 13.17 (s, 1H), 7.70 (s, 1H), 7.63 -7.45 (m, 4H), 4.05 (q, *J* = 7.1 Hz, 2H), 3.71 (s, 2H), 1.14 (t, *J* = 7.1 Hz, 3H). |

### Exemple 16: Préparation du dérivé N°114: 2-(2-(4-chloro phényl)-5-(morpholine-4-carbonyl)thiophén-3-yl)acétate d'éthyle

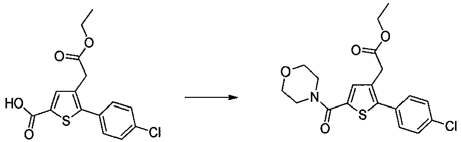

0,44 g (1,244 mmol) d'acide 5-(4-chlorophényl)-4-(2-éthoxy-2-oxoéthyl) thiophène-2-carboxylique ont été solubilisés dans 5 ml de Dichlorométhane, à cette solution ont été ajoutés sous agitation magnétique, 0,327 ml (3,73 mmol) de chlorure d'oxalyle et une goutte de diméthylformamide. Le mélange a été agité à t.a. pendant 2 h avant d'être concentré sous vide. Ce produit a été additionné goute à goute sous agitation magnétique à une solution à une solution de 0,542 ml (6,22 mmol) de morpholine dans 5 ml de Dichlorométhane. Le mélange a été agité à t.a. pendant 16 h. Le milieu réactionnel a été versé dans 20 ml d'eau, la phase aqueuse a été extraite avec 3 x 25 ml de Dichlorométhane. Les phases organiques combinées ont été lavées avec 20 ml d'une solution aqueuse saturée en NaHCO₃, 20 ml d'eau, 20 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de Dichlorométhane / méthanol, 98/2, v/v). 0,359 g (rendement = 68%) 2-(2-(4-chlorophényl)-5-(morpholine-4-carbonyl) thiophén-3-yl)acétate d'éthyle ont été obtenu sous forme d'une huile jaune orangée. LC-MS: m/z = 394 (MH⁺) ; pureté UV à 254 nm = 98%. RMN ¹H (300 MHz, DMSO) δ 7.63 - 7.44 (m, 4H), 7.41 (s, 1H), 4.16 - 3.95 (m, 2H), 3.66 (dd, *J* = 11.0, 7.8 Hz, 10H), 1.14 (t, *J* = 7.1 Hz, 3H).

### Exemple 17 : Préparation du dérivé N°115: 2-(2-(4-chloro phényl)-5-(morpholine-4-carbonyl)thiophén-3-yl)acétate d'éthyle

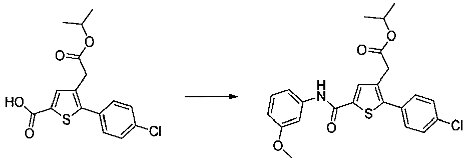

0,5 g (1,476 mmol) d'acide 5-(4-chlorophényl)-4-(2-isopropoxy-2-oxoéthyl)thiophène-2-carboxylique ont été solubilisés dans 10 ml de diméthylformamide, à cette solution ont été ajoutés sous agitation magnétique, 0,311 g (1,623 mmol) d'EDC, 0,249 g (1,623 mmol) de HOBt et 0,411 mol (2,95 mmol) de triéthylamine. Le mélange a été agité à t.a. pendant 30 min avant d'ajouter 0,205 ml (1,77 mmol) de 3-méthoxyaniline, le mélange a ensuite été agité à t.a. pendant 3 h. Le milieu réactionnel a été dilué avec 20 ml d'acétate d'éthyle et lavé 2 x 20 ml d'une solution aqueuse saturée en NaCl. La phase aqueuse a été extraite avec 20 ml d'acétate d'éthyle, les phases organiques combinées ont été lavées avec 20 ml d'une solution aqueuse saturée en NaCl puis séchées sur Na₂SO₄ qui a ensuite été éliminé par filtration et le filtrat obtenu a été concentré sous vide. Le résidu brut a été purifié par trituration dans 10 ml d'éther diisopropylique, un solide marron a été isolé par filtration pour donner 0,529 g (rendement = 80%) de 2-(2-(4-chlorophényl)-5-(morpholine-4-carbonyl)thiophén-3-yl)acétate d'éthyle.
LC-MS: m/z = 444 (MH⁺) ; pureté UV à 254 nm >99%. RMN ¹H (300 MHz, DMSO) δ 10.27 (s, 1H), 8.00 (s, 1H), 7.55 (q, J = 8.6 Hz, 4H), 7.42 (d, J = 2.1 Hz, 1H), 7.34 (d, J = 8.3 Hz, 1H), 7.26 (t, J = 8.1 Hz, 1H), 6.70 (dd, J = 7.8, 1.8 Hz, 1H), 4.90 (dt, J = 12.5, 6.2 Hz, 1H), 3.76 (s, 3H), 3.70 (s, 2H), 1.16 (d, J = 6.3 Hz, 6H).

Les dérivés numéro **116** à **118, 168** et **170** ont été préparés selon le même protocole

| **N°** | **Poids moléculaire g/mol** | **Apparence** | **Pureté LCMS UV à 254 nm** | **Spectrométrie de masse m/z** | | **RMN ¹H (300 MHz, DMSO) δ** |
|---|---|---|---|---|---|---|
| | | | | **MH⁺** | **M-H⁺** | |
| **116** | 457,97 | solide marron clair | 97 | 458 | | 9.12 (t, *J* = 6.0 Hz, 1H), 7.77 (s, 1H), 7.53 (q, J = 8.6 Hz, 4H), 7.25 (t, *J* = 8.1 Hz, 1H), 6.98 - 6.75 (m, 3H), 4.88 (dt, *J* = 12.5, 6.2 Hz, 1H), 4.42 (d, J = 5.9 Hz, 2H), 3.74 (s, 3H), 3.64 (s, 2H), 1.15 (d, *J* = 6.3 Hz, 6H). |
| **117** | 457,97 | solide marron | 96 | 458 | | 9.07 (t, J = 5.9 Hz, 1H), 7.75 (s, 1H), 7.53 (dd, J = 19.6, 8.5 Hz, 4H), 7.24 (d, J = 8.6 Hz, 2H), 6.89 (d, J = 8.6 Hz, 2H), 4.87 (dt, J = 12.5, 6.3 Hz, 1H), 4.37 (d, J = 5.9 Hz, 2H), 3.73 (s, 3H), 3.64 (s, 2H), 1.15 (d, J = 6.2 Hz, 6H). |
| **118** | 443,94 | solide marron | 99 | 444 | 442 | 9.07 (t, J = 5.9 Hz, 1H), 7.75 (s, 1H), 7.53 (dd, J = 19.6, 8.5 Hz, 4H), 7.24 (d, J = 8.6 Hz, 2H), 6.89 (d, J = 8.6 Hz, 2H), 4.87 (dt, J = 12.5, 6.3 Hz, 1H), 4.37 (d, J = 5.9 Hz, 2H), 3.73 (s, 3H), 3.64 (s, 2H), 1.15 (d, J = 6.2 Hz, 6H). |
| **168** | 400,88 | solide | 96% | 401 | 399 | 10.55 (s, 1H), 8.90 (s, 1H), 8.32 (d, J = 3.9 Hz, 1H), 8.15 (d, J = 9.0 Hz, 1H), 8.03 (s, 1H), 7.56 (q, J = 8.7 Hz, 4H), 7.41 (d, J = 12.9 Hz, 1H), 4.09 (q, J = 7.1 Hz, 2H), 3.74 (s, 2H), 1.17 (t, J = 7.1 Hz, 3H) |
| **170** | 394,91 | solide | 99% | 395 | | (CDCl3) 7.42 (s, 1H), 7.34 (s, 4H), 4.11 (q, J = 7.1 Hz, 2H), 3.52 (s, 2H), 3.45 (dd, J = 11.3, 5.2 Hz, 2H), 2.53 - 2.43 (m, 2H), 2.23 (s, 6H), 1.20 (t, J = 7.2 Hz, 3H) |

### Exemple 18: Préparation du dérivé N°119: acide 2-(2-(4-chlorophényl)-5-((3-méthoxyphényl)carbamoyl)thiophén-3-yl) acétique

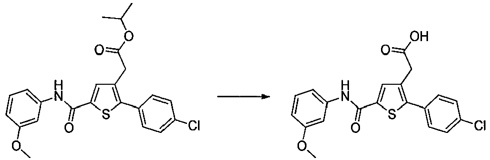

0,1 g (0,225 mmol) de 2-(2-(4-chlorophényl)-5-(morpholine-4-carbonyl)thiophén-3-yl)acétate d'éthyle ont été solubilisés sous agitation magnétique dans 2 ml de méthanol, 0,248 ml (0,248 mmol) d'une solution aqueuse d'hydroxyde de sodium 1 M ont été ajoutés. Le mélange a été agité à t.a. pendant 3 h. 0,01 ml (0,01 mmol) d'une solution aqueuse d'hydroxyde de sodium 1 M ont été ajoutés. Le mélange a de nouveau été agité à t.a. pendant 3 h. Le milieu réactionnel a été neutralisé par addition d'une solution de 0,019 ml (0,338 mmol) d'acide acétique dans 5 ml d'eau. Le mélange a été extrait avec 2 x 10 ml d'acétate d'éthyle, les phases organiques combinées ont été lavées avec 10 ml d'une solution aqueuse saturée en NaCl puis séchées sur Na₂SO₄ qui a ensuite été éliminé par filtration. Le filtrat obtenu a été concentré sous vide pour donner 0,082 g (rendement = 86%) d'acide 2-(2-(4-chlorophényl)-5-((3-méthoxyphényl)carbamoyl)thiophén-3-yl)acétique sous forme d'un solide blanc. LC-MS: m/z = 402 (MH⁺) ; pureté UV à 254 nm = 95%. RMN ¹H (300 MHz, DMSO) δ 12.69 (s, 1H), 10.29 (s, 1H), 8.03 (s, 1H), 7.66 - 7.49 (m, 4H), 7.42 (t, J = 2.1 Hz, 1H), 7.34 (d, J = 8.4 Hz, 1H), 7.25 (t, J = 8.1 Hz, 1H), 6.69 (dd, J = 8.0, 2.1 Hz, 1H), 3.75 (s, 3H), 3.62 (s, 2H).

Les dérivés numéro **120** à **122, 169, 171** et **172** ont été préparés selon le même protocole

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **120** | 120415,89 | mousse marron | 96 | 416 | | 12.62 (s, 1H), 9.13 (t, *J* = 6.0 Hz, 1H), 7.79 (s, 1H), 7.53 (q, *J* = 8.6 Hz, 4H), 7.24 (t, *J* = 8.1 Hz, 1H), 6.95-6.75 (m, 3H), 4.41 (d, *J* = 5.9 Hz, 2H), 3.73 (s, 3H), 3.57 (s, 2H). |
| **121** | 401,86 | solide marron clair | 95 | 402 | | 12.68 (s, 1H), 10.22 (s, 1H), 7.98 (s, 1H), 7.58 (dt, *J* = 14.0, 8.9 Hz, 6H), 6.93 (d, J = 9.1 Hz, 2H), 3.74 (s, 3H), 3.61 (s, 2H). |
| **122** | 122 415,89 | solide marron clair | 96 | 416 | | 12.62 (s, 1H), 9.09 (t, *J* = 5.9 Hz, 1H), 7.78 (s, 1H), 7.54 (q, *J* = 8.6 Hz, 4H), 7.24 (d, *J* = 8.6 Hz, 2H), 6.90 (d, *J* = 8.6 Hz, 2H), 4.37 (d, *J* = 5.8 Hz, 2H), 3.73 (s, 3H), 3.57 (s, 2H). |
| **169** | 372,82 | solide | 99 | 373 | | 9.15 (s, 1H), 8.59 - 8.35 (m, 2H), 8.08 (s, 1H), 7.84 - 7.69 (m, 1H), 7.56 (q, J = 8.5 Hz, 4H) |
| **171** | 366,86 | solide blanc | 93 | 367 | | 8.48 (s, 1H), 7.79 (d, J = 8.5 Hz, 2H), 7.72 (s, 1H), 7.48 (d, *J* = 8.5 Hz, 2H), 2.36 (t, J = 6.7 Hz, 2H), 2.15 (s, 6H), 1.69 (s, 2H) |
| **172** | 434,33 | solide | 91 | 434 | 432 | 10.45 (s, 1H), 8.00 (s, 1H), 7.78 (d, J = 8.9 Hz, 2H), 7.56 (q, J = 8.8 Hz, 4H), 7.42 (d, J = 8.9 Hz, 2H), 4.09 (q, J = 7.1 Hz, 2H), 3.73 (s, 2H), 1.17 (t, J = 7.1 Hz, 3H) |

### Exemple 19 : Préparation du dérivé numéro 123: 2-(2-(4-chlorophényl)-5-(4-méthoxybenzoyl)thiophén-3-yl)propanoate d'éthyle

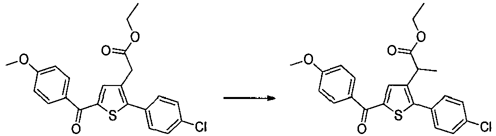

0,25 g (0,597 mmol) de 2-(2-(4-chlorophényl)-5-(4-méthoxybenzoyl)thiophén-3-yl)acétate d'éthyle ont été solubilisés dans 5 ml de tétrahydrofurane sous agitation magnétique. Le mélange a été refroidi à -20°C et 0,663 ml (1,193 mmol) d'une solution de diisopropylamidure de lithium 2M dans le tétrahydrofurane ont été additionnés goutte à goutte. Le mélange a été agité 1 h à -20°C avant d'ajouter 0,039 ml (0,626 mmol) d'iodure de méthyle. Le mélange a été agité 1 h supplémentaire à -20°C. Sans attende le retour à t.a., le mélange a été versé dans 10 ml d'eau. La phase aqueuse a été extraite avec 3 x 10 ml d'acétate d'éthyle, les phases organiques combinées ont été lavées avec 15 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : gradient de Dichlorométhane / éther de pétrole, de 50 à 70% en Dichlorométhane, v/v). 0,145 g (rendement = 55%) de 2-(2-(4-chlorophényl)-5-(4-méthoxybenzoyl)thiophén-3-yl)propanoate d'éthyle ont été obtenus sous forme d'une huile jaune pâle. LC-MS: m/z = 429 (MH⁺) ; pureté UV à 254 nm = 97%. RMN ¹H (300 MHz, DMSO) δ .92 - 7.83 (m, 2H), 7.68 (s, 1H), 7.65 - 7.51 (m, 4H), 7.19 - 7.08 (m, 2H), 4.05 (dt, J = 7.2, 3.4 Hz, 2H), 3.92 - 3.80 (m, 4H), 1.43 (d, J = 7.1 Hz, 3H), 1.13 (t, J = 7.1 Hz, 3H).

Le dérivé numéro **124** a été préparé selon le même protocole

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **124** | 458,95 | solide | 92 | 459 | | 7.87 (d, *J* = 8.8 Hz, 2H), 7.73 (s, 1H), 7.61 (q, *J* = 8.7 Hz, 4H), 7.14 (d, *J* = 8.8 Hz, 2H), 4.19 - 3.56 (m, 8H), 3.20 (s, 3H), 1.15 (t, *J* = 7.1 Hz, 3H). |

### Exemple 20: Préparation du dérivé numéro 125: 2-(2-(4-chlorophényl)-5-(4-méthoxybenzoyl)thiophén-3-yl)propanoate d'isopropyle

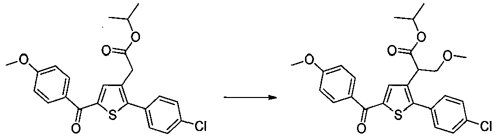

0,2 g (0,382 mmol) de 2-(2-(4-chlorophényl)-5-(4-méthoxybenzoyl) thiophén-3-yl)acétate d'isopropyle ont été solubilisés dans 3 ml de tétrahydrofurane sous agitation magnétique. Le mélange a été refroidi à -80°C et 0,765 ml (0,765 mmol) d'une solution de bis triméthylsilylamidure de lithium 1M dans le tétrahydrofurane ont été additionnés goutte à goutte. Le mélange a été agité 30 min à -50°C avant d'être à nouveau refroidi à -80°C et d'ajouter 0,058 ml (0,765 mmol) de chlorure de méthoxyméthane. Le bain a été retiré et le mélange a été agité pendant 1 h supplémentaire en laissant revenir à t.a.. Le milieu réactionnel a été versé dans 20 ml d'une solution aqueuse saturée en chlorure d'ammonium. La phase aqueuse a été extraite avec 2 x 15 ml d'acétate d'éthyle, les phases organiques combinées ont été lavées avec 15 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : gradient d'éther de pétrole / éther diisopropylique, de 100% à 0% d'éther de pétrole, v/v). 0,062 g (rendement = 33%) de 2-(2-(4-chlorophényl)-5-(4-méthoxybenzoyl) thiophén-3-yl)propanoate d'éthyle ont été obtenus sous forme d'un solide. LC-MS: m/z = 473 (MH⁺) ; pureté UV à 254 nm = 96%. RMN ¹H (300 MHz, DMSO) δ 7.87 (d, J = 8.6 Hz, 2H), 7.72 (s, 1H), 7.61 (d, J = 8.5 Hz, 4H), 7.14 (d, J = 8.6 Hz, 2H), 4.90 (dt, J = 12.4, 6.1 Hz, 1H), 4.02 - 3.76 (m, 5H), 3.72 - 3.60 (m, 1H), 3.20 (s, 3H), 1.15 (dd, J = 14.8, 6.1 Hz, 6H).

### Exemple 21: Préparation du dérivé numéro 126: 2-(2-(4-chlorophényl)-5-(cyclohexanecarbonyl)thiophén-3-yl)-3-phényl propanoate d'isopropyle

### Etape 1: Préparation du 2-(2-(4-chlorophényl)thiophén-3-yl)-3-phénylpropanoate d'éthyle

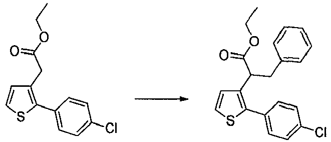

1 g (3,56 mmol) de 2-(2-(4-chlorophényl) thiophén-3-yl)acétate d'éthyle ont été solubilisés sous argon dans 5 ml de tétrahydrofurane. Le mélange a été refroidi à -50°C et 0,663 ml (3,56 mmol) d'une solution de diisopropylamidure de lithium 2M dans le tétrahydrofurane ont été additionnés goutte à goutte sous agitation magnétique. Le mélange réactionnel a été agité 5 min à -20°C avant d'être refroidi à -50°C. 1,272 ml (10,68 mmol) de bromure de benzyle ont été additionnés goutte à goutte. Le mélange a été agité à -50°C pendant 15 min avant que le bain soit retiré puis le mélange agité pendant 1 h en laissant revenir à t.a.. Le milieu réactionnel a été versé dans 30 ml d'un mélange composé d'eau et de glace. La phase aqueuse a été extraite avec 2 x 20 ml de Dichlorométhane, les phases organiques combinées ont été lavées avec 30 ml d'une solution aqueuse d'acide chlorhydrique 1N, 30 ml d'eau puis séchées sur MgSO₄ qui a ensuite été éliminé par filtration. Le filtrat obtenu a été concentré sous vide pour donner 1,285 g (rendement = 94%) de 2-(2-(4-chlorophényl)thiophén-3-yl)-3-phénylpropanoate d'éthyle sous forme d'une huile orangée. LC-MS: m/z = non ionisé. RMN ¹H (300 MHz, DMSO) δ 7.54 (d, J = 5.3 Hz, 1H), 7.43 (d, J = 8.5 Hz, 2H), 7.24 (d, J = 5.3 Hz, 1H), 7.13 (d, J = 7.8 Hz, 6H), 6.99 - 6.86 (m, 2H), 4.04 - 3.91 (m, 2H), 3.85 (t, J = 7.8 Hz, 1H), 3.23 (dd, J = 13.5, 7.6 Hz, 1H), 2.95 (dd, J = 13.5, 8.0 Hz, 1H), 1.03 (t, J = 7.1 Hz, 3H).

### Etape 2 : Préparation de l'acide 2-(2-(4-chlorophényl)thiophén-3-yl)-3-phénylpropanoique

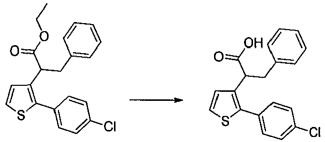

1 g (3,56 mmol) de 2-(2-(4-chlorophényl) thiophén-3-yl)-3-phényl propanoate d'éthyle ont été solubilisés dans 10 ml d'éthanol sous agitation magnétique. 0,311 ml (3,11 mmol) d'une solution aqueuse d'hydroxyde de sodium 10M ont été ajoutés, le mélange a été agité à t.a. pendant 3 j avant d'être concentré sous vide. Une solution aqueuse d'acide chlorhydrique 1N a été ajoutée jusqu'à apparition d'un précipité, la phase aqueuse a été extraite avec 2 x 25 ml d'acétate d'éthyle. Les phases organiques combinées ont été lavées avec 40 ml d'eau, 40 ml d'une solution aqueuse saturée en NaCl puis séchées sur MgSO₄ qui a ensuite été éliminé par filtration. Le filtrat obtenu a été concentré sous vide. Le résidu a été purifié par trituration dans 15 ml d'un mélange d'éther de pétrole et d'éther diisopropylique (1/1, v/v). Le solide a été isolé par filtration, lavé avec 10 ml d'éther de pétrole et séché sous cloche à vide pour donner 0,681 g (rendement = 70%) d'acide 2-(2-(4-chlorophényl)thiophén-3-yl)-3-phényl propanoique. LC-MS: m/z = 340 (M-H⁺) ; pureté UV à 254 nm >99%. RMN ¹H (300 MHz, DMSO) δ 12.56 (s, 1H), 7.58 (d, J = 5.3 Hz, 1H), 7.46 (d, J = 8.4 Hz, 2H), 7.27 (d, J = 5.3 Hz, 1H), 7.17 (dd, J = 10.7, 7.7 Hz, 5H), 6.94 (dd, J = 7.3, 1.9 Hz, 2H), 3.83 (t, J = 7.7 Hz, 1H), 2.93 (dd, J = 13.6, 8.1 Hz, 1H).

### Etape 3: Préparation du 2-(2-(4-chlorophényl)thiophén-3-yl)-3-phénylpropanoate d'isopropyle

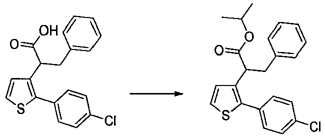

0,55 g (1,604 mmol) d'acide 2-(2-(4-chlorophényl)thiophén-3-yl)-3-phénylpropanoique ont été solubilisés dans 25 ml d'isopropanol, une goutte d'acide sulfurique a été ajoutée à cette solution. Le mélange a été chauffé à reflux pendant 16 h sous agitation magnétique. Après retour à t.a., le mélange a été concentré sous vide, le résidu brut a été repris dans 25 ml et le mélange a été agité à t.a. pendant 16 h. Un précipité a été isolé par filtration ; lavé avec un mélange composé de 10 ml d'eau et de 0,5 ml d'éther de pétrole et séché sous cloche à vide pour donner 0,46 g (rendement = 71%) de 2-(2-(4-chlorophényl)thiophén-3-yl)-3-phényl propanoate d'isopropyle sous forme d'un solide blanc. LC-MS: m/z = non ionisé. RMN ¹H (300 MHz, DMSO) δ 7.57 (d, J = 5.3 Hz, 1H), 7.46 (d, J = 8.5 Hz, 2H), 7.34 - 7.05 (m, 6H), 6.96 (d, J = 5.4 Hz, 2H), 4.78 (dt, J = 12.5, 6.2 Hz, 1H), 3.84 (t, J = 7.8 Hz, 1H), 3.24 (dd, J = 13.5, 8.1 Hz, 1H), 2.96 (dd, J = 13.5, 7.6 Hz, 1H), 1.02 (dd, J = 6.2, 3.4 Hz, 6H).

### Etape 4: Préparation du 2-(2-(4-chlorophényl)-5 (cyclohexane carbonyl)thiophén-3-yl)-3 phénylpropanoate d'isopropyle (dérivé numéro 126)

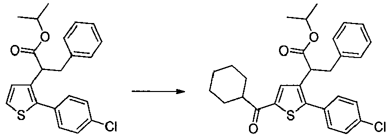

Dans un ballon placé sous un flux d'argon, ont été introduits sous agitation magnétique : 2 ml de Dichlorométhane, 0,285 g (2,14 mmol) de trichlorure d'aluminium, 0,173 g (1,77 mmol) de chlorure de carbonyle de cyclohexane et une solution de 0,429 g (1,07 mmol) de 2-(2-(4-chlorophényl)thiophén-3-yl)-3-phénylpropanoate d'isopropyle dans 3 ml de Dichlorométhane. Le mélange obtenu a été agité à t.a. pendant 16 h puis versé dans 25 ml d'un mélange composé d'eau et de glace et agité pendant 30 min. La phase aqueuse a été extraite avec 2 x 15 ml d'acétate d'éthyle. Les phases organiques combinées ont été lavées avec 15 ml d'une solution aqueuse saturée en NaHCO₃, 15 ml d'eau puis séchées sur MgSO₄ qui a ensuite été éliminé par filtration. Le filtrat obtenu a été concentré sous vide qui a ensuite été éliminé par filtration et le filtrat obtenu a été concentré sous vide. Le résidu brut a été purifié par chromatographie flash sur cartouche de gel de silice (éluant : éther diisopropylique / éther de pétrole, 1/9, v/v). 0,108 g (rendement = 20%) de 2-(2-(4-chlorophényl)-5-(cyclohexanecarbonyl)thiophén-3-yl)-3-phényl propanoate d'isopropyle ont été obtenu sous forme d'un solide. LC-MS: m/z = 495 (MH⁺) ; pureté UV à 254 nm = 97%. RMN ¹H (300 MHz, DMSO) δ 8.07 (s, 1H), 7.51 (d, J = 8.3 Hz, 2H), 7.20 (dd, J = 18.0, 7.5 Hz, 5H), 7.00 (d, J = 7.2 Hz, 2H), 4.81 (dt, J = 12.5, 6.2 Hz, 1H), 3.85 (t, J = 7.8 Hz, 1H), 3.10 (dd, J = 13.4, 8.0 Hz, 1H), 1.76 (t, J = 23.5 Hz, 5H), 1.58 - 1.11 (m, 6H), 1.04 (t, J = 6.0 Hz, 6H).

### Exemple 22: Préparation du dérivé N°127: acide 2-(2-(4-chlorophényl)-5-(4-méthoxybenzoyl)thiophén-3-yl)propanoique

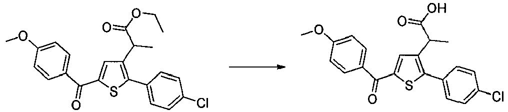

0,087 g (0,197 mmol) de 2-(2-(4-chlorophényl)-5-(4-méthoxybenzoyl)thiophén-3-yl)propanoate d'éthyle ont été solubilisés dans 1 ml d'un mélange de tétrahydrofurane et d'éthanol (1/1, v/v), à cette solution ont été ajoutés sous agitation magnétique 0,197 ml (0,197 mmol) d'une solution aqueuse d'hydroxyde de sodium 1N. Le mélange obtenu a été agité à t.a. pendant 16 h. Le mélange a été concentré sous vide, le résidu obtenu a été repris dans 10 ml d'eau, la phase aqueuse a été extraite avec 10 ml d'éther de méthyle tert-butylique. La phase aqueuse a ensuite été acidifiée jusqu'à pH = 3-4 par addition d'une solution aqueuse d'acide chlorhydrique 1N. La phase aqueuse a été extraite par 3 x 10 ml d'acétate d'éthyle, les phases organiques combinées ont été lavées avec 20 ml d'une solution aqueuse saturée en NaCl puis séchées sur Na₂SO₄ qui a ensuite été éliminé par filtration. Le filtrat obtenu a été concentré sous vide pour donner 0,077 g (rendement = 95%) d'acide 2-(2-(4-chlorophényl)-5-(4-méthoxybenzoyl)thiophén-3-yl)propanoique sous forme d'un solide blanc. LC-MS: m/z = 401 (MH⁺) ; pureté UV à 254 nm = 97%. RMN ¹H (300 MHz, DMSO) δ 7.97 - 7.79 (m, 4H), 7.73 (s, 1H), 7.57 (t, J = 10.0 Hz, 2H), 7.13 (d, J = 8.8 Hz, 2H), 3.88 (s, 3H), 3.46 (d, J = 7.1 Hz, 2H), 1.25 (d, J = 7.0 Hz, 3H).

Le dérivé **128** a été préparé selon le même protocole :

| **N°** | **Poids moléculaire** | **Apparence** | **Pureté LCMS** | **Spectrométrie de masse** m/z | | **RMN ¹H** |
|---|---|---|---|---|---|---|
| | **g/mol** | | **UV à 254 nm** | **MH⁺** | **M-H⁺** | **(300 MHz, CDCl3) δ** |
| **128** | 430,90 | huile | 91 | 431 | | 7.97 - 7.85 (m, 2H), 7.53-7.37 (m, 5H), 7.09 - 6.92 (m, 2H), 4.16 - 4.03 (m, 1H), 3.90 (s, 3H), 3.64 - 3.54 (m, 2H), 3.32 (s, 3H). |

### Exemple 23 : Préparation du dérivé N°129: 2-(2-(4-fluoro-2-méthoxyphényl)-5-(furan-2-carbonyl)thiophén-3-yl)acétate d'éthyle

### Etape 1: Réparation du 2-(thiophén-3-yl)acétate d'éthyle

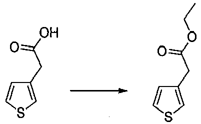

10,75 g (74,1 mmol) d'acide 2-(thiophén-3-yl)acétique ont été solubilisés dans 100 ml d'éthanol, 6 ml (72 mmol) d'acide sulfurique ont été ajoutés à cette solution. Le mélange a été chauffé sous agitation magnétique à reflux pendant 24 h. Après retour à t.a., le mélange a été concentré sous vide, le résidu brut a été traité par 100 ml d'un mélange composé d'eau et de glace. La phase aqueuse a été extraite par 2 x 100 ml d'acétate d'éthyle. Les phases organiques combinées ont été lavées avec 100 ml d'eau, 100 ml d'une solution aqueuse saturée en NaHCO₃ et 100 ml d'une solution aqueuse saturée en NaCl puis séchées sur MgSO₄ qui a ensuite été éliminé par filtration. Le filtrat obtenu a été concentré sous vide pour donner 12,27 g (rendement = 97%) de 2-(thiophén-3-yl)acétate d'éthyle sous forme d'une huile incolore. LC-MS: m/z = non ionisé. RMN ¹H (300 MHz, CDCl₃) δ 7.24 (dd, J = 6.0, 3.0 Hz, 1H), 7.16 - 7.07 (m, 1H), 7.02 (dd, J = 4.9, 1.1 Hz, 1H), 4.13 (q, J = 7.1 Hz, 2H), 3.62 (s, 2H), 1.23 (t, J = 7.1 Hz, 3H).

### Etape 2: Préparation du 2-(2-bromothiophén-3-yl)acétate d'éthyle

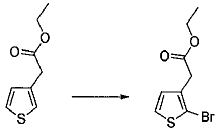

12,2 g (71,7 mmol) de 2-(thiophén-3-yl)acétate d'éthyle ont été solubilisés dans 100 ml de tétrahydrofurane sous agitation magnétique, 121,7 mmol) de N-bromosuccinimide ont été ajoutés et le mélange a été agité à reflux pendant 3 h. Après retour à t.a., le mélange a été concentré sous vide, le résidu brut a été repris dans un minimum d'éther de pétrole pour être filtré sur gel de silice (éluant : Dichlorométhane 100%) et donner 18,38 g (rendement = 88%) de 2-(2-bromothiophén-3-yl)acétate d'éthyle sous forme d'une huile jaune pâle. LC-MS: m/z = non ionisé. RMN ¹H (300 MHz, CDCl₃) δ 7.27 - 7.16 (m, 1H), 7.03 - 6.86 (m, 1H), 4.12 (qd, J = 7.1, 3.2 Hz, 2H), 3.64 - 3.48 (m, 2H), 1.22 (td, J = 7.1, 1.4 Hz, 3H).

### Etape 3: Préparation du 2-(2-(4-tluoro-2-méthoxyphényl) thiophén-3-yl)acétate d'éthyle

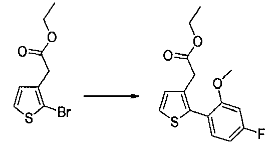

0,7 g (2,53 mmol) de 2-(2-bromothiophén-3-yl)acétate d'éthyle ont été solubilisés sous argon dans 10 ml d'un mélange composé de toluène et d'éthanol (1/1, v/v), ont été ajoutés sous agitation magnétique, 0,645 g (3,79 mmol) d'acide 4-fluoro-2-méthoxyphénylboronique, 8,85 ml (17,7 mmol) d'une solution aqueuse de Na₂CO₃ 2M et 0,148 g (0,126 mmol) de palladium[0] tetrakis(triphénylphosphine). Le mélange a été agité à reflux pendant 3 h. Après retour à t.a., le mélange a été versé dilué avec 15 ml d'eau et 15 ml d'acétate d'éthyle avant d'être filtré sur célite. Après séparation, la phase aqueuse a été extraite avec 20 ml d'acétate d'éthyle. Les phases organiques combinées ont été lavées avec 10 ml d'eau, 10 ml d'une solution aqueuse saturée en NaCl puis séchées sur MgSO₄ qui a ensuite été éliminé par filtration. Le filtrat obtenu a été concentré sous vide. Le résidu brut a été purifié par chromatographie flash sur cartouche de gel de silice (éluant : gradient d'éther de pétrole / acétate d'éthyle, de 100% à 90% d'éther de pétrole, v/v). 0,529 g (rendement = 70%) de 2-(2-(4-fluoro-2-méthoxyphényl)thiophén-3-yl)acétate d'éthyle ont été obtenus sous forme d'une mousse jaune pâle. LC-MS: m/z = 295 (MH⁺) ; pureté UV à 254 nm = 83%. RMN ¹H (300 MHz, CDCl₃) δ 7.51 (d, J = 5.2 Hz, 1H), 7.26 (dd, J = 8.4, 6.9 Hz, 1H), 7.09 - 7.00 (m, 2H), 6.84 (td, J = 8.4, 2.5 Hz, 1H), 4.01 (q, J = 7.1 Hz, 2H), 3.74 (s, 3H), 3.41 (s, 2H), 1.13 (t, J = 7.1 Hz, 3H).

### Etape 4 : Préparation du 2-(2-(4-fluoro-2-méthoxyphényl)-5-(furan-2-carbonyl)thiophén-3-yl)acétate d'éthyle (dérivé numéro 129)

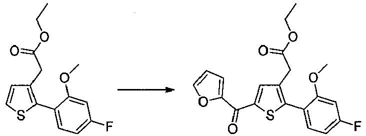

Dans un ballon placé sous un flux d'argon, ont été introduits sous agitation magnétique : 5 ml de Dichlorométhane, 0,583 g (4,37 mmol) de trichlorure d'aluminium, une solution de 0,19 ml (1,924 mmol) de chlorure de 2-furoyle dans 2 ml de Dichlorométhane et une solution de 0,52 g (1,749 mmol) de 2-(2-(4-fluoro-2-méthoxyphényl)thiophén-3-yl)acétate d'éthyle dans 2 ml de Dichlorométhane. Le mélange obtenu a été agité à reflux pendant 3 h puis à t.a. pendant 16 h avant d'être versé dans 15 ml d'un mélange composé d'eau et de glace et d'être agité pendant 30 min. 15 ml de Dichlorométhane ont été ajoutés et le mélange a été filtré sur célite. Après séparation, la phase aqueuse a été extraite avec 20 ml de Dichlorométhane. Les phases organiques combinées ont été lavées avec 20 ml d'eau puis séchées sur MgSO₄ qui a ensuite été éliminé par filtration. Le filtrat obtenu a été concentré sous vide. Le résidu brut a été purifié par chromatographie flash sur cartouche de gel de silice (éluant : gradient d'éther de pétrole / éther diisopropylique, de 100% à 0% d'éther de pétrole, v/v). 0,287 g (rendement = 41%) de 2-(2-(4-fluoro-2-méthoxyphényl)-5-(furan-2-carbonyl)thiophén-3-yl)acétate d'éthyle ont été obtenu sous forme d'un solide. LC-MS: m/z = 389 (MH⁺) ; pureté UV à 254 nm = 98%. RMN ¹H (300 MHz, DMSO) δ 8.19 - 8.11 (m, 2H), 7.56 (d, J = 3.6 Hz, 1H), 7.37 (dd, J = 8.5, 6.8 Hz, 1H), 7.11 (dd, J = 11.4, 2.4 Hz, 1H), 6.91 (td, J = 8.4, 2.5 Hz, 1H), 6.84 (dd, J = 3.6, 1.7 Hz, 1H), 4.04 (q, J = 7.1 Hz, 2H), 3.78 (s, 3H), 3.53 (s, 2H), 1.14 (t, J = 7.1 Hz, 3H).

Les dérivés numéro **130** à **134** ont été préparés selon le même enchaînement de 4 étapes.

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **130** | 376,37 | huile | 98 | 377 | | 8.24 (s, 1H), 8.17 (d, J = 1.0 Hz, 1H), 7.67 - 7.53 (m, 2H), 7.42 - 7.30 (m, 2H), 6.86 (dd, J = 3.6, 1.7 Hz, 1H), 4.02 (q, J = 7.1 Hz, 2H), 3.70 (s, 2H), 1.11 (t, J = 7.1Hz, 3H). |
| **131** | 37,42 | huile marron claire | 92 | 371 | | 8.19 (s, 1H), 8.15 (d, J = 1.0 Hz, 1H), 7.59 (dd, J = 3.6, 0.6 Hz, 1H), 7.49 - 7.38 (m, 1H), 7.13 - 7.02 (m, 3H), 6.84 (dd, J = 3.6, 1.7 Hz, 1H), 4.08 (q, J = 7.1 Hz, 2H), 3.81 (s, 5H), 1.15 (t, J = 7.1 Hz, 3H). |
| **132** | 384,40 | huile | 96 | 385 | | 8.21 - 8.10 (m, 2H), 7.57 (d, J = 4.0 Hz, 1H), 7.15 - 6.96 (m, 3H), 6.84 (dd, J = 3.6, 1.7 Hz, 1H), 6.12 (s, 2H), 4.08 (q, J = 7.1 Hz, 2H), 3.78 (s, 2H), 1.16 (t, J = 7.1 Hz, 3H). |
| **133** | 416,44 | solide blanc | 90 | 417 | | 7.91 (d, J = 8.9 Hz, 2H), 7.79 (s, 1H), 7.61 (s, 1H), 7.42-7.29 (m, 2H), 7.14 (d, J = 8.9 Hz, 2H), 4.01 (q, J = 7.1 Hz, 2H), 3.88 (s, 3H), 3.67 (s, 2H), 1.11 (t, J = 7.1 Hz, 3H). |
| **134** | 370,42 | huile | 92 | 371 | | 8.10 (d, J = 1.7 Hz, 1H), 7.84 (d, J = 8.9 Hz, 2H), 7.55 (d, J = 5.2 Hz, 1H), 7.02 (t, J = 7.1 Hz, 3H), 6.86 (d, J = 1.7 Hz, 1H), 3.99 (q, J = 7.1 Hz, 2H), 3.83 (s, 3H), 3.52 (s, 2H), 1.09 (t, J = 7.1 Hz, 3H). |

### Exemple 24 : Préparation du dérivé numéro 135 : acide 2-(2-(4-chlorophényl)-5-(4-méthoxybenzoyl)thiophén-3-yl)propanoique

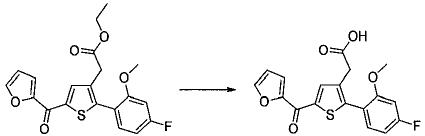

0,207 g (0,522 mmol) de 2-(2-(4-fluoro-2-méthoxyphényl)-5-(furan-2-carbonyl)thiophén-3-yl)acétate d'éthyle ont été solubilisés dans 3 ml d'un mélange composé de tétrahydrofurane et d'éthanol (1/2, v/v), à cette solution ont été ajoutés sous agitation magnétique 0,057 ml (0,575 mmol) d'une solution aqueuse d'hydroxyde de sodium 10N. Le mélange obtenu a été agité à t.a. pendant 3 h. Le mélange a été concentré sous vide, le résidu obtenu a été repris dans 10 ml d'eau, la phase aqueuse a été acidifiée jusqu'à pH = 2-3 par addition d'une solution aqueuse d'acide chlorhydrique 12N. 2 ml d'éther de pétrole ont été ajoutés et le mélange a été agité vigoureusement pendant 15 min. Un précipité a été isolé par filtration, lavé avec 5 ml d'eau et 5 ml d'éther de pétrole avant d'être séché sous cloche à vide pour donner 0,136 g (rendement = 70%) d'acide 2-(2-(4-chlorophényl)-5-(4-méthoxybenzoyl) thiophén-3-yl) propanoique. LC-MS: m/z = 361 (MH⁺) ; pureté UV à 254 nm = 98%. RMN ¹H (300 MHz, DMSO) δ 12.44 (s, 1H), 8.15 (s, 2H), 7.56 (d, J = 3.6 Hz, 1H), 7.38 (dd, J = 8.4, 6.8 Hz, 1H), 7.11 (dd, J = 11.4, 2.4 Hz, 1H), 6.91 (td, J = 8.4, 2.4 Hz, 1H), 6.86 - 6.77 (m, 1H), 3.78 (s, 3H), 3.44 (s, 2H).

Les dérivés **136** et **137** ont été préparés selon le même protocole.

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **136** | 348,32 | solide | 99 | 349 | | 12.57 (s, 1H), 8.22 (s, 1H), 8.17 (dd, J = 1.6, 0.6 Hz, 1H), 7.68 - 7.52 (m, 2H), 7.36 (dd, J = 6.6, 3.2 Hz, 2H), 6.86 (dd, J = 3.7, 1.7 Hz, 1H), 3.59 (s, 2H). |
| **137** | 388,39 | solide | 94 | 389 | | δ 12.52 (s, 1H), 7.91 (d, J = 8.8 Hz, 2H), 7.78 (s, 1H), 7.68 - 7.52 (m, 1H), 7.45-7.27 (m, 2H), 7.14 (d, J = 8.8 Hz, 2H), 3.88 (s, 3H), 3.58 (s, 2H). |

### Exemple 25 : Préparation du dérivé numéro 138 : acide 2-(2-(4-fluoro-2-hydroxyphényl)-5-(furan-2-carbonyl)thiophén-3-yl)acétique

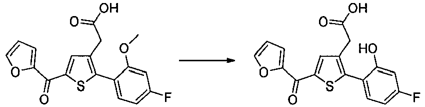

0,122 g (0,816 mmol) de D,L-Méthionine ont été solubilisés dans 0,882 ml (13,69 mmol) d'acide méthanesulfonique sous agitation magnétique. A cette solution, ont été additionnés 0,1 g (0,272 mmol) d'acide 2-(2-(4-chlorophényl)-5-(4-méthoxybenzoyl)thiophén-3-yl)propanoique. Le mélange a été chauffé à 80°C pendant 4 j au cours desquels 0,041 g (0,272 mmol) de D,L-Méthionine ont été ajoutés toutes les 24 h (soit 3 ajouts en 4 j). Après retour à t.a., le mélange a été versé dans 15 ml d'un mélange composé d'eau et de glace. La phase aqueuse a été extraite avec 15 ml d'isobutanol. La phase organique a été lavée avec 15 ml d'eau et 15 ml d'une solution aqueuse saturée en NaCl puis séchée sur MgSO₄ qui a ensuite été éliminé par filtration. Le filtrat obtenu a été concentré sous vide et purifié par HPLC/MS préparative pour donner 0,02 g (rendement = 21%) d'acide 2-(2-(4-fluoro-2-hydroxyphényl)-5-(furan-2-carbonyl)thiophén-3-yl)acétique sous forme d'un solide jaune pâle. LC-MS: m/z = 347 (MH⁺) ; pureté UV à 254 nm = 97%. RMN ¹H (300 MHz, DMSO) δ 11.66 (s, 1H), 8.14 (s, 2H), 7.55 (d, J = 4.0 Hz, 1H), 7.29 (dd, J = 8.9, 6.9 Hz, 1H), 6.83 (dd, J = 3.6, 1.7 Hz, 1H), 6.75 (t, J = 8.1 Hz, 2H), 3.51 (s, 2H).

### Exemple 26: Préparation du dérivé numéro 139: 2-(2-(4-cyanophényl)-5-(furan-2-carbonyl)thiophén-3-yl)acétate d'éthyle

### Etape 1: Préparation du 2-(5-(furan-2-carbonyl)thiophén-3-yl)acétate d'éthyle

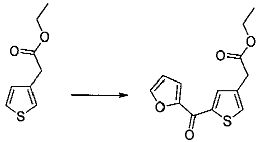

Dans un ballon placé sous un flux d'argon, ont été introduits sous agitation magnétique : 25 ml de Dichlorométhane, 7,84 g (0,585 mmol) de trichlorure d'aluminium, une solution de 3,35 ml (32,3 mmol) de chlorure de 2-furoyle dans 10 ml de Dichlorométhane et une solution de 5 g (29,35 mmol) de 2-(thiophén-3-yl)acétate d'éthyle dans 10 ml de Dichlorométhane. Le mélange obtenu a été agité à reflux pendant 1 h avant d'être versé dans 75 ml d'un mélange composé d'eau et de glace et d'être agité à t.a. pendant 1 h. La phase aqueuse a été extraite avec 2 x 50 ml de Dichlorométhane. Les phases organiques combinées ont été lavées avec 75 ml d'eau puis séchées sur MgSO₄ qui a ensuite été éliminé par filtration. Le filtrat obtenu a été concentré sous vide. Le résidu brut a été purifié par chromatographie flash sur cartouche de gel de silice (éluant : gradient d'éther de pétrole / acétate d'éthyle, de 100% à 80% d'éther de pétrole, v/v). 5,9 g (rendement = 72%) de 2-(5-(furan-2-carbonyl)thiophén-3-yl)acétate d'éthyle ont été obtenu sous forme de solide. LC-MS: m/z = 265 (MH⁺) ; pureté UV à 254 nm = 95%. RMN ¹H (300 MHz, DMSO) δ 8.12 (s, 2H), 7.88 (s, 1H), 7.54 (d, J = 3.6 Hz, 1H), 6.82 (dd, J = 3.6, 1.7 Hz, 1H), 4.11 (q, J = 7.1 Hz, 2H), 3.79 (s, 2H), 1.21 (t, J = 7.1 Hz, 3H).

### Etape 2: Préparation du 2-(2-( 4-cyanophényl)-5-(furan-2-carbonyl)thiophén-3-yl)acétate d'éthyle (dérivé numéro 139)

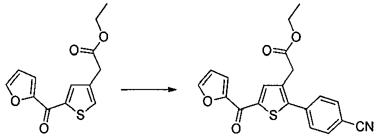

1,189 g (4,27 mmol) de 2-(5-(furan-2-carbonyl)thiophén-3-yl)acétate d'éthyle ont été solubilisés dans 20 ml de DMSO sous agitation magnétique, ensuite 0,999 g (4,27 mmol) de 4-iodobenzonitrile, 0,497 g (8,55 mmol) de fluorure de potassium, 0,6 g (0,855 mmol) de Pd(Cl)₂(PPh₃)₂ et 1,452 g (8,55 mmol) de nitrate d'argent ont été additionnés. Le mélange a été agité à 130°C pendant 10 min. Après retour à t.a. le mélange a été versé dans 100 ml d'un mélange composé d'eau et de glace. La phase aqueuse a été extraite avec 2 x 50 ml d'acétate d'éthyle. Les phases organiques combinées ont été lavées avec 50 ml d'eau et 50 ml d'une solution aqueuse saturée en NaCl puis séchée sur MgSO₄ qui a ensuite été éliminé par filtration. Le filtrat obtenu a été concentré sous vide, repris dans 20 ml de Dichlorométhane et filtré sur gel de silice (éluant Dichlorométhane 100%). Le filtrat obtenu a été concentré sous vide et purifié par chromatographie flash sur cartouche de gel de silice (éluant : gradient d'éther de pétrole / éther diisopropylique, de 100% à 0% d'éther de pétrole, v/v). 254 mg (rendement = 15%) de 2-(2-(4-cyanophényl)-5-(furan-2-carbonyl)thiophén-3-yl)acétate d'éthyle ont été obtenus sous forme d'huile jaune pâle. LC-MS: m/z = 366 (MH⁺) ; pureté UV à 254 nm = 94%. RMN ¹H (300 MHz, DMSO) δ 8.18 (d, J = 15.8 Hz, 2H), 7.99 (d, J = 8.3 Hz, 2H), 7.75 (d, J = 8.3 Hz, 2H), 7.61 (d, J = 3.6 Hz, 1H), 6.85 (dd, J = 3.6, 1.6 Hz, 1H), 4.06 (q, J = 7.1 Hz, 2H), 3.85 (s, 2H), 1.14 (t, J = 7.1 Hz, 3H).

### Exemple 27 : Préparation du dérivé numéro 140 : acide 2-(2-(4-cyanophényl)-5-(furan-2-carbonyl)thiophén-3-yl)acétique

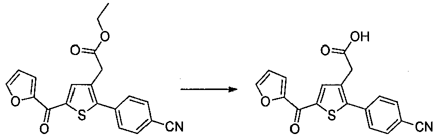

0,189 g (0,491 mmol) de 2-(2-(4-cyanophényl)-5-(furan-2-carbonyl)thiophén-3-yl)acétate d'éthyle ont été solubilisés dans 1,5 ml d'un mélange de tétrahydrofurane et d'éthanol (1/2, v/v), à cette solution ont été ajoutés sous agitation magnétique 0,054 ml (0,541 mmol) d'une solution aqueuse d'hydroxyde de sodium 10N. Le mélange obtenu a été agité à t.a. pendant 1 h. Le mélange a été concentré sous vide, le résidu obtenu a été repris dans 10 ml d'eau, la phase aqueuse a été acidifiée jusqu'à pH = 2-3 par addition d'une solution aqueuse d'acide chlorhydrique 4N. La phase aqueuse a été extraite par 2 x 10 ml d'acétate d'éthyle, les phases organiques combinées ont été lavées avec 20 ml d'eau et 20 ml d'une solution aqueuse saturée en NaCl puis séchées sur MgSO₄ qui a ensuite été éliminé par filtration. Le résidu a été repris dans 10 ml d'eau, agité à t.a. pendant 1 h, le solide obtenu a été isolé par filtration et lavé à l'eau pour donner 166 mg (rendement = 68%) d'acide 2-(2-(4-cyanophényl)-5-(furan-2-carbonyl)thiophén-3-yl)acétique. LC-MS: m/z = non ionisé. RMN ¹H (300 MHz, DMSO) δ 12.69 (s, 1H), 8.21 (s, 1H), 8.16 (d, J = 0.8 Hz, 1H), 8.00 (d, J = 8.2 Hz, 2H), 7.76 (d, J = 8.3 Hz, 2H), 7.61 (d, J = 3.6 Hz, 1H), 6.85 (dd, J = 3.6, 1.6 Hz, 1H), 3.75 (s, 2H).

### Exemple 28: Préparation du dérivé numéro 141: 2-(2-(4-cyanophényl)-5-(furan-2-carbonyl)thiophén-3-yl)-N-(2-(diméthylamino)éthyl)acétamide

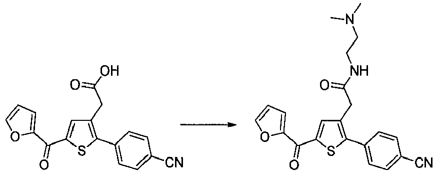

0,125 g (0,371 mmol) d'acide 2-(2-(4-cyanophényl)-5-(furan-2-carbonyl)thiophén-3-yl)acétique ont été solubilisés dans un mélange de 5 ml de Dichlorométhane et 0,1 ml de diméthylformamide. A cette solution, ont été ajoutés sous agitation magnétique à 0°C, 0,045 ml (0,408 mmol) de N,N-dimétyléthane-1,2-diamine, 0,178 g (0,926 mmol) d'EDC.HCl et 0,055 g (0,408 mmol) de HOBt. Le mélange a été agité à t.a. pendant 16 h. 30 ml d'eau ont été ajoutés au milieu réactionnel. La phase aqueuse a été extraite avec 30 ml de Dichlorométhane, la phase organique a été lavée avec 10 ml d'une solution aqueuse saturée en NaHCO₃, 10 ml d'eau et 10 ml d'une solution aqueuse saturée en NaCl puis séchée sur Na₂SO₄ qui a ensuite été éliminé par filtration et le filtrat obtenu a été concentré sous vide. 0,16 g (rendement = 30%) de 2-(2-(4-cyanophényl)-5-(furan-2-carbonyl) thiophén-3-yl)-N-(2-(diméthylamino)éthyl)acétamide ont été obtenus sous forme d'un solide blanc. LC-MS: m/z = 408 (MH⁺) ; pureté UV à 254 nm = 98%. RMN ¹H (300 MHz, DMSO) δ 8.20 (s, 1H), 8.15 (d, J = 1.0 Hz, 2H), 7.98 (d, J = 8.4 Hz, 2H), 7.88 (d, J = 8.4 Hz, 2H), 7.58 (d, J = 3.5 Hz, 1H), 6.86 (dd, J = 3.6, 1.7 Hz, 1H), 3.56 (s, 2H), 3.16 (dd, J = 12.3, 6.4 Hz, 2H), 2.27 (t, J = 6.6 Hz, 2H), 2.13 (s, 6H).

### Exemple 29: Préparation du dérivé numéro 142: 2-(2-(4-cyanophényl)-5-(furan-2-carbonyl)thiophén-3-yl)-N-(2-(diméthylamino)éthyl)acétamide

### Etape 1: Préparation de l'acide 2-(S-(furan-2-carbonyl)thiophén-3-yl)acétique

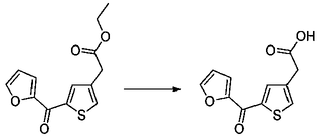

2 g (7,57 mmol) de 2-(5-(furan-2-carbonyl) thiophén-3-yl)acétate d'éthyle ont été solubilisés dans ont été solubilisés dans 10 ml d'éthanol, à cette solution ont été ajoutés sous agitation magnétique 0,832 ml (8,32 mmol) d'une solution aqueuse d'hydroxyde de sodium 10N. Le mélange obtenu a été agité à t.a. pendant 2 h. Le mélange a été concentré sous vide, le résidu obtenu a été repris dans 15 ml d'eau, la phase aqueuse a été lavée 2 x 15 ml d'acétate d'éthyle puis acidifiée jusqu'à pH = 1-2 par addition d'une solution aqueuse d'acide chlorhydrique 12N. La phase aqueuse a été extraite par 2 x 15 ml d'acétate d'éthyle, les phases organiques combinées ont été lavées avec 15 ml d'eau et 15 ml d'une solution aqueuse saturée en NaCl puis séchées sur MgSO₄ qui a ensuite été éliminé par filtration. 1,715 g (rendement = 96%) d'acide 2-(5-(furan-2-carbonyl)thiophén-3-yl)acétique ont été obtenus sous forme d'une huile orangée. LC-MS: m/z = non ionisé. RMN ¹H (300 MHz, DMSO) δ 12.43 (s, 1H), 8.11 (s, 2H), 7.86 (s, 1H), 7.54 (d, J = 3.6 Hz, 1H), 6.81 (dd, J = 3.6, 1.7 Hz, 1H), 3.70 (s, 2H).

### Etape 2 : Préparation du N-(2-(diméthylamino)éthyl)-2-(5-(furan-2-carbonyl)thiophén-3-yl)acétamide

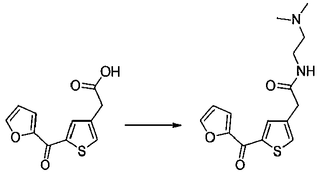

0,416 g (1,761 mmol) d'acide 2-(5-(furan-2-carbonyl)thiophén-3-yl)acétique ont été solubilisés dans un mélange de 5 ml de Dichlorométhane et 0,1 ml de diméthylformamide. A cette solution, ont été ajoutés sous agitation magnétique à 0°C, 0,212 ml (1,937 mmol) de N,N-dimétyléthane-1,2-diamine, 0,844 g (4,4 mmol) d'EDC.HCl et 0,262 g (1,937 mmol) de HOBt. Le mélange a été agité à t.a. pendant 16 h. 30 ml d'eau ont été ajoutés au milieu réactionnel. La phase aqueuse a été extraite avec 30 ml de Dichlorométhane, la phase organique a été lavée avec 10 ml d'une solution aqueuse saturée en NaHCO₃, 10 ml d'eau et 10 ml d'une solution aqueuse saturée en NaCl puis séchée sur Na₂SO₄ qui a ensuite été éliminé par filtration et le filtrat obtenu a été concentré sous vide. 0,16 g (rendement = 30%) de 2-(2-(4-cyanophényl)-5-(furan-2-carbonyl)thiophén-3-yl)-N-(2-(diméthylamino)éthyl)acétamide ont été obtenus sous forme d'un solide blanc. LC-MS: m/z = non ionisé. RMN ¹H (300 MHz, DMSO) δ 8.07 (dd, J = 3.8, 1.2 Hz, 2H), 7.77 (s, 1H), 7.51 (d, J = 3.2 Hz, 1H), 6.79 (dd, J = 3.6, 1.7 Hz, 1H), 3.50 (s, 2H), 3.15 (t, J = 6.6 Hz, 2H), 2.31 (t, J = 6.6 Hz, 2H), 2.13 (s, 6H).

### Etape 3 : Préparation du 2-(2-(3-cyanophényl)-5-(furan-2-carbonyl)thiophén-3-yl)-N-(2-(diméthylamino)éthyl)acétamide (dérivé numéro 142)

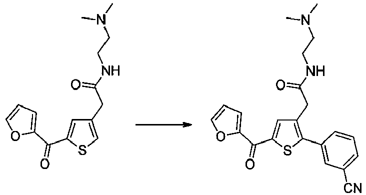

0,16 g (0,522 mmol) de 2-(2-(4-cyanophényl)-5-(furan-2-carbonyl)thiophén-3-yl)-N-(2-(diméthylamino) éthyl)acétamide ont été solubilisés dans 2 ml de DMSO sous agitation magnétique, ensuite 0,12 g (0,522 mmol) de 3-iodobenzonitrile, 0,061 g (1,044 mmol) de fluorure de potassium, 0,073 g (0,104 mmol) de Pd(Cl)₂(PPh₃)₂ et 0,177 g (1,044 mmol) de nitrate d'argent ont été additionnés. Le mélange a été agité à 130°C pendant 10 min. Après retour à t.a. le mélange a été versé dans 100 ml d'un mélange composé d'eau et de glace. La phase aqueuse a été extraite avec 2 x 50 ml d'acétate d'éthyle. Les phases organiques combinées ont été lavées avec 50 ml d'eau et 50 ml d'une solution aqueuse saturée en NaCl puis séchée sur MgSO₄ qui a ensuite été éliminé par filtration. Le filtrat obtenu a été concentré sous vide, repris dans 20 ml de Dichlorométhane et filtré sur gel de silice (éluant Dichlorométhane 100%). Le filtrat obtenu a été concentré sous vide et purifié par chromatographie flash sur cartouche de gel de silice (éluant : gradient d'éther de pétrole / éther diisopropylique, de 100% à 0% d'éther de pétrole, v/v). 45 mg (rendement = 21%) de 2-(2-(4-cyanophényl)-5-(furan-2-carbonyl)thiophén-3-yl)acétate d'éthyle ont été obtenus sous forme d'un solide jaune pâle. LC-MS: m/z = 408 (MH⁺) ; pureté UV à 254 nm = 99%. RMN ¹H (300 MHz, DMSO) δ 8.18 (s, 1H), 8.12 (d, J = 6.4 Hz, 2H), 7.94 (t, J = 7.7 Hz, 2H), 7.71 (t, J = 7.8 Hz, 1H), 7.57 (d, J = 3.6 Hz, 1H), 6.83 (dd, J = 3.6, 1.7 Hz, 1H), 3.18 (t, J = 6.6 Hz, 2H), 2.20 (s, 6H).

### Exemple 30 : Préparation du dérivé numéro 143 : 2-(2-(4-chloro-2-fluorophényl)-5-(furan-2-carbonyl)thiophén-3-yl)acétate d'isopropyle

### Etape 1 : Préparation du 2-(5 (furan-2-carbonyl)thiophén-3-yl)acétate d'isopropyle

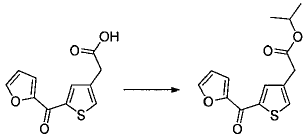

1 g (4,23 mmol) d'acide 2-(5-(furan-2-carbonyl)thiophén-3-yl)acétique ont été solubilisés dans 10 ml d'isopropanol, 1 goutte d'acide sulfurique a été ajoutée à cette solution. Le mélange a été chauffé sous agitation magnétique à reflux pendant 24 h. Après retour à t.a., le mélange a été concentré sous vide, le résidu brut a été repris dans 15 ml de Dichlorométhane, la phase organique a été lavée avec 10 ml d'une solution aqueuse saturée en NaHCO₃ et 10 ml d'eau puis séchées sur MgSO₄ qui a ensuite été éliminé par filtration. Le filtrat obtenu a été concentré sous vide pour donner 0,667 g (rendement = 53%) de 2-(5-(furan-2-carbonyl)thiophén-3-yl)acétate d'isopropyle sous forme d'une huile orangée. LC-MS: m/z = 279 (MH⁺) ; pureté UV à 254 nm=94%

### Etape 2: Préparation du 2-(2-(4-chloro-2-fluorophényl)-5-(furan-2-carbonyl)thiophén-3-yl)acétate d'isopropyle (dérivé numéro 143)

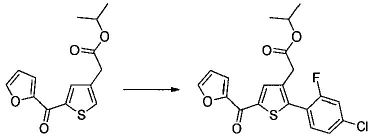

0,66 g (2,29 mmol) de 2-(5-(furan-2-carbonyl)thiophén-3-yl)acétate d'isopropyle ont été solubilisés dans 10 ml de DMSO sous agitation magnétique, ensuite 0,572 g (2,29 mmol) de 4-chloro-2-fluoro-1-iodobenzène, 0,259 g (4,46 mmol) de fluorure de potassium, 0,313 g (0,446 mmol) de Pd(Cl)₂(PPh₃)₂ et 0,757 g (4,46 mmol) de nitrate d'argent ont été additionnés. Le mélange a été agité à 130°C pendant 10 min. Après retour à t.a. le mélange a été versé dans 100 ml d'un mélange composé d'eau et de glace. La phase aqueuse a été extraite avec 2 x 50 ml d'acétate d'éthyle. Les phases organiques combinées ont été lavées avec 50 ml d'eau et 50 ml d'une solution aqueuse saturée en NaCl puis séchée sur MgSO₄ qui a ensuite été éliminé par filtration. Le filtrat obtenu a été concentré sous vide, repris dans 20 ml de Dichlorométhane et filtré sur gel de silice (éluant Dichlorométhane 100%). Le filtrat obtenu a été concentré sous vide et purifié par chromatographie flash sur cartouche de gel de silice (éluant : gradient d'éther de pétrole / Dichlorométhane, de 100% à 0% gradient d'éther de pétrole, v/v). 135 mg (rendement = 21%) de 2-(2-(4-chloro-2-fluorophényl)-5-(furan-2-carbonyl)thiophén-3-yl)acétate d'isopropyle ont été obtenus sous forme d'un solide jaune pâle. LC-MS: m/z = 407 (MH⁺) ; pureté UV à 254 nm = 97%. RMN ¹H (300 MHz, DMSO) δ 8.18 (d, J = 16.2 Hz, 2H), 7.82 - 7.29 (m, 5H), 6.85 (s, 1H), 4.83 (dt, J = 12.2, 6.0 Hz, 1H), 3.64 (s, 2H), 1.12 (d, J = 6.1 Hz, 6H).

### Exemple 31 : Préparation du dérivé numéro 144 : acide 2-(2-(4-chloro-2-fluorophényl)-5-(furan-2-carbonyl)thiophén-3-yl)acétique

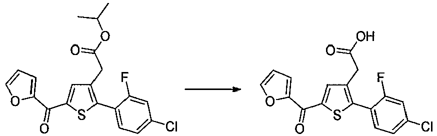

0,115 g (0,283 mmol) de 2-(2-(4-chloro-2-fluorophényl)-5-(furan-2-carbonyl)thiophén-3-yl)acétate d'isopropyle ont été solubilisés dans 3 ml d'isopropanol, à cette solution ont été ajoutés sous agitation magnétique 0,028 ml (0,283 mmol) d'une solution aqueuse d'hydroxyde de sodium 10N. Le mélange obtenu a été agité à t.a. pendant 24 h. Le mélange a été concentré sous vide, le résidu obtenu a été repris dans 15 ml d'eau, la phase aqueuse a été lavée avec 15 ml d'éther méthyle tert-butylique, puis acidifiée jusqu'à pH = 1-2 par addition d'une solution aqueuse d'acide chlorhydrique 4N. La phase aqueuse a été extraite par 2 x 10 ml d'acétate d'éthyle, les phases organiques combinées ont été lavées avec 15 ml d'eau et 15 ml d'une solution aqueuse saturée en NaCl puis séchées sur MgSO₄ qui a ensuite été éliminé par filtration. Le résidu a repris dans 10 ml d'eau, agité à t.a. pendant 1 h, un solide obtenu a été isolé par filtration et lavé à l'eau pour donner 71 mg (rendement = 63%) d'acide 2-(2-(4-chloro-2-fluorophényl)-5-(furan-2-carbonyl)thiophén-3-yl)acétique. LC-MS: m/z = 365 (MH⁺) ; pureté UV à 254 nm = 91%. RMN ¹H (300 MHz, DMSO) δ 12.54 (s, 1H), 8.18 (d, J = 14.1 Hz, 2H), 7.68 (d, J = 10.0 Hz, 1H), 7.58 (dd, J = 13.0, 5.8 Hz, 2H), 7.46 (d, J = 10.3 Hz, 1H), 6.91 - 6.75 (m, 1H), 3.57 (s, 2H).

### Exemple 32: Préparation du dérivé numéro 145: 2-(2-(4-fluorophényl)-5-(furan-2-carbonyl)thiophén-3-yl)acétonitrile Etape 1 : Préparation du 2-(4-fluorophényl)-3-méthylthiophène

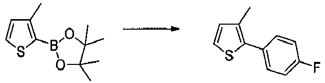

1 g (4,46 mmol) de 4,4,5,5-tetraméthyl-2-(3-méthylthiophén-2-yl)-1,3,2-dioxaborolane ont été solubilisés sous argon dans 15 ml de toluène, ont été ajoutés sous agitation magnétique, 0,781 g (4,46 mmol) de 1-bromo-4-fluorobenzène, 15,62 ml (31,2 mmol) d'une solution aqueuse de Na₂CO₃ 2 M et 0,26 g (0,223 mmol) de palladium[0] tetrakis(triphénylphosphine). Le mélange a été agité à reflux pendant 3 h. Après retour à t.a., le mélange a été filtré sur célite. La phase aqueuse a été extraite avec 20 ml d'acétate d'éthyle. La phase organique a été lavée avec 10 ml d'eau, 10 ml d'une solution aqueuse saturée en NaCl puis séchées sur MgSO₄ qui a ensuite été éliminé par filtration. Le filtrat obtenu a été concentré sous vide. Le résidu brut a été purifié par chromatographie flash sur cartouche de gel de silice (éluant : éther de pétrole 100%). 0,509 g (rendement = 51%) de 2-(4-fluorophényl)-3-méthylthiophène ont été obtenus sous forme d'huile et engagés directement dans l'étape suivante.

### Etape 2 : Préparation du (5-(4-fluorophényl)-4-méthylthiophén-2-yl)(furan-2-yl)méthanone

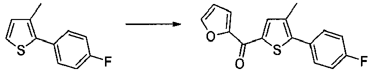

Dans un ballon placé sous un flux d'argon, ont été introduits sous agitation magnétique : 20 ml de Dichlorométhane, 0,5 g (2,237 mmol) de 2-(4-fluorophényl)-3-méthylthiophène, 0,265 ml (2,68 mmol) de chlorure de 2-furoyle Le mélange obtenu a été refroidi à 5°C avant d'ajouter par portions 0,328 g (2,46 mmol) de trichlorure d'aluminium. Le mélange a été agité à t.a. pendant 16 h avant d'être versé sur 150 ml d'un mélange composé d'eau et de Dichlorométhane (2/1, v/v) et d'être agité à t.a. pendant 30 min. Ce mélange a été filtré sur célite. Après séparation, la phase aqueuse a été extraite avec 50 ml de Dichlorométhane, les phases organiques combinées ont été lavées avec 50 ml d'eau puis séchées sur MgSO₄ qui a ensuite été éliminé par filtration. Le filtrat obtenu a été concentré sous vide. Le résidu brut a été purifié par chromatographie flash sur cartouche de gel de silice (éluant : gradient d'éther de pétrole / acétate d'éthyle, de 100% à 75% d'éther de pétrole, v/v). 0,58 g (rendement = 89%) de (5-(4-fluorophényl)-4-méthylthiophén-2-yl)(furan-2-yl)méthanone sous forme de mousse. LC-MS: m/z = 287 (MH⁺) ; pureté UV à 254 nm = 88%. RMN ¹H (300 MHz, DMSO) δ 8.13 (dd, J = 1.6, 0.7 Hz, 1H), 8.09 (s, 1H), 7.62 (ddd, J = 6.3, 4.2, 3.0 Hz, 3H), 7.36 (t, J = 8.9 Hz, 2H), 6.83 (dd, J = 3.6, 1.7 Hz, 1H), 2.34 (s, 3H).

### Etape 3 : Préparation du (4-(bromométhyl)-5-(4-fluorophényl)thiophén-2-yl)(furan-2-yl)méthanone

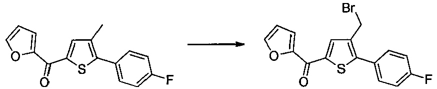

0,56 g (1,741 mmol) de (5-(4-fluorophényl)-4-méthylthiophén-2-yl)(furan-2-yl)méthanone ont été solubilisés dans 15 ml de tétrachlorure de carbone sous agitation magnétique, ont ensuite été additionnés 0,286 g (1,741 mmol) d'AIBN et 0,31 g (1,741 mmol) de N-Bromosuccinimide. Le mélange a été agité à reflux sous une lumière de 300 watts pendant 9 h au cours desquelles 0,043 g (0,26 mmol) d'AIBN et 0,047 g (0,26 mmol) de N-Bromosuccinimide ont été ajoutés à 3 et 6 h. Le milieu réactionnel a été concentré sous vide, le résidu obtenu a été repris dans 50 ml d'un mélange composé d'eau et de glace, la phase aqueuse a été extraite avec 3 x 50 ml d'acétate d'éthyle. Les phases organiques combinées ont été lavées avec 100 ml d'une solution aqueuse saturée en NaCl puis séchées sur MgSO₄ qui a ensuite été éliminé par filtration. Le filtrat obtenu a été concentré sous vide. Le résidu brut a été purifié par chromatographie flash sur cartouche de gel de silice (éluant : gradient d'éther de pétrole / éther diisopropylique, de 100% à 66% d'éther de pétrole, v/v). 0,308 g (rendement = 36%) de (4-(bromométhyl)-5-(4-fluorophényl)thiophén-2-yl)(furan-2-yl)méthanone sous forme de solide. LC-MS: m/z = 367 (MH⁺) ; pureté UV à 254 nm = 75%. RMN ¹H (300 MHz, DMSO) δ 7.98 (s, 1H), 7.89 (dd, J = 12.5, 8.0 Hz, 3H), 7.74 (d, J = 7.7 Hz, 1H), 7.67 (d, J = 5.2 Hz, 1H), 7.26 (d, J = 5.2 Hz, 1H), 4.70 (s, 2H), 1.74 - 1.51 (m, 6H), 1.43 (s, 3H).

### Etape 4 : Préparation du 2-(2-(4-fluorophényl)-5-(furan-2-carbonyl)thiophén-3-yl)acétonitrile (dérivé numéro 145)

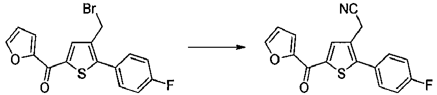

0,3 g (0,616 mmol) de (4-(bromométhyl)-5-(4-fluorophényl)thiophén-2-yl)(furan-2-yl)méthanone ont été solubilisés dans 10 ml de tétrahydrofurane sous agitation magnétique, ont ensuite été additionnés 0,044 g (0,678 mmol) de cyanure de potassium et 1 ml d'eau. Le mélange a été agité à reflux pendant 16 h. Le milieu réactionnel a été versé dans 15 ml d'eau, la phase aqueuse a été extraite avec 2 x 15 ml d'acétate d'éthyle, les phases organiques combinées ont été lavées avec 15 ml d'eau et 15 ml d'une solution aqueuse saturée en NaCl puis séchées sur MgSO₄ qui a ensuite été éliminé par filtration. Le filtrat obtenu a été concentré sous vide. Le résidu brut a été purifié par HLPC/MS préparative. 0,024 g (rendement = 11%) de 2-(2-(4-fluorophényl)-5-(furan-2-carbonyl)thiophén-3-yl)acétonitrile sous forme d'un solide jaune pâle. LC-MS: m/z = 312 (MH⁺) ; pureté UV à 254 nm = 94%. RMN ¹H (300 MHz, DMSO) δ. 8.25 (s, 1H), 8.18 (s, 1H), 7.74 - 7.57 (m, 3H), 7.41 (t, J = 8.8 Hz, 2H), 6.86 (dd, J = 3.6, 1.7 Hz, 1H), 4.13 (s, 2H).

### Exemple 33 : Préparation du dérivé numéro 146 : 2-(2-phényl-5-picolinoylthiophén-3-yl)acétonitrile

### Etape 1: Préparation de l'acide 5-bromo-4-méthylthiophène-2-carboxylique

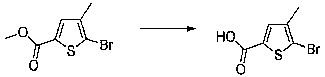

5 g (21,27 mmol) de 5-bromo-4-méthylthiophène-2-carboxylate de méthyle ont été solubilisés dans 250 ml d'éthanol, à cette solution ont été ajoutés sous agitation magnétique 106 ml (106 mmol) d'une solution aqueuse d'hydroxyde de sodium 1N. Le mélange obtenu a été agité à 80°C pendant 2 h. Le mélange a été concentré sous vide, le résidu obtenu a été repris dans 250 ml d'eau et 100 ml d'une solution aqueuse d'acide chlorhydrique 1N ont été additionnés. Un précipité a été isolé par filtration, lavé avec un minimum d'eau et 10 ml de pentane. Le solide blanc a été séché sous cloche à vide et 3,63 g (rendement = 76%) d'acide 5-bromo-4-méthylthiophène-2-carboxylique ont été obtenus. LC-MS: m/z = 219 (M-H⁺); pureté UV à 254 nm >99%. RMN ¹H (300 MHz, DMSO) δ 13.34 (s, 1H), 7.53 (s, 1H), 2.15 (s, 3H).

### Etape 2 : Réparation du 5-bromo-N-méthoxy-N,4-diméthylthiophène-2-carboxamide

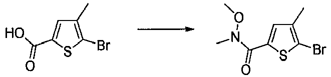

3,63 g (16,26 mmol) d'acide 5-bromo-4-méthylthiophène-2-carboxylique ont été solubilisés dans le Dichlorométhane sous agitation magnétique, ont ensuite été ajoutés 4,27 ml (48,8 mmol) de chlorure d'oxalyle et une goutte de diméthylformamide. Le mélange a été agité à t.a. pendant 2 h avant d'être concentré sous vide. Le résidu a été repris dans 75 ml d'un mélange Dichlorométhane / pyridine (2/1, v/v) puis 2,427 g (24,38 mmol) d'hydrochlorure de N,O-diméthylehydroxylamine ont été ajoutés. Le mélange a été agité à t.a. pendant 3 h avant d'être versé dans 300 ml d'eau. La phase aqueuse a été extraite avec 3 x 150 ml d'acétate d'éthyle. Les phases organiques combinées ont été lavées avec 2 x 150 ml d'une solution aqueuse d'acide chlorhydrique 1N, 150 ml d'eau et 150 ml d'une solution aqueuse saturée en NaCl puis séchées sur Na₂SO₄ qui a ensuite été éliminé par filtration. Le filtrat obtenu a été concentré sous vide. 4,32 g (rendement = 100%) de 5-bromo-N-méthoxy-N,4-diméthylthiophène-2-carboxamide ont été obtenus sous forme d'une huile marron. LC-MS: m/z = 265 (MH⁺) ; pureté UV à 254 nm >99%. RMN ¹H (300 MHz, DMSO) δ 7.64 (s, 1H), 3.76 (s, 3H), 3.26 (s, 3H), 2.17 (s, 3H).

### Etape 3 : Préparation du (5 bromo-4-méthylthiophène-2-yl)(pyridin-2-yl)méthanone

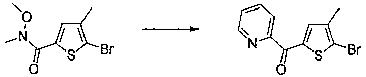

Dans un ballon placé sous argon, ont été introduits à 0°C : 25 ml de tétrahydrofurane, 0,407 ml (3,75 mmol) de 2-Iodopyridine et 3,75 ml (3,75 mmol) de bromure d'éthyle magnésium, le bain froid a été retiré et le mélange a été agité à t.a. pendant 30 min. Ensuite, une solution de 1 g (3,75 mmol) de 5-bromo-N-méthoxy-N,4-diméthylthiophène-2-carboxamide dans 25 ml de tétrahydrofurane a été ajoutée goutte à goutte. Le mélange a été agité à t.a. pendant 3 h avant d'être versé dans 75 ml d'une solution aqueuse saturée de chlorure d'ammonium. La phase organique a été extraite avec 3 x 100 ml d'acétate d'éthyle, 100 ml d'une solution aqueuse saturée en NaHCO₃, 100 ml d'eau, 100 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient d'heptane / acétate d'éthyle, de 95% à 75% d'heptane, v/v). 0,357 g (rendement = 33%) de (5-bromo-4-méthylthiophén-2-yl)(pyridin-2-yl)méthanone ont été obtenus sous forme d'un solide blanc. LC-MS: m/z = 283 (MH⁺) ; pureté UV à 254 nm >99%. RMN ¹H (300 MHz, DMSO) δ 8.79 (s, 1H), 8.08 (d, J = 26.9 Hz, 3H), 7.73 (s, 1H), 2.22 (s, 3H).

### Etape 4 : Préparation du (4-méthyl-5-phénylthiophén-2-yl)(pyridin-2-yl)méthanone

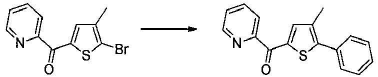

Dans un réacteur adapté aux micro-ondes, ont été introduits sous agitation magnétique : 3 ml d'un mélange composé de diméthyle éther, d'éthanol et d'eau (1/1/1, v/v/v), 0,357 g (1,253 mmol) de 5-bromo-4-méthylthiophén-2-yl)(pyridin-2-yl)méthanone, 0,816 g (2,505 mmol) de carbonate de césium et 0,229 g (1,879 mmol) d'acide phénylboronique. Le mélange a été irradié à 150°C pendant 15 min. Une fois revenu à t.a., le milieu réactionnel a été extrait avec 3 x 10 ml d'acétate d'éthyle. Les phases organiques combinées ont été lavées avec 10 ml d'une solution aqueuse saturée en NaCl, puis séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient d'heptane / acétate d'éthyle, de 100% à 95% d'heptane, v/v). 0,321 g (rendement = 91%) de (4-méthyl-5-phénylthiophén-2-yl)(pyridin-2-yl)méthanone ont été obtenus sous forme d'un solide jaune pâle. LC-MS: m/z = 280 (MH⁺) ; pureté UV à 254 nm >99%. RMN ¹H (300 MHz, DMSO) δ. 8.85 - 8.77 (m, 1H), 8.19 (s, 1H), 8.17 - 8.02 (m, 2H), 7.78 - 7.67 (m, 1H), 7.65 - 7.39 (m, 5H), 2.35 (s, 3H).

### Etape 5: Préparation du (4-(bromométhyl)-5-phénylthiophén-2-yl)(pyridin-2-yl)méthanone

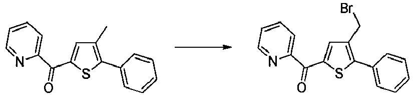

0,157 g (0,556 mmol) de (4-méthyl-5-phénylthiophén-2-yl)(pyridin-2-yl)méthanone ont été solubilisés dans 4,5 ml de tétrachlorure de carbone sous agitation magnétique, ont ensuite été additionnés 0,091 g (0,556 mmol) d'AIBN et 0,099 g (0,556 mmol) de N-Bromosuccinimide. Le mélange a été agité à reflux 6 h. Le milieu réactionnel a été versé dans 30 ml d'un mélange composé d'eau et de glace, la phase aqueuse a été extraite avec 3 x 20 ml d'acétate d'éthyle. Les phases organiques combinées ont été lavées avec 50 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : heptane / acétate d'éthyle, 95% d'heptane, v/v). 0,199 g (rendement = 56%) de (4-(bromométhyl)-5-phénylthiophén-2-yl)(pyridin-2-yl)méthanone ont été obtenus sous forme d'un solide jaune pâle. LC-MS: m/z = 359 (MH⁺) ; pureté UV à 254 nm = 82%. RMN ¹H (300 MHz, DMSO) δ 8.89 - 8.74 (m, 1H), 8.23 - 7.98 (m, 4H), 7.82 - 7.34 (m, 5H), 4.76 (s, 2H).

### Etape 6: Préparation du 2-(2-phényl-5-picolinoylthiophén-3-yl)acétonitrile (dérivé numéro 146)

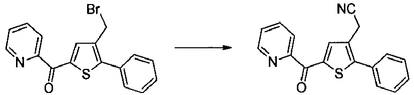

0,135 g (0,309 mmol) de (4-(bromométhyl)-5-phénylthiophén-2-yl)(pyridin-2-yl)méthanone ont été solubilisés dans 6 ml d'éthanol sous agitation magnétique, ont ensuite été additionnés 0,024 g (0,361 mmol) de cyanure de potassium et 0,5 ml d'eau. Le mélange a été agité à t.a. pendant 2 h avant d'être agité à 55°C pendant 90 min. Le milieu réactionnel a été concentré sous vide et le résidu obtenu a été repris dans 20 ml d'un mélange composé d'eau et d'acétate d'éthyle (1/1, v/v). Après séparation, la phase aqueuse a été extraite avec 2 x 15 ml d'acétate d'éthyle, les phases organiques combinées ont été lavées avec 2 x 15 ml d'eau puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : gradient d'heptane / acétate d'éthyle, de 95% à 90% d'heptane, v/v). 0,033 g (rendement = 36%) de 2-(2-phényl-5-picolinoylthiophén-3-yl)acétonitrile ont été obtenus sous forme d'un solide blanc. LC-MS: m/z = 305 (MH⁺) ; pureté UV à 254 nm = 99%. RMN ¹H (300 MHz, DMSO) δ 8.91 - 8.76 (m, 1H), 8.36 (s, 1H), 8.25 - 8.03 (m, 2H), 7.75 (ddd, *J* = 7.2, 4.7, 1.6 Hz, 1H), 7.66 - 7.41 (m, 5H), 4.14 (s, 2H).

Le compose **147** a été préparé selon le même enchainement d'étapes 1 à 6.

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **147** | 338,81 | solide | 98 | 339 | | 8.88 - 8.77 (m, 1H), 8.34 (s, 1H), 8.14 (ddd, J = 9.0, 4.3, 1.3 Hz, 2H), 7.83 - 7.71 (m, 1H), 7.63 (s, 4H), 4.16 (s, 2H). |

### Exemple 34 : Préparation du dérivé numéro 148 : Préparation du 2-(2-(4-chlorophényl)-5-picolinoylthiophén-3-yl)acétate d'éthyle

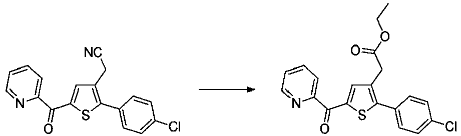

0,2 g (0,578 mmol) de 2-(2-(4-chlorophényl)-5-picolinoylthiophén-3-yl)acétonitrile ont été solubilisés sous agitation magnétique dans 5 ml de méthanol, une suspension blanchâtre a été obtenue et 0,1 ml (1,876 mmol) d'acide sulfurique ont été additionnés. Le mélange a été agité à reflux pendant 8 j. Le milieu réactionnel a été concentré sous vide et le résidu obtenu a été repris dans 20 ml d'un mélange composé d'eau et d'acétate d'éthyle (1/1, v/v). Après séparation, la phase aqueuse a été extraite avec 2 x 15 ml d'acétate d'éthyle, les phases organiques combinées ont été lavées avec 20 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : gradient d'heptane / acétate d'éthyle, de 85% à 70% d'heptane, v/v). 0,215 g (rendement = 60%) de 2-(2-(4-chlorophényl)-5-picolinoylthiophén-3-yl)acétate d'éthyle ont été obtenus sous forme d'un solide blanc. LC-MS: m/z = 372 (MH⁺) ; pureté UV à 254 nm = 99%. RMN ¹H (300 MHz, DMSO) δ 8.86 - 8.77 (m, 1H), 8.26 (s, 1H), 8.19 - 8.04 (m, 2H), 7.73 (s, 1H), 7.58 (d, *J* = 3.0 Hz, 4H), 3.80 (s, 2H), 3.61 (s, 3H).

### Exemple 35 : Préparation du dérivé numéro 149 : Préparation de l'acide 2-(2-(4-chlorophényl)-5-picolinoylthiophén-3-yl)acétique

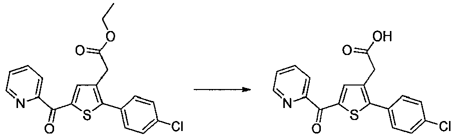

0,099 g (0,264 mmol) de 2-(2-(4-chlorophényl)-5-picolinoylthiophén-3-yl)acétate d'éthyle ont été solubilisés dans 6 ml de méthanol sous agitation magnétique. A cette solution, ont été additionnés 0,527 ml (0,527 mmol) d'une solution aqueuse de NaOH 1N. Le mélange a été agité à t.a. pendant 16 h avant d'être concentré sous vide. Le résidu a été repris dans 5 ml d'eau et une solution aqueuse d'acide chlorhydrique 1N a été additionnée jusqu'à apparition d'un précipité qui a été isolé par filtration et séché sous cloche à vide. 0,07 g (rendement = 73%) d'acide 2-(2-(4-chlorophényl)-5-picolinoylthiophén-3-yl)acétique ont été obtenus. LC-MS: m/z = 358 (M-H⁺) ; pureté UV à 254 nm = 99%. RMN ¹H (300 MHz, DMSO) δ 12.57 (s, 1H), 8.81 (d, *J* = 4.4 Hz, 1H), 8.27 (s, 1H), 8.19 - 8.04 (m, 2H), 7.73 (m, 1H), 7.59 (m, 4H), 3.68 (s, 2H).

### Exemple 36 : Préparation du dérivé numéro 150 : (2-(4-chlorophényl)-5-(furan-2-carbonyl)thiophén-3-yl)acétonitrile

### Etape 1: Préparation du 2-(2-bromothiophén-3-yl)acétonitrile

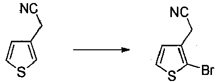

5,9 g (46 mmol) de 2-(thiophén-3-yl)acétonitrile ont été solubilisés dans 25 ml de tétrachlorure de carbone sous agitation magnétique et 8,27 g (46 mmol) de N-bromosuccinimide ont été additionnés. Sous agitation vigoureuse, 0,04 ml (0,460 mmol) d'acide perchlorique ont été ajoutés et le mélange a été agité à t.a. pendant 4,5 h. 0,155 g (1,839 mmol) de bicarbonate de sodium ont été ajoutés au milieu réactionnel. Le solide a été éliminé par filtration et le filtrat concentré sous vide. Le résidu brut a été purifié par chromatographie flash sur cartouche de gel de silice (éluant : heptane / acétate d'éthyle, 9/1, v/v). 8,47 g (rendement = 57%) de 2-(2-bromothiophén-3-yl)acétonitrile ont été obtenus sous forme d'une huile jaune claire. LC-MS: m/z = non ionisé. RMN ¹H (300 MHz, DMSO) δ ¹H NMR (300 MHz, DMSO) δ 7.67 (d, *J* = 5.6 Hz, 1H), 7.08 (d, *J* = 5.6 Hz, 1H), 3.93 (s, 2H).

### Etape 2 : Préparation du 2-(2-bromo-5-(furan-2-carbonyl)thiophén-3-yl)acétonitrile

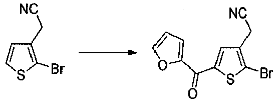

Dans un ballon placé sous un flux d'argon, ont été introduits sous agitation magnétique : 4 ml de 1,2-dichloroéthane, 0,2 g (0,99 mmol) de 2-(2-bromothiophén-3-yl)acétonitrile et 0,145 g (1,089 mmol) de trichlorure d'aluminium. Le mélange a été placé à 0°C sous agitation magnétique et une solution de 0,113 ml (1,089 mmol) de chlorure de 2-furoyle dans 1 ml de 1,2-dichloroéthane a été additionnée goutte à goutte. Le mélange obtenu a été agité à t.a. pendant 4 j puis 0,073 g (0,455 mmol) de trichlorure d'aluminium et 0,057 ml (0,455 mmol) de chlorure de 2-furoyle ont été ajoutés et l'agitation à t.a. a été poursuivie 24 h. Le milieu réactionnel a été versé dans 30 ml d'eau. La phase aqueuse a été extraite avec 2 x 25 ml d'acétate d'éthyle. Les phases organiques combinées ont été lavées avec 30 ml d'une solution aqueuse saturée en NaHCO₃, 30 ml d'une solution aqueuse saturée en NaCl séchées sur Na₂SO₄ qui a ensuite été éliminé par filtration et le filtrat obtenu a été concentré sous vide. 0,211 g (rendement = 71%) de 2-(2-bromo-5-(furan-2-carbonyl)thiophén-3-yl)acétonitrile ont été obtenu sous forme d'un solide jaune. LC-MS: m/z = 296 (MH⁺) ; pureté UV à 254 nm >99%. RMN ¹H (300 MHz, DMSO) δ ¹H NMR (300 MHz, DMSO) δ 8.17 (d, *J* = 0.9 Hz, 1H), 8.14 (s, 1H), 7.61 (d, *J* = 3.7 Hz, 1H), 6.85 (dd, *J* = 3.6, 1.7 Hz, 1H), 4.05 (s, 2H).

### Etape 3: Préparation du 2-(2-(4-chlorophényl)-5 (furan-2-carbonyl)thiophén-3-yl)acétonitrile (dérivé numéro 150)

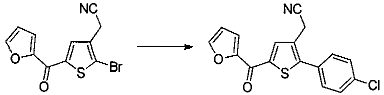

0,207 g (0,699 mmol) de 2-(2-bromo-5-(furan-2-carbonyl)thiophén-3-yl)acétonitrile ont été solubilisés sous argon dans 6 ml d'un mélange composé de toluène et d'éthanol (7/5, v/v), ont été ajoutés sous agitation magnétique, 0,173 g (1,048 mmol) d'acide 4-chlorophénylboronique, 2,342 ml (4,68 mmol) d'une solution aqueuse de Na₂CO₃ 2 M et 0,040 g (0,035 mmol) de palladium[0] tetrakis(triphénylphosphine). Le mélange a été agité à reflux pendant 2,5 h. Après retour à t.a., le mélange a été dilué avec 30 ml d'eau. La phase aqueuse a été extraite avec 2 x 30 ml d'acétate d'éthyle. Les phases organiques combinées ont été lavées avec 10 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : heptane / acétate d'éthyle, 8/2, v/v). 0,169 g (rendement = 72%) de 2-(2-(4-chlorophényl)-5-(furan-2-carbonyl) thiophén-3-yl)acétonitrile ont été obtenus sous forme d'un solide jaune pâle. LC-MS: m/z = 328 (MH⁺) ; pureté UV à 254 nm = 98%. RMN ¹H (300 MHz, DMSO) δ 8.25 (s, 1H), 8.19 (s, 1H), 7.63 (d, J = 3.2 Hz, 5H), 6.86 (dd, J = 3.6,1.7 Hz, 1H), 4.16 (s, 2H).

### Exemple 37 : Préparation du dérivé numéro 151 : Préparation du (4-((2H-tétrazol-5-yl)méthyl)-5-(4-chlorophényl)thiophén-2-yl)(furan-2-yl)méthanone

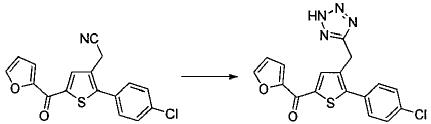

0,149 g (0,45 mmol) de 2-(2-(4-chlorophényl)-5-(furan-2-carbonyl)thiophén-3-yl)acétonitrile ont été solubilisés dans 3 ml de diméthylformamide sous agitation magnétique. A cette solution, ont été ajoutés : 0,088 g (1,35 mmol) d'azoture de sodium et 0,096 g (1,8 mmol) de chlorure d'ammonium. Le mélange a été agité à 160°C pendant 5 h. Après retour à t.a., le milieu réactionnel a été dilué avec 5 ml d'eau, le pH a été ajusté à 2 par addition d'une solution aqueuse d'acide chlorhydrique 1N. La phase aqueuse a été extraite avec 3 x 15 ml d'acétate d'éthyle. Les phases organiques combinées ont été lavées avec 30 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : gradient de Dichlorométhane / méthanol, de 100 à 95% de Dichlorométhane, v/v). 0,015 g (rendement = 8%) de (4-((2H-tétrazol-5-yl)méthyl)-5-(4-chlorophényl)thiophén-2-yl)(furan-2-yl)méthanone ont été obtenus sous forme d'un solide jaune pâle. LC-MS: m/z = 370 (MH⁺) ; pureté UV à 254 nm = 97%. RMN ¹H (300 MHz, DMSO) δ 12.12 (s, 1H), 7.94 (s, 1H), 7.60 (q, *J* = 8.6 Hz, 4H), 7.20 (d, *J* = 16.2 Hz, 2H), 6.42 - 6.22 (m, 1H), 4.35 (s, 2H).

### Exemple 38 : Préparation du dérivé numéro 152 : Préparation du 2-(2-(4-chlorophényl)-5-(furan-2-carbonyl)thiophén-3-yl)-N-hydroxyacétimidamide

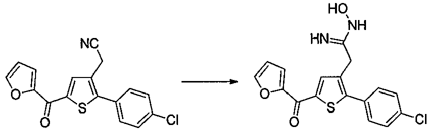

0,1 g (0,299 mmol) de 2-(2-(4-chlorophényl)-5-(furan-2-carbonyl)thiophén-3-yl)acétonitrile ont été solubilisés dans 3 ml de méthanol sous agitation magnétique. A cette solution, ont été ajoutés 0,028 g (0,404 mmol) d'hydrochlorure d'hydroxylamine et 0,043 g (0,314 mmol) de carbonate de potassium. Le mélange a été agité à t.a. pendant 1 h, puis à reflux pendant 5 h. Le milieu réactionnel a été versé dans 20 ml d'eau. La phase aqueuse a été extraite avec 2 x 20 ml d'acétate d'éthyle. Les phases organiques combinées ont été lavées avec 30 ml d'une solution aqueuse saturée en NaCl puis séchées sur Na₂SO₄ qui a ensuite été éliminé par filtration. Le filtrat obtenu a été concentré sous vide. Le résidu brut a été purifié par chromatographie flash sur cartouche de gel de silice (éluant : heptane / acétate d'éthyle, 4/6, v/v). 0,023 g (rendement = 21%) de 2-(2-(4-chlorophényl)-5-(furan-2-carbonyl) thiophén-3-yl)-N-hydroxy acétimidamide ont été obtenus sous forme d'un solide jaune pâle. LC-MS: m/z = 361 (MH⁺) ; pureté UV à 254 nm >99%. RMN ¹H (300 MHz, DMSO) δ 9.11 (s, 1H), 8.19 (s, 1H), 8.14 (s, 1H), 7.69 (d, J = 8.5 Hz, 2H), 7.65 - 7.50 (m, 3H), 6.84 (dd, J = 3.5, 1.6 Hz, 1H), 5.66 (s, 2H), 3.39 (s, 2H).

### Exemple 39 : Préparation du dérivé numéro 153 : Préparation du 3-((2-(4-chlorophényl)-5-(furan-2-carbonyl)thiophén-3-yl)méthyl)-1,2,4-oxadiazol-5(2H)-one

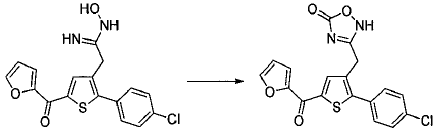

0,05 g (0,139 mmol) de 2-(2-(4-chlorophényl)-5-(furan-2-carbonyl)thiophén-3-yl)-N-hydroxyacétimidamide ont été solubilisés dans 1 ml de 1,4-dioxane sous agitation magnétique. A cette solution, ont été ajoutés : 0,028 g (0,173 mmol) de carbonyle diimidazole et 0,023 ml (0,152 ml) de DBU. Le mélange a été agité à 105°C pendant 30 min. Le milieu réactionnel a été versé dans 10 ml d'eau. La phase aqueuse a été lavée avec 5 ml d'acétate d'éthyle, le pH de la phase aqueuse a été ajusté à 2 par addition d'une solution aqueuse d'acide chlorhydrique 1N. La phase aqueuse a été extraite avec 2 x 10 ml d'acétate d'éthyle. Les phases organiques combinées ont été lavées avec 10 ml d'une solution aqueuse saturée en NaCl puis séchées sur Na₂SO₄ qui a ensuite été éliminé par filtration. 0,02 g (rendement = 36%) de 3-((2-(4-chlorophényl)-5-(furan-2-carbonyl) thiophén-3-yl)méthyl)-1,2,4-oxadiazol-5(2H)-one ont été obtenus sous forme d'un solide jaune pâle. LC-MS: m/z = 387 (MH⁺) ; pureté UV à 254 nm = 96%. RMN ¹H (300 MHz, DMSO) δ 12.40 (s, 1H), 8.19 (s, 1H), 8.16 (d, J = 1.1 Hz, 1H), 7.66 - 7.57 (m, 5H), 6.85 (dd, J = 3.6, 1.7 Hz, 1H), 3.99 (s, 2H).

### Exemple 40 : Préparation du dérivé N°173 : 5-(4-chlorophényl)-4-[2-(2-diméthylaminoéthylamino)-2-oxo-éthyl]-N-(3-pyridyl)thiophène-2-carboxamide

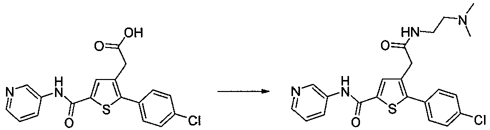

80 mg (0,215 mmol) d'acide 2-[2-(4-chlorophényl)-5-(3-pyridylcarbamoyl)-3-thiényl]acétique et 0,049 mL (0,429 mmol) de N,N-diméthyléthane-1,2-diamine ont été solubilisés dans 2 ml de DMPU. A cette solution, ont été ajoutés sous agitation magnétique, 0,045 mL (0,322 mol) de triéthylamine et 112 mg (0,215 mmol) de PyBOP. Le mélange a été agité à t.a. pendant 4 heures. 10 mL d'eau a été ajouté, l'agitation a été maintenue pendant 30 mn, et le solide obtenu a été filtré sur fritté, lavé avec de l'eau et séché sous vide. 0,053 g (rendement = 53%) de 5-(4-chlorophényl)-4-[2-(2-diméthylamino éthylamino)-2-oxo-éthyl]-N-(3-pyridyl)thiophène-2-carboxamide ont été obtenus sous forme d'un solide. LC-MS: m/z = 443 (MH+), pureté UV à 254 nm = 95%. RMN 1H (300 MHz, DMSO) δ 10.58 (s, 1H), 8.90 (s, 1H), 8.32 (d, J = 4.6 Hz, 1H), 8.16 (d, J = 8.5 Hz, 2H), 8.02 (s, 1H), 7.59 (q, J = 8.6 Hz, 4H), 7.40 (d, J = 12.9 Hz, 1H), 3.51 (s, 2H), 2.31 (s, 6H)

### Exemple 41: Préparation du dérivé N°174 : 2-[2-(3,4-dichlorophényl)-5-(4-méthoxyphényl)sulfonyl-3-thiényl]acétate d'éthyle

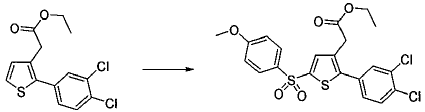

Dans un ballon placé sous un flux d'argon, ont été introduits sous agitation magnétique : 2 ml de Dichlorométhane, 0,1 g (0,317 mmol) de 2-(2-(3,4-dichlorophényl)thiophén-3-yl)acétate d'éthyle et 0,131 g (0,634 mmol) de chlorure de 4-méthoxybenzen-1-sulfonyl. Le mélange a été placé à 5°C sous agitation magnétique et 0,085 g (0,634 mmol) de chlorure d'aluminium ont été additionnés par portions. Le mélange obtenu a été agité à t.a. pendant 24 heures puis versé sur de la glace et agité pendant 1 heure. La phase aqueuse a été extraite avec 2 x 5 ml de Dichlorométhane. Les phases organiques combinées ont été séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient de éther de pétrole / Dichlorométhane, 100% à 0% de éther de pétrole, v/v). 0,120 g (rendement = 76%) de 2-[2-(3,4-dichlorophényl)-5-(4-méthoxyphényl)sulfonyl-3-thiényl]acétate d'éthyle ont été obtenu sous forme d'une huile marron claire. LC-MS: m/z = 486 (MH⁺) pureté UV à 254 nm = 98%. RMN 1H (300 MHz, DMSO) δ 7.93 (d, J = 8.9 Hz, 2H), 7.82 - 7.72 (m, 3H), 7.45 (dd, J = 8.3, 2.1 Hz, 1H), 7.18 (d, J = 9.0 Hz, 2H), 4.01 (q, J = 7.1 Hz, 2H), 3.85 (s, 3H), 3.74 (s, 2H), 1.10 (t, J = 7.1 Hz, 3H)

### Exemple 42: Préparation du dérivé N°175: acide 2-[2-(3,4-dichlorophényl)-5-(4-méthoxyphényl)sulfonyl-3-thiényl]acétique

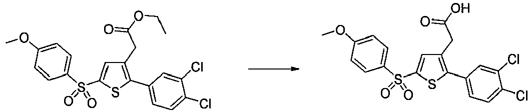

0,090 g (0,185 mmol) de 2-[2-(3,4-dichlorophényl)-5-(4-méthoxyphényl)sulfonyl-3-thiényl]acétate d'éthyle ont été solubilisés dans 2 ml d'éthanol et 2 ml de THF, à cette solution ont été ajoutés sous agitation magnétique 0,020 ml (0,204 mmol) d'une solution aqueuse d'hydroxyde de sodium à 30% en masse. Le mélange obtenu a été agité à t.a. pendant 16 heures. Le mélange a été concentré sous vide, le résidu obtenu a été repris dans 10 ml d'eau. Le pH de la phase aqueuse a ensuite été abaissé par addition d'une solution aqueuse d'acide chlorhydrique 1N jusqu'à apparition d'un précipité. La solution a été agitée pendant 2h après avoir ajouté 1 ml d'éther diisopropylique. Le solide a été isolé par filtration, lavé avec 2 x 5 ml d'eau et 1 ml d'éther diisopropylique et séché sous cloche à vide pour donner 0,072 g (rendement = 84%) d'acide 2-[2-(3,4-dichlorophényl)-5-(4-méthoxyphényl)sulfonyl-3-thiényl]acétique sous forme d'un solide blanc. Pureté UV à 254 nm = 99%. RMN 1H (300 MHz, DMSO) δ 12.69 (s, 1H), 7.93 (d, J = 8.9 Hz, 2H), 7.76 (d, J = 9.6 Hz, 3H), 7.46 (dd, J = 8.3, 2.1 Hz, 1H), 7.18 (d, J = 8.9 Hz, 2H), 3.85 (s, 3H), 3.63 (s, 2H)

### Exemple 43 : Préparation du dérivé N°176 : 2-[5-(5-chlorofuran-2-carbonyl)-2-(4-chlorophényl)-3-thiényl]acétate d'éthyle

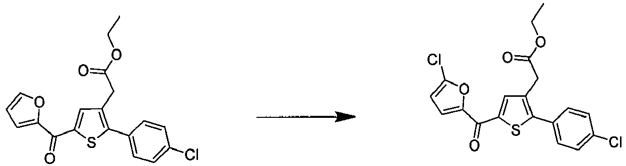

200 mg (0.534 mmol) de 2-[2-(4-chlorophényl)-5-(furan-2-carbonyl)-3-thiényl]acétate d'éthyle sont dissous dans 3 ml d'acétonitrile, avant addition de 142,4 mg (1,068 mmol) de N-chloro-succinimide. La réaction est portée à reflux pendant 15 heures. Le mélange est lavé par 5 ml d'eau, séché sur Na₂SO₄ qui a ensuite été éliminé par filtration et le filtrat obtenu a été concentré sous vide. Le résidu brut a été purifié par chromatographie flash sur cartouche de gel de silice (éluant : gradient Heptane / Dichlorométhane, 100% à 0% d'Heptane, v/v). 78 mg (rendement = 31%) de 2-[5-(5-chlorofuran-2-carbonyl)-2-(4-chlorophényl)-3-thiényl]acétate d'éthyle ont été obtenus sous forme d'une huile peu colorée. LC-MS: m/z = 409 (MH⁺) pureté UV à 254 nm = 99%. RMN 1H (300 MHz, DMSO) δ 8.14 (s, 1H), 7.69 (d, J = 3.7 Hz, 1H), 7.64 - 7.43 (m, 4H), 6.93 (d, J = 3.7 Hz, 1H), 4.11 - 4.00 (m, 2H), 3.82 (d, J = 12.5 Hz, 2H), 1.14 (t, J = 7.1 Hz, 3H)

### Exemple 44 : Préparation du dérivé N°177 : 2-[5-[[(4-chlorobenzoyl)amino]carbamoyl]-2-(4-chlorophényl)-3-thiényl] acétate d'éthyle

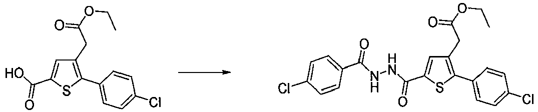

100 mg (0,308 mmol) d'acide 5-(4-chlorophényl)-4-(2-éthoxy-2-oxo-éthyl)thiophène-2-carboxylique, 0,172 ml (0,985 mmol) de triéthylamine et 52,5 mg (0,308 mmol) de 4-chlorobenzohydrazide sont chargés dans 2 ml de DMF. La solution est refroidie à 0°C et 240 mg (0,462 mmol) de PyBOP sont additionnés. Le mélange est agité à t.a. pendant 4 heures. La solution est versée sur 10 ml d'eau additionnée de 1 ml d'éther diisopropylique. Après 30 minutes d'agitation vigoureuse, le solide obtenu est filtré puis lavé avec de l'eau et de l'éther diisopropylique. 123 mg (rendement = 78%) de 2-[5-[[(4-chlorobenzoyl)amino]carbamoyl]-2-(4-chlorophényl)-3-thiényl] acétate d'éthyle ont été obtenu sous forme d'un solide. LC-MS: m/z = 477 (MH⁺) pureté UV à 254 nm = 93%. RMN 1H (300 MHz, DMSO) δ 10.68 (s, 2H), 7.93 (d, J = 8.5 Hz, 2H), 7.86 (s, 1H), 7.57 (dt, J = 17.0, 8.6 Hz, 6H), 4.09 (q, J = 7.1 Hz, 2H), 3.72 (s, 2H), 1.17 (t, J = 7.1 Hz, 3H)

Les dérivés **178** et **179** ont été préparés selon le même protocole :

| **N°** | **Poids moléculaire** | **Apparence** | **Pureté LCMS** | **Spectro métrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **178** | 472,94 | solide | 99 | 473 | 471 | 10.61 (d, J = 32.2 Hz, 2H), 7.87 (s, 1H), 7.66 - 7.35 (m, 7H), 7.17 (d, J = 7.8 Hz, 1H), 4.10 (q, J = 7.1 Hz, 2H), 3.82 (s, 3H), 3.72 (s, 2H), 1.18 (t, J = 7.1 Hz, 3H) |
| **179** | 443,90 | solide | 95 | 444 | 442 | 10.84 (d, J = 33.9 Hz, 2H), 8.80 (d, J = 6.0 Hz, 2H), 7.97 - 7.73 (m, 3H), 7.56 (q, J = 8.6 Hz, 4H), 4.09 (q, J = 7.1 Hz, 2H), 3.73 (s, 2H), 1.17 (t, J = 7.1 Hz, 3H) |

### Exemple 45 : Préparation du dérivé N°180 : 2-[2-(4-chlorophényl)-5-(furan-2-carbonyl)-3-thiényl]-N-éthyl-thioacétamide

### Étape 1 : Réparation de l'acide 2-[2-(4-chlorophényl)-3-thiényl]acétique

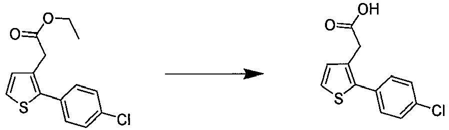

1,5 g (5,34 mmol) de 2-[2-(4-chlorophényl)-3-thiényl]acétate d'éthyle ont été chargés dans 8 ml d'éthanol avant addition de 0,588 ml (5,88 mmol) d'une solution aqueuse d'hydroxyde de sodium à 30% en masse. La solution a été agitée à t.a. pendant 18 heures. Le mélange a été concentré sous vide, le résidu obtenu a été repris dans 15 ml d'eau. Le pH de la phase aqueuse a ensuite été abaissé par addition d'une solution aqueuse d'acide chlorhydrique 1N jusqu'à apparition d'un précipité. Le solide a été isolé par filtration, lavé avec 2 x 5 ml d'eau et séché sous cloche à vide. 1,227g (rendement = 88%) d'acide 2-[2-(4-chlorophényl)-3-thiényl]acétique ont été obtenus sous forme d'un solide. LC-MS: m/z = 251 (MH⁻) pureté UV à 254 nm = 93%.

### Etape 2 : Préparation du 2-[2-(4-chlorophényl)-3-thiényl]-N-ethyl-acétamide

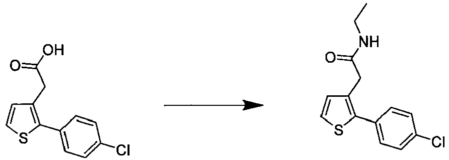

0,6 g (2,374 mmol) d'acide 2-[2-(4-chlorophényl)-3-thiényl]acétique, 0,290 g (3,55 mmol) de chlorhydrate d'éthanamine, 1,236 g (2,374 mmol) de PyBOP et 0,827 ml (5,94 mmol) de triéthylamine ont été chargés dans 2 ml de DMF. La solution a été agitée à t.a. pendant 18 heures. Le mélange a été concentré sous vide, le résidu obtenu a été repris dans 10 ml d'une solution aqueuse d'acide chlorhydrique 1N, puis agité pendant 1 heure. Le solide a été isolé par filtration, lavé avec 2 x 5 ml d'eau et séché sous cloche à vide. 0,559 g (rendement = 79%) de 2-[2-(4-chlorophényl)-3-thiényl]-N-éthyl-acétamide ont été obtenus sous forme d'un solide. LC-MS: m/z = 280 (MH⁺) pureté UV à 254 nm = 94%. RMN 1H (300 MHz, DMSO) δ 8.11 (s, 1H), 7.62 (d, J = 8.5 Hz, 2H), 7.57 - 7.46 (m, 3H), 7.08 (d, J = 5.2 Hz, 1H), 3.39 (s, 2H), 3.14 - 3.02 (m, 2H), 1.01 (t, J = 7.2 Hz, 3H).

### Etape 3 : Préparation de 2-[2-(4-chlorophényl)-3-thiényl]-N-éthyl-thioacétamide

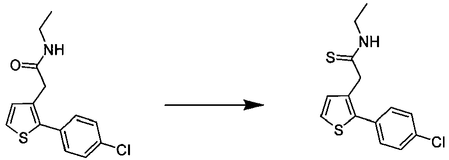

559 mg (1,998 mmol) de 2-[2-(4-chlorophényl)-3-thiényl]-N-éthyl-acétamide et 808 mg (1,998 mmol) de réactif de Lawesson ont été ajoutés à 5 ml de toluène. La solution a été agitée et chauffée à reflux pendant 4 heures. 10 ml d'eau a été additionnée, et le mélange a été extrait avec 2 fois 15 ml d'acétate d'éthyle. Les phases organiques réunies ont été lavées avec 10 ml d'eau et 10 ml de saumure, séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient Éther de pétrole / Éther diisopropylique, 100% à 0% d'éther de pétrole, v/v). 476 mg (rendement = 77%) ont été obtenus sous forme d'huile. LC-MS: m/z = 296 (MH⁺) pureté UV à 254 nm = 95%. RMN 1H (300 MHz, DMSO) δ 10.22 (s, 1H), 7.66 (d, J = 8.6 Hz, 2H), 7.56 - 7.47 (m, 3H), 7.02 (d, J = 5.2 Hz, 1H), 3.86 (s, 2H), 3.59 - 3.47 (m, 2H), 1.14 (t, J = 7.3 Hz, 3H).

### Etape 4 : Préparation de 2-[2-(4-chlorophényl)-5-(furan-2-carbonyl)-3-thiényl]-N-éthyl-thioacétamide (dérivé numéro 180)

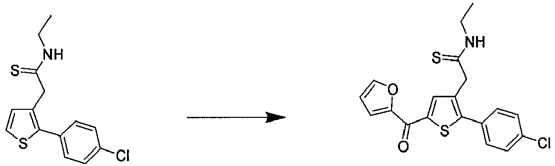

215 mg (0,727 mmol) de 2-[2-(4-chlorophényl)-3-thiényl]-N-éthyl-thioacétamide ont été dissous dans 3 ml de dichlorométhane. A +5°C, 0,107 ml (1,090 mmol) de chlorure de 2-furan-carbonyl ont été additionnés, puis, toujours à +5°C, 97 mg (0,727 mmol) de chlorure d'aluminium ont été ajoutés. La solution a été agitée à t.a. pendant 18 heures. Le mélange a été versé dans 200 ml d'eau, agitée pendant 3 heures et filtrée sur de la célite, puis la solution a été extraite 2 fois avec 15 ml de dichlorométhane. Les phases organiques réunies ont été lavées avec 10 ml d'eau, séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient Éther de pétrole / Dichlorométhane, 100% à 0% d'éther de pétrole, v/v). 51 mg (rendement = 17%) de 2-[2-(4-chlorophényl)-5-(furan-2-carbonyl)-3-thiényl]-N-éthyl-thioacétamide ont été obtenus sous forme d'un solide. LC-MS: m/z = 390 (MH⁺) pureté UV à 254 nm = 94%. RMN 1H (300 MHz, DMSO) δ 10.27 (s, 1H), 8.14 (d, J = 6.6 Hz, 2H), 7.70 (d, J = 8.6 Hz, 2H), 7.62 - 7.49 (m, 3H), 6.89 - 6.81 (m, 1H), 3.94 (s, 2H), 3.67 - 3.46 (m, 2H), 1.15 (t, J = 7.2 Hz, 3H).

### Exemple 46: Préparation du dérivé N°182 : N-(N-(tert-butoxycarbonyl)carbamimidoyl)-2-(2-(4-chlorophényl)-5-(4-méthoxybenzoyl)thiophén-3-yl)acétamide

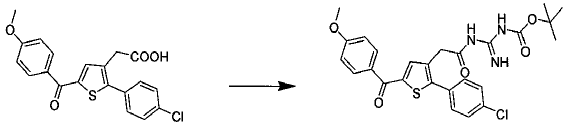

142 mg (0,337 mmol) d'acide 2-(2-(4-chlorophényl)-5-(4-méthoxybenzoyl)thiophén-3-yl)acétique, 175 mg (0,337 mmol) de PyBOP et 64,4 mg (0,404 mmol) de Boc-guanidine ont été ajoutés à 1 ml de DMF. 0,141 ml (1,011 mmol) de triéthylamine ont ensuite été additionnées. Le mélange a été agité à t.a. pendant 4 heures. La solution a été versée sur 10 ml d'eau et 5 ml d'éther diisopropylique sous forte agitation. Le solide obtenu a été filtré puis lavé avec de l'eau et de l'éther diisopropylique. 172 mg (rendement = 90%) de N-(N-(tert-butoxycarbonyl)carbamimidoyl)-2-(2-(4-chlorophényl)-5-(4-méthoxybenzoyl)thiophén-3-yl)acétamide ont été obtenus sous forme d'un solide blanc. LC-MS: m/z = 462 (MH⁺) pureté UV à 254 nm = 99%. RMN 1H (300 MHz, DMSO) δ 11.05 (s, 1H), 8.70 (s, 2H), 7.93 - 7.77 (m, 5H), 7.57 (d, J = 8.4 Hz, 1H), 7.13 (d, J = 8.8 Hz, 2H), 3.88 (s, 3H), 3.75 (s, 2H), 1.39 (s, 9H).

Les dérivés **181** et **183** ont été préparés selon le même protocole

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **181** | 487,96 | solide blanc | 99 | 388 [M+1-BOC] | 486 | 11.15 (s, 1H), 8.70 (s, 2H), 8.33 - 8.03 (m, 2H), 7.64 - 7.55 (m, 5H), 6.83 (dd, J = 3.6, 1.7 Hz, 1H), 3.75 (s, 2H), 1.39 (s, 9H) |
| **183** | 528,02 | solide jaune pâle | 95 | 428 [M+1-BOC] | 526 | 11.08 (s, 1H), 8.77 (d, J = 42.4 Hz, 2H), 7.94 - 7.82 (m, 2H), 7.76 (s, 1H), 7.59 (s, 4H), 7.19 - 7.03 (m, 2H), 3.86 (s, 3H), 3.72 (s, 2H), 1.38 (s, 9H) |

### Exemple 47 : Préparation du dérivé N°185 : Chlorhydrate de N-carbamimidoyl-2-(2-(4-chlorophényl)-5-(4-méthoxybenzoyl)thiophén-3-yl)acétamide

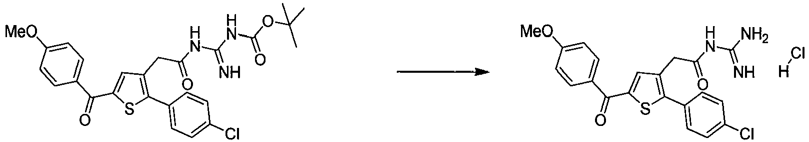

481 mg (0,911 mmol) de N-(N-(tert-butoxycarbonyl)carbamimidoyl)-2-(2-(4-chlorophényl)-5-(4-méthoxybenzoyl)thiophén-3-yl)acétamide ont été dissous dans 2 ml de Dichlorométhane puis 2 ml (26 mmol) d'acide trifluoroacétique a été ajouté. Le mélange a été agité à t.a. pendant 2 heures. 0,25 ml (1 mmol) d'une solution d'acide chlorhydrique à 4 mole par litre dans du dioxane a été additionné avant d'évaporer les solvants sous vide. Le solide obtenu a été trituré dans 5 ml de propan-2-ol bouillant, et après refroidissement de la solution, le solide a été filtré et lavé avec 1 ml de propan-2-ol puis séché sous vide. 421 mg (rendement = 99%) de chlorhydrate de N-carbamimidoyl-2-(2-(4-chlorophényl)-5-(4-méthoxybenzoyl)thiophén-3-yl)acétamide a été obtenu sous forme d'un solide jaune. LC-MS: m/z = 428 (MH⁺) pureté UV à 254 nm = 99%. RMN 1H (300 MHz, DMSO) δ 11.78 (s, 1H), 8.36 (s, 4H), 7.88 (d, J = 8.8 Hz, 2H), 7.81 (s, 1H), 7.66 - 7.45 (m, 4H), 7.12 (d, J = 8.9 Hz, 2H), 3.88 (d, J = 7.2 Hz, 5H).

Les dérivés **184** et **186** ont été préparés selon le même protocole

| **N°** | **Poids moléculaire** | **Apparence** | **Pureté LCMS** | **Spectrométrie de masse m/z** | | **RMN ¹H** |
|---|---|---|---|---|---|---|
| | **g/mol** | | **UV à 254 nm** | **MH⁺** | **M-H⁺** | **(300 MHz, DMSO) δ** |
| **184** | 498,80 | solide blanc | 97 | 462 | | 11.88 (s, 1H), 8.25 (d, J = 45.7 Hz, 4H), 7.95 - 7.81 (m, 4H), 7.78 (d, J = 8.3 Hz, 1H), 7.54 (dd, J = 8.3, 2.1 Hz, 1H), 7.12 (d, J = 8.9 Hz, 2H), 3.91 (s, 2H), 3.87 (s, 3H) |
| **186** | 424,30 | solide gris | 95 | 388 | 386 | 11.89 (s, 1H), 8.43 (s, 3H), 8.25 (s, 1H), 8.15 (d, J = 1.0 Hz, 1H), 7.59 (d, J = 5.9 Hz, 5H), 7.02 (s, 1H), 6.85 (dd, J = 3.6, 1.7 Hz, 1H), 3.93 (s, 2H) |

### Exemple 48 : Préparation du dérivé N°187: 2-(2-(4-chlorophényl)-5-(4-méthoxybenzoyl)thiophén-3-yl)acrylate d'éthyle

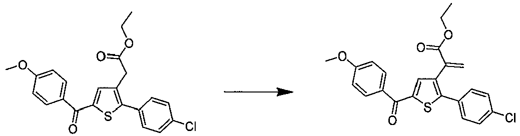

Sous une atmosphère d'argon, 1,5 g (3,62 mmol) de 2-(2-(4-chlorophényl)-5-(4-méthoxybenzoyl)thiophén-3-yl)acétate d'éthyle a été dissout dans 20 ml de tétrahydrofurane, et après refroidissement à -78°C, 10,85 ml (10,85 mmol) d'une solution de lithiumbis(triméthylsilyl)amide à une mole par litre dans du tétrahydrofurane a été coulée au goutte à goutte. La solution a été agitée à -78°C pendant 30 minutes avant l'addition lente de 0,984 ml (10,85 mmol) de bromo(méthoxy)méthane. Le mélange a été agité en laissant la température remonter à t.a. pendant 16 heures. Le mélange a été ensuite versé sur 50 ml d'eau, extrait avec 3 fractions de 15 ml de dichlorométhane, les phases organiques réunies ont été lavées avec 20 ml d'eau, séchées sur Na₂SO₄ qui a ensuite été éliminé par filtration et le filtrat obtenu a été concentré sous vide. Le résidu brut a été purifié par chromatographie flash sur cartouche de gel de silice (éluant : gradient Heptane / Acétate d'éthyle, 100% à 0% heptane, v/v). 0,696 g (rendement = 41%) de 2-(2-(4-chlorophényl)-5-(4-méthoxybenzoyl) thiophén-3-yl)acrylate d'éthyle ont été obtenus sous forme d'un solide. LC-MS: m/z = 427 (MH⁺) pureté UV à 254 nm = 90.6%. RMN 1H (300 MHz, DMSO) δ 7.93 (d, J = 8.9 Hz, 2H), 7.70 (s, 1H), 7.56 - 7.51 (m, 2H), 7.48 - 7.41 (m, 2H), 7.11 (d, J = 8.9 Hz, 2H), 6.39 (d, J = 0.8 Hz, 1H), 6.11 (d, J = 0.7 Hz, 1H), 3.93 - 3.78 (m, 5H), 0.91 (t, J = 7.1 Hz, 3H).

### Test de stimulation de la sécrétion d'insuline par des cellules INS-1

Les différents composés ont été testés sur la lignée béta pancréatique INS-1 pour évaluer leur capacité à potentialiser la sécrétion d'insuline en réponse au glucose. Très brièvement, les cellules sont cultivées dans du milieu de culture, RPMI 1640 10 mM glucose contenant 1 mM de pyruvate de sodium, 50 µM de 2-mercaptoéthanol, 2 mM de glutamine, 10 mM d'HEPES, 100 IU/mL de pénicilline, 100 µg/mL de streptomycine et 10% de sérum de veau foetal inactivé, comme décrit par Asfari et al. [13]. Pour le test de sécrétion d'insuline, les cellules INS-1 sont ensemencées et cultivées en plaque 96 puits. Après 3 jours de culture à 37°C dans une atmosphère humide (95% air/ 5% CO₂), le milieu est retiré et les cellules incubées pendant 16h dans du milieu contenant 5mM de glucose et 1% de sérum de veau foetal inactivé. Le jour du test, les cellules sont lavées avec un tampon Krebs (pH 7,4) contenant 0,1% d'albumine bovine puis pré-incubées pendant 30 min à 37°C dans ce même tampon contenant 2,8 mM de glucose. Enfin, les cellules sont lavées à nouveau avec du tampon Krebs puis incubées pendant 1 h dans le tampon du test de sécrétion (Krebs pH 7,4 contenant 0,1% d'albumine bovine et 3,5 mM de glucose et les molécules à évaluer). A la fin du test, le surnageant cellulaire est récupéré pour y mesurer l'insuline sécrétée à l'aide d'un kit ELISA utilisant un anticorps anti-insuline de rat (ELISA Alpco Cat n° 80-INSRTH-E10). Chaque condition est testée en triplicate. Le glucose 3,5 mM, le GLP-1 à 10⁻⁷M et le mélange Forskoline 10⁻⁷/ IBMX 10⁻⁵M sont utilisés comme contrôles positifs du test. Un composé stimule la sécrétion d'insuline si ce facteur est supérieur ou égal à 130% du contrôle pour une dose de glucose donnée.

| **N°** | **INS-1 % du ctrl @ 50 µM** | **INS-1 % du ctrl @ 10 µM** | **N°** | **INS-1 % du ctrl @ 50 µM** | **INS-1 % du ctrl @ 10 µM** |
|---|---|---|---|---|---|
| **3** | 318 | 152 | **104** | 203 | 153 |
| **10** | 210 | 144 | **106** | 151 | 148 |
| **13** | | 155 | **108** | | 135 |
| **14** | 165 | 152 | **114** | | 150 |
| **15** | 160 | 150 | **123** | 166 | 168 |
| **22** | 207 | 152 | **124** | 169 | 177 |
| **28** | 138 | 136 | **125** | 126 | 154 |
| **30** | | 157 | **130** | 323 | 179 |
| **33** | 280 | 199 | **136** | 158 | 120 |
| **35** | 148 | 128 | **139** | 247 | 183 |
| **37** | 107 | 157 | **140** | 188 | 123 |
| **39** | 389 | 143 | **142** | 175 | 117 |
| **44** | 223 | 180 | **143** | 233 | 127 |
| 52 | 140 | 127 | **145** | 145 | 125 |
| 84 | 153 | 139 | **148** | 189 | 134 |
| **85** | | 146 | **155** | 143 | |
| **89** | | 171 | **157** | 140 | |
| **91** | 152 | 184 | **158** | 201 | 132 |
| **93** | | 149 | **175** | 145 | 106 |
| **95** | 147 | 144 | **176** | 246 | 144 |
| **96** | | 177 | **187** | 120 | 152 |

Les dérivés de formule I testés ont donc un effet significatif sur la potentialisation de la sécrétion d'insuline en réponse au glucose par les cellules INS-1 β pancréatiques. Les valeurs sont comprises entre 132% et plus de 300% d'activation indépendamment de la dose considérée.

### Test d'inhibition de la production hépatique de glucose

Les hépatocytes sont isolés à partir de foie de rat Wistar à jeun depuis 24 h après perfusion de collagénase dans la veine porte. Les hépatocytes fraichement isolés sont ensemencés en plaques 6 puits coatées au collagène et contenant du milieu d'attachement (Milieu Williams). Après attachement, le milieu est remplacé par du milieu RPMI 1640 sans glucose contenant de l'hydrocortisone (7.10⁻⁵M) pour une durée de 16 à 18 h. Le jour suivant, le test de production hépatique de glucose est réalisé en milieu krebs pendant 3 h. Les conditions basales sont les cellules incubées avec du krebs seul, les conditions stimulées sont les cellules placées en Krebs+ lactate+ pyruvate, les conditions produits sont les cellules exposées aux composés chimiques dans un milieu krebs/lactate/pyruvate. Dans le cas où les composés sont dissous dans le DMSO, toutes les conditions du test sont réalisées en présence d'une concentration finale de 0,1% en DMSO. Le control positif du test est le mercaptopicolinate connu pour son action inhibitrice sur la production hépatique de glucose via la phosphoénolpyruvate carboxykinase. Pour les traitements à court terme, les composés sont incubés 3 h. Pour les traitements à long terme, les composés sont incubés 20 h au moment de la mise en culture des hépatocytes en RPMI puis de nouveau ajoutés lors du test de production hépatique pour 3 h. A la fin des 3 h d'incubation, le surnageant est récupéré pour la mesure de glucose par une méthode colorimétrique utilisant la glucose oxydase. Les cellules sont lysées par une solution aqueuse de NaOH à 0,1% afin de mesurer la quantité de protéine par la méthode Lowry. Les résultats sont exprimés en mmole de glucose par mg de protéine Un composé inhibe la production hépatique de glucose si ce facteur est inférieur ou égal à 75% du contrôle pour une dose de glucose donnée.

**Incubation 3 h**

| **N°** | **HGP % du ctrl @ 100 µM** | **HGP % du ctrl @ 50 µM** | **N°** | **HGP % du ctrl @ 100 µM** | **HGP % du ctrl @ 50 µM** |
|---|---|---|---|---|---|
| **2** | 62 | 83 | **82** | 26 | 67 |
| **3** | 74 | 82 | | | |
| **4** | 75 | 77 | **86** | 72 | 77 |
| **5** | 50 | 68 | **87** | 73 | 77 |
| **6** | 55 | 72 | **98** | 74 | 88 |
| **7** | 70 | 84 | **108** | 26 | 88 |
| **10** | 39 | 64 | **109** | 27 | 69 |
| **12** | 53 | 70 | **110** | 33 | 72 |
| **13** | 69 | 84 | **115** | 75 | 66 |
| **14** | 73 | 91 | **116** | 67 | 62 |
| **16** | 63 | 69 | **121** | 66 | 80 |
| **17** | 73 | 76 | **123** | 63 | 101 |
| **23** | 73 | 79 | **127** | 23 | 54 |
| **26** | 71 | 86 | **143** | 72 | 89 |
| **38** | 70 | 74 | **144** | 52 | 74 |
| **43** | 73 | 82 | **148** | 49 | 80 |
| **44** | 69 | 83 | **153** | 38 | 95 |
| **45** | 73 | 82 | **154** | 63 | 81 |
| **46** | 55 | 74 | **155** | 54 | 65 |
| **48** | 73 | 91 | **156** | 7 | 21 |
| **49** | 38 | 57 | **157** | 74 | 97 |
| **50** | 75 | 97 | **158** | | 59 |
| **51** | 54 | 90 | **159** | | 40 |
| **52** | 44 | 61 | **166** | 65 | 71 |
| **54** | 74 | 85 | **167** | 23 | 20 |
| **56** | 70 | 94 | **174** | 77 | 73 |
| **57** | 51 | 77 | **175** | 20 | 51 |
| **58** | 25 | 51 | **176** | 36 | 76 |
| **59** | 35 | 67 | **178** | 72 | 92 |
| **60** | 53 | 74 | **179** | 69 | 91 |
| **61** | 66 | 78 | **184** | 80 | 68 |
| **63** | 28 | 56 | **185** | 28 | 54 |
| **65** | 66 | 93 | **186** | 60 | 69 |
| **69** | 60 | 86 | **V** | 63 | 81 |
| **80** | 67 | 83 | **Y** | 15 | 26 |
| **81** | 54 | 106 | **Z** | 19 | 67 |

Les dérivés de formule I testés ont un effet significatif sur l'inhibition de la production hépatique de glucose. Les plus fortes inhibitions sont obtenues avec les dérivés **49, 58, 63, 127, 156, 167, 175, 185** et **Y** aux deux doses et les dérivés **158** et **159** à la dose de 50 µM.

**Incubation 20 h**

| **N°** | **HGP % du ctrl @ 100 µM** | **HGP % du ctrl @ 50 µM** | **N°** | **HGP % du ctrl @ 100 µM** | **HGP % du ctrl @ 50 µM** |
|---|---|---|---|---|---|
| **13** | 35 | 58 | **106** | 45 | 74 |
| **15** | 32 | 63 | **124** | 60 | 92 |
| **35** | 70 | 100 | **128** | 16 | 41 |
| **44** | 61 | 71 | **130** | 40 | 53 |
| **84** | 40 | 85 | **136** | 43 | 64 |
| **100** | 20 | 43 | **149** | 13 | 47 |
| **104** | 50 | 76 | | | |

Les dérivés de formule I testés ont un effet significatif sur l'inhibition de la production hépatique de glucose. Les plus fortes inhibitions sont obtenues aux deux doses avec les dérivés **13, 100, 128, 130** et **149.**

### Etude de l'effet des composés sur la sécrétion d'insuline en réponse au glucose au niveau de pancréas perfusés isolés de rats diabétiques N0STZ

### Matériel et méthode :

Le pancréas est prélevé sur des rats rendus diabétiques par injection de Streptozotocine le jour de la naissance [14] et anesthésiés au Pentobarbital (Nembutal^{©} : 45 mg/kg ; voie intrapéritonéale). Ces rats présentent un défaut spécifique de la réponse insulinique au glucose [15], comme observé chez l'homme diabétique de type 2. L'isolement et la perfusion du pancréas ont été réalisés selon une modification [16] du protocole décrit par Sussman et al. [17]. L'effet des composés ou des substances de référence est testé pendant 35 minutes (de t = 20 min. à t = 55 min.) dans du tampon Krebs en l'absence (G0) ou en présence de glucose à 2,8 mM (G2.8 mM), puis 20 minutes (de t = 55 min. à t = 75 min.) en présence de glucose 16,5 mM. La concentration d'insuline sécrétée dans le milieu est mesurée par un dosage Elisa (ELISA Alpco Cat n° 80-INSRTH-E10). Les résultats sont exprimés comme la moyenne +/-ESM (écart standard moyen) de plusieurs expériences.

**Tableau de résultats :**

| **N°** | **Concentration testée (µM)** | **Pic de sécrétion d'insuline à G16.5mM** | |
|---|---|---|---|
| | | **Produit testé (µU/min.)** | **Groupe Contrôle (µU/min.)** |
| **10** | 10 | 2 028 ± 278 | 560 ± 71 |
| **10** | 1 | 1 052 ± 187 | 560 ± 71 |
| **13** | 10 | 1489 | 560 ± 71 |
| **56** | 10 | 845 ± 133 | 454 ± 52 |
| **124** | 10 | 1278 ± 66 | 454 ± 52 |
| **157** | 10 | 989 ± 86 | 560 ± 71 |

Les dérivés de formule I testés ont un effet significatif sur la restauration de la sécrétion d'insuline en réponse au glucose au niveau de pancréas perfusés isolés de rats diabétiques N0STZ. Les plus fortes sécrétions sont obtenues avec les composés **10, 13** et **124.**

### Etude de l'activité antidiabétique du composé 10 chez le rat GK (Goto-Kakisaki)

L'activité antidiabétique du composé **10** a été évaluée chez le rat GK, un modèle de diabète de type 2 non obèse. Ce modèle a été obtenu par croisement de rats Wistar sélectionnés sur la base d'une légère intolérance au glucose [18]. Ces rats présentent la majorité des dysfonctions observées dans le diabète de type 2 chez l'homme [19]: hyperglycémie, intolérance au glucose, insulinorésistance, et réponse insulinique au glucose détériorée. Ces animaux sont élevés chez Metabrain et sont hébergés dans une animalerie à température régulée (22 ± 2°C) sous humidité constante (50 ± 20%) avec un cycle jour/nuit de 12h (lumière 7h-19h) et ont accès à volonté à la nourriture et la boisson. Les conditions d'hébergement et d'expérimentation répondent aux règles européennes concernant la santé et le traitement éthique des animaux de laboratoire (ETS123). Dans cette étude, les rats utilisés sont des rats GK femelles âgées de 16 semaines mises à jeun 2 heures avant le début de l'étude (état postabsorptif). Un test de tolérance au glucose par voie intraveineuse (IVGTT) est réalisé sur 2 groupes de rats : Un groupe traité avec le composé **10** par voie orale à la dose unique de 20 mg/kg et un groupe contrôle traité par voie orale avec le véhicule. Le test de tolérance est réalisé 1h après l'administration orale du composé **10** sur les animaux préalablement anesthésiés au pentobarbital (45 mg/kg par voie intrapéritonéale). Une prise de sang est effectuée à T0 juste avant l'administration de la charge en glucose (0,5 g/kg par voie intraveineuse) et aux temps T5, T10, T15, T20 et T30 min après la charge en glucose. Les échantillons de sang sont centrifugés afin de recueillir le plasma pour la détermination de la glycémie. Les résultats présentés ci-dessus sont exprimés
• en pourcentage de diminution de la glycémie à T0 pour le groupe traité avec le composé **10** comparé au groupe contrôle
• en pourcentage de diminution de l'AUC (aire sous courbe de la glycémie en fonction du temps) pour le groupe traité avec le composé **10** comparé au groupe contrôle

| **Composé 10** | **% d'activité** | **Signification statistique (test de t de Student)** |
|---|---|---|
| **Glycémie à T0** | -11% | *p*=0,0499 |
| **AUC** | -35% | *P*=0,0326 |

Ces résultats montrent que le composé **10** administré en dose unique à 20 mg/kg est capable de réduire l'hyperglycémie basale et l'intolérance au glucose d'un animal diabétique de type 2.

### BIBLIOGRAPHIE

[1] WO 2008051197;
[2] Park et al., Bioorganic & Medicinal Chemistry (2006), 14(2), 395-408;
[3] Shengwu Jishu Tongxun (2007), 18(4), 625-627 ;
[4] US 20090163545;
[5] Floquet et al., Bioorganic & Medicinal Chemistry Letters (2007), 17(7), 1966-1970;
[6] Khimiko-Farmatsevticheskii Zhurnal (1987), 21(11), 1320-6;
[7] WO 2002095361;
[8] Pang et al., PLoS ONE (2010), 5(4), e10129 ;
[9] Tang et al., PLoS ONE (2007) 2(8), e761 ;
[10] US 20120114696
[11] Merino et al., Bioorganic & Medicinal Chemistry (2006), 14(2), 3583-3591;
[12] Pang et al., PLoS ONE (2009), 4(11), e7730 ;
[13] Asfari et al., Endocrinology 130: 167-178, 1992 ;
[14] Portha et al., Diabetes, 23, (1974), 889-895 ;
[15] Giroix et al., Diabetes, 32, (1983), 445-451 ;
[16] Assan et al., Nature, 239, (1972), 125-126 ;
[17] Sussman et al., Diabetes, 15, (1966), 466-472 ;
[18] Goto et al., Proc. Jpn. Acad. 51, 80-85, 1975 ;
[19] Portha et al., Mol. Cell. Endocrinol., 297 : 73-85, 2009 ;

## Revendications

1. Dérivé thiophène de formule générale I suivante : 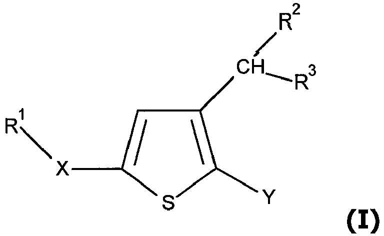 dans laquelle :
**Y** représente un groupe aryle, un groupe hétéroaryle, ou un groupe benzo-1,3-dioxole, le groupe aryle ou hétéroaryle étant éventuellement substitué par un ou plusieurs groupes choisi parmi -CN ; un atome d'halogène -O(alkyle en C₁-C₆) , le groupe alkyle étant éventuellement substitué par un ou plusieurs atomes d'halogène, ou par un groupe -O(alkyle en C₁-C₆); alkyle en C₁-C₆ substitué par un ou plusieurs atomes d'halogène, par un groupe -O(alkyle en C₁-C₆), ou par un groupe -OH ; -SO₂(alkyle en C₁-C₆) ; -CONRaRb dans lequel **Ra** représente un atome d'hydrogène ou un groupe alkyle en C₁-C₆ et **Rb** représente un groupe alkyle en C₁-C₆ ; ou -OH ;
X représente un groupe -SO₂ ou un groupe 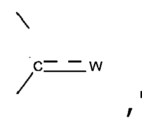 dans lequel représente une liaison et **W** représente un atome d'oxygène ou le groupe -NOR⁴, dans lequel **R⁴** représente un atome d'hydrogène, un groupe alkyle en C₁-C₆ ou un groupe (alkyle en C₁-C₆)aryle le groupe aryle étant éventuellement substitué par un ou plusieurs groupes choisi parmi -CN ; un atome d'halogène; -O(alkyle en C₁-C₆₀), le groupe alkyle étant éventuellement substitué par un ou plusieurs atomes d'halogène, ou par un groupe -O(alkyle en C₁-C₆); alkyle en C₁-C₆ substitué par un ou plusieurs atomes d'halogène, par un groupe -O(alkyle en C₁-C₆), ou par un groupe -OH ; -SO₂(alkyle en C₁-C₆) ; -CONRa'Rb' dans lequel **Ra'** représente un atome d'hydrogène ou une groupe alkyle en C₁-C₆ et **Rb'** représente un groupe alkyle en C₁-C₆ ; ou -OH, ou
est absent et **W** représente -OH ;
**R¹** représente
- un groupe alkyle en C₁-C₆, le groupe alkyle étant éventuellement substitué par un atome d'halogène;
- un groupe cycloalkyle en C₃-C₆;
- un groupe (alkyle en C₁-C₆)O(alkyle en C₁-C₆) ;
- un groupe (alkyle en C₁-C₆)NR(alkyle en C₁-C₆) dans lequel **R** représente un atome d'hydrogène ou un groupe alkyle en C₁-C₆ ;
- un groupe aryle, le groupe aryle étant éventuellement substitué par un ou plusieurs groupes choisi parmi -CN ; un atome d'halogène; -O(alkyle en C₁-C₆₀), le groupe alkyle étant éventuellement substitué par un ou plusieurs atomes d'halogène, ou par un groupe -O(alkyle en C₁-C₆); -SO₂(alkyle en C₁-C₆) ; -CONRa"Rb" dans lequel **Ra"** représente un atome d'hydrogène ou un groupe alkyle en C₁-C₆ et **Rb"** représente un groupe alkyle en C₁-C₆; ou alkyle en C₁-C₆), le groupe alkyle étant éventuellement substitué par un ou plusieurs atomes d'halogène, par un groupe -O(alkyle en C₁-C₆), ou par un groupe -OH ;
- un groupe (alkyle en C₁-C₆)aryle ; le groupe aryle étant éventuellement substitué par un ou plusieurs groupes choisi parmi -CN ; un atome d'halogène; -O(alkyle en C₁-C₆) ; ou alkyle en C₁-C₆;
- un groupe -NH-aryle ; le groupe aryle étant éventuellement substitué par un ou plusieurs groupes choisi parmi -CN ; un atome d'halogène; -O(alkyle en C₁-C₆); ou alkyle en C₁-C₆;
- un groupe -NH(alkyle en C₁-C₆)aryle ; le groupe aryle étant éventuellement substitué par un ou plusieurs groupes choisi parmi -CN ; un atome d'halogène; -O(alkyle en C₁-C₆) ; ou alkyle en C₁-C₆;
- un groupe hétéroaryle, éventuellement substitué par un atome d'halogène ;
- un groupe -OH ;
- un groupe morpholine ; ou
- un groupe N-Phénylepipérazine ;
- un groupe NH-NH-CO-aryle dans lequel le groupe aryle est éventuellement substitué par un ou plusieurs groupes choisi parmi un atome d'halogène, et un groupe -O(alkyle en C₁-C₆) ;
- un groupe NH-NH-CO-hétéroaryle,
**R²** représente un atome d'hydrogène, un groupe alkyle en C₁-C₆, un groupe (alkyle en C₁-C₆)aryle ou un groupe (alkyle en C₁-C₆)O(alkyle en C₁-C₆) ;
**R³** représente
- un groupe -COOR⁵, dans lequel **R⁵** représente un atome d'hydrogène, un groupe alkyle en C**₁**-C**₆** ou le groupe glucopyranose ;
- un groupe -COSR⁶, dans lequel **R⁶** représente un atome d'hydrogène ou un groupe alkyle en C₁-C₆;
- un groupe -CONR⁷R⁸, dans lequel **R⁷** représente un atome d'hydrogène ou un groupe alkyle en C₁-C₆ et **R⁸** représente un atome d'hydrogène ; un groupe alkyle en C₁-C₆ éventuellement substitué par un groupe -OH ; un groupe -OH ; un groupe -O(alkyle en C₁-C₆) ; un groupe -NH₂ ; un groupe -(alkyle en C₁-C₆)NR⁹R¹⁰ dans lequel **R⁹** et **R¹⁰** représentent tous les deux un groupe alkyle en C₁-C₆ ; un groupe -(alkyle en C₁-C₆)COOH ; un groupe-(alkyle en C₁-C₆)COO(alkyle en C₁-C₆) ; un groupe aryle; ou un groupe hétéroaryle ;
- un groupe -CSNR¹¹R¹² dans lequel **R¹¹** et **R¹²** représentent indépendamment l'un de l'autre un atome d'hydrogène ou un groupe alkyle en en C₁-C₆);
- un groupe -CN ;
- un groupe -C(=NH)NHOH ;
- un groupe -COmorpholine ;
- un groupe -COpyrolidine ;
- un groupe -CON-Me-pipérazine ;
- un groupe -COguanidine ou -COguanidine-BOC ;
- un groupe tétrazole ; ou
- un groupe oxadiazolone ;
ou un énantiomère, diastéréoisomère, hydrate, solvate, tautomère, mélange racémique ou sel pharmaceutiquement acceptable de celui-ci, à l'exception des composés (a) à (z1) de formule suivante :

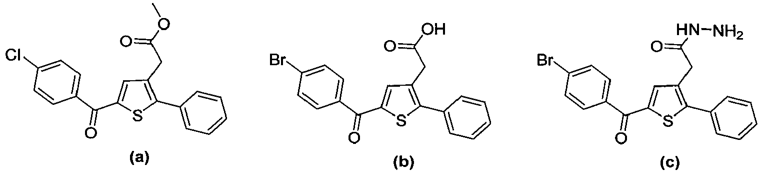

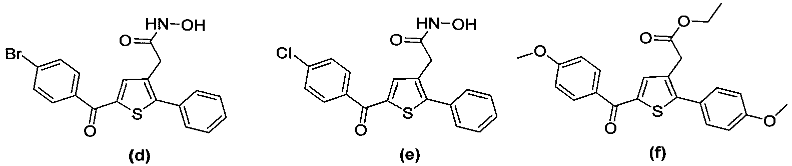

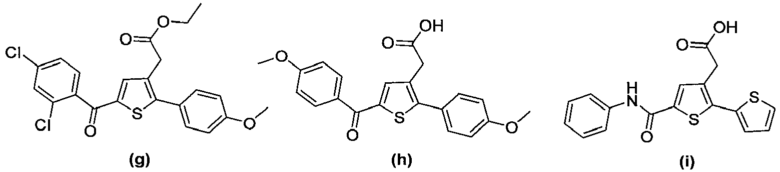

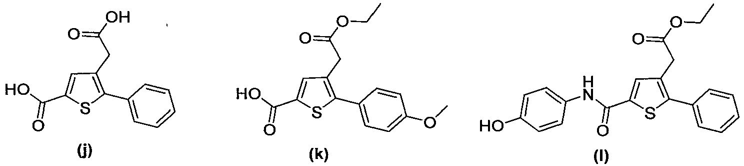

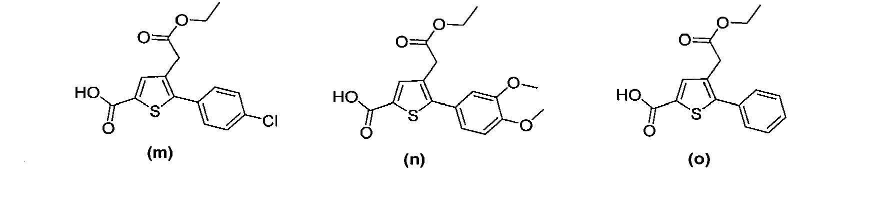

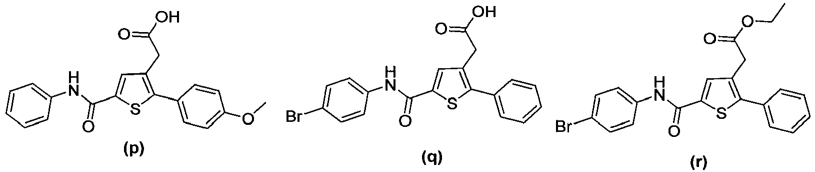

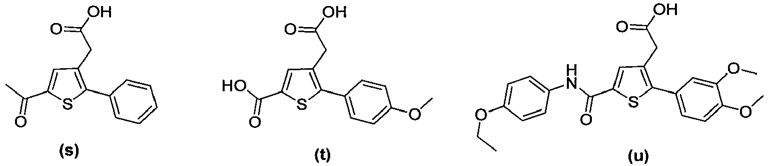

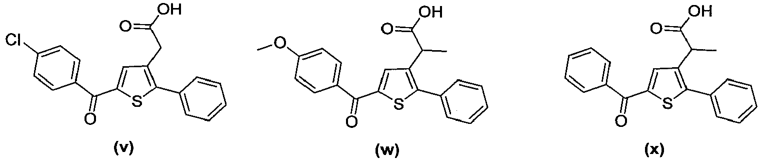

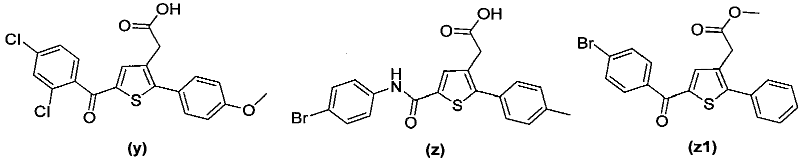

2. Dérivé thiophène selon la revendication 1 **caractérisé en ce que** 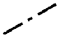 représente une liaison et **W** représente un atome d'oxygène ou le groupe -NOR⁴, dans lequel **R⁴** représente un atome d'hydrogène ou un groupe alkyle en C₁-C₆, avantageusement **W** représente un atome d'oxygène.

3. Dérivé thiophène selon l'une quelconque des revendications 1 ou 2 **caractérisé en ce que R³** représente
- un groupe -COguanidine ;
- un groupe -COOR⁵, dans lequel **R⁵** représente un atome d'hydrogène ou un groupe alkyle en C₁-C₆;
- un groupe -CONR⁷R⁸, dans lequel **R⁷** représente un atome d'hydrogène et **R⁸** représente un atome d'hydrogène ; un groupe alkyle en C₁-C₆ éventuellement substitué par un groupe -OH ; un groupe -OH ; un groupe -O(alkyle en C₁-C₆) ou un groupe -(alkyle en C₁-C₆)NR⁹R¹⁰ dans lequel **R⁹** et **R¹⁰** représentent tous les deux un groupe alkyle en C₁-C₆ ; ou
- un groupe -COmorpholine ;
avantageusement **R³** représente un groupe -COOR⁵, dans lequel **R⁵** représente un atome d'hydrogène ou un groupe alkyle en C₁-C₆.

4. Dérivé thiophène selon l'une quelconque des revendications 1 à 3 **caractérisé en ce que Y** représente un groupe aryle, avantageusement phényle, le groupe aryle étant éventuellement substitué, , par un ou plusieurs groupes choisis parmi -CN ; un atome d'halogène, avantageusement choisi parmi Cl ou F ; ou -O(alkyle en C₁-C₆) avantageusement -OMe.

5. Dérivé thiophène selon la revendication 4 **caractérisé en ce que Y** représente un groupe phényle, le groupe phényle étant substitué par un ou plusieurs atome d'halogène, avantageusement Cl.

6. Dérivé thiophène selon l'une quelconque des revendications 1 à 5 **caractérisé en ce que R¹** représente
- un groupe cycloalkyle en C₃-C₆ ;
- un groupe aryle, avantageusement phényle, le groupe aryle étant éventuellement substitué par un ou plusieurs groupes choisis parmi -CN ; un atome d'halogène, avantageusement choisi parmi Cl ou F ; -O(alkyle en C₁-C₆) avantageusement -OMe; ou alkyle en C₁-C₆ avantageusement méthyle;
- un groupe hétéroaryle éventuellement substitué par un atome d'halogène, avantageusement furanyle, pyridyle ou thiazolyle ; ou
- un groupe morpholine.

7. Dérivé thiophène selon la revendication 6 **caractérisé en ce que R¹** représente
- un groupe phényle, le groupe phényle étant éventuellement substitué par un ou plusieurs groupes -O(alkyle en C₁-C₆) avantageusement -OMe; ou
- un groupe furanyle, pyridyle ou thiazolyle, le groupe furanyle étant éventuellement substitué par un atome d'halogène.

8. Dérivé thiophène selon l'une quelconque des revendications 1 à 7 **caractérisé en ce qu'**il est choisi parmi les composés suivants :
Acide 2-(2-(4-chlorophényl)-5-((éthoxyimino)(4-méthoxyphényl)méthyl) thiophén-3-yl) acétique (**108**) ;
Acide 2-(2-(4-chlorophényl)-5-((hydroxyimino)(4-méthoxyphényl)méthyl) thiophèn-3-yl) acétique (**109**) ;
Acide 2-(2-(4-chlorophényl)-5-((méthoxyimino)(4-méthoxyphényl)méthyl) thiophén-3-yl) acétique (**110**) ;
2-(2-(4-chlorophényl)-5-((éthoxyimino)(4-méthoxyphényl)méthyl) thiophén-3-yl) acétate d'éthyle (**102**) ;
2-(2-(4-chlorophényl)-5-((hydroxyimino)(4-méthoxyphényl)méthyl) thiophén-3-yl)acétate d'éthyle (**104**) ;
2-(2-(4-chlorophényl)-5-((méthoxyimino)(4-méthoxyphényl)méthyl) thiophén-3-yl)acétate d'éthyle (**106**) ;
Acide 2-(2-(2,3-difluorophényl)-5-(4-méthoxybenzoyl)thiophén-3-yl) acétique (**137**) ;
Acide 2-(2-(2,3-difluorophényl)-5-(furan-2-carbonyl)thiophén-3-yl) acétique (**136**) ;
Acide 2-(2-(3,4-dichlorophényl)-5-(4-méthoxybenzoyl)thiophén-3-yl) acétique (**81**);
2-(2-(3-cyanophényl)-5-(furan-2-carbonyl)thiophén-3-yl)-N-(2-(diméthyl amino)éthyl)acétamide (**142**) ;
Acide 2-(2-(4-chloro-2-fluorophényl)-5-(furan-2-carbonyl)thiophén-3-yl) acétique (**144**) ;
Acide 2-(2-(4-chlorophényl)-5-(3-méthoxybenzoyl)thiophén-3-yl) acétique **(59)** ;
Acide 2-(2-(4-chlorophényl)-5-(4-méthoxybenzoyl)thiophén-3-yl)-3-méthoxypropanoïque (**128**) ;
2-(2-(4-chlorophényl)-5-(4-méthoxybenzoyl)thiophén-3-yl)acétamide (**86**);
Acide 2-(2-(4-chlorophényl)-5-(4-méthoxybenzoyl)thiophén-3-yl) acétique **(60)** ;
2-(2-(4-chlorophényl)-5-(4-méthoxybenzoyl)thiophén-3-yl)-N-(2-(diméthyl amino)éthyl)acétamide (**89**) ;
2-(2-(4-chlorophényl)-5-(4-méthoxybenzoyl)thiophén-3-yl)-N-(2-hydroxy éthyl)acétamide (**96**) ;
2-(2-(4-chlorophényl)-5-(4-méthoxybenzoyl)thiophén-3-yl)-N-hydroxy acétamide (**100**) ;
Acide 2-(2-(4-chlorophényl)-5-(4-méthoxybenzoyl)thiophén-3-yl) propanoïque (**127**) ;
Acide 2-(2-(4-chlorophényl)-5-(cyclohexanecarbonyl)thiophén-3-yl)acétique (**61**) ;
2-(2-(4-chlorophényl)-5-(furan-2-carbonyl)thiophén-3-yl)-1-morpholino éthanone (**91**)**;**
Acide 2-(2-(4-chlorophényl)-5-(furan-2-carbonyl)thiophén-3-yl) acétique (**56**) 2-(2-(4-chlorophényl)-5-(furan-2-carbonyl)thiophén-3-yl)-N-(2-(diméthyl amino)éthyl)acétamide (**95**) ;
2-(2-(4-chlorophényl)-5-(furan-2-carbonyl)thiophén-3-yl)-N-éthoxy acétamide (**98**) ;
Acide 2-(2-(4-chlorophényl)-5-picolinoylthiophén-3-yl) acétique (**149**) ;
Acide 2-(5-(2,3-difluorobenzoyl)-2-(4-méthoxyphényl)thiophén-3-yl) acétique (**64**) ;
Acide 2-(5-(2,4-difluorobenzoyl)-2-(4-méthoxyphényl)thiophén-3-yl) acétique (**65**) ;
Acide 2-(5-(2-fluorobenzoyl)-2-(4-méthoxyphényl)thiophén-3-yl) acétique (**67**) ;
Acide 2-(5-(3,5-difluorobenzoyl)-2-(4-méthoxyphényl)thiophén-3-yl) acétique (**70**) ;
Acide 2-(5-(3-chlorobenzoyl)-2-(4-méthoxyphényl)thiophén-3-yl) acétique (**71**) ;
Acide 2-(5-(3-chlorobenzoyl)-2-(4-chlorophényl)thiophén-3-yl) acétique (**58**) ;
Acide 2-(5-(3-fluoro-4-méthoxybenzoyl)-2-(4-méthoxyphényl)thiophén-3-yl) acétique (**73**) ;
Acide 2-(5-(3-fluorobenzoyl)-2-(4-méthoxyphényl)thiophén-3-yl) acétique (**72**) ;
Acide 2-(5-(3-méthoxybenzoyl)-2-(4-méthoxyphényl)thiophén-3-yl) acétique **(75) ;**
Acide 2-(5-(4-cyanobenzoyl)-2-(4-méthoxyphényl)thiophén-3-yl) acétique (**76**) ;
Acide 2-(5-(4-fluoro-3-méthylbenzoyl)-2-(4-méthoxyphényl)thiophén-3-yl) acétique (**74**) ;
Acide 2-(5-(4-fluorobenzoyl)-2-(4-méthoxyphényl)thiophén-3-yl) acétique (**77**) ;
Acide 2-(5-(4-méthoxybenzoyl)-2-phénylthiophén-3-yl) acétique (**51**) ;
Acide 2-(5-(cyclohexanecarbonyl)-2-phénylthiophén-3-yl) acétique (**52**) ;
Acide 2-(5-(furan-2-carbonyl)-2-(4-méthoxyphényl)thiophén-3-yl) acétique (**68**) ;
Acide 2-(5-(furan-2-carbonyl)-2-phénylthiophén-3-yl) acétique (**48**) ;
Acide 2-(5-benzoyl-2-(4-chlorophényl)thiophén-3-yl) acétique (**63**) ;
Acide 2-(5-benzoyl-2-(4-méthoxyphényl)thiophén-3-yl) acétique (**78**) ;
2-(2-(2,3-difluorophényl)-5-(4-méthoxybenzoyl)thiophén-3-yl)acétate d'éthyle (**133**) ;
2-(2-(2,3-difluorophényl)-5-(furan-2-carbonyl)thiophén-3-yl)acétate d'éthyle (**130**) ;
2-(2-(4-chlorophényl)-5-(3-méthoxybenzoyl)thiophén-3-yl)acétate d'éthyle (**12**) ;
2-(2-(4-chlorophényl)-5-(4-méthoxybenzoyl)thiophén-3-yl)-3-méthoxypropanoate d'éthyle (**124**) ;
2-(2-(4-chlorophényl)-5-(4-méthoxybenzoyl)thiophén-3-yl)acétate d'éthyle (**13**) ;
2-(2-(4-chlorophényl)-5-(4-méthoxybenzoyl)thiophén-3-yl) propanoate d'éthyle (**123**) ;
2-(2-(4-chlorophényl)-5-(cyclohexanecarbonyl)thiophén-3-yl)acétate d'éthyle (**14**) ;
2-(2-(4-chlorophényl)-5-(furan-2-carbonyl)thiophén-3-yl)acétate d'éthyle (**10**) ;
2-(2-(4-chlorophényl)-5-(morpholine-4-carbonyl)thiophén-3-yl)acétate d'éthyle (**114**) ;
2-(2-(4-cyanophényl)-5-(furan-2-carbonyl)thiophén-3-yl)acétate d'éthyle **(139)** ;
2-(2-(4-méthoxyphényl)-5-(thiazole-4-carbonyl)thiophén-3-yl)acétate d'éthyle (**33**) ;
2-(5-(2,3-difluorobenzoyl)-2-(4-méthoxyphényl)thiophén-3-yl)acétate d'éthyle (**18**)**;**
2-(5-(2,4-difluorobenzoyl)-2-(4-méthoxyphényl)thiophén-3-yl)acétate d'éthyle (**19**)**;**
2-(5-(2-fluorobenzoyl)-2-(4-méthoxyphényl)thiophén-3-yl)acétate d'éthyle (**21**) ;
2-(5-(3,5-difluorobenzoyl)-2-(4-méthoxyphényl)thiophén-3-yl)acétate d'éthyle (**24**) ;
2-(5-(3-chlorobenzoyl)-2-(4-chlorophényl)thiophén-3-yl)acétate d'éthyle (**11**) ;
2-(5-(3-chlorobenzoyl)-2-(4-méthoxyphényl)thiophén-3-yl)acétate d'éthyle (**25**) ;
2-(5-(3-fluoro-4-méthoxybenzoyl)-2-(4-méthoxyphényl)thiophén-3-yl) acétate d'éthyle (**27**) ;
2-(5-(3-fluorobenzoyl)-2-(4-méthoxyphényl)thiophén-3-yl)acétate d'éthyle (**26**) ;
2-(5-(3-méthoxybenzoyl)-2-(4-méthoxyphényl)thiophén-3-yl)acétate d'éthyle (**30**) ;
2-(5-(4-cyanobenzoyl)-2-(4-méthoxyphényl)thiophén-3-yl)acétate d'éthyle (**31**) ;
2-(5-(4-fluoro-3-méthylbenzoyl)-2-(4-méthoxyphényl)thiophén-3-yl) acétate d'éthyle (**29**) ;
2-(5-(4-fluorobenzoyl)-2-(4-méthoxyphényl)thiophén-3-yl)acétate d'éthyle (**32**) ;
2-(5-(furan-2-carbonyl)-2-(4-méthoxyphényl)thiophén-3-yl)acétate d'éthyle (**22**) ;
2-(5-benzoyl-2-(4-chlorophényl)thiophén-3-yl)acétate d'éthyle (**15**) ;
2-(5-benzoyl-2-(4-méthoxyphényl)thiophén-3-yl)acétate d'éthyle (**35**) ;
2-(2-(3,4-dichlorophényl)-5-(4-méthoxybenzoyl)thiophén-3-yl) acétate d'isopropyle **(40) ;**
2-(2-(3,4-dichlorophényl)-5-(furan-2-carbonyl)thiophén-3-yl) acétate d'isopropyle **(39) ;**
2-(2-(4-chloro-2-fluorophényl)-5-(furan-2-carbonyl)thiophén-3-yl)acétate d'isopropyle **(143) ;**
2-(2-(4-chlorophényl)-5-(4-méthoxybenzoyl)thiophén-3-yl)-3-méthoxypropanoate d'isopropyle **(125) ;**
2-(2-(4-chlorophényl)-5-picolinoylthiophén-3-yl)acétate de méthyle **(148) ;**
2-(5-(4-méthoxybenzoyl)-2-phénylthiophén-3-yl)acétate de méthyle **(5) ;**
2-(5-(cyclohexanecarbonyl)-2-phénylthiophén-3-yl)acétate de méthyle **(6)** ;
2-(5-(furan-2-carbonyl)-2-phénylthiophén-3-yl)acétate de méthyle **(3) ;**
2-(2-(4-chlorophényl)-5-(4-méthoxybenzoyl)thiophén-3-yl) acétate d'isopropyle **(43) ;**
2-(2-(4-chlorophényl)-5-(furan-2-carbonyl)thiophén-3-yl)acétate d'isopropyle **(44) ;**
2-(2-(4-chlorophényl)-5-(4-méthoxybenzoyl)thiophén-3-yl) acétate de tert-butyle **(84) ;**
2-(2-(4-chlorophényl)-5-(furan-2-carbonyl)thiophén-3-yl)acétate de tert-butyle **(83) ;**
*N*-(*N*-(tert-butoxycarbonyl)carbamimidoyl)-2-(2-(4-chlorophényl)-5-(4-méthoxybenzoyl)thiophén-3-yl)acétamide **(46) ;**
Acide 2-(5-(3-chlorobenzoyl)-2-phénylthiophén-3-yl)acétique **(49) ;**
Acide 2-(5-(3-méthoxybenzoyl)-2-phénylthiophén-3-yl)acétique **(50) ;**
2-(2-(4-chlorophényl)-5-(4-méthoxybenzoyl)thiophén-3-yl)-1-(4-méthylpipérazin-1-yl)éthanone **(93) ;**
Acide 2-(2-(4-cyanophényl)-5-(furan-2-carbonyl)thiophén-3-yl)acétique **(140) ;**
Acide 2-[5-benzoyl-2-(4-chlorophényl)-3-thiényl]acétique **(154) ;**
2-[5-(4-chloro-2-méthoxy-benzoyl)-2-(4-chlorophényl)-3-thiényl]acétate d'éthyle **(155) ;**
Acide 2-[5-(4-chloro-2-méthoxy-benzoyl)-2-(4-chlorophényl)-3-thiényl]acétique **(156) ;**
2-[2-(3,4-dichlorophényl)-5-(4-méthoxybenzoyl)-3-thiényl]acétate d'éthyle **(157) ;**
2-[2-(3,4-dichlorophényl)-5-(furan-2-carbonyl)-3-thiényl]acétate d'éthyle **(158) ;**
Acide 2-[2-(3,4-dichlorophényl)-5-(furan-2-carbonyl)-3-thiényl]acétique **(159) ;**
2-[2-(3,4-dichlorophényl)-5-(4-méthoxybenzoyl)-3-thiényl]-N-(2-hydroxyéthyl)acétamide **(165) ;**
2-[2-(3,4-dichlorophényl)-5-(4-méthoxybenzoyl)-3-thiényl]-N-(2-diméthylaminoéthyl)acétamide **(166) ;**
Acide 2-[2-(4-chlorophényl)-5-[C-(3,4-dichlorophényl)-N-ethoxy-carbonimidoyl]-3-thiényl]acétique **(167) ;**
Acide 2-[2-(3,4-dichlorophényl)-5-(4-méthoxyphényl)sulfonyl-3-thiényl]acétique **(175) ;**
2-[5-(5-chlorofuran-2-carbonyl)-2-(4-chlorophényl)-3-thiényl]acétate d'éthyle **(176) ;**
N-[N-[2-[2-(3,4-dichlorophényl)-5-(4-méthoxybenzoyl)-3-thiényl]acétyl]carbamimidoyl]carbamate de tert-butyle **(182) ;**
N-[N-[2-[2-(4-chlorophényl)-5-(4-méthoxybenzoyl)-3-thiényl]acétyl]carbamimidoyl]carbamate de tert-butyle **(183) ;**
chlorhydrate de N-carbamimidoyl-2-[2-(3,4-dichlorophényl)-5-(4-méthoxybenzoyl)-3-thiényl]acétamide **(184) ;**
chlorhydrate de N-carbamimidoyl-2-[2-(4-chlorophényl)-5-(4-méthoxybenzoyl)-3-thiényl]acétamide **(185) ;**
chlorhydrate de N-carbamimidoyl-2-[2-(4-chlorophényl)-5-(furan-2-carbonyl)-3-thiényl]acétamide **(186) ;**
2-[2-(4-chlorophényl)-5-(4-méthoxybenzoyl)-3-thiényl]prop-2-énoate d'éthyle **(187).**

9. Composition pharmaceutique comprenant un dérivé selon l'une quelconque des revendications 1 à 8 et un excipient pharmaceutiquement acceptable.

10. Composition pharmaceutique selon la revendication 9 comprenant en outre un autre agent antidiabétique, en particulier la metformine.

11. Dérivé selon l'une quelconque des revendications 1 à 8 ou choisi parmi les composés de formule (f) à (p), (r), (s), (u), (y) et (z) tels que définis dans la revendication 1 pour utilisation à titre de médicament.

12. Dérivé selon l'une quelconque des revendications 1 à 8 ou choisi parmi les composés de formule (a) à (z1), tels que définis dans la revendication 1 pour utilisation à titre de médicament destiné au traitement et/ou à la prévention du diabète, de ses complications et/ou des pathologies associés, avantageusement du diabète de type 2 et de l'hyperglycémie.

## Patentansprüche

1. Thiophen-Derivat der folgenden allgemeinen Formel I: 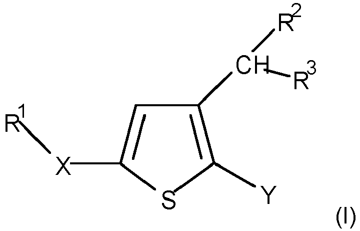 worin:
Y eine Arylgruppe, eine Heteroarylgruppe oder eine Benzo-1,3-dioxol-Gruppe darstellt, wobei die Aryl- oder Heteroarylgruppe eventuell substituiert ist durch eine oder mehrere Gruppen, ausgewählt aus -CN; einem Halogenatom; -O(C₁-C₆-Alkyl), wobei die Alkylgruppe eventuell substituiert ist durch ein oder mehrere Halogenatome, oder durch eine -O(C₁-C₆-Alkyl)-Gruppe; C₁-C₆-Alkyl substituiert durch ein oder mehrere Halogenatome, durch eine -O(C₁-C₆-Alkyl)-Gruppe, oder durch eine Gruppe -OH; -SO₂(C₁-C₆-Alkyl); -CONRaRb, worin Ra ein Wasserstoffatom oder eine C₁-C₆-Alkylgruppe darstellt und Rb eine C₁-C₆-Alkylgruppe darstellt; oder -OH,
X eine -SO₂-Gruppe oder eine 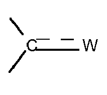 -Gruppe darstellt, worin 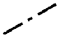 eine Bindung darstellt und W ein Sauerstoffatom oder die -NOR⁴-Gruppe darstellt, worin R⁴ ein Wasserstoffatom, eine C₁-C₆-Alkylgruppe oder eine (C₁-C₆-Alkyl)Arylgruppe darstellt, wobei die Arylgruppe eventuell substituiert ist durch eine oder mehrere Gruppen, ausgewählt aus -CN; einem Halogenatom; -O(C₁-C₆-Alkyl), wobei die Alkylgruppe eventuell substituiert ist durch ein oder mehrere Halogenatome, oder durch eine -O(C₁-C₆-Alkyl)-Gruppe; C₁-C₆-Alkyl substituiert durch ein oder mehrere Halogenatome, durch eine -O(C₁-C₆-Alkyl)-Gruppe, oder durch eine Gruppe -OH; -SO₂(C₁-C₆-Alkyl); -CONRa'Rb', worin Ra' ein Wasserstoffatom oder eine C₁-C₆-Alkylgruppe darstellt und Rb' eine C₁-C₆-Alkylgruppe darstellt; oder -OH, oder
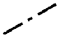 nicht vorhanden ist und W -OH darstellt,
R¹ folgendes darstellt
- eine C₁-C₆-Alkylgruppe, wobei die Alkylgruppe eventuell durch ein Halogenatom substituiert ist,
- eine C₃-C₆-Cycloalkylgruppe,
- eine (C₁-C₆-Alkyl)O(C₁-C₆-Alkyl)-Gruppe,
- eine (C₁-C₆-Alkyl)NR(C₁-C₆-Alkyl)-Gruppe, worin R ein Wasserstoffatom oder eine C₁-C₆-Alkylgruppe darstellt,
- eine Arylgruppe, wobei die Arylgruppe eventuell substituiert ist durch eine oder mehrere Gruppen, ausgewählt aus -CN; einem Halogenatom; -O(C₁-C₆-Alkyl), wobei die Alkylgruppe eventuell substituiert ist durch ein oder mehrere Halogenatome, oder durch eine Gruppe -O(C₁-C₆-Alkyl); -SO₂(C₁-C₆-Alkyl); -CONRa"Rb", worin Ra" ein Wasserstoffatom oder eine C₁-C₆-Alkylgruppe darstellt und Rb" eine C₁-C₆-Alkylgruppe darstellt; oder C₁-C₆-Alkyl, wobei die Alkylgrupe eventuell substituiert ist durch ein oder mehrere Halogenatome, durch eine -O(C₁-C₆-Alkyl)-Gruppe oder durch eine -OH-Gruppe,
- eine (C₁-C₆-Alkyl)Arylgruppe, wobei die Arylgruppe eventuell substituiert ist durch eine oder mehrere Gruppen, ausgewählt aus -CN, einem Halogenatom, -O(C₁-C₆-Alkyl), oder C₁-C₆-Alkyl,
- eine -NH-Arylgruppe, wobei die Arylgruppe eventuell substituiert ist durch eine oder mehrere Gruppen, ausgewählt aus -CN, einem Halogenatom, -O(C₁-C₆-Alkyl), oder C₁-C₆-Alkyl,
- eine -NH(C₁-C₆-Alkyl)Arylgruppe, wobei die Arylgruppe eventuell substituiert ist durch eine oder mehrere Gruppen, ausgewählt aus -CN, einem Halogenatom, - O(C₁-C₆-Alkyl), oder C₁-C₆-Alkyl,
- eine Heteroarylgruppe, eventuell substituiert durch ein Halogenatom,
- eine -OH-Gruppe,
- eine Morpholingruppe, oder
- eine N-Phenylpiperazin-Gruppe,
- eine NH-NH-CO-Arylgruppe, bei der die Arylgruppe eventuell substituiert ist durch eine oder mehrere Gruppen, ausgewählt aus einem Halogenatom, und einer
- O(C₁-C₆-Alkyl)-Gruppe,
- eine NH-NH-CO-Heteroarylgruppe,
R² ein Wasserstoffatom, eine C₁-C₆-Alkylgruppe, eine (C₁-C₆-Alkyl)Arylgruppe oder eine (C₁-C₆-Alkyl)O(C₁-C₆-Alkyl)-Gruppe darstellt,
R³ folgendes darstellt
- eine -COOR⁵-Gruppe, bei der R⁵ ein Wasserstoffatom, eine C₁-C₆-Alkylgruppe oder die Glucopyranose-Gruppe darstellt,
- eine -COSR⁶-Gruppe, bei der R⁶ ein Wasserstoffatom oder eine C₁-C₆-Alkylgruppe darstellt,
- eine -CONR⁷R⁸-Gruppe, bei der R⁷ ein Wasserstoffatom oder eine C₁-C₆-Alkylgruppe darstellt und R⁸ ein Wasserstoffatom, eine C₁-C₆-Alkylgruppe, eventuell substituiert durch eine -OH-Gruppe, eine -OH-Gruppe, eine -O(C₁-C₆-Alkyl)-Gruppe, eine -NH₂-Gruppe, eine -(C₁-C₆-Alkyl)NR⁹R¹⁰-Gruppe, bei der R⁹ und R ¹⁰ beide eine C₁-C₆-Alkylgruppe darstellen, eine -(C₁-C₆-Alkyl)COOH-Gruppe, eine -(C₁-C₆-Alkyl)COO(C₁-C₆-Alkyl)-Gruppe, eine Arylgruppe, oder eine Heteroarylgruppe darstellt,
- eine -CSNR¹¹R¹²-Gruppe, bei der R¹¹ und R¹² unabhängig voneinander ein Wasserstoffatom oder eine C₁-C₆-Alkylgruppe darstellen,
- eine -CN-Gruppe,
- eine -C(=NH)NHOH-Gruppe,
- eine -CO Morpholin-Gruppe,
- eine -CO Pyrolidin-Gruppe,
- eine -CON-Me-Piperazin-Gruppe,
- eine -CO Guanidin- oder -CO Boc-Guanidin-Gruppe,
- eine Tetrazolgruppe, oder
- eine Oxadiazolon-Gruppe,
oder ein Enantiomer, Diastereoisomer, Hydrat, Solvat, Tautomer, eine racemische Mischung oder ein pharmazeutisch akzeptables Salz davon,
mit Ausnahme der Verbindungen (a) bis (z1) folgender Formel:

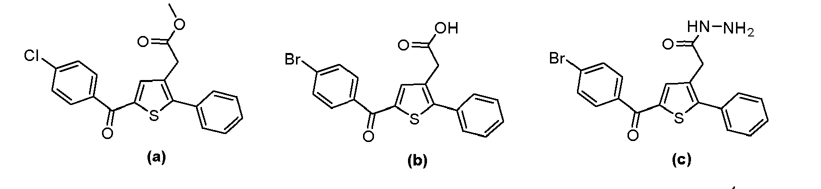

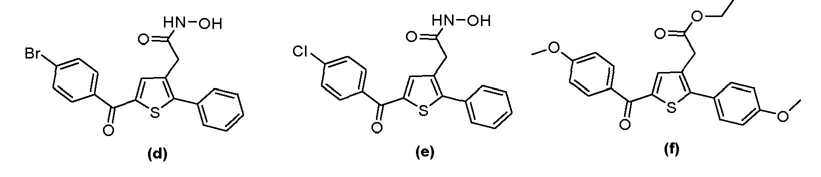

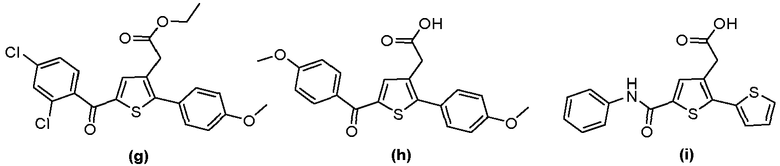

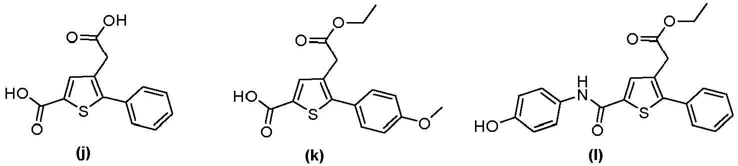

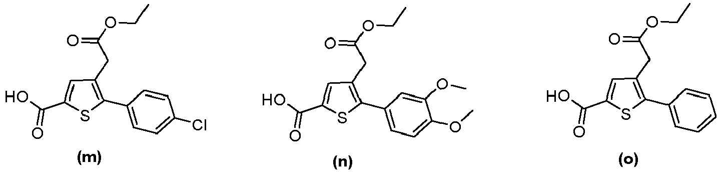

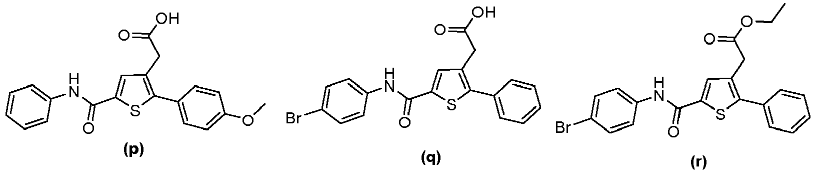

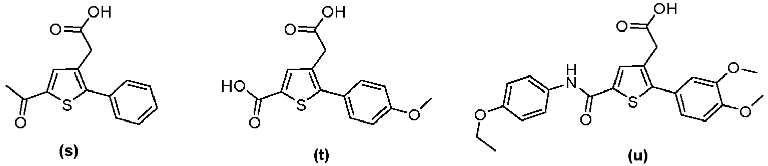

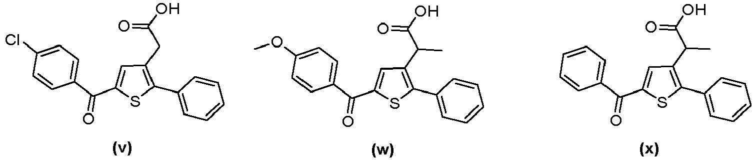

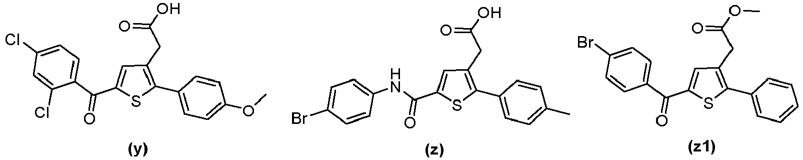

2. Thiophen-Derivat nach Anspruch 1, **dadurch gekennzeichnet, dass** eine Bindung darstellt und W ein Sauerstoffatom oder die -NOR⁴-Gruppe darstellt, worin R⁴ ein Wasserstoffatom oder eine C₁-C₆-Alkylgruppe darstellt, vorteilhafterweise W ein Sauerstoffatom darstellt.

3. Thiophen-Derivat nach einem der Ansprüche 1 oder 2, **dadurch gekennzeichnet, dass** R³ darstellt
- eine - CO Guanidin-Gruppe,
- eine -COOR⁵-Gruppe, bei der R⁵ ein Wasserstoffatom oder eine C₁-C₆-Alkylgruppe darstellt,
- eine -CONR⁷ R⁸-Gruppe, bei der R⁷ ein Wasserstoffatom darstellt und R⁸ ein Wasserstoffatom, eine C₁-C₆-Alkylgruppe, eventuell substituiert durch eine -OH-Gruppe, eine -OH-Gruppe, eine -O(C₁-C₆-Alkyl)-Gruppe oder eine -(C₁-C₆-Alkyl)NR⁹R¹⁰-Gruppe darstellt, bei der R⁹ und R¹⁰ beide eine C₁-C₆-Alkylgruppe darstellen, oder
- eine -CO Morpholin-Gruppe,
vorteilhafterweise R³ eine -COOR⁵-Gruppe darstellt, bei der R⁵ ein Wasserstoffatom oder eine C₁-C₆-Alkylgruppe darstellt.

4. Thiophen-Derivat nach einem der Ansprüche 1 bis 3, **dadurch gekennzeichnet, dass** Y eine Aryl-, vorteilhafterweise Phenylgruppe darstellt, wobei die Arylgruppe eventuell substituiert ist durch eine oder mehrere Gruppen, ausgewählt aus -CN, einem Halogenatom, das vorteilhafterweise aus Cl oder F ausgewählt ist, oder -O(C₁-C₆-Alkyl), vorteilhafterweise -OMe.

5. Thiophen-Derivat nach Anspruch 4, **dadurch gekennzeichnet, dass** Y eine Phenylgruppe darstellt, wobei die Phenylgruppe durch ein oder mehrere Halogenatome, vorteilhafterweise Cl, substituiert ist.

6. Thiophen-Derivat nach einem der Ansprüche 1 bis 5, **dadurch gekennzeichnet, dass** R¹ darstellt
- eine C₃-C₆-Cycloalkylgruppe,
- eine Aryl-, vorteilhafterweise Phenylgruppe, wobei die Arylgruppe eventuell substituiert ist durch eine oder mehrere Gruppen, ausgewählt aus -CN, einem Halogenatom, das vorteilhafterweise aus Cl oder F ausgewählt ist, -O(C₁-C₆-Alkyl), vorteilhafterweise -OMe, oder C₁-C₆-Alkyl, vorteilhafterweise Methyl,
- eine Heteroarylgruppe, die eventuell substituiert ist durch ein Halogenatom, vorteilhafterweise Furanyl, Pyridyl oder Thiazolyl, oder
- eine Morpholin-Gruppe.

7. Thiophen-Derivat nach Anspruch 6, **dadurch gekennzeichnet, dass** R¹ darstellt
- eine Phenylgruppe, wobei die Phenylgruppe eventuell durch eine oder mehrere -O(C₁-C₆-Alkyl)-Gruppen, vorteilhafterweise -OMe, substituiert ist, oder
- eine Furanyl-, Pyridyl- oder Thiazolylgruppe, wobei die Furanylgruppe eventuell durch ein Halogenatom substituiert ist.

8. Thiophen-Derivat nach einem der Ansprüche 1 bis 7, **dadurch gekennzeichnet, dass** es aus den folgenden Verbindungen ausgewählt ist:
2-(2-(4-Chlorphenyl)-5-((ethoxyimino)(4-methoxyphenyl)methyl) thiophen-3-yl) essigsäure (108),
2-(2-(4-chlorphenyl)-5-((hydroxyimino)(4-methoxyphenyl)methyl) thiophen-3-yl) essigsäure (109),
2-(2-(4-chlorphenyl)-5-((methoxyimino)(4-methoxyphenyl)methyl) thiophen-3-yl) essigsäure (110),
2-(2-(4-chlorphenyl)-5-((ethoxyimino)(4-methoxyphenyl)methyl) thiophen-3-yl) ethylacetat (102),
2-(2-(4-chlorphenyl)-5-((hydroxyimino)(4-methoxyphenyl)methyl) thiophen-3-yl) ethylacetat (104),
2-(2-(4-chlorphenyl)-5-((methoxyimino)(4-methoxyphenyl)methyl) thiophen-3-yl) ethylacetat (106),
2-(2-(2,3-difluorphenyl)-5-(4-methoxybenzoyl)thiophen-3-yl) essigsäure (137),
2-(2-(2,3-difluorphenyl)-5-(furan-2-carbonyl)thiophen-3-yl) essigsäure (136),
2-(2-(3,4-dichlorphenyl)-5-(4-methoxybenzoyl)thiophen-3-yl) essigsäure (81),
2-(2-(3-cyanophenyl)-5-(furan-2-carbonyl)thiophen-3-yl)-N-(2-(dimethyl amino)ethyl) acetamid (142),
2-(2-(4-chlor-2-fluorphenyl)-5-(furan-2-carbonyl)thiophen-3-yl) essigsäure (144),
2-(2-(4-chlorphenyl)-5-(3-methoxybenzoyl)thiophen-3-yl) essigsäure (59),
2-(2-(4-chlorphenyl)-5-(4-methoxybenzoyl)thiophen-3-yl)-3-methoxypropionsäure (128),
2-(2-(4-chlorphenyl)-5-(4-methoxybenzoyl)thiophen-3-yl) acetamid (86),
2-(2-(4-chlorphenyl)-5-(4-methoxybenzoyl)thiophen-3-yl) essigsäure (60),
2-(2-(4-chlorphenyl)-5-(4-methoxybenzoyl)thiophen-3-yl)-N-(2-(dimethyl amino)ethyl) acetamid (89),
2-(2-(4-chlorphenyl)-5-(4-methoxybenzoyl)thiophen-3-yl)-N-(2-hydroxy ethyl) acetamid (96),
2-(2-(4-chlorphenyl)-5-(4-methoxybenzoyl)thiophen-3-yl)-N-hydroxy acetamid (100),
2-(2-(4-chlorphenyl)-5-(4-methoxybenzoyl)thiophen-3-yl) propionsäure (127),
2-(2-(4-chlorphenyl)-5-(cyclohexancarbonyl)thiophen-3-yl) essigsäure (61),
2-(2-(4-chlorphenyl)-5-(furan-2-carbonyl)thiophen-3-yl)-1-morpholin ethanon (91),
2-(2-(4-chlorphenyl)-5-(furan-2-carbonyl)thiophen-3-yl) essigsäure (56),
2-(2-(4-chlorphenyl)-5-(furan-2-carbonyl)thiophen-3-yl)-N-(2-(dimethyl amino)ethyl) acetamid (95),
2-(2-(4-chlorphenyl)-5-(furan-2-carbonyl)thiophen-3-yl)-N-ethoxy acetamid (98),
2-(2-(4-chlorphenyl)-5-picolinoylthiophen-3-yl) essigsäure (149),
2-(5-(2,3-difluorbenzoyl)-2-(4-methoxyphenyl)thiophen-3-yl) essigsäure (64),
2-(5-(2,4-difluorbenzoyl)-2-(4-methoxyphenyl)thiophen-3-yl) essigsäure (65),
2-(5-(2-fluorbenzoyl)-2-(4-methoxyphenyl)thiophen-3-yl) essigsäure (67),
2-(5-(3,5-difluorbenzoyl)-2-(4-methoxyphenyl)thiophen-3-yl) essigsäure (70),
2-(5-(3-chlorbenzoyl)-2-(4-methoxyphenyl)thiophen-3-yl) essigsäure (71),
2-(5-(3-chlorbenzoyl)-2-(4-chlorphenyl)thiophen-3-yl) essigsäure (58),
2-(5-(3-fluor-4-methoxybenzoyl)-2-(4-methoxyphenyl)thiophen-3-yl) essigsäure (73),
2-(5-(3-fluorbenzoyl)-2-(4-methoxyphenyl)thiophen-3-yl) essigsäure (72),
2-(5-(3-methoxybenzoyl)-2-(4-methoxyphenyl)thiophen-3-yl) essigsäure (75),
2-(5-(4-cyanobenzoyl)-2-(4-methoxyphenyl)thiophen-3-yl) essigsäure (76),
2-(5-(4-fluor-3-methylbenzoyl)-2-(4-methoxyphenyl)thiophen-3-yl) essigsäure (74),
2-(5-(4-fluorbenzoyl)-2-(4-methoxyphenyl)thiophen-3-yl) essigsäure (77),
2-(5-(4-methoxybenzoyl)-2-phenylthiophen-3-yl) essigsäure (51),
2-(5-(cyclohexancarbonyl)-2-phenylthiophen-3-yl) essigsäure (52),
2-(5-(furan-2-carbonyl)-2-(4-methoxyphenyl)thiophen-3-yl) essigsäure (68),
2-(5-(furan-2-carbonyl)-2-phenylthiophen-3-yl) essigsäure (48),
2-(5-benzoyl-2-(4-chlorphenyl)thiophen-3-yl) essigsäure (63),
2-(5-benzoyl-2-(4-methoxyphenyl)thiophen-3-yl) essigsäure (78),
2-(2-(2,3-difluorphenyl)-5-(4-methoxybenzoyl)thiophen-3-yl) ethylacetat (133),
2-(2-(2,3-difluorphenyl)-5-(furan-2-carbonyl)thiophen-3-yl) ethylacetat (130),
2-(2-(4-chlorphenyl)-5-(3-methoxybenzoyl)thiophen-3-yl) ethylacetat (12),
2-(2-(4-chlorphenyl)-5-(4-methoxybenzoyl)thiophen-3-yl)-3-methoxy-ethylpropanoat (124),
2-(2-(4-chlorphenyl)-5-(4-methoxybenzoyl)thiophen-3-yl) ethylacetat (13),
2-(2-(4-chlorphenyl)-5-(4-methoxybenzoyl)thiophen-3-yl) ethylpropanoat (123),
2-(2-(4-chlorphenyl)-5-(cyclohexancarbonyl)thiophen-3-yl) ethylacetat (14),
2-(2-(4-chlorphenyl)-5-(furan-2-carbonyl)thiophen-3-yl) ethylacetat (10),
2-(2-(4-chlorphenyl)-5-(morpholine-4-carbonyl)thiophen-3-yl) ethylacetat (114),
2-(2-(4-cyanophenyl)-5-(furan-2-carbonyl)thiophen-3-yl) ethylacetat (139),
2-(2-(4-methoxyphenyl)-5-(thiazol-4-carbonyl)thiophen-3-yl) ethylacetat (33),
2-(5-(2,3-difluorbenzoyl)-2-(4-methoxyphenyl)thiophen-3-yl) ethylacetat (18),
2-(5-(2,4-difluorbenzoyl)-2-(4-methoxyphenyl)thiophen-3-yl) ethylacetat (19),
2-(5-(2-fluorbenzoyl)-2-(4-methoxyphenyl)thiophen-3-yl) ethylacetat (21),
2-(5-(3,5-difluorbenzoyl)-2-(4-methoxyphenyl)thiophen-3-yl) ethylacetat (24),
2-(5-(3-chlorbenzoyl)-2-(4-chlorphenyl)thiophen-3-yl) ethylacetat (11),
2-(5-(3-chlorbenzoyl)-2-(4-methoxyphenyl)thiophen-3-yl) ethylacetat (25),
2-(5-(3-fluor-4-methoxybenzoyl)-2-(4-methoxyphenyl)thiophen-3-yl) ethylacetat (27),
2-(5-(3-fluorbenzoyl)-2-(4-methoxyphenyl)thiophen-3-yl) ethylacetat (26),
2-(5-(3-methoxybenzoyl)-2-(4-methoxyphenyl)thiophen-3-yl) ethylacetat (30),
2-(5-(4-cyanobenzoyl)-2-(4-methoxyphenyl)thiophen-3-yl) ethylacetat (31),
2-(5-(4-fluor-3-methylbenzoyl)-2-(4-methoxyphenyl)thiophen-3-yl) ethylacetat (29),
2-(5-(4-fluorbenzoyl)-2-(4-methoxyphenyl)thiophen-3-yl) ethylacetat (32),
2-(5-(furan-2-carbonyl)-2-(4-methoxyphenyl)thiophen-3-yl) ethylacetat (22),
2-(5-benzoyl-2-(4-chlorphenyl)thiophen-3-yl) ethylacetat (15),
2-(5-benzoyl-2-(4-methoxyphenyl)thiophen-3-yl) ethylacetat (35),
2-(2-(3,4-dichlorphenyl)-5-(4-methoxybenzoyl)thiophen-3-yl) isopropylacetat (40),
2-(2-(3,4-dichlorphenyl)-5-(furan-2-carbonyl)thiophen-3-yl) isopropylacetat (39),
2-(2-(4-chlor-2-fluorphenyl)-5-(furan-2-carbonyl)thiophen-3-yl) isopropylacetat (143),
2-(2-(4-chlorphenyl)-5-(4-methoxybenzoyl)thiophen-3-yl)-3-methoxy-isopropylpropanoat (125),
2-(2-(4-chlorphenyl)-5-picolinoylthiophen-3-yl) methylacetat (148),
2-(5-(4-methoxybenzoyl)-2-phenylthiophen-3-yl) methylacetat (5),
2-(5-(cyclohexancarbonyl)-2-phenylthiophen-3-yl) methylacetat (6),
2-(5-(furan-2-carbonyl)-2-phenylthiophen-3-yl) methylacetat (3),
2-(2-(4-chlorphenyl)-5-(4-methoxybenzoyl)thiophen-3-yl) isopropylacetat (43),
2-(2-(4-chlorphenyl)-5-(furan-2-carbonyl)thiophen-3-yl) isopropylacetat (44),
2-(2-(4-chlorphenyl)-5-(4-methoxybenzoyl)thiophen-3-yl) tert-Butylacetat (84),
2-(2-(4-chlorphenyl)-5-(furan-2-carbonyl)thiophen-3-yl) tert-Butylacetat (83),
*N*-(*N*-(tert-butoxycarbonyl)carbamimidoyl)-2-(2-(4-chlorphenyl)-5-(4-methoxybenzoyl)thiophen-3-yl) acetamid (46),
2-(5-(3-chlorbenzoyl)-2-phenylthiophen-3-yl) essigsäure (49),
2-(5-(3-methoxybenzoyl)-2-phenylthiophen-3-yl) essigsäure (50),
2-(2-(4-ch lorphenyl)-5-(4-methoxybenzoyl)thiophen-3-yl)-1-(4-methylpiperazin-1-yl) ethanon (93),
2-(2-(4-cyanophenyl)-5-(furan-2-carbonyl)thiophen-3-yl) essigsäure (140),
2-[5-benzoyl-2-(4-chlorphenyl)-3-thienyl] essigsäure (154),
2-[5-(4-chlor-2-methoxy-benzoyl)-2-(4-chlorphenyl)-3-thienyl] ethylacetat (155),
2-[5-(4-chlor-2-methoxy-benzoyl)-2-(4-chlorphenyl)-3-thienyl] essigsäure (156),
2-[2-(3,4-dichlorphenyl)-5-(4-methoxybenzoyl)-3-thienyl] ethylacetat (157),
2-[2-(3,4-dichlorphenyl)-5-(furan-2-carbonyl)-3-thienyl] ethylacetat (158),
2-[2-(3,4-dichlorphenyl)-5-(furan-2-carbonyl)-3-thienyl] essigsäure (159),
2-[2-(3,4-dichlorphenyl)-5-(4-methoxybenzoyl)-3-thienyl]-N-(2-hydroxyethyl) acetamid (165),
2-[2-(3,4-dichlorphenyl)-5-(4-methoxybenzoyl)-3-thienyl]-N-(2-dimethylaminoethyl) acetamid (166),
2-[2-(4-chlorphenyl)-5-[C-(3,4-dichlorphenyl)-N-ethoxy-carbonimidoyl]-3-thienyl] essigsäure (167),
2-[2-(3,4-dichlorphenyl)-5-(4-methoxyphenyl) sulfonyl-3-thienyl] essigsäure (175),
2-[5-(5-chlorfuran-2-carbonyl)-2-(4-chlorphenyl)-3-thienyl] ethylacetat (176),
N-[N-[2-[2-(3,4-dichlorphenyl)-5-(4-methoxybenzoyl)-3-thienyl] acetyl] carbamimidoyl] tert-Butylcarbamat (182),
N-[N-[2-[2-(4-chlorphenyl)-5-(4-methoxybenzoyl)-3-thienyl] acetyl] carbamimidoyl] tert-Butylcarbamat (183),
N-carbamimidoyl-2-[2-(3,4-dichlorphenyl)-5-(4-methoxybenzoyl)-3-thienyl]acetamid-Hydrochlorid (184),
N-carbamimidoyl-2-[2-(4-chlorphenyl)-5-(4-methoxybenzoyl)-3-thienyl]acetamid-Hydrochlorid (185),
N-carbamimidoyl-2-[2-(4-chlorphenyl)-5-(furan-2-carbonyl)-3-thienyl] acetamid-Hydrochlorid (186),
Ethyl-2-[2-(4-chlorphenyl)-5-(4-methoxybenzoyl)-3-thienyl] prop-2-enoat (187).

9. Pharmazeutische Zusammensetzung, die ein Derivat nach einem der Ansprüche 1 bis 8 und einen pharmazeutisch akzeptablen Hilfsstoff umfasst.

10. Pharmazeutische Zusammensetzung nach Anspruch 9, die ferner ein weiteres Antidiabetikum, insbesondere Metformin, umfasst.

11. Derivat nach einem der Ansprüche 1 bis 8 oder ausgewählt aus den Verbindungen der Formel (f) bis (p), (r), (s), (u), (y) und (z), wie sie in Anspruch 1 definiert sind, für die Verwendung als Medikament.

12. Derivat nach einem der Ansprüche 1 bis 8 oder ausgewählt aus den Verbindungen der Formel (a) bis (z1), wie sie in Anspruch 1 definiert sind, für die Verwendung als Medikament, das für die Behandlung und/oder die Prävention von Diabetes, ihren Komplikationen und/oder assoziierten Pathologien, vorteilhafterweise von Diabetes Typ 2 und von Hyperglykämie bestimmt ist.

## Claims

1. A thiophene derivative of the following general formula I: 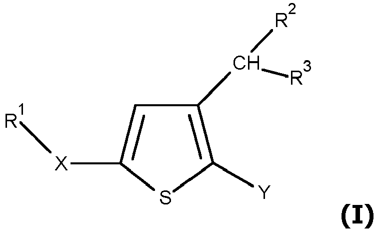 wherein:
Y represents an aryl group, a heteroaryl group, or a benzo-1,3-dioxole group,
the aryl or heteroaryl group being optionally substituted with one or more groups selected from -CN;
a halogen atom, -O(C₁-C₆ alkyl), the alkyl group being optionally substituted with one or more halogen atoms or with a -O(C₁-C₆ alkyl) group;
a C₁-C₆ alkyl substituted with one or more halogen atoms, with a -O(C₁-C₆ alkyl) group or with a -OH group; -SO₂(C₁-C₆ alkyl);
-CONRaRb wherein **Ra** represents a hydrogen atom or a C₁-C₆ alkyl group and **Rb** represents a C₁-C₆ alkyl group;
or -OH;
**X** represents a -SO₂ group or a 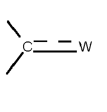 group, wherein 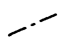 represents a bond and **W** represents an oxygen atom or the-NOR⁴ group, wherein **R⁴** represents a hydrogen atom, a C₁-C₆ alkyl group or a (C₁-C₆ alkyl)aryl group, the aryl group being optionally substituted with one or more groups selected from -CN; a halogen atom; -O(C₁-C₆ alkyl), the alkyl group being optionally substituted with one or more halogen atoms or with a -O(C₁-C₆ alkyl) group; a C₁-C₆ alkyl substituted with one or more halogen atoms, with a -O(C₁-C₆ alkyl) group or with a OH group; -SO₂(C₁-C₆ alkyl); -CONRa'Rb' wherein **Ra'** represents a hydrogen atom or a C₁-C₆ alkyl group and **Rb'** represents a C₁-C₆ alkyl group; or -OH or
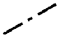 is absent and **W** represents -OH;
**R¹** represents
- a C₁-C₆ alkyl group, the alkyl group being optionally substituted with a halogen atom;
- a C₃-C₆ cycloalkyl group;
- a (C₁-C₆ alkyl)O(C₁-C₆ alkyl) group;
- a (C₁-C₆ alkyl)NR(C₁-C₆ alkyl) group wherein **R** represents a hydrogen atom or a C₁-C₆ alkyl group;
- an aryl group, the aryl group being optionally substituted with one or more groups selected from -CN; a halogen atom; -O(C₁-C₆ alkyl), the alkyl group being optionally substituted with one or more halogen atoms or with a -O(C₁-C₆ alkyl) group; -SO₂(C₁-C₆ alkyl); -CONRa"Rb" wherein **Ra"** represents a hydrogen atom or a C₁-C₆ alkyl group and **Rb"** represents a C₁-C₆ alkyl group; or C₁-C₆ alkyl, the alkyl group being optionally substituted with one or more halogen atoms, with a -O(C₁-C₆ alkyl) group or with an -OH group;
- a (C₁-C₆ alkyl)aryl group; the aryl group being optionally substituted with one or more groups selected from -CN; a halogen atom; -O(C₁-C₆ alkyl);
or C₁-C₆ alkyl;
- an -NH-aryl group; the aryl group being optionally substituted with one or more groups selected from -CN; a halogen atom; -O(C₁-C₆ alkyl); or C₁-C₆ alkyl;
- an -NH(C₁-C₆ alkyl)aryl group; the aryl group being optionally substituted with one or more groups selected from -CN; a halogen atom; -O(C₁-C₆ alkyl); or C₁-C₆ alkyl;
- a heteroaryl group, optionally substituted with a halogen atom;
- an -OH group;
- a morpholine group; or
- an N-phenylpiperazine group;
- an NH-NH-CO-aryl group wherein the aryl group is optionally substituted with one or more groups selected from a halogen atom and a -O(C₁-C₆ alkyl) group;
- an NH-NH-CO-heteroaryl group,
**R²** represents a hydrogen atom, a C₁-C₆ alkyl group, a (C₁-C₆ alkyl)aryl group or a (C₁-C₆ alkyl)O(C₁-C₆ alkyl) group;
**R³** represents
- a -COOR⁵ group, wherein **R⁵** represents a hydrogen atom, a C₁-C₆ alkyl group or the glucopyranose group;
- a -COSR⁶ group, wherein **R⁶** represents a hydrogen atom or a C₁-C₆ alkyl group;
- a -CONR⁷R⁸ group, wherein **R⁷** represents a hydrogen atom or a C₁-C₆ alkyl group and **R⁸** represents a hydrogen atom; a C₁-C₆ alkyl group optionally substituted with an -OH group; an -OH group; an -O(C₁-C₆ alkyl) group; an -NH₂ group; a -( C₁-C₆ alkyl)NR⁹R¹⁰ group wherein **R⁹** and **R¹⁰** both represent a C₁-C₆ alkyl group; a -(C₁-C₆ alkyl)COOH group; a -(C₁-C₆ alkyl)COO(C₁-C₆ alkyl) group; an aryl group; or a heteroaryl group;
- a -CSNR¹¹R¹² group wherein **R¹¹** and **R¹²** represent independently of each other a hydrogen atom or a C₁-C₆ alkyl group;
- a -CN group;
- a -C(=NH)NHOH group;
- a -COmorpholine group;
- a -COpyrolidine group;
- a -CON-Me-piperazine group;
- a -COguanidine or -COguanidine-BOC group;
- a tetrazole group; or
- a oxadiazolone group;
or an enantiomer, a diastereoisomer, hydrate, solvate, tautomer, racemic mixture or pharmaceutically acceptable salt thereof,
except for the compounds (a) to (z1) of the following formulae:

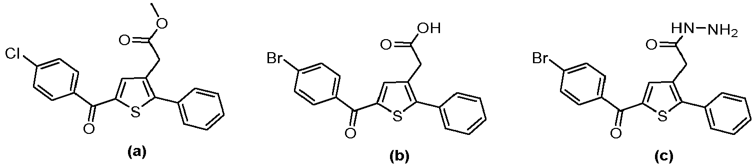

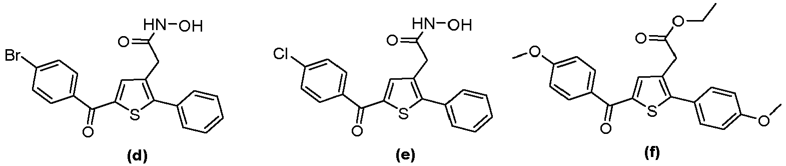

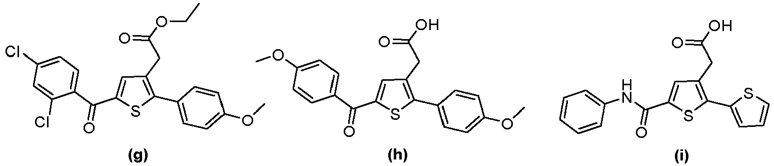

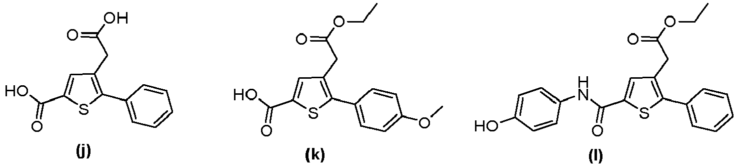

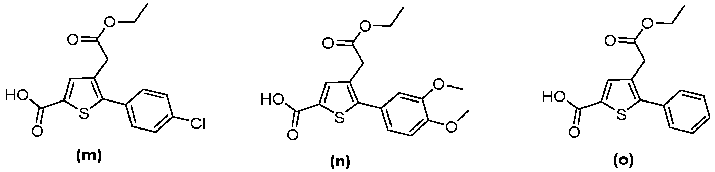

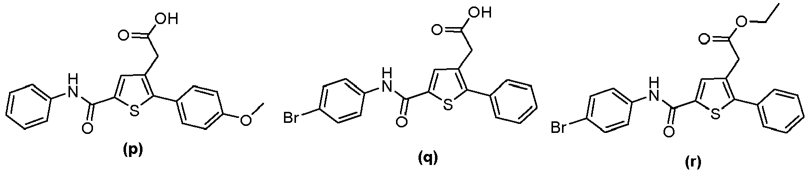

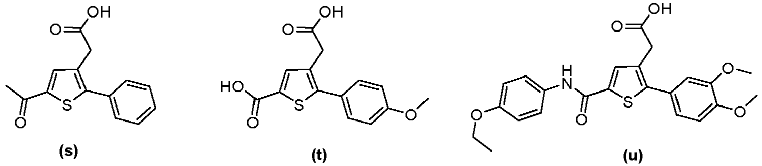

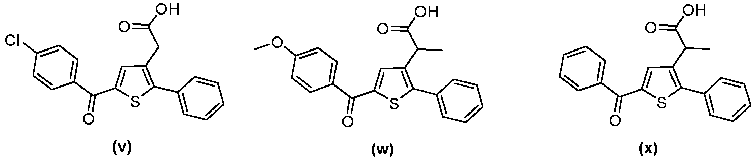

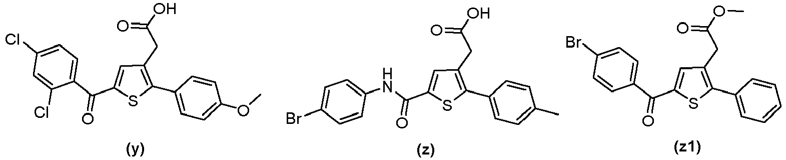

2. The thiophene derivative according to claim 1, **characterized in that** 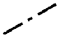
represents a bond and **W** represents an oxygen atom or the-NOR⁴ group, wherein **R⁴** represents a hydrogen atom or a C₁-C₆ alkyl group, advantageously **W** represents an oxygen atom.

3. The thiophene derivative according to any of claims 1 or 2 **characterized in that R³** represents
- a -COguanidine group;
- a -COOR⁵ group, wherein **R⁵** represents a hydrogen atom or a C₁-C₆ alkyl group;
- a -CONR⁷R⁸ group, wherein **R⁷** represents a hydrogen atom and **R⁸** represents a hydrogen atom; a C₁-C₆ alkyl group optionally substituted with an -OH group; an -OH group; an -O(C₁-C₆ alkyl) group or a -(C₁-C₆ alkyl)NR⁹R¹⁰ group wherein **R⁹** and **R¹⁰** both represent a C₁-C₆ alkyl group; or
- a -COmorpholine group;
advantageously **R³** represents a -COOR⁵ group, wherein **R⁵** represents a hydrogen atom or a C₁-C₆ alkyl group.

4. The thiophene derivative according to any of claims 1 to 3 **characterized in that Y** represents an aryl group, advantageously a phenyl, the aryl group being optionally substituted, with one or several groups selected from -CN; a halogen atom, advantageously selected from Cl or F; or -O(C₁-C₆ alkyl), advantageously -OMe .

5. The thiophene derivative according to claim 4 **characterized in that Y** represents a phenyl group, the phenyl group being substituted with one or more halogen atoms, advantageously Cl.

6. The thiophene derivative according to any of claims 1 to 5 **characterized in that R¹** represents
- a C₃-C₆ cycloalkyl group;
- an aryl group, advantageously a phenyl, the aryl group being optionally substituted with one or more groups selected from -CN; a halogen atom, advantageously selected from Cl or F; -O(C₁-C₆ alkyl), advantageously -OMe; or C₁-C₆ alkyl, advantageously methyl;
- a heteroaryl group optionally substituted with a halogen atom, advantageously a furanyl, pyridyl or thiazolyl; or
- a morpholine group.

7. The thiophene derivative according to claim 6 **characterized in that R¹** represents
- a phenyl group, the phenyl group being optionally substituted with one or more -O(C₁-C₆ alkyl) groups, advantageously -OMe; or
- a furanyl, pyridyl or thiazolyl group, the furanyl group being optionally substituted with a halogen atom.

8. The thiophene derivative according to any of claims 1 to 7, **characterized in that** it is selected from the following compounds:
2-(2-(4-chlorophenyl)-5-((ethoxyimino)(4-methoxyphenyl)methyl) thiophen-3-yl) acetic acid **(108);**
2-(2-(4-chlorophenyl)-5-((hydroxyimino)(4-methoxyphenyl)methyl) thiophen-3-yl) acetic acid **(109);**
2-(2-(4-chlorophenyl)-5-((methoxyimino)(4-methoxyphenyl)methyl) thiophen-3-yl) acetic acid **(110);**
ethyl 2-(2-(4-chlorophenyl)-5-((ethoxyimino)(4-methoxyphenyl)methyl) thiophen-3-yl) acetate **(102);**
ethyl 2-(2-(4-chlorophenyl)-5-((hydroxyimino)(4-methoxyphenyl)methyl)-thiophen-3-yl)acetate **(104);**
ethyl 2-(2-(4-chlorophenyl)-5-((methoxyimino)(4-methoxyphenyl)methyl) thiophen-3-yl)acetate **(106);**
2-(2-(2,3-difluorophenyl)-5-(4-methoxybenzoyl)thiophen-3-yl) acetic acid **(137);**
2-(2-(2,3-difluorophenyl)-5-(furan-2-carbonyl)thiophen-3-yl) acetic acid **(136);**
2-(2-(3,4-dichlorophenyl)-5-(4-methoxybenzoyl)thiophen-3-yl) acetic acid **(81);**
2-(2-(3-cyanophenyl)-5-(furan-2-carbonyl)thiophen-3-yl)-N-(2-(dimethyl amino)ethyl)acetamide **(142);**
2-(2-(4-chloro-2-fluorophenyl)-5-(furan-2-carbonyl)thiophen-3-yl) acetic acid **(144);**
2-(2-(4-chlorophenyl)-5-(3-methoxybenzoyl)thiophen-3-yl) acetic acid **(59);**
2-(2-(4-chlorophenyl)-5-(4-methoxybenzoyl)thiophen-3-yl)-3-methoxy-propanoic acid **(128);**
2-(2-(4-chlorophenyl)-5-(4-methoxybenzoyl)thiophen-3-yl)acetamide **(86);**
2-(2-(4-chlorophenyl)-5-(4-methoxybenzoyl)thiophen-3-yl) acetic acid **(60);**
2-(2-(4-chlorophenyl)-5-(4-methoxybenzoyl)thiophen-3-yl)-N-(2-(dimethyl amino)ethyl)acetamide **(89);**
2-(2-(4-chlorophenyl)-5-(4-methoxybenzoyl)thiophen-3-yl)-N-(2-hydroxy ethyl)acetamide **(96);**
2-(2-(4-chlorophenyl)-5-(4-methoxybenzoyl)thiophen-3-yl)-N-hydroxy acetamide **(100);**
2-(2-(4-chlorophenyl)-5-(4-methoxybenzoyl)thiophen-3-yl) propanoic acid **(127);**
2-(2-(4-chlorophenyl)-5-(cyclohexanecarbonyl)thiophen-3-yl)acetic acid **(61);**
2-(2-(4-chlorophenyl)-5-(furan-2-carbonyl)thiophen-3-yl)-1-morpholino ethanone **(91);**
2-(2-(4-chlorophenyl)-5-(furan-2-carbonyl)thiophen-3-yl) acetic acid **(56)** 2-(2-(4-chlorophenyl)-5-(furan-2-carbonyl)thiophen-3-yl)-N-(2-(dimethyl amino)ethyl)acetamide **(95);**
2-(2-(4-chlorophenyl)-5-(furan-2-carbonyl)thiophen-3-yl)-N-ethoxy acetamide **(98);**
2-(2-(4-chlorophenyl)-5-picolinoylthiophen-3-yl) acetic acid **(149);**
2-(5-(2,3-difluorobenzoyl)-2-(4-methoxyphenyl)thiophen-3-yl) acetic acid **(64);**
2-(5-(2,4-difluorobenzoyl)-2-(4-methoxyphenyl)thiophen-3-yl) acetic acid **(65);**
2-(5-(2-fluorobenzoyl)-2-(4-methoxyphenyl)thiophen-3-yl) acetic acid **(67);**
2-(5-(3,5-difluorobenzoyl)-2-(4-methoxyphenyl)thiophen-3-yl) acetic acid **(70);**
2-(5-(3-chlorobenzoyl)-2-(4-methoxyphenyl)thiophen-3-yl) acetic acid **(71);**
2-(5-(3-chlorobenzoyl)-2-(4-chlorophenyl)thiophen-3-yl) acetic acid **(58);**
2-(5-(3-fluoro-4-methoxybenzoyl)-2-(4-methoxyphenyl)thiophen-3-yl) acetic acid **(73);**
2-(5-(3-fluorobenzoyl)-2-(4-methoxyphenyl)thiophen-3-yl) acetic acid **(72);**
2-(5-(3-methoxybenzoyl)-2-(4-methoxyphenyl)thiophen-3-yl) acetic acid **(75);**
2-(5-(4-cyanobenzoyl)-2-(4-methoxyphenyl)thiophen-3-yl) acetic acid **(76);**
2-(5-(4-fluoro-3-methylbenzoyl)-2-(4-methoxyphenyl)thiophen-3-yl) acetic acid **(74);**
2-(5-(4-fluorobenzoyl)-2-(4-methoxyphenyl)thiophen-3-yl) acetic acid **(77);**
2-(5-(4-methoxybenzoyl)-2-phenylthiophen-3-yl) acetic acid **(51);**
2-(5-(cyclohexanecarbonyl)-2-phenylthiophen-3-yl) acetic acid **(52);**
2-(5-(furan-2-carbonyl)-2-(4-methoxyphenyl)thiophen-3-yl) acetic acid **(68);**
2-(5-(furan-2-carbonyl)-2-phenylthiophen-3-yl) acetic acid **(48);**
2-(5-benzoyl-2-(4-chlorophenyl)thiophen-3-yl) acetic acid **(63);**
2-(5-benzoyl-2-(4-methoxyphenyl)thiophen-3-yl) acetic acid **(78);**
ethyl 2-(2-(2,3-difluorophenyl)-5-(4-methoxybenzoyl)thiophen-3-yl)acetate **(133);**
ethyl 2-(2-(2,3-difluorophenyl)-5-(furan-2-carbonyl)thiophen-3-yl)acetate **(130);**
ethyl 2-(2-(4-chlorophenyl)-5-(3-methoxybenzoyl)thiophen-3-yl)acetate **(12);**
ethyl 2-(2-(4-chlorophenyl)-5-(4-methoxybenzoyl)thiophen-3-yl)-3-methoxy-propanoate **(124);**
ethyl 2-(2-(4-chlorophenyl)-5-(4-methoxybenzoyl)thiophen-3-yl)acetate **(13);**
ethyl 2-(2-(4-chlorophenyl)-5-(4-methoxybenzoyl)thiophen-3-yl) propanoate **(123);**
ethyl 2-(2-(4-chlorophenyl)-5-(cyclohexanecarbonyl)thiophen-3-yl)acetate **(14);**
ethyl 2-(2-(4-chlorophenyl)-5-(furan-2-carbonyl)thiophen-3-yl)acetate **(10);**
ethyl 2-(2-(4-chlorophenyl)-5-(morpholine-4-carbonyl)thiophen-3-yl)acetate **(114);**
ethyl 2-(2-(4-cyanophenyl)-5-(furan-2-carbonyl)thiophen-3-yl)acetate **(139);**
ethyl 2-(2-(4-methoxyphenyl)-5-(thiazole-4-carbonyl)thiophen-3-yl)acetate **(33);**
ethyl 2-(5-(2,3-difluorobenzoyl)-2-(4-methoxyphenyl)thiophen-3-yl)acetate **(18);**
ethyl 2-(5-(2,4-difluorobenzoyl)-2-(4-methoxyphenyl)thiophen-3-yl)acetate **(19);**
ethyl 2-(5-(2-fluorobenzoyl)-2-(4-methoxyphenyl)thiophen-3-yl)acetate **(21);**
ethyl 2-(5-(3,5-difluorobenzoyl)-2-(4-methoxyphenyl)thiophen-3-yl)acetate **(24);**
ethyl 2-(5-(3-chlorobenzoyl)-2-(4-chlorophenyl)thiophen-3-yl)acetate **(11);**
ethyl 2-(5-(3-chlorobenzoyl)-2-(4-methoxyphenyl)thiophen-3-yl)acetate **(25);**
ethyl 2-(5-(3-fluoro-4-methoxybenzoyl)-2-(4-methoxyphenyl)thiophen-3-yl) acetate **(27);**
ethyl 2-(5-(3-fluorobenzoyl)-2-(4-methoxyphenyl)thiophen-3-yl)acetate **(26);**
ethyl 2-(5-(3-methoxybenzoyl)-2-(4-methoxyphenyl)thiophen-3-yl)acetate **(30);**
ethyl 2-(5-(4-cyanobenzoyl)-2-(4-methoxyphenyl)thiophen-3-yl)acetate **(31);**
ethyl 2-(5-(4-fluoro-3-methylbenzoyl)-2-(4-methoxyphenyl)thiophen-3-yl) acetate **(29);**
ethyl 2-(5-(4-fluorobenzoyl)-2-(4-methoxyphenyl)thiophen-3-yl)acetate **(32);**
ethyl 2-(5-(furan-2-carbonyl)-2-(4-methoxyphenyl)thiophen-3-yl)acetate **(22);**
ethyl 2-(5-benzoyl-2-(4-chlorophenyl)thiophen-3-yl)acetate **(15);**
ethyl 2-(5-benzoyl-2-(4-methoxyphenyl)thiophen-3-yl)acetate **(35);**
isopropyl 2-(2-(3,4-dichlorophenyl)-5-(4-methoxybenzoyl)thiophen-3-yl) acetate **(40);**
isopropyl 2-(2-(3,4-dichlorophenyl)-5-(furan-2-carbonyl)thiophen-3-yl) acetate **(39);**
isopropyl 2-(2-(4-chloro-2-fluorophenyl)-5-(furan-2-carbonyl)thiophen-3-yl)acetate **(143);**
isopropyl 2-(2-(4-chlorophenyl)-5-(4-methoxybenzoyl)thiophen-3-yl)-3-methoxypropanoate **(125);**
methyl 2-(2-(4-chlorophenyl)-5-picolinoylthiophen-3-yl)acetate **(148);**
methyl 2-(5-(4-methoxybenzoyl)-2-phenylthiophen-3-yl)acetate **(5);**
methyl 2-(5-(cyclohexanecarbonyl)-2-phenylthiophen-3-yl)acetate **(6);**
methyl 2-(5-(furan-2-carbonyl)-2-phenylthiophen-3-yl)acetate **(3);**
isopropyl 2-(2-(4-chlorophenyl)-5-(4-methoxybenzoyl)thiophen-3-yl) acetate **(43);**
isopropyl 2-(2-(4-chlorophenyl)-5-(furan-2-carbonyl)thiophen-3-yl)acetate **(44);**
tert-butyl 2-(2-(4-chlorophenyl)-5-(4-methoxybenzoyl)thiophen-3-yl) acetate **(84);**
tert-butyl 2-(2-(4-chlorophenyl)-5-(furan-2-carbonyl)thiophen-3-yl)acetate **(83);**
*N*-(*N*-(tert-butoxycarbonyl)carbamimidoyl)-2-(2-(4-chlorophenyl)-5-(4-methoxybenzoyl)thiophen-3-yl)acetamide **(46);**
2-(5-(3-chlorobenzoyl)-2-phenylthiophen-3-yl)acetic acid **(49);**
2-(5-(3-methoxybenzoyl)-2-phenylthiophen-3-yl) acetic acid **(50);**
2-(2-(4-chlorophenyl)-5-(4-methoxybenzoyl)thiophen-3-yl)-1-(4-methyl piperazin-1-yl)ethanone **(93);**
2-(2-(4-cyanophenyl)-5-(furan-2-carbonyl)thiophen-3-yl) acetic acid **(140);**
2-[5-benzoyl-2-(4-chlorophenyl)-3-thienyl] acetic acid **(154);**
ethyl 2-[5-(4-chloro-2-methoxy-benzoyl)-2-(4-chlorophenyl)-3-thienyl] acetate **(155);**
2-[5-(4-chloro-2-methoxy-benzoyl)-2-(4-chlorophenyl)-3-thienyl] acetic acid **(156);**
ethyl 2-[2-(3,4-dichlorophenyl)-5-(4-methoxybenzoyl)-3-thienyl]acetate **(157);**
ethyl 2-[2-(3,4-dichlorophenyl)-5-(furan-2-carbonyl)-3-thienyl]acetate **(158);**
2-[2-(3,4-dichlorophenyl)-5-(furan-2-carbonyl)-3-thienyl] acetic acid **(159);**
2-[2-(3,4-dichlorophenyl)-5-(4-methoxybenzoyl)-3-thienyl]-N-(2-hydroxyethyl)acetamide **(165);**
2-[2-(3,4-dichlorophenyl)-5-(4-methoxybenzoyl)-3-thienyl]-N-(2-dimethylaminoethyl)acetamide **(166);**
2-[2-(4-chlorophenyl)-5-[C-(3,4-dichlorophenyl)-N-ethoxy-carbonimidoyl]-3-thienyl] acetic acid **(167);**
2-[2-(3,4-dichlorophenyl)-5-(4-methoxyphenyl)sulfonyl-3-thienyl]acetic acid **(175);**
ethyl 2-[5-(5-chlorofuran-2-carbonyl)-2-(4-chlorophenyl)-3-thienyl]acetate **(176);**
tert-butyl N-[N-[2-[2-(3,4-dichlorophenyl)-5-(4-methoxybenzoyl)-3-thienyl]-acetyl]carbamimidoyl]carbamate **(182);**
tert-butyl N-[N-[2-[2-(4-chlorophenyl)-5-(4-methoxybenzoyl)-3-thienyl]-acetyl]carbamimidoyl]carbamate **(183);**
N-carbamimidoyl-2-[2-(3,4-dichlorophenyl)-5-(4-methoxybenzoyl)-3-thienyl]acetamide hydrochloride **(184);**
N-carbamimidoyl-2-[2-(4-chlorophenyl)-5-(4-methoxybenzoyl)-3-thienyl]acetamide hydrochloride **(185);**
N-carbamimidoyl-2-[2-(4-chlorophenyl)-5-(furan-2-carbonyl)-3-thienyl]acetamide hydrochloride **(186);**
ethyl 2-[2-(4-chlorophenyl)-5-(4-methoxybenzoyl)-3-thienyl]prop-2-enoate **(187).**

9. A pharmaceutical composition comprising a derivative according to any of claims 1 to 8 and a pharmaceutically acceptable excipient.

10. The pharmaceutical composition according to claim 9, further comprising another antidiabetic agent, in particular metformin.

11. The derivative according to any of claims 1 to 8 or selected from compounds of formulae (f) to (p), (r), (s), (u), (y) and (z) as defined in claim 1 for use as a drug.

12. The derivative according to any of claims 1 to 8 or selected from compounds of formulae (a) to (z1), as defined in claim 1 for use as a drug intended for treating and/or preventing diabetes, its complications and/or associated pathologies, advantageously diabetes of type II and hyperglycemia.
